(19) Europäisches Patentamt
European Patent Office
Office européen des brevets

(11) EP 2 861 223 B1

(12) **EUROPEAN PATENT SPECIFICATION**

(45) Date of publication and mention of the grant of the patent:
**03.08.2022 Bulletin 2022/31**

(21) Application number: **13804115.7**

(22) Date of filing: **14.06.2013**

(51) International Patent Classification (IPC):
***A61K 9/00*** (2006.01) ***A61K 31/135*** (2006.01)
***A61K 9/20*** (2006.01) ***A61P 21/00*** (2006.01)
***A61P 21/02*** (2006.01) ***A61P 25/00*** (2006.01)
***A61P 25/04*** (2006.01) ***A61P 25/20*** (2006.01)
***A61P 25/22*** (2006.01) ***A61P 25/24*** (2006.01)

(52) Cooperative Patent Classification (CPC):
**A61K 9/006; A61K 9/2009; A61K 31/135; A61P 21/00; A61P 21/02; A61P 25/00; A61P 25/04; A61P 25/20; A61P 25/22; A61P 25/24;** A61K 9/2018; A61K 9/2059

(86) International application number:
**PCT/US2013/046023**

(87) International publication number:
**WO 2013/188847 (19.12.2013 Gazette 2013/51)**

(54) **COMPOSITIONS AND METHODS FOR TRANSMUCOSAL ABSORPTION**

ZUSAMMENSETZUNGEN UND VERFAHREN ZUR TRANSMUKOSALEN ABSORPTION

COMPOSITIONS ET PROCÉDÉS POUR L'ABSORPTION TRANSMUCOSALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**15.06.2012 US 201261660593 P**
**03.07.2012 US 201261667774 P**
**12.11.2012 US 201261725402 P**
**15.03.2013 US 201361792900 P**

(43) Date of publication of application:
**22.04.2015 Bulletin 2015/17**

(60) Divisional application:
**22180843.9**

(73) Proprietor: **Tonix Pharma Holdings Limited**
**Hamilton HM 10 (BM)**

(72) Inventors:
- **BRITTAIN, Harry**
  **Milford, New Jersey 08848 (US)**
- **LEDERMAN, Seth**
  **New York, NY 10022 (US)**
- **REINER, Giorgio**
  **22100 Como (IT)**

(74) Representative: **Haley Guiliano International LLP**
**26-28 Bedford Row**
**London WC1R 4HE (GB)**

(56) References cited:
**WO-A1-01/89476**
**WO-A1-99/18937**
**WO-A1-2011/062614**
**WO-A2-2014/145156**
**US-A1- 2003 077 227**
**US-A1- 2003 077 227**
**US-A1- 2010 098 832**
**US-A1- 2010 266 682**
**US-A1- 2010 266 682**
**US-A1- 2011 319 389**
**US-A1- 2011 319 389**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Background

Cyclobenzaprine, or 3-(5H-dibenzo[a,d]cyclohepten-5-ylidene)-N,N-dimethyl-1-propanamine, was first approved by the U.S. Food and Drug Administration in 1977 for the treatment of acute muscle spasms of local origin. (Katz, W., et al., Clinical Therapeutics 10:216-228 (1988)). Amitriptyline, or 3-(10,11-dihydro-5H-dibenzo [a,d] cycloheptene-5-ylidene)-*N,N*-dimethyl-1-propanamine, was first approved by the U.S. Food and Drug Administration for the treatment of depression.

Subsequent studies have shown cyclobenzaprine to also be effective in the treatment of fibromyalgia syndrome, post-traumatic stress disorder (PTSD), traumatic brain injury (TBI), generalized anxiety disorder and depression. Furthermore, the utility of cyclobenzaprine as an agent for improving the quality of sleep, as a sleep deepener, or for treating sleep disturbances has been investigated. However, while FDA-approved therapeutics address pain and mood, but there are currently no FDA-approved treatments that address the disturbed sleep and fatigue associated with fibromyalgia syndrome. Treatment with cyclobenzaprine may be particularly useful in treating sleep disturbances caused by, exacerbated by, or associated with fibromyalgia syndrome, prolonged fatigue, chronic fatigue, chronic fatigue syndrome, a sleep disorder, a psychogenic pain disorder, chronic pain syndrome (type II), the administration of a drug, autoimmune disease, stress or anxiety, or for treating an illness caused by or exacerbated by sleep disturbances, and symptoms of such illness (see, for example, U.S. Patent Nos. 6,395,788 and 6,358,944). Compositions formulated as buccal sprays or soft bite gelatin capsules are described in US 2003/077227 A1, which describes the formulation of a wide range of drugs, including anxiolytic agents and anti-depression agents. US 2003/077227 A1 does not, however, describe a composition comprising cyclobenzaprine and a basifying agent.

Despite the broad therapeutic usefulness of cyclobenzaprine, cyclobenzaprine is absorbed slowly into the blood stream after oral administration and should be taken approximately one to two hours before an effect is desired. If the effect is desired sooner, the patient must wait for the effect to occur, which is not desirable for use as a sleep aid and is not desirable for a patient with symptoms of muscle spasms seeking relief. In part because oral cyclobenzaprine has a slow onset of action, in desperation fibromyalgia patients sometimes try to manage the non-restorative sleep associated with fibromyalgia through the use of prescription sedatives or hypnotics, which are not effective for treating the sleep quality problems associated with fibromyalgia and can be addictive. Despite the broad therapeutic usefulness of cyclobenzaprine, cyclobenzaprine often causes fatigue, somnolence, a groggy feeling, or cognitive impairment in individuals, which is not desirable during normal periods of wakefulness. Cyclobenzaprine also is only recommended for short-term usage, as it has not been shown to provide benefits during long-term administration. In part because cyclobenzaprine is not a chronic treatment, in desperation fibromyalgia patients sometimes try to manage the pain associated with fibromyalgia through the use of opiate analgesics, which are not effective for treating fibromyalgia pain and can be addictive. Studies with cyclobenzaprine in various formulations, have led to the conclusion that the slow and delayed absorption of cyclobenzaprine after oral administration leads to undesirable characteristics for a bedtime medication designed to target the sleeping brain in daily therapy. Thus, improved cyclobenzaprine formulations are desirable.

### Summary of the Invention

The present invention provides a compostion suitable for oral transmucosal absorption comprising a pharmaceutically acceptable cyclobenzaprine salt and a basifying agent. In embodiments, the oral transmucosal absorption is sublingual absorption. In embodiments, the composition is in a form selected from the group consisting of a sublingual tablet, a sublingual film, a sublingual powder and a sublingual spray solution. In embodiments, the pharmaceutically acceptable cyclobenzaprine salt is cyclobenzaprine HCl. In embodiments, the basifying agent is selected from the group consisting of potassium dihydrogen phosphate, dipotassium hydrogen phosphate, tripotassium phosphate, sodium carbonate, sodium bicarbonate, calcium carbonate, calcium bicarbonate, TRIS buffer, sodium dihydrogen phosphate, disodium hydrogen phosphate, trisodium phosphate, potassium carbonate, potassium bicarbonate, potassium acetate, sodium acetate, dipotassium citrate, tripotassium citrate, disodium citrate and trisodium citrate. In a preferred embodiment, the basifying agent is dipotassium hydrogen phosphate. In another preferred embodiment, the basifying agent is potassium dihydrogen phosphate.

In another aspect, the invention provides a composition of the invention for use in a method for treating a disease or condition in an individual, the method comprising administering said composition by transmucosal absorption, wherein the disease or condition is post-traumatic stress disorder (PTSD).

In a further aspect, the invention provides a composition of the invention for use in a method for treating a disease or condition in an individual, the method comprising administering said composition by transmucosal absorption, wherein the disease or condition is selected from the group consisting of fibromyalgia, depression, traumatic brain injury, sleep disorder, non-restorative sleep, chronic pain, muscle spasm, and anxiety disorder.

Detailed Description

The present disclosure (which includes the invention as set out in the appended claims and above) is directed generally to a composition comprising cyclobenzaprine and a basifying agent, wherein the composition is suitable for oral transmucosal absorption.

The basifying agent can optionally be selected from the group consisting of potassium dihydrogen phosphate, dipotassium hydrogen phosphate, tripotassium phosphate, sodium carbonate, sodium bicarbonate, calcium carbonate, calcium bicarbonate, TRIS buffer, sodium dihydrogen phosphate, disodium hydrogen phosphate, trisodium phosphate, potassium carbonate, potassium bicarbonate, potassium acetate, sodium acetate, dipotassium citrate, tripotassium citrate and trisodium citrate.

The transmucosal absorption is oral absorption. The composition can optionally be suitable for sublingual administration. For example, the composition can be in a form selected from the group consisting of a sublingual tablet, a sublingual film, a sublingual powder, and a sublingual spray solution.

The composition can optionally be suitable for buccal administration. For example, the composition can be in a form selected from the group consisting of a buccal tablet, a lozenge, a buccal powder, and a buccal spray solution.

In certain embodiments not claimed, the transmucosal absorption is intranasal absorption. In further embodiments not claimed, the composition is in a form of a nasal spray solution. In certain embodiments not claimed, the transmucosal absorption is pulmonary absorption. In further embodiments not claimed, the composition is in a form selected from the group consisting of an aerosolized composition and an inhalable dry powder.

A composition can optionally be characterized in that, when administered by transmucosal absorption, the cyclobenzaprine has a dnC* of greater than or equal to 50 $\pm$ 25% $\times$ $10^{-9}$ $mL^{-1}$ 10 minutes after administration. A composition can optionally be characterized in that, when administered by transmucosal absorption, the cyclobenzaprine has a dnC* of greater than or equal to 125 $\pm$ 25% $\times$ $10^{-9}$ $mL^{-1}$ 15 minutes after administration. A composition can optionally be characterized in that, when administered by transmucosal absorption, the cyclobenzaprine has a dnC* of greater than or equal to 150 $\pm$ 25% $\times$ $10^{-9}$ $mL^{-1}$ 20 minutes after administration. A composition can optionally be characterized in that, when administered by transmucosal absorption, the cyclobenzaprine has a dnC* of greater than or equal to 300 $\pm$ 25% $\times$ $10^{-9}$ $mL^{-1}$ 30 minutes after administration. A composition can optionally be characterized in that, when administered by transmucosal absorption, the cyclobenzaprine has a dnC* of greater than or equal to 450 $\pm$ 25% $\times$ $10^{-9}$ $mL^{-1}$ 45 minutes after administration. A composition can optionally be characterized in that, when administered by transmucosal absorption, the cyclobenzaprine has a dnC* of greater than or equal to 600 $\pm$ 25% $\times$ $10^{-9}$ $mL^{-1}$ 1 hour after administration. A composition can optionally be characterized in that, when administered by transmucosal absorption, the cyclobenzaprine has a dnC* of greater than or equal to 700 $\pm$ 25% $\times$ $10^{-9}$ $mL^{-1}$ 2 hours after administration. A composition can optionally be characterized in that, when administered by transmucosal absorption, the cyclobenzaprine has a dnC* of greater than or equal to 750 $\pm$ 25% $\times$ $10^{-9}$ $mL^{-1}$ 2.5 (150 min) hours after administration. A composition can optionally be characterized in that, when administered by transmucosal absorption, the cyclobenzaprine has a dnC* of greater than or equal to 850 $\pm$ 25% $\times$ $10^{-9}$ $mL^{-1}$ 3 hours after administration. A composition can optionally be characterized in that, when administered by transmucosal absorption, the cyclobenzaprine has a dnC* of greater than or equal to 900 $\pm$ 25% $\times$ $10^{-9}$ $mL^{-1}$ 3.3 hours (200 min) after administration. A composition can optionally be characterized in that, when administered by transmucosal absorption, the cyclobenzaprine has a dnC* of greater than or equal to 950 $\pm$ 25% $\times$ $10^{-9}$ $mL^{-1}$ 3.7 (220 min) hours after administration. A composition can optionally be characterized in that, when administered by transmucosal absorption, the cyclobenzaprine has a dnC* of greater than or equal to 1000 $\pm$ 25% $\times$ $10^{-9}$ $mL^{-1}$ 4 hours after administration. A composition can optionally be characterized in that, when administered by transmucosal absorption, the cyclobenzaprine has a dnC* of greater than or equal to 1000 $\pm$ 25% $\times$ $10^{-9}$ $mL^{-1}$ 4.33 (260 min) hours after administration. A composition can optionally be characterized in that, when administered by transmucosal absorption, the cyclobenzaprine has a dnC* of greater than or equal to 1050 $\pm$ 25% $\times$ $10^{-9}$ $mL^{-1}$ 4.67 hours (280 min) after administration. A composition can optionally be characterized in that, when administered by transmucosal absorption, the cyclobenzaprine has a dnC* of greater than or equal to 1000 $\pm$ 25% $\times$ $10^{-9}$ $mL^{-1}$ 5 hours after administration. A composition can optionally be characterized in that, when administered by transmucosal absorption, the cyclobenzaprine has a dnC* of greater than or equal to 1000 $\pm$ 25% $\times$ $10^{-9}$ $mL^{-1}$ 5.5 hours after administration. A composition can optionally be characterized in that, when administered by transmucosal absorption, the cyclobenzaprine has a dnC* of greater than or equal to 900 $\pm$ 25% $\times$ $10^{-9}$ $mL^{-1}$ 6 hours after administration. A composition can optionally be characterized in that, when administered by transmucosal absorption, the cyclobenzaprine has a dnC* of greater than or equal to 700 $\pm$ 25% $\times$ $10^{-9}$ $mL^{-1}$ 8 hours after administration. A composition can optionally be characterized in that, when administered by transmucosal absorption, the cyclobenzaprine has a dnC* of less than or equal to 650 $\pm$ 25% $\times$ $10^{-9}$ $mL^{-1}$ 10 hours after administration. A composition can optionally be characterized in that, when administered by transmucosal absorption, the cyclobenzaprine has a dnC* of less than or equal to 500 $\pm$ 25% $\times$ $10^{-9}$ $mL^{-1}$ 12 hours after administration. A composition can optionally be characterized in that, when administered by transmucosal absorption, the cyclobenzaprine has a dnC* of less than or equal to 500 $\pm$ 25% $\times$ $10^{-9}$ $mL^{-1}$ 14 hours after administration. A composition can optionally be characterized in that, when administered by transmucosal absorption, the cyclobenzaprine has a dnC* of less than or equal to 350 ± 25% × $10^{-9}$ $mL^{-1}$ 16 hours after administration. A composition can optionally be characterized in that, when administered by transmucosal absorption, the cyclobenzaprine has a dnC* of less than or equal to 350 ± 25% × $10^{-9}$ $mL^{-1}$ 18 hours after administration. A composition can optionally be characterized in that, when administered by transmucosal absorption, the cyclobenzaprine has a dnC* of less than or equal to 300 ± 25% × $10^{-9}$ $mL^{-1}$ 20 hours after administration. A composition can optionally be characterized in that, when administered by transmucosal absorption, the cyclobenzaprine has a dnC* of less than or equal to 300 ± 25% × $10^{-9}$ $mL^{-1}$ 22 hours after administration. A composition can optionally be characterized in that, when administered by transmucosal absorption, the cyclobenzaprine has a dnC* of less than or equal to 300 ± 25% × $10^{-9}$ $mL^{-1}$ 24 hours after administration. A composition can optionally be characterized in that, when administered by transmucosal absorption, the cyclobenzaprine has a dnC* of less than or equal to 200 ± 25% × $10^{-9}$ $mL^{-1}$ 36 hours after administration. A composition can optionally be characterized in that, when administered by transmucosal absorption, the cyclobenzaprine has a dnC* of less than or equal to 150 ± 25% × $10^{-9}$ $mL^{-1}$ 48 hours after administration. A composition can optionally be characterized in that, when administered by transmucosal absorption, the cyclobenzaprine has a dnC* of less than or equal to 100 ± 25% × $10^{-9}$ $mL^{-1}$ 72 hours after administration. A composition can optionally be characterized in that, when administered by transmucosal absorption, the cyclobenzaprine has a dnC* of greater than or equal to 2.0 ± 25% × $10^{-9}$ $mL^{-1}$ 30 minutes after administration. A composition can optionally be characterized in that, when administered by transmucosal absorption, the cyclobenzaprine has a dnC* of greater than or equal to 2.0 ± 25% × $10^{-9}$ $mL^{-1}$ 45 minutes after administration. A composition can optionally be characterized in that, when administered by transmucosal absorption, the cyclobenzaprine has a dnC* of greater than or equal to 2.0 ± 25% × $10^{-9}$ $mL^{-1}$ 1 hour after administration. A composition can optionally be characterized in that, when administered by transmucosal absorption, the cyclobenzaprine has a dnC* of greater than or equal to 2.0 ± 25% × $10^{-9}$ $mL^{-1}$ 2 hours after administration. A composition can optionally be characterized in that, when administered by transmucosal absorption, the cyclobenzaprine has a dnC* of greater than or equal to 2.0 ± 25% × $10^{-9}$ $mL^{-1}$ 3 hours after administration. A composition can optionally be characterized in that, when administered by transmucosal absorption, the cyclobenzaprine has a dnC* of less than or equal to 5.0 ± 25% × $10^{-7}$ $mL^{-1}$ 8 hours after administration. A composition can optionally be characterized in that, when administered by transmucosal absorption, the cyclobenzaprine has a dnC* of less than or equal to 5.0 ± 25% × $10^{-7}$ $mL^{-1}$ 10 hours after administration. A composition can optionally be characterized in that, when administered by transmucosal absorption, the cyclobenzaprine has a dnC* of less than or equal to 5.0 ± 25% × $10^{-7}$ $mL^{-1}$ 12 hours after administration. A composition can optionally be characterized in that, when administered by transmucosal absorption, the cyclobenzaprine has a dnC* of less than or equal to 5.0 ± 25% × $10^{-7}$ $mL^{-1}$ 14 hours after administration. A composition can optionally be characterized in that, when administered by transmucosal absorption, the cyclobenzaprine has a dnC* of less than or equal to 5.0 ± 25% × $10^{-7}$ $mL^{-1}$ 16 hours after administration. A composition can optionally be characterized in that, when administered by transmucosal absorption, the cyclobenzaprine has a dnC* of less than or equal to 5.0 ± 25% × $10^{-7}$ $mL^{-1}$ 18 hours after administration. A composition can optionally be characterized in that, when administered by transmucosal absorption, the cyclobenzaprine has a dnC* of less than or equal to 5.0 ± 25% × $10^{-7}$ $mL^{-1}$ 20 hours after administration. A composition can optionally be characterized in that, when administered by transmucosal absorption, the cyclobenzaprine has a dnC* of less than or equal to 5.0 ± 25% × $10^{-7}$ $mL^{-1}$ 22 hours after administration. A composition can optionally be characterized in that, when administered by transmucosal absorption, the cyclobenzaprine has a dnC* of less than or equal to 5.0 ± 25% × $10^{-7}$ $mL^{-1}$ 24 hours after administration.

Optionally, a composition is characterized in that, when administered by transmucosal absorption, the cyclobenzaprine has a $dnAUC_{0-8h}$ of greater than or equal to 5 ± 25% × $10^{-6}$ $mL^{-1}$ hr. A composition can optionally be characterized in that, when administered by transmucosal absorption, the cyclobenzaprine has a $dnAUC_{0-\infty h}$ of greater than or equal to 20 ± 25% × $10^{-6}$ $mL^{-1}$ hr. A composition can optionally be characterized in that, when administered by transmucosal absorption, the cyclobenzaprine has a $dnC_{max}$* of greater than or equal to 1.0 ± 25% × $10^{-6}$ $mL^{-1}$. A composition can optionally be characterized in that, when administered by transmucosal absorption, the cyclobenzaprine has a partial AUCo-20min of greater than or equal to 37 ± 25% ng hr $L^{-1}$, an $AUC_{0-30min}$ of greater than or equal to 128 ± 25% ng hr $L^{-1}$, an $AUC_{0-45min}$ of greater than or equal to 333 ± 25% ng hr $L^{-1}$, an $AUC_{0-1h}$ of greater than or equal to 614 ± 25% ng hr $L^{-1}$, an $AUC_{0-2h}$ of greater than or equal to 2098 ± 25% ng hr $L^{-1}$, an $AUC_{0-2.5h}$ of greater than or equal to 2955 ± 25% ng hr $L^{-1}$, an $AUC_{0-3h}$ of greater than or equal to 3931 ± 25% ng hr $L^{-1}$. A composition can optionally be characterized in that, when administered by transmucosal absorption, the cyclobenzaprine has a partial AUCo-20min of greater than or equal to 23 ± 25% ng hr L-1, an $AUC_{0-30min}$ of greater than or equal to 86 ± 25% ng hr $L^{-1}$, an $AUC_{0-45min}$ of greater than or equal to 223 ± 25% ng hr $L^{-1}$, an $AUC_{0-1h}$ of greater than or equal to 405 ± 25% ng hr $L^{-1}$, an $AUC_{0-2h}$ of greater than or equal to 1478 ± 25% ng hr $L^{-1}$, an $AUC_{0-2.5h}$ of greater than or equal to 2167 ± 25% ng hr $L^{-1}$. Optionally, the $dnAUC_{0-20min}$ is about 0.02 ± 25% × $10^{-6}$ hr $mL^{-1}$, the $dnAUC_{0-30min}$ is about 0.05 ± 25% × $10^{-6}$ hr $mL^{-1}$, the $dnAUC_{0-45min}$ is about 0.15 ± 25% × $10^{-6}$ hr $mL^{-1}$, the $dnAUC_{0-1h}$ is about 0.25 ± 25% × $10^{-6}$ hr $mL^{-1}$, the $dnAUC_{0-2h}$ is about 0.9 ± 25% × $10^{-6}$ hr $mL^{-1}$, the $dnAUC_{0-2.5h}$ is about 1.2 ± 25% × $10^{-6}$ hr $mL^{-1}$, the $dnAUC_{0-3h}$ is about 1.5 ± 25% × $10^{-6}$ hr $mL^{-1}$, the $dnAUC_{3.3h}$ is about 1.8 ± 25% x $10^{-6}$ hr $mL^{-1}$, $dnAUC_{0-3.7h}$ is about 2.3 ± 25%

$\times 10^{-6}$ hr $mL^{-1}$, the $dnAUC_{0-4h}$ is about 2.6 $\pm$ 25% $\times 10^{-6}$ hr $mL^{-1}$, the $dnAUC_{0-4.3h}$ is about 3.0 $\pm$ 25% $\times 10^{-6}$ hr $mL^{-1}$, the $dnAUC_{0-4\cdot7h}$ is about 3.3 $\pm$ 25% $\times 10^{-6}$ hr $mL^{-1}$, the $dnAUC_{0-5h}$ is about 3.7 $\pm$ 25% $\times 10^{-6}$ hr $mL^{-1}$, the $dnAUC_{0-5.5h}$ is about 4.2 $\pm$ 25% $\times 10^{-6}$ hr $mL^{-1}$, the $dnAUC_{0-6h}$ is about 4.7 $\pm$ 25% $\times 10^{-6}$ hr $mL^{-1}$, the $dnAUC_{0-8h}$ is 6.30 $\pm$ 25% $\times 10^{-6}$ hr $mL^{-1}$, the $dnAUC_{0-12h}$ is about 20 $\pm$ 25% $\times 10^{-6}$ hr $mL^{-1}$, and the $dnAUC_{0-\infty h}$ is about 25 $\pm$ 25% $\times 10^{-6}$ hr $mL^{-1}$.

A composition can optionally be characterized in that, when administered by transmucosal absorption, the cyclobenzaprine has a $dbmnAUC_{0-20min}$ of greater than or equal to1.0 $\pm$ 25% $\times 10^{-6}$ kg hr $mL^{-1}$. A composition can optionally be characterized in that, when administered by transmucosal absorption, the cyclobenzaprine has a $dbmnAUC_{0-30min}$ of greater than or equal to 3.5 $\pm$ 25% $\times 10^{-6}$ kg hr $mL^{-1}$. A composition can optionally be characterized in that, when administered by transmucosal absorption, the cyclobenzaprine has a $dbmnAUC_{0-45min}$ of greater than or equal to 10 $\pm$ 25% $\times 10^{-6}$ kg hr $mL^{-1}$. A composition can optionally be characterized in that, when administered by transmucosal absorption, the cyclobenzaprine has a $dbmnAUC_{0-1h}$ of greater than or equal to 18 $\pm$ 25% $\times 10^{-6}$ kg hr $mL^{-1}$. A composition can optionally be characterized in that, when administered by transmucosal absorption, the cyclobenzaprine has a $dbmnAUC_{0-2h}$ of greater than or equal to 60 $\pm$ 25% $\times 10^{-6}$ kg hr $mL^{-1}$. A composition can optionally be characterized in that, when administered by transmucosal absorption, the cyclobenzaprine has a $dbmnAUC_{0-2.5h}$ of greater than or equal to 85 $\pm$ 25% $\times 10^{-6}$ kg hr $mL^{-1}$. A composition can optionally be characterized in that, when administered by transmucosal absorption, the cyclobenzaprine has a $dbmnAUC_{0-3h}$ of greater than or equal to 115 $\pm$ 25% $\times 10^{-6}$ kg hr $mL^{-1}$. A composition can optionally be characterized in that, when administered by transmucosal absorption, the cyclobenzaprine has a $dbmnAUC_{3.3h}$ of greater than or equal to 135 $\pm$ 25% $\times 10^{-6}$ kg hr $mL^{-1}$. A composition can optionally be characterized in that, when administered by transmucosal absorption, the cyclobenzaprine has a$dbmnAUC_{0-37h}$ of greater than or equal to 160 $\pm$ 25% $\times 10^{-6}$ kg hr $mL^{-1}$. A composition can optionally be characterized in that, when administered by transmucosal absorption, the cyclobenzaprine has a $dbmnAUC_{0-4h}$ of greater than or equal to 180 $\pm$ 25% $\times 10^{-6}$ kg hr $mL^{-1}$. A composition can optionally be characterized in that, when administered by transmucosal absorption, the cyclobenzaprine has a $dbmnAUC_{0-4.3h}$ of greater than or equal to 210 $\pm$ 25% $\times 10^{-6}$ kg hr $mL^{-1}$. A composition can optionally be characterized in that, when administered by transmucosal absorption, the cyclobenzaprine has a $dbmnAUC_{0-4.7h}$ of greater than or equal to 230 $\pm$ 25% $\times 10^{-6}$ kg hr $mL^{-1}$. A composition can optionally be characterized in that, when administered by transmucosal absorption, the cyclobenzaprine has a $dbmnAUC_{0-5h}$ of greater than or equal to 250 $\pm$ 25% $\times 10^{-6}$ kg hr $mL^{-1}$. A composition can optionally be characterized in that, when administered by transmucosal absorption, the cyclobenzaprine has a $dbmnAUC_{0-5.5h}$ of greater than or equal to 290 $\pm$ 25% $\times 10^{-6}$ kg hr $mL^{-1}$. A composition can optionally be characterized in that, when administered by transmucosal absorption, the cyclobenzaprine has a $dbmnAUC_{0-6h}$ of greater than or equal to 330 $\pm$ 25% $\times 10^{-6}$ kg hr $mL^{-1}$. A composition can optionally be characterized in that, when administered by transmucosal absorption, the cyclobenzaprine has a $dbmnAUC_{0-8h}$ is 440 $\pm$ 25% $\times 10^{-6}$ kg hr $mL^{-1}$. A composition can optionally be characterized in that, when administered by transmucosal absorption, the cyclobenzaprine has a $dbmnAUC_{0-12h}$ of greater than or equal to 1500 $\pm$ 25% $\times 10^{-6}$ kg hr $mL^{-1}$. A composition can optionally be characterized in that, when administered by transmucosal absorption, the cyclobenzaprine has a $dbmnAUC_{0-Inf}$ of greater than or equal to 1800 $\pm$ 25% $\times 10^{-6}$ kg hr $mL^{-1}$

A composition can optionally be characterized in that, when administered by transmucosal absorption, the cyclobenzaprine has a plasma concentration of 50% or less of the $C_{max}$ 8 hours after administration. A composition can optionally be characterized in that, when administered by transmucosal absorption, the cyclobenzaprine has a plasma concentration of 50% or less of the $C_{max}$ 12 hours after administration.

Cyclobenzaprine may be present in a composition of the disclosure in an amount from 0.1 mg to 10 mg, for example, from 0.1 mg to 5 mg. The cyclobenzaprine can optionally be present in an amount of about 2.4 mg, less than about 2.4 mg, about 4.8 mg, or less than about 4.8 mg. The cyclobenzaprine can optionally be present in an amount of about 2.8 mg, less than about 2.8 mg, about 5.6 mg, or less than about 5.6 mg. The cyclobenzaprine can optionally be present in an amount of about 4.5 mg, less than about 5 mg, about 9 mg, or less than about 10 mg.

A composition can optionally be characterized in that, when administered by transmucosal absorption, the composition affords a $C_{max}$ of cyclobenzaprine greater than or equal to 10 ng/mL. A composition can optionally be characterized in that, when administered by transmucosal absorption, the composition affords a $C_{max}$ of cyclobenzaprine greater than or equal to 15 ng/mL, greater than or equal to 20 ng/mL, greater than or equal to 25 ng/mL or greater than or equal to 30 ng/mL. A composition can optionally be characterized in that, when administered by transmucosal absorption, the composition affords a $C_{max}$ of cyclobenzaprine greater than or equal to 2.5 ng/mL, greater than or equal to 3 ng/mL, greater than or equal to 4 ng/mL, greater than or equal to 10 ng/mL. A composition can optionally be characterized in that, when administered by transmucosal absorption, the composition affords a $C_{max}$ of cyclobenzaprine greater than or equal to 2.74 ng/mL, greater than or equal to 3.20 ng/mL, greater than or equal to 5.13 ng/mL or greater than or equal to 10.27 ng/mL.

A composition can optionally be characterized in that, when administered by transmucosal absorption, the composition affords a $C_{max}$ of cyclobenzaprine greater than or equal to 10 ng/mL, greater than or equal to 15 ng/mL, greater than or equal to 20 ng/mL, greater than or equal to 25 ng/mL, or greater than or equal to 30 ng/mL above the baseline level of cyclobenzaprine in the individual immediately prior to administration. In relation to repeated, repetitive, daily and chronic dosing, a composition can optionally be characterized in that, when administered by transmucosal absorption, the composition affords a $C_{max}$ of cyclobenzaprine greater than or equal to 10 ng/mL, greater than or equal to 15 ng/mL, greater than or equal to 20 ng/mL, greater than or equal to 25 ng/mL, or greater than or equal to 30 ng/mL above the baseline level of cyclobenzaprine in the individual immediately prior to administration. A composition can optionally be characterized in that, when administered by transmucosal absorption, the composition affords a $C_{max}$ of cyclobenzaprine greater than or equal to 2.74 ng/ml, greater than or equal to 3.20 ng/ml, greater than or equal to 5.13 ng/ml, or greater than or equal to 10.27 ng/ml above the baseline level of cyclobenzaprine in the individual immediately prior to administration. In relation to repeated, repetitive, daily and chronic dosing, a composition can optionally be characterized in that, when administered by transmucosal absorption, the composition affords a $C_{max}$ of cyclobenzaprine greater than or equal to 10 ng/ml, greater than or equal to 15 ng/ml, greater than or equal to 20 ng/ml, greater than or equal to 25 ng/ml, or greater than or equal to 30 ng/ml above the baseline level of cyclobenzaprine in the individual immediately prior to administration.

A composition can optionally be characterized in that, when administered by transmucosal absorption, the composition affords a $t_{max}$ of cyclobenzaprine of less than 4.70 hours.

A composition can optionally be characterized in that, when administered by transmucosal absorption, the composition affords a plasma level of cyclobenzaprine that decreases by at least 50%, by at least 60%, by at least 70%, by at least 80% the $C_{max}$ by 8 hours after administration.

The present disclosure also relates to the composition of the invention for use in a method for treating a disease or condition in an individual in need thereof wherein the disease or condition is post-traumatic stress disorder (PTSD). For example, administration of the composition can optionally treat the development of PTSD following a traumatic event, the initiation of PTSD following a traumatic event, the consolidation of PTSD following a traumatic event, or the perpetuation of PTSD following a traumatic event. Alternatively, the disease or condition can optionally be selected from the group consisting of fibromyalgia, depression, traumatic brain injury, sleep disorder, non-restorative sleep, chronic pain, muscle spasm, acute pain, and anxiety disorder. Throughout the specification, reference to a method of treatment means the composition of the invention for use in a method of treatment.

The basifying agent useful in methods disclosed herein can optionally be selected from the group consisting of potassium dihydrogen phosphate, dipotassium hydrogen phosphate, tripotassium phosphate, sodium carbonate, sodium bicarbonate, calcium carbonate, calcium bicarbonate, TRIS buffer, sodium dihydrogen phosphate, disodium hydrogen phosphate, trisodium phosphate, potassium carbonate, potassium bicarbonate, potassium acetate, sodium acetate, tripotassium citrate, dipotassium citrate, trisodium citrate and disodium citrate.

The oral absorption in a method disclosed herein can optionally be sublingual absorption. For example, the composition can optionally be in a form selected from the group consisting of a sublingual tablet, a sublingual film, a sublingual powder, and a sublingual spray solution.

The oral absorption in a method disclosed herein can optionally be buccal absorption. For example, the composition can optionally be selected from the group consisting of a buccal tablet, a lozenge, a buccal powder, and a buccal spray solution.

In some embodiments not claimed, the transmucosal absorption useful in a method of the invention is intranasal absorption. In certain embodiments not claimed, the composition is in a form of a nasal spray solution.

In some embodiments not claimed, the transmucosal absorption useful in a method of the invention is pulmonary absorption. In certain embodiments not claimed, the composition is in a form selected from the group consisting of an aerosolized composition and an inhalable dry powder.

In a method disclosed herein, the cyclobenzaprinemay optionally have a dnC* of greater than or equal to 8.0 $\pm$ 25% $\times$ $10^{-7}$ $mL^{-1}$ 15 minutes after administration, greater than or equal to 1.0 $\pm$ 25% $\times$ $10^{-6}$ $mL^{-1}$ 30 minutes after administration, greater than or equal to 1.0 $\pm$ 25% $\times$ $10^{-6}$ $mL^{-1}$ 45 minutes after administration, greater than or equal to 1.0 $\pm$ 25% $\times$ $10^{-6}$ $mL^{-1}$ 1 hour after administration, greater than or equal to 1.0 $\pm$ 25% $\times$ $10^{-6}$ $mL^{-1}$ 2 hours after administration, or greater than or equal to1.0 $\times$ $mL^{-1}$ 3 hours after administration. The cyclobenzaprine when used in a method disclosed herein can optionally have a dnC* of less than or equal to 5.0 $\pm$ 25% $\times$ $10^{-7}$ $mL^{-1}$ 8 hours after administration. The cyclobenzaprine when used in a method disclosed herein can optionally have a dnC* of less than or equal to 5.0 $\pm$ 25% $\times$ $10^{-7}$ $mL^{-1}$ 10 hours after administration. The cyclobenzaprine when used in a method disclosed herein can optionally have a dnC* of less than or equal to 5.0 $\pm$ 25% $\times$ $10^{-7}$ $mL^{-1}$ 12 hours after administration. The cyclobenzaprine when used in a method disclosed herein can optionally havea dnC* of less than or equal to 5.0 $\pm$ 25% $\times$ $10^{-7}$ $mL^{-1}$ 14 hours after administration. The cyclobenzaprine when used in a method disclosed herein can optionally have a dnC* of less than or equal to 5.0 $\pm$ 25% $\times$ $10^{-7}$ $mL^{-1}$ 16 hours after administration. The cyclobenzaprinewhen used in a method disclosed herein can optionally have a dnC* of less than or equal to 5.0 $\pm$ 25% $\times$ $10^{-7}$ $mL^{-1}$ 18 hours after administration. The cyclobenzaprine when used in a method disclosed herein can optionally have a dnC* of less than or equal to 5.0 $\pm$ 25% $\times$ $10^{-7}$ $mL^{-1}$ 20 hours after administration. The cyclobenzaprine when used in a method disclosed herein can optionally havea dnC* of less than or equal to 5.0 $\pm$ 25% $\times$ $10^{-7}$ $mL^{-1}$ 22 hours after administration. The cyclobenzaprine when used in a method disclosed herein can optionally have a dnC* of less than or equal to 5.0

$\pm$ 25% $\times$ $10^{-7}$ $mL^{-1}$ 24 hours after administration. The cyclobenzaprine when used in a method disclosed herein can optionally have a dnC* of greater than or equal to 50 $\pm$ 25% $\times$ $10^{-9}$ $mL^{-1}$ 10 minutes after administration. The cyclobenzaprine when used in a method disclosed herein can optionally havea dnC* of greater than or equal to 150 $\pm$ 25% $\times$ $10^{-9}$ $mL^{-1}$ 20 minutes after administration. The cyclobenzaprine when used in a method disclosed herein can optionally havea dnC* of greater than or equal to 300 $\pm$ 25% $\times$ $10^{-9}$ $mL^{-1}$ 30 minutes after administration. The cyclobenzaprine when used in a method disclosed herein can optionally have a dnC* of greater than or equal to 450 $\pm$ 25% $\times$ $10^{-9}$ $mL^{-1}$ 45 minutes after administration. The cyclobenzaprine when used in a method disclosed herein can optionally havea dnC* of greater than or equal to 600 $\pm$ 25% $\times$ $10^{-9}$ $mL^{-1}$ 1 hour after administration. The cyclobenzaprine when used in a method disclosed herein can optionally havea dnC* of greater than or equal to 700 $\pm$ 25% $\times$ $10^{-9}$ $mL^{-1}$ 2 hours after administration. The cyclobenzaprine when used in a method disclosed herein can optionally havea dnC* of greater than or equal to 750 $\pm$ 25% $\times$ $10^{-9}$ $mL^{-1}$ 2.5 hours after administration. The cyclobenzaprine when used in a method disclosed herein can optionally have a dnC* of greater than or equal to 850 $\pm$ 25% x $10^{-9}$ $mL^{-1}$ 3 hours after administration. The cyclobenzaprine when used in a method disclosed herein can optionally havea dnC* of greater than or equal to 900 $\pm$ 25% $\times$ $10^{-9}$ $mL^{-1}$ 3.3 hours after administration. The cyclobenzaprine when used in a method disclosed herein can optionally have a dnC* of greater than or equal to 950 $\pm$ 25% $\times$ $10^{-9}$ $mL^{-1}$ 3.7 hours after administration. The cyclobenzaprine when used in a method disclosed herein can optionally havea dnC* of greater than or equal to 1000 $\pm$ 25% $\times$ $10^{-9}$ $mL^{-1}$ 4 hours after administration. The cyclobenzaprine when used in a method disclosed herein can optionally havea dnC* of greater than or equal to 1050 $\pm$ 25% $\times$ $10^{-9}$ $mL^{-1}$ 4.33 hours after administration. The cyclobenzaprine when used in a method disclosed herein can optionally have a dnC* of less than or equal to 1050 $\pm$ 25% $\times$ $10^{-9}$ $mL^{-1}$ 4.67 hours after administration. The cyclobenzaprine when used in a method disclosed herein can optionally havea dnC* of less than or equal to 1000 $\pm$ 25% $\times$ $10^{-9}$ $mL^{-1}$ 5 hours after administration. The cyclobenzaprine when used in a method disclosed herein can optionally havea dnC* of less than or equal to 1000 $\pm$ 25% $\times$ $10^{-9}$ $mL^{-1}$ 5.5 hours after administration. The cyclobenzaprine when used in a method disclosed herein can optionally havea dnC* of less than or equal to 900 $\pm$ 25% $\times$ $10^{-9}$ $mL^{-1}$ 6 hours after administration. The cyclobenzaprine when used in a method disclosed herein can optionally havea dnC* of less than or equal to 700 $\pm$ 25% $\times$ $10^{-9}$ $mL^{-1}$ 8 hours after administration. The cyclobenzaprine when used in a method disclosed herein can optionally havea dnC* of less than or equal to 500 $\pm$ 25% $\times$ $10^{-9}$ $mL^{-1}$ 12 hours after administration. The cyclobenzaprine when used in a method disclosed herein can optionally havea dnC* of less than or equal to 350 $\pm$ 25% $\times$ $10^{-9}$ $mL^{-1}$ 16 hours after administration. The cyclobenzaprine when used in a method disclosed herein can optionally have a dnC* of less than or equal to 300 $\pm$ 25% $\times$ $10^{-9}$ $mL^{-1}$ 24 hours after administration. The cyclobenzaprine when used in a method disclosed herein can optionally havea dnC* of less than or equal to 180 $\pm$ 25% $\times$ $10^{-9}$ $mL^{-1}$ 36 hours after administration. The cyclobenzaprine when used in a method disclosed herein can optionally havea dnC* of less than or equal to 140 $\pm$ 25% $\times$ $10^{-9}$ $mL^{-1}$ 48 hours after administration. The cyclobenzaprine when used in a method disclosed herein can optionally havea dnC* of less than or equal to 90 $\pm$ 25% $\times$ $10^{-9}$ $mL^{-1}$ 72 hours after administration.

In a methoddisclosed herein, the cyclobenzaprine can optionally have a $dnAUC_{0-8h}$ of greater than or equal to 5 $mL^{-1}$ hr. The cyclobenzaprine can optionally have a $dnAUC_{0-\infty h}$ of greater than or equal to 20 $mL^{-1}$ hr. In a method disclosed herein, the cyclobenzaprine can optionally have a $dnC_{max}$* of greater than or equal to 1.0 $\pm$ 25% $\times$ $10^{-6}$ $mL^{-1}$. In a method disclosed herein, the cyclobenzaprine can optionally have a $dnAUC_{0-8h}$ of greater than or equal to 6.3 $\pm$ 25% $\times$ $10^{-6}$ hr $mL^{-1}$. The cyclobenzaprine can optionally have a $dnAUC_{0-\infty h}$ of greater than or equal to 25 $\pm$ 25% $\times$ $10^{-6}$ hr $mL^{-1}$. In a method disclosed herein, the cyclobenzaprine can optionally have a $dnC_{max}$* of greater than or equal to 1.1 $\pm$ 25% $\times$ $10^{-6}$ $mL^{-1}$.

In a method disclosed herein, the cyclobenzaprine can optionally have a plasma concentration of 50% or less of the $C_{max}$ 4 hours after administration, 50% or less of the $C_{max}$ 6 hours after administration, 50% or less of the $C_{max}$ 8 hours after administration, or 50% or less of the $C_{max}$ 12 hours after administration.

In a method disclosed herein, the cyclobenzaprine can optionally be present in the composition in an amount from 0.1 mg to 10 mg. The cyclobenzaprine can optionally be present in the composition in an amount from 0.1 mg to 5 mg, for example, in an amount of about 2.4 mg, in an amount of less than about 2.4 mg, in an amount of about 4.8 mg, or in an amount of less than about 4.8 mg or in an amount of about 2.8 mg, in an amount of less than about 2.8 mg, in an amount of about 5.6 mg, or in an amount of less than about 5.6 mg, in an amount of about 9.0 mg, in an amount of less than about 10 mg

In a method disclosed herein, a composition can optionally afford a $C_{max}$ of cyclobenzaprine greater than or equal to 10 ng/mL, greater than or equal to 15 ng/mL, greater than or equal to 20 ng/mL, greater than or equal to 25 ng/mL, greater than or equal to 30 ng/mL, greater than or equal to 40 ng/mL, greater than or equal to 50 ng/mL, greater than or equal to 60 ng/mL, greater than or equal to 70 ng/mL, greater than or equal to 80 ng/mL, greater than or equal to 90 ng/mL, greater than or equal to 100 ng/mL, greater than or equal to 120 ng/mL, greater than or equal to 140 ng/mL, greater than or equal to 150 ng/mL, greater than or equal to 160 ng/mL, greater than or equal to 170 ng/mL, greater than or equal to 180 ng/mL, greater than or equal to 190 ng/mL, greater than or equal to 200 ng/mL, greater than or equal to 220 ng/mL, greater than or equal to 240 ng/mL, greater than or equal to 260 ng/mL, or greater than or equal to 280 ng/mL. In a method disclosed herein, a composition can optionally afford a $C_{max}$ of cyclobenzaprine greater than or equal to 2.74 ng/ml, greater than or equal to 3.20 ng/ml, greater than or equal to 5.13 ng/ml, greater than or equal to 10.27 ng/ml, greater than or equal to 2 ng/ml, greater than or equal to 3 ng/ml.

In a method disclosed herein, a composition can optionally afford a $C_{max}$ of cyclobenzaprine greater than or equal to 10 ng/mL above the baseline level, greater than or equal to 15 ng/mL above the baseline level, greater than or equal to 20 ng/mL above the baseline level, greater than or equal to 25 ng/mL above the baseline level, greater than or equal to 30 ng/mL above the baseline level, greater than or equal to 40 ng/mL above the baseline level, greater than or equal to 50 ng/mL above the baseline level, greater than or equal to 60 ng/mL above the baseline level, greater than or equal to 70 ng/mL above the baseline level, greater than or equal to 80 ng/mL above the baseline level, greater than or equal to 90 ng/mL above the baseline level, greater than or equal to 100 ng/mL above the baseline level, greater than or equal to 120 ng/mL above the baseline level, greater than or equal to 140 ng/mL above the baseline level, greater than or equal to 150 ng/mL above the baseline level, greater than or equal to 160 ng/mL above the baseline level, greater than or equal to 170 ng/mL above the baseline level, greater than or equal to 180 ng/mL above the baseline level, greater than or equal to 190 ng/mL above the baseline level, greater than or equal to 200 ng/mL above the baseline level, greater than or equal to 220 ng/mL above the baseline level, greater than or equal to 240 ng/mL above the baseline level, greater than or equal to 260 ng/mL above the baseline level, or greater than or equal to 280 ng/mL above the baseline level of cyclobenzaprine in the individual immediately prior to administration. In relation to repeated, repetitive, daily and chronic dosing, the composition can optionally afford a $C_{max}$ of cyclobenzaprine greater than or equal to 10 ng/mL above the baseline level, greater than or equal to 15 ng/mL above the baseline level, greater than or equal to 20 ng/mL above the baseline level, greater than or equal to 25 ng/mL above the baseline level, greater than or equal to 30 ng/mL above the baseline level, greater than or equal to 40 ng/mL above the baseline level, greater than or equal to 50 ng/mL above the baseline level, greater than or equal to 60 ng/mL above the baseline level, greater than or equal to 70 ng/mL above the baseline level, greater than or equal to 80 ng/mL above the baseline level, greater than or equal to 90 ng/mL above the baseline level, greater than or equal to 100 ng/mL above the baseline level, greater than or equal to 120 ng/mL above the baseline level, greater than or equal to 140 ng/mL above the baseline level, greater than or equal to 150 ng/mL above the baseline level, greater than or equal to 160 ng/mL above the baseline level, greater than or equal to 170 ng/mL above the baseline level, greater than or equal to 180 ng/mL above the baseline level, greater than or equal to 190 ng/mL above the baseline level, greater than or equal to 200 ng/mL above the baseline level, greater than or equal to 220 ng/mL above the baseline level, greater than or equal to 240 ng/mL above the baseline level, greater than or equal to 260 ng/mL above the baseline level, or greater than or equal to 280 ng/mL above the baseline level, of cyclobenzaprine in the individual immediately prior to administration. In a method disclosed herein, a composition can optionally afford a $C_{max}$ of cyclobenzaprine greater than or equal to 2.74 ng/ml above the baseline level, greater than or equal to 3.20 ng/ml above the baseline level, greater than or equal to 5.13 ng/ml above the baseline level, greater than or equal to 10.27 ng/ml above the baseline level, greater than or equal to 2 ng/ml above the baseline level, greater than or equal to 3 ng/ml above the baseline level, 10 ng/ml, greater than or equal to 15 ng/ml above the baseline level, greater than or equal to 20 ng/ml above the baseline level, greater than or equal to 25 ng/ml above the baseline level, greater than or equal to 30 ng/ml above the baseline level, greater than or equal to 40 ng/ml above the baseline level.

In a method disclosed herein, a composition can optionally afford a $t_{max}$ of cyclobenzaprine of less than 4 hours, less than 3 hours, less than 2 hours, less than 1 hour, less than 45 minutes, less than 30 minutes, less than 15 minutes, less than 10 minutes, or less than 5 minutes.

In a method dislosed herein, the composition can optionally afford a plasma level of cyclobenzaprine that decreases by at least 50% of the $C_{max}$ by 8 hours after administration, by at least 60% of the $C_{max}$ by 8 hours after administration, by at least 70% of the $C_{max}$ by 8 hours after administration, by at least 80% of the $C_{max}$ by 8 hours after administration, by at least 90% of the $C_{max}$ by 8 hours after administration, or by at least 95% of the $C_{max}$ by 8 hours after administration.

A composition comprising cyclobenzaprine for transmucosal administration can optionally comprise from about 2 to about 20 mg of cyclobenzaprine or a salt thereof, the formulation affording a $C_{max}$ of cyclobenzaprine from about 20 to about 200 ng/mL from about 0.05 to about 2.5 hours after administration, and a minimum cyclobenzaprine plasma concentration from about 1 to about 5 ng/mL from about 22 to about 26 hours after administration, wherein the composition is administered for four days or more of daily administration. A composition comprising cyclobenzaprine for transmucosal administration can optionally comprise from about 2 to about 20 mg of cyclobenzaprine or a salt thereof, the formulation affording a $C_{max}$ of cyclobenzaprine from about 1.0 ng/ml to about 30.0 ng/ml from about 2 to about 5.0 hours after administration, and a minimum plasma concentration from about 1 to about 5 ng/ml from about 22 to about 26 hours after administration, wherein the composition is administered for four days or more of daily administration, and wherein the composition is administered within two hours of sleep.ng/ml.

A composition comprising cyclobenzaprine for transmucosal administration can optionally comprise from about 2 to about 20 mg of cyclobenzaprine or a salt thereof, the formulation affording a $dnC_{min(24)}*$ of cyclobenzaprine from about $100 \pm 25\% \times 10^{-9}$ $mL^{-1}$ to about $1000 \pm 25\% \times 10^{-9}$ $mL^{-1}$ about 22 to about 26 hours after administration, wherein the composition is administered for four days or more of daily administration, and wherein the composition is administered within two hours of sleep. A composition comprising cyclobenzaprine for transmucosal administration can optionally comprise from about 2 to about 20 mg of cyclobenzaprine or a salt thereof, the formulation affording a $dnC_{min(24)}{}^*$ of cyclobenzaprine less then or equal to 300 ± 25% × $10^{-9}$ $mL^{-1}$ (calculated using the mean $C_{min(24)}$ of 706.55 pg/mL at 24 hours and the $dnC_{min(24)}{}^*$ was ((706.55 pg/mL)/(2.4 mg)) = 294.40 pg/(mg mL), or 300 ± 25% × $10^{-9}$ $mL^{-1}$) at 24 hours or between 22 to about 26 hours after administration, wherein the composition is administered for four days or more of daily administration, and wherein the composition is administered within two hours of sleep.

A method for reducing the symptoms of fibromyalgia in a human patient can optionally comprise administering a transmucosal dosage formulation comprising from about 2 to about 20 mg of cyclobenzaprine or a salt thereof, said formulation affording a $C_{max}$ of cyclobenzaprine from about 20 to about 200 ng/mL from about 0.05 to about 2.5 hours after administration, and a minimum plasma concentration from about 1 to about 5 ng/mL from about 22 to about 26 hours after administration, wherein the composition is administered for four days or more of daily administration, and wherein the composition is administered within two hours of sleep.

A method for reducing the symptoms of fibromyalgia in a human patient can optionally comprise administering a transmucosal dosage formulation comprising of about 2.4 mg of cyclobenzaprine or a salt thereof, said formulation affording in a single dose study, a $C_{max}$ of cyclobenzaprine of about 2.74 μg $mL^{-1}$ at about 4.70 hours after administration, and a minimum plasma concentration of about 706.55 ng $mL^{-1}$ at about 24 hours after administration, wherein the composition is administered for four days or more of daily administration, and wherein the composition is administered within two hours of sleep.

A method for reducing the symptoms of PTSD in a human patient can optionally comprise administering a transmucosal dosage formulation comprising from about 2 to about 20 mg of cyclobenzaprine or a salt thereof, said formulation affording a $C_{max}$ of cyclobenzaprine from about 1.0 ng/ml to about 30.0 ng/ml from about 2 to about 5.0 hours after administration, and a minimum plasma concentration from about 1 to about 5 ng/ml from about 22 to about 26 hours after administration, wherein the composition is administered for four days or more of daily administration, and wherein the composition is administered within two hours of sleep.

A method for reducing the symptoms of muscle spasm and acute painful musculoskeletal conditions, including local pain and restriction of movement, in a human patient can optionally comprise administering a transmucosal dosage formulation comprising from about 2 to about 20 mg of cyclobenzaprine or a salt thereof, said formulation affording a $C_{max}$ of cyclobenzaprine from about 1.0 ng/ml to about 30.0 ng/ml from about 2 to about 5.0 hours after administration.

## Brief Description of the Drawings

Figure 1a depicts a linear plot of mean plasma cyclobenzaprine concentration ± standard deviation and mean plasma norcyclobenzaprine concentration ± standard deviation in 10 healthy human subjects after oral (PO) treatment with 5 mg of cyclobenzaprine HCl immediate release tablets under fasting conditions.

Figure 1b depicts a log plot of mean plasma cyclobenzaprine concentration ± standard deviation and mean plasma norcyclobenzaprine concentration ± standard deviation in 10 healthy human subjects after PO treatment with 5 mg of cyclobenzaprine HCl immediate release tablets under fasting conditions.

Figure 2 depicts a linear plot of mean cyclobenzaprine concentration ± standard deviation in female beagle dog plasma over time. Cyclobenzaprine was administered orally (PO) by nasogastric (NG) tube into the stomach, sublingually, or intravenously (IV).

Figure 3 depicts a semilogarithmic plot of mean cyclobenzaprine concentration ± standard deviation in female beagle dog plasma over time. Cyclobenzaprine was administered orally (PO) by NG tube, sublingually, or intravenously.

Figure 4 depicts mean cyclobenzaprine concentration time profiles after IV administration of cyclobenzaprine HCl ± standard deviation in female beagle dog plasma, comparing the mean of dogs treated with and without pre-anesthesia with propofol (IV Investigation) with the mean of dogs from the IV data in Figure 2.

Figure 5 depicts mean cyclobenzaprine concentration time profiles after sublingual administration of cyclobenzaprine HCl solution ± standard deviation in female beagle dog plasma, comparing the mean of dogs treated with and without pre-anesthesia with propofol (Sublingual Investigation) with the mean of dogs from the sublingual data in Figure 2.

Figure 6 depicts a linear plot of mean cyclobenzaprine concentration ± standard deviation in female beagle dog plasma over time. Cyclobenzaprine was administered sublingually in tablets that either contained or lacked the basifying agent $K_2HPO_4$.

Figure 7 depicts a log plot of mean cyclobenzaprine concentration ± standard deviation in female beagle dog plasma over time. Cyclobenzaprine was administered sublingually in tablets with and without the basifying agent, $K_2HPO_4$.

Figure 8 depicts a chart showing daily assessments performed over the course of a study of cyclobenzaprine HCl administration to humans.

Figure 9 depicts a chart showing hourly assessments performed during a study of cyclobenzaprine HCl 5 mg immediate release tablets after PO administration to humans.

Figure 10 depicts a chart showing cyclobenzaprine plasma concentration-time profiles from 0 to 1 hr after administration of sublingual (SL, Subject 7 only), oral (PO, group mean), and intravenous (IV, group mean) doses of cyclobenzaprine.

Figure 11 depicts a chart showing cyclobenzaprine plasma concentration-time profiles from 0 to 24 hrs after administration of sublingual (Subject 7 only), oral (group mean), and intravenous (group mean) doses of cyclobenzaprine.

Figure 12 depicts a chart showing mean cyclobenzaprine plasma concentration-time profiles from 0 to 24 hrs after administration of sublingual cyclobenzaprine at pH 7.1 (mean for all subjects except Subjects 7 and 10), pH 3.5 (mean for all subjects except Subject 4), and oral (group mean) cyclobenzaprine.

Figure 13 depicts a chart showing norcyclobenzaprine plasma concentration-time profiles from 0 to 24 hrs after administration of sublingual (Subject 7 only), oral (group mean), and intravenous (group mean) doses of cyclobenzaprine.

Figure 14 depicts a chart showing cyclobenzaprine plasma concention from 0 to 2 hrs after administration of 2.4 mg (Figure 14a) and 4.8 mg (Figure 14b) of sublingual cyclobenzaprine and 5 mg of oral cyclobenzaprine.

Figure 15 depicts a chart showing cyclobenzaprine plasma concention from 0 to 8 hrs after administration of 4.8 mg of sublingual cyclobenzaprine and 5 mg of oral cyclobenzaprine.

Figure 16 depicts a chart showing cyclobenzaprine plasma concention from 0 to 8 hrs after administration of 2.4 and 4.8 mg of sublingual cyclobenzaprine.

Figure 17 depicts charts showing cyclobenzaprine plasma concention from 0 to 2 hrs (Figure 17a) amd 0 to 8 hrs (Figure 17b) after administration of 2.4 mg of sublingual cyclobenzaprine with and without phosphate.

Figure 18 depicts equilibrium binding studies of cyclobenzaprine (circles) and norcyclobenzaprine (triangles) on serotonin ($5\text{-}HT_{1A}$, Figure 18a; $5\text{-}HT_{2A}$, Figure 18b; $5\text{-}HT_{2B}$, Figure 18c; $5\text{-}HT_{2C}$, Figure 18d;), histamine $H_1$ ($H_1$) (Figure 18e), adrenergic $\alpha_{1A}$ ($\alpha_{1A}$) (Figure 18f), muscarinic $M_1$ ($M_1$) (Figure 18g), and dopamine $D_1$ ($D_1$) (Figure 18h) receptors expressed in the central nervous system of humans.

Figure 19 depicts equilibrium binding studies of cyclobenzaprine (circles) and norcyclobenzaprine (triangles) on the norepinephrine (NE), 5-HT, and dopamine (D) transporters expressed in the central nervous system of humans.

Figure 20 depicts G-protein-dependent signal transduction studies of cyclobenzaprine (circles) and norcyclobenzaprine (triangles) on serotonin ($5\text{-}HT_{1A}$, Figure 20a; $5\text{-}HT_{2A}$, Figure 20b; $5\text{-}HT_{2B}$, Figure 20c; $5\text{-}HT_{2C}$, Figure 20d;), histamine $H_1$ ($H_1$) (Figure 20e), adrenergic $\alpha_{1A}$ ($\alpha_{1A}$) (Figure 20f), muscarinic $M_1$ ($M_1$) (Figure 20g), and dopamine $D_1$ ($D_1$) (Figure 20h) receptors expressed in the central nervous system of humans.

Figure 21 depicts G-protein-independent signal transduction studies of cyclobenzaprine (circles) and norcyclobenzaprine (triangles) on serotonin ($5\text{-}HT_{2A}$,) (Figure 21d), histamine $H_1$ ($H_1$) (Figure 21a), adrenergic $\alpha_{1B}$ ($\alpha_{1A}$) (Figure 21b), and muscarinic $M_1$ ($M_1$) (Figure 21c), receptors expressed in the central nervous system of humans.

Definitions

Unless otherwise defined herein, scientific and technical terms used in this specification shall have the meanings that are commonly understood by those of ordinary skill in the art. Generally, nomenclature used in connection with, and techniques of, pharmacology, cell and tissue culture, molecular biology, cell and cancer biology, neurobiology, neurochemistry, virology, immunology, microbiology, genetics and protein and nucleic acid chemistry, described herein, are those well known and commonly used in the art.

The methods and techniques disclosed herein are generally performed, unless otherwise indicated, according to conventional methods well known in the art and as described in various general and more specific references that are cited and discussed throughout this specification.

Chemistry terms used herein are used according to conventional usage in the art, as exemplified by "The McGraw-Hill Dictionary of Chemical Terms", Parker S., Ed., McGraw-Hill, San Francisco, C.A. (1985).

In case of conflict between the present specification and any other publications, patents and published patent applications referred to in this application the present specification, including its specific definitions, will control.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to." "Including" and "including but not limited to" are used interchangeably.

A "patient", "subject", or "individual" are used interchangeably and refer to either a human or a non-human animal. These terms include mammals, such as humans, primates, livestock animals (including bovines, porcines, etc.), companion animals (e.g., canines, felines, etc.) and rodents (e.g., mice and rats).

"Treating" a condition or patient refers to taking steps to obtain beneficial or desired results, including clinical results. Beneficial or desired clinical results include, but are not limited to, alleviation or amelioration of one or more symptoms associated with a disease or condition described herein.

"Administering" or "administration of" a substance, a compound or an agent to a subject can be carried out using one of a variety of methods known to those skilled in the art. For example, a compound or an agent can be administered sublingually or intranasally, by inhalation into the lung or rectally. Administering can also be performed, for example, once, a plurality of times, and/or over one or more extended periods. In some aspects, the administration includes both direct administration, including self-administration, and indirect administration, including the act of prescribing a drug. For example, as used herein, a physician who instructs a patient to self-administer a drug, or to have the drug administered by another and/or who provides a patient with a prescription for a drug is administering the drug to the patient.

Compositions and methods for administering compounds for transmucosal absorption in accordance with the invention are disclosed herein. The compositions and methods have a number of surprising pharmacokinetic benefits in comparison to the oral administration of a compound, which results predominantly in the absorption of compounds in the stomach, small intestine and colon.

Each embodiment described herein may be used individually or in combination with any other embodiment described herein.

Compounds

. The cyclobenzaprine can optionally be micronized. Alternatively, the cyclobenzaprine is not micronized. The cyclobenzaprine can optionally be present in one or more crystal isoforms.

As used herein, "cyclobenzaprine" includes cyclobenzaprine and pharmaceutically acceptable salts of cyclobenzaprine (e.g., cyclobenzaprine HCl). Cyclobenzaprine may be modified by the covalent addition of lysine or by binding to albumin.

Doses

A "therapeutically effective amount" of a drug or agent is an amount of a drug or an agent that, when administered to a subject will have the intended therapeutic effect, e.g. reducing the symptoms of fibromyalgia or post-traumatic stress disorder (PTSD) or treating the development of fibromyalgia or post-traumatic stress disorder (PTSD). The full therapeutic effect does not necessarily occur by administration of one dose, and may occur only after administration of a series of doses. Generally, cyclobenzaprine therapy can be carried out indefinitely to alleviate the symptoms of interest and frequency of dosage may be changed to be taken as needed. Thus, a therapeutically effective amount may be administered in one or more administrations. The precise effective amount needed for a subject will depend upon, for example, the subject's size, health and age, the nature and extent of the cognitive impairment, and the therapeutics or combination of therapeutics selected for administration, and the mode of administration. The skilled worker can readily determine the effective amount for a given situation by routine experimentation. Generally, a therapeutically effective amount of cyclobenzaprine administered to a subject is between 0.1 mg and 20.0 mg, between 0.1 mg and 5.0 mg, between 0.1 mg and 4.0 mg, or between 0.1 and 3.0 mg, or between 1 and 50 mg or between 1 and 75 mg. For example, a therapeutically effective amount can optionally be about 0.1 mg, 0.5 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, 5.0 mg, 5.1 mg, 5.2 mg, 5.3 mg, 5.4 mg, 5.5 mg, 6.0 mg, 6.5 mg, 7.0 mg, 7.5 mg, 8.0 mg, 8.5 mg, 9.0 mg, 9.5 mg, 10.0 mg, 11.0 mg, 12.0 mg, 13.0 mg, 14.0 mg, 15.0 mg, 16.0 mg, 17.0 mg, 18.0 mg, 19.0 mg, or 20.0 mg. In further examples, a therapeutically effective amount can optionally be about 21.0 mg, 22.0 mg, 23.0 mg, 24.0 mg, 25.0 mg, 26.0 mg, 27.0 mg, 28.0 mg, 28.0 mg, 29.0 mg, 30.0 mg, 31.0 mg, 32.0 mg, 33.0 mg, 34.0 mg, 35.0 mg, 36.0 mg, 37.0 mg, 38.0 mg, 39.0 mg, 40.0 mg, 41.0 mg, 42.0 mg, 43.0 mg, 44.0 mg, 45.0 mg, 46.0 mg, 47.0 mg, 48.0 mg, 49.0 mg, or 50.0 mg.

## Administration

Appropriate methods of administering a substance, a compound or an agent to a subject will depend, for example, on the age of the subject, whether the subject is active or inactive at the time of administering, whether the subject is experiencing symptoms of a disease or condition at the time of administering, the extent of the symptoms, and the chemical and biological properties of the compound or agent (e.g. solubility, digestibility, bioavailability, stability and toxicity). A compound can optionally be administered for oral transmucosal absorption. Absorption properties of compounds disclosed herein through oral transmucosal delivery cannot be predicted without experimentation. The suitability of compounds disclosed herein for oral transmucosal absorption is a surprising feature. A composition can optionally be suitable for oral transmucosal absorption. A composition can optionally be formulated for oral transmucosal absorption.

Methods of administering compositions for transmucosal absorption are well known in the art. For example, a composition may be administered for buccal absorption through buccal tablets, lozenges, buccal powders, and buccal spray solutions. A composition may be administered for sublingual absorption through sublingual tablets, sublingual films, liquids, sublingual powders, and sublingual spray solutions.

Doses and dosing regimens can be determined by one of skill in the art according to the needs of a subject to be treated. The skilled worker may take into consideration factors such as the age or weight of the subject, the severity of the disease or condition being treated, and the response of the subject to treatment. A composition disclosed herein can be administered, for example, as needed or on a daily basis. A composition disclosed herein can be administered immediately prior to sleep or several hours before sleep. Administration prior to sleep may be beneficial by providing the therapeutic effect before the onset of the symptoms of the disease or condition being treated. Dosing may take place over varying time periods. For example, a dosing regimen may last for 1 week, 2 weeks, 3 weeks, 4 weeks, 5 weeks, 6 weeks, 7 weeks, 8 weeks, 9 weeks, 10 weeks, 11 weeks, 12 weeks, or longer. A dosing regimen will optionally last 1 month, 2 months, 3 months, 4 months, 5 months, 6 months, 7 months, 8 months, 9 months, 10 months, 11 months, 12 months, or longer.

## Therapeutic uses

The compounds described herein may be employed for treating or preventing the development of fibromyalgia syndrome, also known as fibrositis (see, e.g., Moldofsky et al,. J Rheumatol 38(12):2653-2663 (2011) and Thomas, J Rheumatol 38(12):2499-2500 (2011)). Fibromyalgia is a chronic, non-inflammatory rheumatic disorder. The American College of Rheumatology (ACR) published a classification criteria for fibromyalgia in 1990 (Wolfe, F., et al., Arthritis and Rheumatism 33:160-172 (1990)). Subsequently, a modification to the ACR criteria been published (Wolfe et al., J Rheumatol 38(6):1113-22 (2011)). Diagnostic criteria have also been published by an international network of working groups called, "Outcome Measures in Rheumatology" clinical trials or OMERACT (Mease P, et al. J Rheumatol. 2009;36(10):2318-29). Fibromyalgia is traditionally characterized by stiffness or diffuse pain, aches, muscle soreness, sleep disturbances or fatigue. The pain is generally widespread and generally localized at specific "tender points," which may bring on widespread pain and muscle spasm when touched. Other symptoms include mental and emotional disturbances such as poor concentration and irritability, neuropsychiatric symptoms such as depression and anxiety, joint swelling, headache, numbness. Fibromyalgia is associated with nonrefreshing sleep, tiredness, sleepiness, reflux, mental fog and cognitive impairments including difficulty multi-tasking. Fibromyalgia also is often comorbid with sleep disorders, fatigue, non-restorative sleep, anxiety, and depression. Compositions and methods disclosed herein can be used to treat any one of the above-identified conditions, and any combination thereof.

Some practitioners further classify fibromyalgia into two categoriesprimary or secondary-concomitant fibromyalgia. Generally, primary fibromyalgia syndrome can be considered fibromyalgia occurring in the absence of another significant condition whereas secondary-concomitant fibromyalgia can be considered fibromyalgia occurring in the presence of another significant medical disorder, which may have been caused by or is merely associated with the patient's fibromyalgia. Secondary or concomitant fibromyalgia can include fibromyalgia in patients with classical or definite rheumatoid arthritis, osteoarthritis of the knee or hand, low back pain syndromes, cervical pain syndromes, cancer pain syndromes, temporomandibular joint disorders, migraine headaches, menopause, post-traumatic stress disorder and interstitial cystitis or painful bladder syndrome (or combinations thereof).

The compounds described herein also may be employed for treating or preventing the development (either the initiation, consolidation or perpetuation) of a PTSD symptom following a traumatic event. A traumatic event is defined as a direct personal experience that involves actual or threatened death or serious injury, or other threat to one's physical integrity, or witnessing an event that involves death, injury, or a threat to the physical integrity of another person; or learning about unexpected or violent death, serious harm, or threat of death or injury experienced by a family member or other close associate. Traumatic events that are experienced directly include, but are not limited to, military combat, violent personal assault (sexual assault, physical attack, robbery, mugging), being kidnapped, being taken hostage, terrorist attack, torture, incarceration as a prisoner of war or in a concentration camp, natural or manmade disasters, severe automobile accidents, or being diagnosed with a life-threatening illness. For children, sexually traumatic events may include developmentally inappropriate sexual experiences without threatened or actual violence or injury. Witnessed events include, but are not limited to, observing the serious injury or unnatural death of another person due to violent assault, accident, war, or disaster or unexpectedly witnessing a dead body or body parts. Events experienced by others that are learned about may include, but are not limited to, violent personal assault, serious accident, or serious injury experienced by a family member or a close friend, learning about the sudden, unexpected death of a family member or a close friend, or learning that one's child has a life-threatening disease. The disorder may be especially severe or long lasting when the stressor is of human design (e.g., torture or rape). The initiation of a PTSD symptom typically occurs immediately following the traumatic event, during which the symptoms of PTSD appear and become increasingly severe. One theory of how PTSD develops is that there is a type of "learning" or reinforcement process during which the memories of the trauma are engrained in the mind. As these memories become more fixed (a process called consolidation), symptoms such as flashbacks and nightmares grow in severity and frequency. Interventions during this critical time may prevent some patients from developing full-blown PTSD. The consolidation of a PTSD symptom typically occurs during the weeks and months following a traumatic event. A person's memories of that event become consolidated into highly vivid and concrete memories that are re-experienced with increasing frequency either as flashbacks or nightmares. During this time, hyperarousal symptoms and avoidant behavior can become increasingly severe and disabling. The perpetuation of a PTSD symptom occurs once traumatic memories are consolidated, and the re-experienced symptoms (flashbacks and nightmares) and hyperarousal symptoms become persistent and remain at a level that is functionally disabling to the patient.

Compositions and methods disclosed herein may be used to treat different phases of PTSD development at various time intervals after a traumatic event. For example, treating the initiation phase of PTSD may require the administration of a composition as disclosed herein soon after the traumatic event, for example within the first week, within the second week, within the third week, or within the fourth week or later. By contrast, when treating the consolidation phase of PTSD, the skilled worker may be able to administer a composition as disclosed herein later after the traumatic event and later during the development of the symptoms, for example, within the first month, within the second month, or within the third month or later. The perpetuation phase of PTSD may be treated with a composition as disclosed herein administered 3 months or longer after the traumatic event, for example within the third month, within the fourth month, within the fifth month, or later. As a result of treatment at the initiation, consolidation, or perpetuation phase, PTSD symptoms will be ameliorated or be eliminated.

Compositions and methods disclosed herein also can be used to treat traumatic brain injury (TBI). TBI is associated with sleep disorders, sleep disturbances, fatigue, non-restorative sleep, anxiety, and depression. Compositions and methods disclosed herein also can be used to treat any of the above conditions, in combination with or independently of treating TBI.

Compositions and methods disclosed herein also can be used to chronic traumatic encephalopathy (CTE). CTE is associated with sleep disorders, sleep disturbances, fatigue, non-restorative sleep, anxiety, and depression. Compositions and methods disclosed herein also can be used to treat any of the above conditions, in combination with or independently of treating CTE.

Compositions and methods disclosed herein may be used to treat sleep disorders or sleep disturbances. A "sleep disorder" may be any one of four major categories of sleep dysfunction (DSM-IV, pp. 551-607; *see also* The International Classification of Sleep Disorders: (ICSD) Diagnostic and Coding Manual, 1990, American Sleep Disorders Association). One category, primary sleep disorders, comprises sleep disorders that do not result from another mental disorder, a substance, or a general medical condition. They include without limitation primary insomnia, primary hypersomnia, narcolepsy, circadian rhythm sleep disorder, nightmare disorder, sleep terror disorder, sleepwalking disorder, REM sleep behavior disorder, sleep paralysis, day/night reversal and other related disorders; substance-induced sleep disorders; and sleep disorders due to a general medical condition. Primary insomnia non-restorative sleep is described by the DSM-IV-TR as a type of primary insomnia wherein the predominant problem is waking up feeling unrefreshed or nonrefreshed. A second category comprises those sleep disorders attributable to substances, including medications and drugs of abuse. A third category comprises sleep disturbances arising from the effects of a general medical condition on the sleep/wake system. A fourth category of sleep disorders comprises those resulting from an identifiable mental disorder such as a mood or anxiety disorder. A fifth category of sleep disorders comprises those described as non-restorative sleep. One definition of non-restorative sleep is in the DSM-IV-TR as a type of primary insomnia (A1.3) wherein the predominant problem is waking up feeling unrefreshed or nonrefreshed. Symptoms of each category of sleep disorder are known in the art. A "sleep disturbance" may be an impairment in refreshing sleep. Such a clinical diagnosis may be made based on a patient's self described feeling of fatigue upon waking or the patient's report of poor quality sleep. Such impediments to good quality sleep may be described as shallow sleep or frequent awakenings which may be associated with an increase in the Cyclic Alternating Pattern (CAP) A2 or A3 rate or cycle duration or an increase in the normalized CAP A2 + A3 which is determined by CAP (A2+A3)/CAP (A1+A2+A3) in non-REM sleep (see, e.g., Moldofsky et al,. J Rheumatol 38(12):2653-2663 (2011) and Thomas, J Rheumatol 38(12):2499-2500 (2011)), alpha rhythm contamination in non-REM sleep, or absence of delta waves during deeper physically restorative sleep. Such "sleep disturbances" may or may not rise to the level of a "sleep disorder" as defined in the DSM-IV, although they may share one or more symptom in common. Symptoms of sleep disturbances are known in the art. Among the known symptoms are groggy or spacey feelings, tiredness, feelings of being run down, and having difficulty concentrating during waking hours. Among the sleep-related conditions that may be treated with the methods and compositions disclosed herein are dyssomnias (e.g., intrinsic sleep disorders such as sleep state misperception, psychophysiological insomnia, idiopathic insomnia, obstructive sleep apnea syndrome, central sleep apnea syndrome, central alveolar hypoventilation syndrome, restless leg syndrome, and periodic limb movement disorder; extrinsic sleep disorders such as environmental sleep disorder, adjustment sleep disorder, limit-setting sleep disorder, stimulant-dependent sleep disorder, alcohol-dependent sleep disorder, toxin-induced sleep disorder, sleep onset association disorder, hypnotic dependent sleep disorder, inadequate sleep hygiene, altitude insomnia, insufficient sleep syndrome, nocturnal eating syndrome, and nocturnal drinking syndrome; and circadian rhythm sleep disorders such as jet lag syndrome, delayed sleep phase syndrome, advanced sleep phase syndrome, shift work sleep disorder, non-24 hour sleep-wake disorder, and irregular sleep-wake patterns), parasomnias (e.g., arousal disorders such as sleepwalking, confusional arousals, and sleep terrors and sleep-wake transition disorders such as rhythmic movement disorder, sleep talking and sleep starts, and nocturnal leg cramps), and sleep disorders associated with medical or psychiatric conditions or disorders.

Basifying agents

Compositions of the invention include a basifying agent in addition to a compound useful in the disclosed compositions. As used herein, a "basifying agent" refers to an agent (e.g., a substance that increases the local pH of the liquid near a mucosal surface including potassium dihydrogen phosphate (monopotassium phosphate, monobasic potassium phosphate, $KH_2PO_4$), dipotassium hydrogen phosphate (dipotassium phosphate, dibasic potassium phosphate, $K_2HPO_4$), tripotassium phosphate ($K_3PO_4$), sodium dihydrogen phosphate (monosodium phosphate, monobasic sodium phosphate, $NaH_2PO_4$), disodium hydrogen phosphate (disodium phosphate, dibasic sodium phosphate, $Na_2HPO_4$), trisodium phosphate ($Na_3PO_4$), bicarbonate or carbonate salts, dipotassium citrate, tripotassium citrate, disodium citrate, trisodium citrate, borate, hydroxide, silicate, nitrate, dissolved ammonia, the conjugate bases of some organic acids (including bicarbonate), and sulfide) that raises the pH of a solution containing a compound (e.g., cyclobenzaprine HCl) useful in the compositions and methods disclosed herein. The solution of interest is the layer of aqueous material overlying a mucous membrane. Therefore the basifying agent is sometimes an ingredient (and excipient) in a tablet, and the basifying agent exerts its effects during the time the tablet is being dispersed in the mucous material, while parts of the formulation are dissolving in the mucous material and for a period of time after the tablet is dissolved in the mucous material. Surprisingly, the addition of a basifying agent to a composition of the invention improves the pharmacokinetic properties of the composition. This is exemplified by cyclobenzaprine HCl as one particular compound useful in the methods and compositions of the invention. A basifying agent with particular effects on cyclobenzaprine HCl is dipotassium hydrogen phosphate ($K_2HPO_4$). Another basifying agent with particular effects on cyclobenzaprine HCl is potassium dihydrogen phosphate ($KH_2PO_4$). Another basifying agent with particular effects on cyclobenzaprine HCl is disodium hydrogen phosphate ($Na_2HPO_4$). Another basifying agent with particular effects on cyclobenzaprine HCl is tripotassium citrate. Another basifying agent with particular effects on cyclobenzaprine HCl is trisodium citrate.

Cyclobenzaprine HCl has an acid dissociation constant (or pKa) for the amine group of approximately 8.5 at 25° C, indicating that at pH 8.5, the compound is 50% ionized or protonated (and 50% un-ionized or free base) (M. L.

Cotton, G. R. B. Down, Anal. Profiles Drug Subs. 17, 41-72 (1988)). The pH of an aqueous solution of cyclobenzaprine HCl from 10 gm/100 mL (0.32 molar) to 30 gm/100 mL (0.96 molar) is between approximately 3.1 and 3.3, thereby providing a condition wherein nearly all the cyclobenzaprine is ionized and soluble. The skilled worker, with this knowledge in hand, would therefore look to maintain cyclobenzaprine at a low pH, maximizing its solubility. However, ionized cyclobenzaprine may not have optimal absorption by and across mucosal surfaces because of its charge. This problem is solved by combining the cyclobenzaprine with a basifying agent. Indeed, we have discovered that combining cyclobenzaprine HCl with a basifying agent such as dipotassium hydrogen phosphate ($K_2HPO_4$) or potassium dihydrogen phosphate ($KH_2PO_4$) improves the pharmacokinetic properties of a composition comprising cyclobenzaprine for transmucosal absorption. In experiments with oral and intravenous solutions containing cyclobenzaprine and a basifying agent, the pH was adjusted to about pH 7.1 to pH 7.4. In experiments with tablet formulations containing cyclobenzaprine and a basifying agent, the addition of the basifying agent results in a higher pH when the tablet is dissolved in water. Combining cyclobenzaprine with a basifying agent also enhances the uptake of cyclobenzaprine by transmucosal absorption. The effects of a basifying agent like $K_2HPO_4$ or $KH_2PO_4$ on the transmucosal pharmacokinetic properties of a composition comprising cyclobenzaprine are remarkable because cyclobenzaprine HCl in a solution at 2.5 mg/ml at 7.4 containing $K_2HPO_4$ or $KH_2PO_4$ appears to be close to its saturation point where cyclobenzaprine falls out of solution, or becomes insoluble, presumably because the concentration of cyclobenzaprine free base increases relative to ionized cyclobenzaprine. Without wishing to be bound by theory, it is possible that the basifying agent increases the pH of the microenvironment local to the mucosal membrane and brings more of the cyclobenzaprine into an un-ionized or free-base state at the mucosal surface, which helps drive cyclobenzaprine across mucosa and into the bloodstream, thereby offsetting any decrease in the solubility of cyclobenzaprine resulting from the basifying agent action in the solution near the mucous membrane. Without wishing to be bound by theory, the basifying agent may create a transition state involving hydration of the free base, such that the free base is formed *in situ* near the mucosal surface and crosses the mucosal membrane.

A basifying agent useful in the compositions and methods disclosed herein may be any agent that increases the pH of a solution containing a compound useful in the disclosed methods and compositions. Exemplary basifying agents include potassium dihydrogen phosphate (monopotassium phosphate, monobasic potassium phosphate, $KH_2PO_4$), dipotassium hydrogen phosphate (dipotassium phosphate, dibasic potassium phosphate, $K_2HPO_4$), tripotassium phosphate ($K_3PO_4$), sodium dihydrogen phosphate (monosodium phosphate, monobasic sodium phosphate, $NaH_2PO_4$), disodium hydrogen phosphate (disodium phosphate, dibasic sodium phosphate, $Na_2HPO_4$), trisodium phosphate ($Na_3PO_4$), sodium carbonate, sodium bicarbonate, calcium carbonate, calcium bicarbonate, TRIS buffer, potassium carbonate, potassium bicarbonate, potassium acetate, sodium acetate, potassium citrate and sodium citrate. Optionally, a composition disclosed herein has a molar ratio of a compound (e.g., cyclobenzaprine) to a basifying agent of 1:1, 1:2, 1:3, 1:4, 1:5 1:6, 1.7, 1.8, 1.9 or 2.0. For example, the ratio of cyclobenzaprine HCl (2.4 mg, MW 275.387) to $K_2HPO_4$ (1.05 mg, MW 174.2) can optionally be 0.69.

Cyclobenzaprine and amitriptyline metabolism

Cyclobenzaprine rapidly distributes out of the vasculature after intravenous (IV) bolus administration in humans (Hucker et al., J Clin Pharmacol 17:719-727 (1977), Hucker et al., Drug Metab Dispos 6:659-672 (1978), Till at al., Annu Rev Pharmacol Toxicol 40:581-616 (2000), and Winchell et al., J Clin Pharmacol 42:61-69 (2002)). The amount of cyclobenzaprine in plasma within 3 to 30 minutes was less than 5% of the theoretical initial concentration ($C_{init}$) of each infused dose, and a 3 minute time point resulted in a relatively higher $C_{init}$. Together, this information shows that cyclobenzaprine is rapidly partitioned out of plasma. Since greater than 95% of injected cyclobenzaprine is cleared from plasma before the first time point of any of the four studies listed above, they are not informative about the half-life of the first phase of IV cyclobenzaprine distributing out of plasma, beyond establishing that an upper limit for the half-life of the first phase is clearly less than 3-5 minutes.

Neither cyclobenzaprine nor amitriptyline is an effective long-term treatment for fibromyalgia in any formulation that has been tested, for example currently available formulations were not effective over six months of treatment (Carette, S. Arthritis Rheum. 1994 Jan;37(1):32-40). Cyclobenzaprine is not an effective treatment for fibromyalgia in a twelve week study (Bennett et al., Arthritis Rheum. 31: 1535-1542 (1988)). In general, cyclobenzaprine is not recommended for long-term use. We hypothesized that the side effects of fatigue, somnolence and grogginess have overwhelmed the treatment effects from cyclobenzaprine or amitriptyline, but it was not known how to shorten the plasma half-life of either cyclobenzaprine or amitriptyline. In addition, we have shown for the first time that the ineffectiveness of cyclobenzaprine may be due to the metabolism of cyclobenzaprine by the liver. Whereas amitriptyline was known to be metabolized to nortriptyline, and the delayed plasma half life of nortriptyline has been reported (Bhatt, Biomed Chromatogr 24(11):1247-54 (2010)), the manner in which nortriptyline accumulation may decrease efficacy of bedtime amitriptyline as a chronic treatment was not understood.

Cyclobenzaprine is extensively metabolized and in humans, excreted predominantly by the kidney as the N+-glucuronide conjugate. Glucuronidation of an aliphatic tertiary amine group in a molecule results in a quaternary ammonium-linked glucuronide metabolite (i.e. N+-glucuronide) (Hucker et al., Drug Metab Dispos 6:659-672 (1978), Hawes, Drug Metab Dispos 26:830-837 (1998)). Amitriptyline has been studied in more detail and more recently than cyclobenzaprine, and the enzyme UDP-glucuronysl-transferase (UGT) UGT2B10 was found to be the high-affinity component (Zhou et al., Drug Metab Dispos 38:863-870 (2010)), while UGT1A4 is the low-affinity enzyme for glucuronidation in human liver microsomes (HLM) (Breyer-Pfaff et al., Drug Metab Dispos 25:340-345 (1997), Nakajima et al., Drug Metab Dispos 30:636-642 (2002)). It is likely that cyclobenzaprine is similarly metabolized to cyclobenzaprine-N+-glucuronide by UGT2B10 and, to a lesser extent, by UGT1A4.

Cyclobenzaprine also is N-demethylated to 3-(5H-dibenzo[a,d]cyclohepten-5-ylidene)-N-methyl-1-propanamine (norcyclobenzaprine), predominantly by hepatic enzymes P450 3A4 and 1A2 (Wong et al., J Anal Toxicol 19:218-224 (1995)). Amitriptyline is similarly subject to P450 mediated N-demethylation to nortriptyline. Nortriptyline represents a significant percentage of the plasma amitriptyline plus nortriptyline content in subjects who ingest amitriptyline either as a single dose or chronically, whereas norcyclobenzaprine had not previously been measured in human plasma except in cases of overdose (Hucker et al., Drug Metab Dispos 6:659-672 (1978), Wong et al., J Anal Toxicol 19:218-224 (1995)).

Previously, a number of studies had been performed and published showing cyclobenzaprine, amitriptyline and nortriptyline binding to various receptors in the central nervous system and peripheral tissues. We undertook a systematic analysis of the binding affinities of these molecules (cyclobenzaprine, amitriptyline and nortriptyline) and of norcyclobenzaprine (which had never been studied before to our knowledge) and determined the $K_i$ of these molecules on a variety of receptors. Cyclobenzaprine, norcyclobenzaprine, amitriptyline and nortriptyline binding to receptors was studied by methods described in: adrenergic alpha-2A (Langin et al., Eur J Pharmacol 167: 95-104 (1989), -2B, and -2C receptors (Devedjian et al., Eur J Pharmacol 252: 43-49 (1994)), the histamine H1 receptor (Smit et al., Brit. J. Pharmacol 117: 1071-1080 (1996)), the muscarinic M1 and M2 receptors (Dorje et al., J Pharmacol Exp Ther 256: 727-733 (1991)), and the 5-HT1A (Mulheron et al., J Biol Chem 269: 12954-12962 (1994)), 5-HT2A (Bonhaus et al., Brit J Pharmacol 115: 622-628 (1995)) and 5-HT2C receptors (Stam et al., Eur J Pharmacol 269: 339-348 (1994)). The binding constants $K_i$ are listed in Table 1, below. In vitro, cyclobenzaprine and norcyclobenzaprine exhibited high affinity binding ($K_i$) to receptors: 5HT2a (5.2 and 13 nM) and 5HT2c (5.2 and 43 nM, respectively), alpha-adrenergic alpha-lA (5.6 and 34 nM), alpha-2B ($K_i$ = 21 and 150 nM) and alpha-2C ($K_i$ = 21 and 48 nM,); H1 (1.3 nM and 5.6 nM); and M1 (7.9 nM and 30 nM). Like cyclobenzaprine, norcyclobenzaprine is a functional antagonist at 5HT2a ($IC_{50}$ = 92 nM) by $Ca^+$ mobilization. Cyclobenzaprine is also an antagonist on 5HT2b ($IC_{50}$ = 100 nM). Cyclobenzaprine and norcyclobenzaprine are functional antagonists on 5HT2c ($IC_{50}$ = 0.44 $\mu$M and 1.22 $\mu$M) and on alpha-2A ($IC_{50}$ = 4.3 $\mu$M and 6.4 $\mu$M). By contrast, both cyclobenzaprine and norcyclobenzaprine are functional agonists on 5HT1a ($EC_{50}$ = 5.3 $\mu$M and 3.2 $\mu$M). Cyclobenzaprine's antagonist activity on 5HT2b is consistent with the lack of any association with heart valve pathology. Antagonists of 5HT2a and H-1 are known to have effects on sleep and sleep maintenance. Adrenergic antagonists may have effects on autonomic dysfunction.

Without wishing to be bound by theory, we hypothesize that the primary activity of cyclobenzaprine that relates to effects on fibromyalgia, PTSD, TBI and sleep disturbances is the binding to 5-HT2a. Plasma cyclobenzaprine and norcyclobenzaprine were measured over 168 hr in ten healthy, fasting subjects who received 5 mg oral (PO) immediate release cyclobenzaprine HCl. The oral bioavailability of cyclobenzaprine was similar to published results ($C_{max}$ = 4.12 ng $mL^{-1}$, tmax = 3.5 h, $AUC_{0-\infty}$ = 103.1 ng hr $mL^{-1}$), but plasma norcyclobenzaprine was unexpectedly high and persistent (Cmax = 1.27ng $mL^{-1}$, $t_{max}$ = 24.0 h, $AUC_{0-\infty}$ = 169.5 ng hr $mL^{-1}$). We have calculated for the first time the half-life of norcyclobenzaprine in human plasma after oral ingestion of a 5 mg immediate release tablet of cyclobenzaprine HCl (72.8 hours), which is significantly longer than the half-life of cyclobenzaprine (31.0 hours) in the same study.

Without wishing to be bound by theory, we hypothesize that the accumulation of norcyclobenzaprine interferes with the efficacy of cyclobenzaprine treatment over extended periods of time for bedtime or once daily use in which the goal of therapy is to have cyclobenzaprine levels change dynamically over the course of the day. The accumulation of biologically active norcyclobenzaprine may affect responses to cyclobenzaprine therapy in a chronic bedtime dosing regimen. Without wishing to be bound by theory, we hypothesize the chronic occupancy of 5-HT2A and other receptors leads to adaptation by a variety of mechanisms which control plasticity of neuronal signaling and responsiveness. We found that norcyclobenzaprine has 13.2 nM Ki for 5-HT2a and cyclobenzaprine has 5.1 nM $K_i$ for 5HT2A, which indicates that norcyclobenzaprine competes with the binding of cyclobenzaprine to 5-HT2A. Similarly, nortriptyline has 16 nM $K_i$ for 5-HT2A, and amitriptyline has 2.5 nM $K_i$ for 5-HT2A, which indicates that nortriptyline competes with the binding of amitriptyline to 5-HT2A. Although cyclobenzaprine and amitriptyline are more avid 5-HT2A binders than norcyclobenzaprine and nortriptyline, the concentrations of norcyclobenzaprine and nortriptyline become significant because of their longer half lives and accumulation, particularly with repeated dosing on a daily dosing schedule. By administering cyclobenzaprine or amitriptyline for transmucosal absorption, the cyclobenzaprine or amitriptyline avoids first pass metabolism in the liver, thereby reducing or eliminating the formation of norcyclobenzaprine or nortriptyline, respectively, by p450 metabolism in the gut and liver. Thus, cyclobenzaprine and amitriptyline can be effectively administered over longer treatment regimens than currently possible without accumulation of the demethylated metabolite. Without wishing to be bound by theory, we hypothesize the long half lives of norcyclobenzaprine and notriptyline are due to the instability of norcyclobenzaprine-N+-glucuronide and notriptyline (which we have observed in attempting to synthesize norcyclobenzaprine-N+-glucuronide) and possibly the inability of human enzymes, including UDP-glucuronysl-transferase (UGT) UGT2B10 and UGT1A4 to form the-N+-glucuronide metabolites which can be exreted in the kidney. Because norcyclobenzaprine had not been measured in plasma of animals after therapeutic dosing and norcyclobenzaprine's long half-life was not previously known, and because norcyclobenzaprine was not known to bind to 5-HT2A or to other receptors in the CNS and peripheral tissues, the benefit of transmucosal dosing on decreasing norcyclobenzaprine and increasing the therapeutic potential of cyclobenzaprine were surprising and novel. By contrast, the long half life of nortriptyline was known after either ingestion of amitriptyline or nortriptyline and the long half life of nortriptyline in plasma and at the site of action is believed to be an advantage in the treatment of depression and major depressive disorder.

Table 1: Binding affinities of cyclobenzaprine, norcyclobenzaprine, amitriptyline and nortriptyline for various receptors

| | | | | Ki (nM) | | | | | |
|---|---|---|---|---|---|---|---|---|---|
| | Alpha2A | Alpha2B | Alpha2C | H1 | M1 | M2 | 5-HT1A | 5-HT2A | 5-HT2C |
| Cyclo | 360 | 21 | 25 | 1.3 | 7.9 | 250 | 1100 | 5.2 | 43 |
| Norcyclo | 1800 | 150 | 48 | 5.6 | 30 | 76 | 76 | 13 | 43 |
| Amitrip | 250 | 3.6 | 14 | 0.6 | 4.3 | 61 | 220 | 2.5 | 28 |
| Nortrip | 1000 | 23 | 15 | 2.8 | 24 | 140 | 100 | 16 | 16 |
| | Alpha1A | Alpha1B | D1 | D2S | D3 | D4.4 | D5 5-HT5A | 5-HT6 | 5-HT7 |
| Cyclo | 5.6 | 9.1 | 12 120 | | 34 | 180 60 | 730 | 480 | 67 |
| Norcyclo | 34 | 11 | 57 410 | | 98 | 250 280 | 1600 | 1400 | 140 |

Pharmacokinetic properties

Transmucosal absorption of a compound useful in the compositions and methods disclosed herein has a number of beneficial effects on the pharmacokinetic properties of the compound in addition to the benefit of avoiding the production of norcyclobenzaprine. Transmucosal delivery allows a compound disclosed herein to be absorbed more rapidly than if administered orally, resulting in a shorter time to therapeutic concentrations of cyclobenzaprine in the plasma. Optionally, the compositions disclosed herein can afford therapeutic concentrations of cyclobenzaprine in the plasma at less than 3.3 hours, less than 3 hours, less than 2.5 hours, less than 2 hours, less than 1 hour, less than 45 minutes, less than 30 minutes, or less than 20 minutes. Compositions disclosed herein can optionally afford increased concentrations of cyclobenzaprine in the plasma, relative to oral doses, at time less than or equal to 3.3 hours, less than or equal to 3 hours, less than or equal to 2.5 hours, less than or equal to 2 hours, less than or equal to 1 hour, less than or equal to 45 minutes, less than or equal to 30 minutes, or less than or equal to 20 minutes. Optionally, the compositions disclosed herein can afford increased AUCs of cyclobenzaprine in the plasma, relative to oral doses, at times 0 to 3.3 hours, 0 to 3 hours, 0 to 2.5 hours, 0 to 2 hours, 0 to 1 hour, 0 to 45 minutes, 0 to 30 minutes, or 0 to 20 minutes. Optionally, the compositions disclosed herein can afford increased dose-normalized concentrations (dnC*) of cyclobenzaprine in the plasma, relative to oral doses, at time less than or equal to 3.3 hours, less than or equal to 3 hours, less than or equal to 2.5 hours, less than or equal to 2 hours, less than or equal to 1 hour, less than or equal to 45 minutes, less than or equal to 30 minutes, or less than or equal to 20 minutes. Optionally, the compositions disclosed herein can afford increased dose normalized AUCs (dnAUC*) of cyclobenzaprine in the plasma, relative to oral doses, at times 0 to 3.3 hours, 0 to 3 hours, 0 to 2.5 hours, 0 to 2 hours, 0 to 1 hour, 0 to 45 minutes, 0 to 30 minutes, or 0 to 20 minutes. Transmucosal delivery allows a compound disclosed herein to be absorbed more rapidly than if administered orally, resulting in a shorter time to maximum concentration, or $t_{max}$. For example, the compositions disclosed herein can afford a $t_{max}$ of cyclobenzaprine of less than 5 hours, less than 4 hours, less than 3.5 hours, less than 3 hours, less than 2.5 hours, less than 2 hours, less than 1.5 hours, less than 1 hour, less than 45 minutes, less than 30 minutes, less than 15 minutes, less than 10 minutes, or less than 5 minutes. Compositions disclosed herein can afford a $t_{max}$ of cyclobenzaprine of about 5 hours, about 4 hours, about 3 hours, about 2.5 hours, about 2 hours, about 1.5 hours, about 1 hour, about 45 minutes, about 30 minutes, about 15 minutes, about 10 minutes, or about 5 minutes.

Transmucosal absorption also produces higher plasma concentrations of a compound as compared to oral administration. A plasma concentration may be an individual plasma concentration or a mean plasma concentration when observing multiple individuals. The higher plasma concentrations produced by transmucosal absorption may be determined by measuring the plasma concentration of the compound being administered or by calculating the ratio of the plasma concentration and the dose administered, which is the dose-normalized plasma concentration (C) or dnC*, measured in $mL^{-1}$. The dnC* is calculated by determining the ratio of plasma level to dose administered. For example, if 2.4 mg of cyclobenzaprine is administered and the plasma level is 2.4 ng/mL at 3 hours, the dnC* at 3 hours is ((2.4 ng/mL)/(2.4 mg)) = 1.0 $\times$ $10^{-6}$ $mL^{-1}$. The dnC* can be measured either at fixed time points or at a variable time point, e.g., the time point corresponding to $C_{max}$. The dose normalized concentration of cyclobenzaprine dnC* of cyclobenzaprine in plasma after ingestion of 5 mg immediate release cyclobenzaprine was: at 20 min was 0.00; at 30 min was 1.95 $\times$ $10^{-9}$ $mL^{-1}$; at 45 min was 19.31 $\times$ $10^{-9}$ $mL^{-1}$; at 1 hour was 50.00 $\times$ $10^{-9}$ $mL^{-1}$; at 2 hour was 378.65 $\times$ $10^{-9}$ $mL^{-1}$; at 2.5 hours (150 min) was 510.94 $\times$ $10^{-9}$ $mL^{-1}$; at 3 hours was 625.29 $\times$ $10^{-9}$ $mL^{-1}$ $\times$ $10^{-9}$ $mL^{-1}$; at 3.3 hours (200 min) was 698.49 $\times$ $10^{-9}$ $mL^{-1}$; at 3.67 hours (220 min) was 818.31 $\times$ $10^{-9}$ $mL^{-1}$; at 4 hours was 848.33 $\times$ $10^{-9}$ $mL^{-1}$; at 4.33 hours (260 min) was 968.09 $\times$ $10^{-9}$ $mL^{-1}$; at 4.67 hours (280 min) was 933.95 $\times$ $10^{-9}$ $mL^{-1}$; at 5 hours was 932.86 $\times$ $10^{-9}$ $mL^{-1}$; at 5.5 hours (330 min) was 920.94 $\times$ $10^{-9}$ $mL^{-1}$; at 6 hours was 953.40 $\times$ $10^{-9}$ $mL^{-1}$; at 8 hours was 801.23 $\times$ $10^{-9}$ $mL^{-1}$; at 12 hours was 516.73 $\times$ $10^{-9}$ $mL^{-1}$; at 16 hours was 347.39 $\times$ $10^{-9}$ $mL^{-1}$; at 24 hours was 320.44 $\times$ $10^{-9}$ $mL^{-1}$; at 36 hours was 233.66 $\times$ $10^{-9}$ $mL^{-1}$; at; at 48 hours was 199.41 $\times$ $10^{-9}$ $mL^{-1}$; and at 72 hours was 136.80. The dose normalized concentration of cyclobenzaprine dnC* of cyclobenzaprine in plasma after ingestion of 2.4 mg sublingual cyclobenzaprine with phosphate was: at 20 min was 157.60 $\times$ $10^{-9}$ $mL^{-1}$; at 30 min was 301.60 $\times$ $10^{-9}$ $mL^{-1}$; at 45 min was 432.58x $10^{-9}$ $mL^{-1}$; at 1 hour was 598.85x $10^{-9}$ $mL^{-1}$; at 2 hour was 683.58x $10^{-9}$ $mL^{-1}$; at 2.5 hours (150 min) was 727.67x $10^{-9}$ $mL^{-1}$; at 3 hours was 840.33x $10^{-9}$ $mL^{-1}$ $\times$ $10^{-9}$ $mL^{-1}$; at 3.3 hours (200 min) was 923.58 $\times$ $10^{-9}$ $mL^{-1}$; at 3.67 hours (220 min) was 952.71x $10^{-9}$ $mL^{-1}$; at 4 hours was 1012.35x $10^{-9}$ $mL^{-1}$; at 4.33 hours (260 min) was 1030.10$\times$ $10^{-9}$ $mL^{-1}$; at 4.67 hours (280 min) was 1038.58x $10^{-9}$ $mL^{-1}$; at 5 hours was 990.90x $10^{-9}$ $mL^{-1}$; at 5.5 hours (330 min) was 1046.42x $10^{-9}$ $mL^{-1}$; at 6 hours was 911.07x $10^{-9}$ $mL^{-1}$; at 8 hours was 696.33x $10^{-9}$ $mL^{-1}$; at 12 hours was 504.90x $10^{-9}$ $mL^{-1}$; at 16 hours was 354.04x $10^{-9}$ $mL^{-1}$; at 24 hours was 294.40x $10^{-9}$ $mL^{-1}$; at 36 hours was 184.19$\times$ $10^{-9}$ $mL^{-1}$; at; at 48 hours was 143.37x $10^{-9}$ $mL^{-1}$; and at 72 hours was 88.23. For example, dnC* can be measured 5 minutes after administration, 10 minutes after administration, 15 minutes after administration, 20 minutes after administration, 30 minutes after administration, 45 minutes after administration, 1 hour after administration, 2 hours after administration, 3 hours after administration, 4 hours after administration, 5 hours after administration, 6 hours after administration, 7 hours after administration, 8 hours after administration, 9 hours after administration, 10 hours after administration, 11 hours after administration, or 12 hours after administration. For example, a dnC* value may be about, or greater than about, 8.0 $\pm$ 25% $\times$ $10^{-7}$ $mL^{-1}$, 0.001 $\pm$ 25% $\times$ $10^{-6}$ $mL^{-1}$, 0.01 $\pm$ 25% $\times$ $10^{-6}$ $mL^{-1}$, 0.05 $\pm$ 25% $\times$ $10^{-6}$ $mL^{-1}$, 0.1 $\pm$ 25% $\times$ $10^{-6}$ $mL^{-1}$, 0.5 $\pm$ 25% $\times$ $10^{-6}$ $mL^{-1}$, 1.0 $\pm$ 25% $\times$ $10^{-6}$ $mL^{-1}$, 5.0 $\pm$ 25% $\times$ $10^{-6}$ $mL^{-1}$, 10.0 $\pm$ 25% $\times$ $10^{-6}$ $mL^{-1}$, 50.0 $\pm$ 25% $\times$ $10^{-6}$ $mL^{-1}$, or 100.0 $\pm$ 25% $\times$ $10^{-6}$ $mL^{-1}$, 125.0 $\pm$ 25% $\times$ $10^{-6}$ $mL^{-1}$, 150.0 $\pm$ 25% $\times$ $10^{-6}$ $mL^{-1}$, 175.0 $\pm$ 25% $\times$ $10^{-6}$ $mL^{-1}$, 200.0 $\pm$ 25% $\times$ $10^{-6}$ $mL^{-1}$, 300.0 $\pm$ 25% $\times$ $10^{-6}$ $mL^{-1}$, 400.0 $\pm$ 25% $\times$ $10^{-6}$ $mL^{-1}$, 500.0 $\pm$ 25% $\times$ $10^{-6}$ $mL^{-1}$, 600.0 $\pm$ 25% $\times$ $10^{-6}$ $mL^{-1}$o or 700.0 $\pm$ 25% $\times$ $10^{-6}$ $mL^{-1}$. For example, a dnC* value may be about, or greater than or equal to 50 $\pm$ 25% $\times$ $10^{-9}$ $mL^{-1}$ 10 minutes after administration, greater than or equal to 125 $\pm$ 25% $\times$ $10^{-9}$ $mL^{-1}$ 15 minutes after administration, greater than or equal to 150 $\pm$ 25% $\times$ $10^{-9}$ $mL^{-1}$ 20 minutes after administration, greater than or equal to 300 $\pm$ 25% $\times$ $10^{-9}$ $mL^{-1}$ 30 minutes after administration, greater than or equal to 450 $\pm$ 25% $\times$ $10^{-9}$ $mL^{-1}$ 45 minutes after administration, greater than or equal to 600 $\pm$ 25% $\times$ $10^{-9}$ $mL^{-1}$ 1 hour after administration, greater than or equal to 700 $\pm$ 25% $\times$ $10^{-9}$ $mL^{-1}$ 2 hours after administration, greater than or equal to 750 $\pm$ 25% $\times$ $10^{-9}$ $mL^{-1}$ 2.5 hours after administration, greater than or equal to 850 $\pm$ 25% $\times$ $10^{-9}$ $mL^{-1}$ 3 hours after administration, greater than or equal to 900 $\pm$ 25% $\times$ $10^{-9}$ $mL^{-1}$ 3.3 hours after administration, greater than or equal to 950 $\pm$ 25% $\times$ $10^{-9}$ $mL^{-1}$ 3.7 hours after administration, greater than or equal to 1000 $\pm$ 25% $\times$ $10^{-9}$ $mL^{-1}$ 4 hours after administration, greater than or equal to 1050 $\pm$ 25% $\times$ $10^{-9}$ $mL^{-1}$ 4.33 hours after administration, greater than or equal to 1050 $\pm$ 25% $\times$ $10^{-9}$ $mL^{-1}$ 4.67 hours after administration, less than or equal to 1000 $\pm$ 25% $\times$ $10^{-9}$ $mL^{-1}$ 5 hours after administration, less than or equal to 1000 $\pm$ 25% $\times$ $10^{-9}$ $mL^{-1}$ 5.5 hours after administration, less than or equal to 900 $\pm$ 25% $\times$ $10^{-9}$ $mL^{-1}$ 6 hours after administration, less than or equal to 700 $\pm$ 25% $\times$ $10^{-9}$ $mL^{-1}$ 8 hours after administration, less than or equal to 650 $\pm$ 25% $\times$ $10^{-9}$ $mL^{-1}$ 10 hours after administration, less than or equal to 500 $\pm$ 25% $\times$ $10^{-9}$ $mL^{-1}$ 12 hours after administration, less than or equal to 400 $\pm$ 25% $\times$ $10^{-9}$ $mL^{-1}$ 14 hours after administration, less than or equal to 350 $\pm$ 25% $\times$ $10^{-9}$ $mL^{-1}$ 16 hours after administration, less than or equal to 340 $\pm$ 25% $\times$ $10^{-9}$ $mL^{-1}$ 18 hours after administration, less than or equal to 320 $\pm$ 25% $\times$ $10^{-9}$ $mL^{-1}$ 20 hours after administration, less than or equal to 310 $\pm$ 25% $\times$ $10^{-9}$ $mL^{-1}$ 22 hours after administration, less than or equal to 300 $\pm$ 25% $\times$ $10^{-9}$ $mL^{-1}$ 24 hours after administration, less than or equal to 180 $\pm$ 25% $\times$ $10^{-9}$ $mL^{-1}$ 36 hours after administration, less than or equal to 140 $\pm$ 25% $\times$ $10^{-9}$ $mL^{-1}$ 48 hours after administration, or less than or equal to 90 $\pm$ 25% $\times$ $10^{-9}$ $mL^{-1}$ 72 hours after administration. Optionally, dnC* can be measured 10 hours after administration, 11 hours after administration, 12 hours after administration, 13 hours after administration, 14 hours after administration, 15 hours after administration, 16 hours after administration, 17 hours after administration, 18 hours after administration, 19 hours after administration, 20 hours after administration, 21 hours after administration, 22 hours after administration, 23 hours after administration, 24 hours after administration, or 36 hours after administration. For example, a dnC* value may be about, or less than about, 1.0 $\pm$ 25% $\times$ $10^{-9}$ $mL^{-1}$, 1.0 $\pm$ 25% $\times$ $10^{-8}$ $mL^{-1}$, 0.7 $\pm$ 25% $\times$ $10^{-7}$ $mL^{-1}$, 1.0 $\pm$ 25% $\times$ $10^{-7}$ $mL^{-1}$, 2.0 $\pm$ 25% $\times$ $10^{-7}$ $mL^{-1}$, 3.0 $\pm$ 25% $\times$ $10^{-7}$ $mL^{-1}$, 4.0 $\pm$ 25% $\times$ $10^{-7}$ $mL^{-1}$, 5.0 $\pm$ 25% $\times$ $10^{-7}$ $mL^{-1}$, 1.0 $\pm$ 25% $\times$ $10^{-6}$ $mL^{-1}$, or 5.0 $\pm$ 25% $\times$ $10^{-6}$ $mL^{-1}$ Optionally, the dnC* value can relate to single dosing. The dnC* value can also relate to a multi-dose regimen (e.g., repeated daily administration). Optionally, the plasma concentration used to calculate the dnC* may be adjusted to reflect a baseline plasma concentration (e.g., a baseline plasma level because of repeated daily administration). $C_{max}$ is defined as the peak plasma concentration of a compound disclosed herein after administration. If a dnC* value is calculated at the time point corresponding to $C_{max}$, the value may alternatively be referred to as a dose normalized $C_{max}$ or $dnC_{max}$*. Optionally, a $dnC_{max}$* is greater than or equal to 1.0 ± 25% × $10^{-6}$ $mL^{-1}$, greater than or equal to 1.5 ± 25% × $10^{-6}$ $mL^{-1}$, greater than or equal to 2.0 ± 25% × $10^{-6}$ $mL^{-1}$, greater than or equal to 2.5 ± 25% × $10^{-6}$ $mL^{-1}$, greater than or equal to 3.0 ± 25% × $10^{-6}$ $mL^{-1}$, greater than or equal to 3.5 ± 25% x $10^{-6}$ $mL^{-1}$, greater than or equal to 4.0 ± 25% × $10^{-6}$ $mL^{-1}$, greater than or equal to 4.5 ± 25% × $10^{-6}$ $mL^{-1}$, greater than or equal to 5.0 ± 25% × $10^{-6}$ $mL^{-1}$.

Transmucosal absorption also produces higher plasma concentrations of a compound as compared to oral administration. A plasma concentration may be an individual plasma concentration or a mean plasma concentration when observing multiple individuals. The higher plasma concentrations produced by transmucosal absorption may be determined by measuring the plasma concentration of the compound being administered or by calculating the ratio of the plasma concentration*body mass product and the dose administered, which is the dose- and body mass-normalized plasma concentration (C) or dbmnC*, measured in kg $mL^{-1}$. The dbmnC* is calculated by determining the ratio of plasma level times body mass product to dose administered. For example, if 2.4 mg of cyclobenzaprine is administered to a 70 kg animal and the plasma level is 4.8 ng/mL at 15 minutes, the dbmnC* at 15 minutes is ((4.8 ng/mL) × (70 kg)/(2.4 mg)) = $dbmnC_{(0.25h)}$* = 140.0 × $10^{-6}$ kg $mL^{-1}$. The dbmnC* can be measured either at fixed time points or at a variable time point, e.g., the time point corresponding to $C_{max}$. For example, dbmnC* can be measured 5 minutes after administration, 10 minutes after administration, 15 minutes after administration, 30 minutes after administration, 45 minutes after administration, 1 hour after administration, 2 hours after administration, 3 hours after administration, 4 hours after administration, 5 hours after administration, 6 hours after administration, 7 hours after administration, 8 hours after administration, 9 hours after administration, 10 hours after administration, 11 hours after administration, or 12 hours after administration. For example, a dbmnC* value may be about, or greater than about, 80.0 ± 25% × $10^{-7}$ kg $mL^{-1}$, 0.01 ± 25% x $10^{-6}$ kg $mL^{-1}$, 0.1 ± 25% × $10^{-6}$ kg $mL^{-1}$, 0.5 ± 25% x $10^{-6}$ kg $mL^{-1}$, 1.0 ± 25% × $10^{-6}$ kg $mL^{-1}$, 5.0 ± 25% × $10^{-6}$ kg $mL^{-1}$, 10.0 ± 25% × $10^{-6}$ kg $mL^{-1}$, 50.0 ± 25% × $10^{-6}$ kg $mL^{-1}$, 100.0 ± 25% × $10^{-6}$ kg $mL^{-1}$, 500.0 ± 25% × $10^{-6}$ kg $mL^{-1}$, or 1000.0 ± 25% × $10^{-6}$ kg $mL^{-1}$, 1250.0 ± 25% × $10^{-6}$ kg $mL^{-1}$, 1500.0 ± 25% × $10^{-6}$ kg $mL^{-1}$, 1750.0 ± 25% × $10^{-6}$ kg $mL^{-1}$, 2000.0 ± 25% × $10^{-6}$ kg $mL^{-1}$, 3000.0 ± 25% × $10^{-6}$ kg $mL^{-1}$, 4000.0 ± 25% × $10^{-6}$ kg $mL^{-1}$, 5000.0 ± 25% × $10^{-6}$ kg $mL^{-1}$, 6000.0 ± 25% × $10^{-6}$ kg $mL^{-1}$o or 7000.0 ± 25% × $10^{-6}$ kg $mL^{-1}$. Optionally, dbmnC* can be measured 10 hours after administration, 11 hours after administration, 12 hours after administration, 13 hours after administration, 14 hours after administration, 15 hours after administration, 16 hours after administration, 17 hours after administration, 18 hours after administration, 19 hours after administration, 20 hours after administration, 21 hours after administration, 22 hours after administration, 23 hours after administration, 24 hours after administration, or 36 hours after administration. For example, a dbmnC* value may be about, or less than about, 1.0 ± 25% × $10^{-9}$ $mL^{-1}$, 1.0 ± 25% × $10^{-8}$ $mL^{-1}$, 0.7 ± 25% × $10^{-7}$ $mL^{-1}$, 1.0 ± 25% × $10^{-7}$ $mL^{-1}$, 2.0 ± 25% × $10^{-7}$ $mL^{-1}$, 3.0 ± 25% × $10^{-7}$ $mL^{-1}$, 4.0 ± 25% × $10^{-7}$ $mL^{-1}$, 5.0 ± 25% × $10^{-7}$ $mL^{-1}$, 1.0 ± 25% × $10^{-6}$ $mL^{-1}$, or 5.0 ± 25% × $10^{-6}$ $mL^{-1}$. Optionally, the dbmnC* value can relate to single dosing. The dbmnC* value can also relate to a multi-dose regimen (e.g., repeated daily administration). Optionally, the plasma concentration used to calculate the dbmnC* may be adjusted to reflect a baseline plasma concentration (e.g., a baseline plasma level because of repeated daily administration). $C_{max}$ is defined as the peak plasma concentration of a compound disclosed herein after administration. If a dbmnC* value is calculated at the time point corresponding to $C_{max}$, the value may alternatively be referred to as a dose and body mass normalized $C_{max}$ or $dbmnC_{max}$*. Optionally, a $dbmnC_{max}$* is greater than or equal to 10.0 ± 25% × $10^{-6}$ kg $mL^{-1}$, greater than or equal to 15 ± 25% × $10^{-6}$ kg $mL^{-1}$, greater than or equal to 20 ± 25% × $10^{-6}$ kg $mL^{-1}$, greater than or equal to 25 ± 25% × $10^{-6}$ kg $mL^{-1}$, greater than or equal to 30 ± 25% × $10^{-6}$ kg $mL^{-1}$, greater than or equal to 35 ± 25% × $10^{-6}$ kg $mL^{-1}$, greater than or equal to 40 ± 25% × $10^{-6}$ kg $mL^{-1}$, greater than or equal to 45 ± 25% × $10^{-6}$ kg $mL^{-1}$, greater than or equal to 50 ± 25% × $10^{-6}$ kg $mL^{-1}$. A $dbmnC_{max}$* can optionally also be greater than or equal to 100.0 ± 25% × $10^{-6}$ kg $mL^{-1}$, greater than or equal to 150 ± 25% × $10^{-6}$ kg $mL^{-1}$, greater than or equal to 200 ± 25% × $10^{-6}$ kg $mL^{-1}$, greater than or equal to 250 ± 25% × $10^{-6}$ kg kg $mL^{-1}$, greater than or equal to 300 ± 25% × $10^{-6}$ $mL^{-1}$, greater than or equal to 350 ± 25% × $10^{-6}$ kg $mL^{-1}$, greater than or equal to 400 ± 25% × $10^{-6}$ kg $mL^{-1}$, greater than or equal to 450 ± 25% × $10^{-6}$ kg $mL^{-1}$, greater than or equal to 500 ± 25% × $10^{-6}$ kg $mL^{-1}$.

As described above, $C_{max}$ is defined as the peak plasma concentration of a compound disclosed herein after administration. By administering a composition as disclosed herein for transmucosal absorption, it is possible to obtain higher $C_{max}$ values than if the composition were administered orally. Optionally, a composition affords a $C_{max}$ of a compound greater than or equal to 10 ng/mL, greater than or equal to 11 ng/mL, greater than or equal to 12 ng/mL, greater than or equal to 13 ng/mL, greater than or equal to 14 ng/mL, greater than or equal to 15 ng/mL, greater than or equal to 16 ng/mL, greater than or equal to 17 ng/mL, greater than or equal to 18 ng/mL, greater than or equal to 19 ng/mL, greater than or equal to 20 ng/mL, greater than or equal to 21 ng/mL, greater than or equal to 22 ng/mL, greater than or equal to 23 ng/mL, greater than or equal to 24 ng/mL, greater than or equal to 25 ng/mL, greater than or equal to 26 ng/mL, greater than or equal to 27 ng/mL, greater than or equal to 28 ng/mL, greater than or equal to 29 ng/mL, greater than or equal to 30 ng/mL, greater than or equal to 31 ng/mL, greater than or equal to 32 ng/mL, greater than or equal to 33 ng/mL, greater than or equal to 34 ng/mL, greater than or equal to 35 ng/mL, greater than or equal to 36 ng/mL, greater than or equal to 37 ng/mL, greater than or equal to 38 ng/mL, greater than or equal to 39 ng/mL, greater than or equal to 40 ng/mL, greater than or equal to 50 ng/mL, greater than or equal to 60 ng/mL, greater than or equal to 70 ng/mL, greater than or equal to 80 ng/mL, greater than or equal to 90 ng/mL, greater than or equal to 100 ng/mL, greater than or equal to 120 ng/mL, greater than or equal to 140 ng/mL, greater than or equal to 160 ng/mL, greater than or equal to 180 ng/mL, or greater than or equal to 200 ng/mL.

A $C_{max}$ may be measured either after administration of a first dose or after the administration of any dose of a composition disclosed herein. However, because the compositions and methods disclosed herein may be used to prolong a therapeutic regimen, plasma levels of a compound useful in the compositions and methods may not return to 0 between dosing (i.e., there may be a baseline level of a compound in circulation). Accordingly, a composition may afford a $C_{max}$ that can be compared to a baseline level of the compound rather than taken as an absolute numerical value compared to a starting plasma concentration of 0 ng/mL. Optionally, a composition affords a $C_{max}$ of a compound greater than or equal to 10 ng/mL, greater than or equal to 11 ng/mL, greater than or equal to 12 ng/mL, greater than or equal to 13 ng/mL, greater than or equal to 14 ng/mL, greater than or equal to 15 ng/mL, greater than or equal to 16 ng/mL, greater than or equal to 17 ng/mL, greater than or equal to 18 ng/mL, greater than or equal to 19 ng/mL, greater than or equal to 20 ng/mL, greater than or equal to 21 ng/mL, greater than or equal to 22 ng/mL, greater than or equal to 23 ng/mL, greater than or equal to 24 ng/mL, greater than or equal to 25 ng/mL, greater than or equal to 26 ng/mL, greater than or equal to 27 ng/mL, greater than or equal to 28 ng/mL, greater than or equal to 29 ng/mL, greater than or equal to 30 ng/mL, greater than or equal to 31 ng/mL, greater than or equal to 32 ng/mL, greater than or equal to 33 ng/mL, greater than or equal to 34 ng/mL, greater than or equal to 35 ng/mL, greater than or equal to 36 ng/mL, greater than or equal to 37 ng/mL, greater than or equal to 38 ng/mL, greater than or equal to 39 ng/mL, greater than or equal to 40 ng/mL, greater than or equal to 50 ng/mL, greater than or equal to 60 ng/mL, greater than or equal to 70 ng/mL, greater than or equal to 80 ng/mL, greater than or equal to 90 ng/mL, greater than or equal to 100 ng/mL, greater than or equal to 120 ng/mL, greater than or equal to 140 ng/mL, greater than or equal to 160 ng/mL, greater than or equal to 180 ng/mL, or greater than or equal to 200 ng/mL above a baseline level (e.g., plasma concentration) of the compound as measured immediately prior to a second administration. As used herein, "immediately prior to administration" means within 1 hour, 45 minutes, 30 minutes, 15 minutes, 10 minutes, 5 minutes, or 1 minute of administration.

As a result of the higher $C_{max}$ values achieved through sublingual administration, the area under the curve (AUC) for plasma concentration of a compound over time also is greater in comparison to the AUC afforded by an oral administration. AUC can be measured between two specific time points (e.g., $AUC_{0-8h}$) or over an extrapolated period of time from 0 to infinity ($AUC_{0-0\infty h}$, $AUC_{0-\infty}$ or $AUC_{inf}$). AUC is typically given in units of ng hr $mL^{-1}$, so for example in the experiment on human subjects who received 5 mg immediate release cyclobenzaprine tablets in Figure 1, the $AUC_{0-\infty h}$ was determined to be 103.1 $\pm$ 35.8 ng hr $mL^{-1}$ and the $AUC_{0-168h}$ was 92.2 $\pm$ 29.9 ng hr $mL^{-1}$. In another example, a dose of a 2.4 mg cyclobenzaprine sublingual tablet with basifying agent from 0 to 0.75 h resulted in an $AUC_{0-0.75\,h}$ of 135.6 ng hr $mL^{-1}$ in an experiment on Beagles. In the same experiment, for the sublingual tablet with basifying agent, the $AUC_{0-\infty h}$ was 179.0 $\pm$ 50.2 ng hr $mL^{-1}$ and the $AUC_{0-10h}$ was 176.6 $\pm$ 49.9 ng hr $mL^{-1}$. In the Beagle experiment, for the sublingual 2.4 mg tablet lacking the basifying agent, the $AUC_{0-0.75\,h}$ was 82.4 ng hr $mL^{-1}$, the $AUC_{0-\infty h}$ was 155.4 $\pm$ 64.6 ng hr $mL^{-1}$ and the $AUC_{0-10h}$ was 151.6 $\pm$ 64.0 ng hr $mL^{-1}$. In a Beagle experiment (Figures 2 and 3) for i.v. cyclobenzaprine at an average dose of 1.79 mg, the $AUC_{0-\infty h}$ was 44.9 $\pm$ 4.15 ng hr $mL^{-1}$ and the $AUC_{0-24h}$ was 43.5 $\pm$ 3.77 ng hr $mL^{-1}$. In a Beagle experiment (Figures 2 and 3) for sublingual solution cyclobenzaprine at an average dose of 1.79 mg, the $AUC_{0-\infty h}$ was 129.1 $\pm$ 36.4 ng hr $mL^{-1}$ and the $AUC_{0-24h}$ was 126.9 $\pm$ 37.1 ng hr $mL^{-1}$. By contrast, in an example from the literature, administering 2.5, 5.0 or 10 mg (10 mg in a 70 kg human being equivalent to 0.14 mg/kg in Beagles) cyclobenzaprine immediate release tablets to humans resulted in $AUC_{0-8\,h}$ of 11.1, 23.0, and 45.9 ng hr $mL^{-1}$, respectively and $AUC_{0-\infty h}$ of 44.2, 89.5, and 178.2 ng hr $mL^{-1}$, respectively (Winchell G.A. et al. "Cyclobenzaprine pharmacokinetics, including the effects of age, gender and hepatic insufficiency", J. Clin. Pharmacol 2002 42:61). For example, at a 2.4 mg dosing, the $AUC_{0-20min}$ is about 0.04 ng hr $mL^{-1}$, $AUC_{0-30min}$ is about 0.13 ng hr $mL^{-1}$, $AUC_{0-45min}$ is about 0.33 ng hr $mL^{-1}$, $AUC_{0-1h}$ is about 0.61 ng hr $mL^{-1}$, $AUC_{0-2h}$ is about 2.10 ng hr $mL^{-1}$, $AUC_{0-2.5h}$ is about 2.95 ng hr $mL^{-1}$, $AUC_{0-3h}$ is about 3.93 ng hr $mL^{-1}$, $AUC_{0-3.3h}$ is about 4.66 ng hr $mL^{-1}$, $AUC_{0-3.7h}$ is about 5.46 ng hr $mL^{-1}$, $AUC_{0-4h}$ is about 6.27 ng hr $mL^{-1}$, $AUC_{0-4.3h}$ is about 7.12 ng hr $mL^{-1}$, $AUC_{0-4.7h}$ is about 7.99 ng hr $mL^{-1}$, $AUC_{0-0-5h}$ is about 8.81 ng hr $mL^{-1}$, $AUC_{0-5.5h}$ is about 10.06 ng hr $mL^{-1}$, $AUC_{0-6h}$ is about 11.27 ng hr $mL^{-1}$, $AUC_{0-8h}$ is about 15.11 ng hr $mL^{-1}$, $AUC_{0-12h}$ is about 50.30 ng hr $mL^{-1}$, $AUC_{0-Inf}$ is about 60.97 ng hr $mL^{-1}$. Optionally (with TNX-102 SL 2.8), the $AUC_{0-20min}$ is about 0.04 ng hr $mL^{-1}$, $AUC_{0-30min}$ is about 0.15 ng hr $mL^{-1}$, $AUC_{0-45min}$ is about 0.39 ng hr $mL^{-1}$, $AUC_{0-1h}$ is about 0.72 ng hr $mL^{-1}$, $AUC_{0-2h}$ is about 2.45 ng hr $mL^{-1}$, $AUC_{0-2.5h}$ is about 3.45 ng hr $mL^{-1}$, $AUC_{0-3h}$ is about 4.59 ng hr $mL^{-1}$, $AUC_{0-3.3h}$ is about 5.44 ng hr $mL^{-1}$, $AUC_{0-3.7h}$ is about 6.37 ng hr $mL^{-1}$, $AUC_{0-4h}$ is about 7.32 ng hr $mL^{-1}$, $AUC_{0-4.3h}$ is about 8.30 ng hr $mL^{-1}$, $AUC_{0-4.7h}$ is about 9.32 ng hr $mL^{-1}$, $AUC_{0-0-5h}$ is about 10.27 ng hr $mL^{-1}$, $AUC_{0-5.5h}$ is about 11.74 ng hr $mL^{-1}$, $AUC_{0-6h}$ is about 13.14 ng hr $mL^{-1}$, $AUC_{0-8h}$ is about 17.63 ng hr $mL^{-1}$, $AUC_{0-12h}$ is about 58.68 ng hr $mL^{-1}$, $AUC_{0-Inf}$ is about 71.13 ng hr $mL^{-1}$. AUC also can be compared to the dose administered to generate an AUC to dose ratio which is sometimes referred to as dose normalized AUC, or dnAUC. The dose normalized

$dnAUC_{0-\infty h}$ for the human data described above and in Figure 1 is 20.6 $\times$ $10^{-6}$ hr $mL^{-1}$. The dose normalized $AUC_{0-0.75 h}$ ($dnAUC_{0-0.75 h}$) for the Beagle data described above is $dnAUC_{0-0.75 h}$ = 135.6 6 ng hr $mL^{-1}$/2.4 mg = 56.5 $\times$ $10^{-6}$ hr $mL^{-1}$. In a Beagle experiment (Figures 2 and 3) for IV cyclobenzaprine at an average dose of 1.79 mg, the $dnAUC_{0-\infty h}$ was 25.04 $\times$ $10^{-6}$ hr $mL^{-1}$ and the $dnAUC_{0-24h}$ was 24.2 $\times$ $10^{-6}$ hr $mL^{-1}$. In a Beagle experiment (Figures 2 and 3) for sublingual cyclobenzaprine in solution at an average dose of 1.79 mg, the $dnAUC_{0-\infty h}$ was 71.95 $\times$ $10^{-6}$ hr $mL^{-1}$ and the $dnAUC_{0-24h}$ was 70.72 $\times$ $10^{-6}$ hr $mL^{-1}$. The dose normalized $AUC_{0-8 h}$ ($dnAUC_{0-8h}$) for the human data described above (from Winchell et al) is 4.4 $\times$ $10^{-6}$ hr $mL^{-1}$, 4.6 $\times$ $10^{-6}$ hr $mL^{-1}$ and 4.6 $\times$ $10^{-6}$ hr $mL^{-1}$ for the 2.5, 5.0 and 10 mg cyclobenzaprine doses, respectively. The dose normalized $dnAUC_{0-\infty h}$ for the human data described above (from Winchell et al.) is 17.7 $\times$ $10^{-6}$ hr $mL^{-1}$, 17.9 $\times$ $10^{-6}$ hr $mL^{-1}$, and 17.8 $\times$ $10^{-6}$ hr $mL^{-1}$ for the 2.5, 5.0 and 10 mg cyclobenzaprine doses, respectively. Optionally, the $dnAUC_{0-20mm}$ is about 0.02 $\pm$ 25% $\times$ $10^{-6}$ hr $mL^{-1}$, the $dnAUC_{0-30min}$ is about 0.05 $\pm$ 25% x $10^{-6}$ hr $mL^{-1}$, the $dnAUC_{0-45min}$ is about 0.15 $\pm$ 25% x $10^{-6}$ hr $mL^{-1}$, the $dnAUC_{0-1h}$ is about 0.25 $\pm$ 25% x $10^{-6}$ hr $mL^{-1}$, the $dnAUC_{0-2h}$ is about 0.90 $\pm$ 25% $\times$ $10^{-6}$ hr $mL^{-1}$, the $dnAUC_{0-2.5h}$ is about 1.2 $\pm$ 25% $\times$ $10^{-6}$ hr $mL^{-1}$, the $dnAUC_{0-3h}$ is about 1.6 $\pm$ 25% $\times$ $10^{-6}$ hr $mL^{-1}$, the $dnAUC_{3.3h}$ is about 1.8 $\pm$ 25% $\times$ $10^{-6}$ hr $mL^{-1}$, $dnAUC_{0-3.7h}$ is about 2.3 $\pm$ 25% x $10^{-6}$ hr $mL^{-1}$, the $dnAUC_{0-4h}$ is about 2.6 $\pm$ 25% x $10^{-6}$ hr $mL^{-1}$, the $dnAUC_{0-4.3h}$ is about 3.0 $\pm$ 25% $\times$ $10^{-6}$ hr $mL^{-1}$, the $dnAUC_{0-4.7h}$ is about 3.3 $\pm$ 25% $\times$ $10^{-6}$ hr $mL^{-1}$, the $dnAUC_{0-5h}$ is about 3.7 $\pm$ 25% x $10^{-6}$ hr $mL^{-1}$, the $dnAUC_{0-5.5h}$ is about 4.2 $\pm$ 25% $\times$ $10^{-6}$ hr $mL^{-1}$, the $dnAUC_{0-6h}$ is about 4.7 $\pm$ 25% $\times$ $10^{-6}$ hr $mL^{-1}$, the $dnAUC_{0-8h}$ is 6.3 $\pm$ 25% $\times$ $10^{-6}$ hr $mL^{-1}$, the $dnAUC_{0-12h}$ is about 20 $\pm$ 25% $\times$ $10^{-6}$ hr $mL^{-1}$, and the $dnAUC_{0-\infty h}$ is about 25 $\pm$ 25% $\times$ $10^{-6}$ hr $mL^{-1}$. Optionally, a $dnAUC_{0-8h}$ is greater than or equal to 5 $\pm$ 25% $\times$ $10^{-6}$ hr $mL^{-1}$, greater than or equal to 6 $\pm$ 25% $\times$ $10^{-6}$ hr $mL^{-1}$, greater than or equal to 7 $\pm$ 25% $\times$ $10^{-6}$ hr $mL^{-1}$, greater than or equal to 8 $\pm$ 25% $\times$ $10^{-6}$ hr $mL^{-1}$, greater than or equal to 9 $\pm$ 25% $\times$ $10^{-6}$ hr $mL^{-1}$, greater than or equal to 10 $\pm$ 25% $\times$ $10^{-6}$ hr $mL^{-1}$, greater than or equal to 11 $\pm$ 25% x $10^{-6}$ hr $mL^{-1}$, greater than or equal to 12 $\pm$ 25% $\times$ $10^{-6}$ hr $mL^{-1}$, greater than or equal to 13 $\pm$ 25% $\times$ $10^{-6}$ hr $mL^{-1}$, greater than or equal to 14 $\pm$ 25% $\times$ $10^{-6}$ hr $mL^{-1}$, greater than or equal to 15 $\pm$ 25% $\times$ $10^{-6}$ hr $mL^{-1}$, greater than or equal to 16 $\pm$ 25% $\times$ $10^{-6}$ hr $mL^{-1}$, greater than or equal to 17 $\pm$ 25% $\times$ $10^{-6}$ hr $mL^{-1}$, greater than or equal to 18 $\pm$ 25% $\times$ $10^{-6}$ hr $mL^{-1}$, or greater than or equal to 19 $\pm$ 25% $\times$ $10^{-6}$ hr $mL^{-1}$. Optionally, a $dnAUC_{0-8h}$ is greater than or equal to 20 $\pm$ 25% $\times$ $10^{-6}$ hr $mL^{-1}$, greater than or equal to 22 $\pm$ 25% $\times$ $10^{-6}$ hr $mL^{-1}$, greater than or equal to 24 $\pm$ 25% $\times$ $10^{-6}$ hr $mL^{-1}$, greater than or equal to 26 $\pm$ 25% $\times$ $10^{-6}$ hr $mL^{-1}$, greater than or equal to 28 $\pm$ 25% $\times$ $10^{-6}$ hr $mL^{-1}$, or greater than or equal to 30 $\pm$ 25% $\times$ $10^{-6}$ hr $mL^{-1}$. Optionally, a $dnAUC_{0-8h}$ is greater than or equal to 40 $\pm$ 25% $\times$ $10^{-6}$ hr $mL^{-1}$, greater than or equal to 50 $\pm$ 25% $\times$ $10^{-6}$ hr $mL^{-1}$, greater than or equal to 60 $\pm$ 25% $\times$ $10^{-6}$ hr $mL^{-1}$, greater than or equal to 70 $\pm$ 25% $\times$ $10^{-6}$ hr $mL^{-1}$, greater than or equal to 80 $\pm$ 25% $\times$ $10^{-6}$ hr $mL^{-1}$, or greater than or equal to 90 $\pm$ 25% $\times$ $10^{-6}$ hr $mL^{-1}$. Optionally, a $dnAUC_{0-8h}$ is greater than or equal to 100 $\pm$ 25% $\times$ $10^{-6}$ hr $mL^{-1}$, greater than or equal to 120 $\pm$ 25% x $10^{-6}$ hr $mL^{-1}$, greater than or equal to 140 $\pm$ 25% $\times$ $10^{-6}$ hr $mL^{-1}$, greater than or equal to 160 $\pm$ 25% $\times$ $10^{-6}$ hr $mL^{-1}$, greater than or equal to 180 $\pm$ 25% $\times$ $10^{-6}$ hr $mL^{-1}$, or greater than or equal to 200 $\pm$ 25% $\times$ $10^{-6}$ hr $mL^{-1}$. Optionally a $dnAUC_{0-10h}$ is greater than or equal to 5 $\pm$ 25% $\times$ $10^{-6}$ hr $mL^{-1}$, greater than or equal to 6 $\pm$ 25% $\times$ $10^{-6}$ hr $mL^{-1}$, greater than or equal to 7 $\pm$ 25% $\times$ $10^{-6}$ hr $mL^{-1}$, greater than or equal to 8 $\pm$ 25% $\times$ $10^{-6}$ hr $mL^{-1}$, greater than or equal to 9 $\pm$ 25% $\times$ $10^{-6}$ hr $mL^{-1}$, greater than or equal to 10 $\pm$ 25% $\times$ $10^{-6}$ hr $mL^{-1}$, greater than or equal to 11 $\pm$ 25% $\times$ $10^{-6}$ hr $mL^{-1}$, greater than or equal to 12 $\pm$ 25% $\times$ $10^{-6}$ hr $mL^{-1}$, greater than or equal to 13 $\pm$ 25% $\times$ $10^{-6}$ hr $mL^{-1}$, greater than or equal to 14 $\pm$ 25% x $10^{-6}$ hr $mL^{-1}$, greater than or equal to 15 $\pm$ 25% $\times$ $10^{-6}$ hr $mL^{-1}$, greater than or equal to 16 $\pm$ 25% $\times$ $10^{-6}$ hr $mL^{-1}$, greater than or equal to 17 $\pm$ 25% $\times$ $10^{-6}$ hr $mL^{-1}$, greater than or equal to 18 $\pm$ 25% $\times$ $10^{-6}$ hr $mL^{-1}$, or greater than or equal to 19 $\pm$ 25% $\times$ $10^{-6}$ hr $mL^{-1}$. Optionally, a $dnAUC_{0-10h}$ is greater than or equal to 20 $\pm$ 25% $\times$ $10^{-6}$ hr $mL^{-1}$, greater than or equal to 22 $\pm$ 25% $\times$ $10^{-6}$ hr $mL^{-1}$, greater than or equal to 24 $\pm$ 25% $\times$ $10^{-6}$ hr $mL^{-1}$, greater than or equal to 26 $\pm$ 25% $\times$ $10^{-6}$ hr $mL^{-1}$, greater than or equal to 28 $\pm$ 25% $\times$ $10^{-6}$ hr $mL^{-1}$, or greater than or equal to 30 $\pm$ 25% $\times$ $10^{-6}$ hr $mL^{-1}$. Optionally, a $dnAUC_{0-10h}$ is greater than or equal to 40 $\pm$ 25% $\times$ $10^{-6}$ hr $mL^{-1}$, greater than or equal to 50 $\pm$ 25% $\times$ $10^{-6}$ hr $mL^{-1}$, greater than or equal to 60 $\pm$ 25% $\times$ $10^{-6}$ hr $mL^{-1}$, greater than or equal to 70 $\pm$ 25% $\times$ $10^{-6}$ hr $mL^{-1}$, greater than or equal to 80 $\pm$ 25% $\times$ $10^{-6}$ hr $mL^{-1}$, or greater than or equal to 90 $\pm$ 25% $\times$ $10^{-6}$ hr $mL^{-1}$. Optionally, a $dnAUC_{0-10h}$ is greater than or equal to 100 $\pm$ 25% $\times$ $10^{-6}$ hr $mL^{-1}$, greater than or equal to 120 $\pm$ 25% $\times$ $10^{-6}$ hr $mL^{-1}$, greater than or equal to 140 $\pm$ 25% $\times$ $10^{-6}$ hr $mL^{-1}$, greater than or equal to 160 $\pm$ 25% $\times$ $10^{-6}$ hr $mL^{-1}$, greater than or equal to 180 $\pm$ 25% $\times$ $10^{-6}$ hr $mL^{-1}$, or greater than or equal to 200 $\pm$ 25% $\times$ $10^{-6}$ hr $mL^{-1}$. Optionally, a $dnAUC_{0-12h}$ is greater than or equal to 20 $\pm$ 25% $\times$ $10^{-6}$ hr $mL^{-1}$, greater than or equal to 30 $\pm$ 25% $\times$ $10^{-6}$ hr $mL^{-1}$, greater than or equal to 40 $\pm$ 25% $\times$ $10^{-6}$ hr $mL^{-1}$, greater than or equal to 50 $\pm$ 25% $\times$ $10^{-6}$ hr $mL^{-1}$, greater than or equal to 60 $\pm$ 25% $\times$ $10^{-6}$ hr $mL^{-1}$, or greater than or equal to 70 $\pm$ 25% $\times$ $10^{-6}$ hr $mL^{-1}$. Optionally, a $dnAUC_{0-12h}$ is greater than or equal to 80 $\pm$ 25% $\times$ $10^{-6}$ hr $mL^{-1}$, greater than or equal to 90 $\pm$ 25% $\times$ $10^{-6}$ hr $mL^{-1}$, greater than or equal to 100 $\pm$ 25% $\times$ $10^{-6}$ hr $mL^{-1}$, greater than or equal to 120 $\pm$ 25% $\times$ $10^{-6}$ hr $mL^{-1}$, greater than or equal to 160 $\pm$ 25% $\times$ $10^{-6}$ hr $mL^{-1}$, or greater than or equal to 180 $\pm$ 25% $\times$ $10^{-6}$ hr $mL^{-1}$. Optionally, a $dnAUC_{0-24h}$ is greater than or equal to 24 $\pm$ 25% $\times$ $10^{-6}$ hr $mL^{-1}$, or greater than or equal to 25 $\pm$ 25% $\times$ $10^{-6}$ hr $mL^{-1}$, greater than or equal to 30 $\pm$ 25% $\times$ $10^{-6}$ hr $mL^{-1}$, greater than or equal to 35 $\pm$ 25% $\times$ $10^{-6}$ hr $mL^{-1}$, greater than or equal to 40 $\pm$ 25% $\times$ $10^{-6}$ hr $mL^{-1}$, greater than or equal to 50 $\pm$ 25% $\times$ $10^{-6}$ hr $mL^{-1}$, greater than or equal to 60 $\pm$ 25% $\times$ $10^{-6}$ hr $mL^{-1}$, greater than or equal to 70 $\pm$ 25% $\times$ $10^{-6}$ hr $mL^{-1}$, greater than or equal to 80 $\pm$ 25% $\times$ $10^{-6}$ hr $mL^{-1}$, or greater than or equal to 90 $\pm$ 25% $\times$ $10^{-6}$ hr $mL^{-1}$. Optionally, $dnAUC_{0-24h}$

is greater than or equal to 100 ± 25% × $10^{-6}$ hr $mL^{-1}$, greater than or equal to 110 ± 25% × $10^{-6}$ hr $mL^{-1}$, greater than or equal to 120 ± 25% × $10^{-6}$ hr $mL^{-1}$, greater than or equal to 130 ± 25% × $10^{-6}$ hr $mL^{-1}$, greater than or equal to 140 ± 25% × $10^{-6}$ hr $mL^{-1}$, or greater than or equal to 150 ± 25% × $10^{-6}$ hr $mL^{-1}$. Optionally, a $dnAUC_{0-24h}$ is greater than or equal to 160 ± 25% × $10^{-6}$ hr $mL^{-1}$, greater than or equal to 170 ± 25% x $10^{-6}$ hr $mL^{-1}$, greater than or equal to 180 ± 25% × $10^{-6}$ hr $mL^{-1}$, greater than or equal to 190 ± 25% × $10^{-6}$ hr $mL^{-1}$, greater than or equal to 200 ± 25% × $10^{-6}$ hr $mL^{-1}$, or greater than or equal to 210 ± 25% × $10^{-6}$ hr $mL^{-1}$. Optionally, a $dnAUC_{0-24h}$ is greater than or equal to 220 ± 25% × $10^{-6}$ hr $mL^{-1}$, greater than or equal to 240 ± 25% × $10^{-6}$ hr $mL^{-1}$, greater than or equal to 250 ± 25% x $10^{-6}$ hr $mL^{-1}$, greater than or equal to 260 ± 25% × $10^{-6}$ hr $mL^{-1}$, greater than or equal to 270 ± 25% × $10^{-6}$ hr $mL^{-1}$, or greater than or equal to 280 ± 25% × $10^{-6}$ hr $mL^{-1}$. Optionally, a $dnAUC_{0-\infty h}$ is greater than or equal to 24 ± 25% × $10^{-6}$ hr $mL^{-1}$, or greater than or equal to 25 ± 25% × $10^{-6}$ hr $mL^{-1}$, greater than or equal to 30 ± 25% × $10^{-6}$ hr $mL^{-1}$, greater than or equal to 35 ± 25% × $10^{-6}$ hr $mL^{-1}{}_1$ greater than or equal to 40 ± 25% × $10^{-6}$ hr $mL^{-1}$, greater than or equal to 50 ± 25% × $10^{-6}$ hr $mL^{-1}$, greater than or equal to 60 ± 25% × $10^{-6}$ hr $mL^{-1}$, greater than or equal to 70 ± 25% × $10^{-6}$ hr $mL^{-1}$, greater than or equal to 80 ± 25% × $10^{-6}$ hr $mL^{-1}$, or greater than or equal to 90 ± 25% × $10^{-6}$ hr $mL^{-1}$. Optionally, $dnAUC_{0-\infty h}$ is greater than or equal to 100 ± 25% × $10^{-6}$ hr $mL^{-1}$, greater than or equal to 110 ± 25% × $10^{-6}$ hr $mL^{-1}$, greater than or equal to 120 ± 25% × $10^{-6}$ hr $mL^{-1}$, greater than or equal to 130 ± 25% × $10^{-6}$ hr $mL^{-1}$, greater than or equal to 140 ± 25% × $10^{-6}$ hr $mL^{-1}$, or greater than or equal to 150 ± 25% x $10^{-6}$ hr $mL^{-1}$. Optionally, a $dnAUC_{0-\infty h}$ is greater than or equal to 160 ± 25% × $10^{-6}$ hr $mL^{-1}$, greater than or equal to 170 ± 25% × $10^{-6}$ hr $mL^{-1}$, greater than or equal to 180 ± 25% × $10^{-6}$ hr $mL^{-1}$, greater than or equal to 190 ± 25% × $10^{-6}$ hr $mL^{-1}$, greater than or equal to 200 ± 25% × $10^{-6}$ hr $mL^{-1}$, or greater than or equal to 210 ± 25% × $10^{-6}$ hr $mL^{-1}$. Optionally, a $dnAUC_{0-\infty h}$ is greater than or equal to 220 ± 25% × $10^{-6}$ hr $mL^{-1}$, greater than or equal to 240 ± 25% × $10^{-6}$ hr $mL^{-1}$, greater than or equal to 250 ± 25% × $10^{-6}$ hr $mL^{-1}$, greater than or equal to 260 ± 25% × $10^{-6}$ hr $mL^{-1}$, greater than or equal to 270 ± 25% × $10^{-6}$ hr $mL^{-1}$, or greater than or equal to 280 ± 25% × $10^{-6}$ hr $mL^{-1}$.

The product of AUC and body mass also can be compared to the dose administered to generate a ratio between the AUC times body mass product to dose which is herein referred to as dose- and body mass- normalized AUC, or dbmnAUC. The dose- and body mass- normalized $dbmnAUC_{0-\infty h}$ for the human data described above and in Figure 1 is approximately, for 70 kg humans, 140.6 x $10^{-6}$ kg hr $mL^{-1}$. The dose- and body mass- normalized $AUC_{0-0.75 h}$ ($dbmnAUC_{0-0.75 h}$) for the Beagle data described above (average body mass of Beagles was 12.5 kg) is $dbmnAUC_{0-0.75 h}$ = 12.5 kg × 135.6 ng hr $mL^{-1}$/2.4 mg = 708 × $10^{-6}$ kg hr $mL^{-1}$. In a Beagle experiment (Figures 2 and 3) for IV cyclobenzaprine at an average dose of 1.79 mg, the $dbmnAUC_{0-\infty h}$ was 12.5 kg × 25.04 × $10^{-6}$ hr $mL^{-1}$ and the $dbmnAUC_{0-24h}$ was 314 × $10^{-6}$ kg hr $mL^{-1}$. In a Beagle experiment (Figures 2 and 3) for sublingual cyclobenzaprine in solution at an average dose of 1.79 mg, the $dbmnAUC_{0-\infty h}$ was 12.5 kg x 71.95 × $10^{-6}$ hr $mL^{-1}$ and the $dbmnAUC_{0-24h}$ was 886 × $10^{-6}$ kg hr $mL^{-1}$. The $dbmnAUC_{0-8 h}$ for the human data described above (from Winchell et al.), assuming 70 kg humans, is approximately 70 kg x 4.4 ± 25% × $10^{-6}$ hr $mL^{-1}$ = 308 ± 25% × $10^{-6}$ kg hr $mL^{-1}$, 322 ± 25% × $10^{-6}$ kg hr $mL^{-1}$ and 322 ± 25% × $10^{-6}$ kg hr $mL^{-1}$ for the 2.5, 5.0 and 10 mg cyclobenzaprine doses, respectively. The dose normalized $dbmnAUC_{0-\infty h}$ for the human data described above (from Winchell et al.) is 70 kg × 17.7 ± 25% × $10^{-6}$ hr $mL^{-1}$ = 1239 ± 25% × $10^{-6}$ kg hr $mL^{-1}$, 1253 ± 25% × $10^{-6}$ kg hr $mL^{-1}$, and 1246 ± 25% × $10^{-6}$ kg hr $mL^{-1}$ for the 2.5, 5.0 and 10 mg cyclobenzaprine doses, respectively. Optionally, the $dbmnAUC_{0-20min}$ is about 1.1 ± 25% × $10^{-6}$ kg hr $mL^{-1}$, the $dbmnAUC_{0-30min}$ is about 3.7 ± 25% × $10^{-6}$ kg hr $mL^{-1}$, the $dbmnAUC_{0-45min}$ is about 9.7 ± 25% × $10^{-6}$ kg hr $mL^{-1}$, the $dbmnAUC_{0-1h}$ is about 18 ± 25% × $10^{-6}$ kg hr $mL^{-1}$, the $dbmnAUC_{0-2h}$ is about 62 ± 25% × $10^{-6}$ kg hr $mL^{-1}$, the $dbmnAUC_{0-2.5h}$ is about 86 ± 25% × $10^{-6}$ kg hr $mL^{-1}$, the $dbmnAUC_{0-3h}$ is about 115 ± 25% × $10^{-6}$ kg hr $mL^{-1}$, the $dbmnAUC_{3.3h}$ is about 135 ± 25% × $10^{-6}$ kg hr $mL^{-1}$, $dbmnAUC_{0-3.7h}$ is about 160 ± 25% × $10^{-6}$ kg hr $mL^{-1}$, the $dbmnAUC_{0-4h}$ is about 180 ± 25% × $10^{-6}$ kg hr $mL^{-1}$, the $dbmnAUC_{0-4.3h}$ is about 210 ± 25% x $10^{-6}$ kg hr $mL^{-1}$, the dbmnAUCo-$_{4.7h}$ is about 230 ± 25% × $10^{-6}$ kg hr $mL^{-1}$, the $dbmnAUC_{0-5h}$ is about 260 ± 25% × $10^{-6}$ kg hr $mL^{-1}$, the $dbmnAUC_{0-5.5h}$ is about 290 ± 25% × $10^{-6}$ kg hr $mL^{-1}$, the $dbmnAUC_{0-6h}$ is about 330 ± 25% × $10^{-6}$ kg hr $mL^{-1}$, the $dbmnAUC_{0-8h}$ is 440 ± 25% × $10^{-6}$ kg hr $mL^{-1}$, the $dbmnAUC_{0-12h}$ is about 1500 ± 25% × $10^{-6}$ kg hr $mL^{-1}$, and the $dbmnAUC_{0-Inf}$ is about 1800 ± 25% × $10^{-6}$ kg hr $mL^{-1}$. Optionally, a $dbmnAUC_{0-8h}$ is greater than or equal to 350 ± 25% × $10^{-6}$ kg hr $mL^{-1}$. Optionally, a $dbmnAUC_{0-8h}$ is greater than or equal to 400 ± 25% × $10^{-6}$ hr $mL^{-1}$, greater than or equal to 500 ± 25% × $10^{-6}$ kg hr $mL^{-1}$, greater than or equal to 600 ± 25% × $10^{-6}$ kg hr $mL^{-1}$, greater than or equal to 700 ± 25% × $10^{-6}$ kg hr $mL^{-1}$, greater than or equal to 800 ± 25% × $10^{-6}$ kg hr $mL^{-1}$, or greater than or equal to 900 ± 25% × $10^{-6}$ kg hr $mL^{-1}$. Optionally, a $dbmnAUC_{0-8h}$ is greater than or equal to 1000 ± 25% × $10^{-6}$ kg hr $mL^{-1}$, greater than or equal to 1200 ± 25% × $10^{-6}$ kg hr $mL^{-1}$, greater than or equal to 1400 ± 25% × $10^{-6}$ kg hr $mL^{-1}$, greater than or equal to 1600 ± 25% × $10^{-6}$ kg hr $mL^{-1}$, greater than or equal to 1800 ± 25% × $10^{-6}$ kg hr $mL^{-1}$, or greater than or equal to 2000 ± 25% × $10^{-6}$ kg hr $mL^{-1}$. Optionally, a dbmnAUCo-$_{10h}$ is greater than or equal to 400 ± 25% × $10^{-6}$ kg hr $mL^{-1}$, greater than or equal to 500 ± 25% × $10^{-6}$ kg hr $mL^{-1}$, greater than or equal to 600 ± 25% × $10^{-6}$ kg hr $mL^{-1}$, greater than or equal to 700 ± 25% × $10^{-6}$ kg hr $mL^{-1}$, greater than or equal to 800 ± 25% × $10^{-6}$ kg hr $mL^{-1}$, or greater than or equal to 900 ± 25% × $10^{-6}$ kg hr $mL^{-1}$. Optionally, a $dbmnAUC_{0-10h}$ is greater than or equal to 1000 ± 25% × $10^{-6}$ kg hr $mL^{-1}$, greater than or equal to 1200 ± 25% × $10^{-6}$ kg hr $mL^{-1}$, greater than or equal to 1400 ± 25% × $10^{-6}$ kg hr $mL^{-1}$, greater than or equal to 1600 ± 25% × $10^{-6}$ kg hr $mL^{-1}$, greater than or equal to 1800 ± 25% × $10^{-6}$ kg hr

$mL^{-1}$, or greater than or equal to 2000 ± 25% × $10^{-6}$ kg hr $mL^{-1}$. Optionally, a $dbmnAUC_{0-12h}$ is greater than or equal to 500 ± 25% × $10^{-6}$ kg hr $mL^{-1}$, greater than or equal to 600 ± 25% × $10^{-6}$ kg hr $mL^{-1}$, greater than or equal to 700 ± 25% × $10^{-6}$ kg hr $mL^{-1}$, greater than or equal to 800 ± 25% × $10^{-6}$ kg hr $mL^{-1}$, or greater than or equal to 900 ± 25% × $10^{-6}$ kg hr $mL^{-1}$. Optionally, a $dbmnAUC_{0-12h}$ is greater than or equal to 1000 ± 25% × $10^{-6}$ kg hr $mL^{-1}$, greater than or equal to 1200 ± 25% × $10^{-6}$ kg hr $mL^{-1}$, greater than or equal to 1400 ± 25% × $10^{-6}$ kg hr $mL^{-1}$, greater than or equal to 1600 ± 25% × $10^{-6}$ kg hr $mL^{-1}$, greater than or equal to 1800 ± 25% × $10^{-6}$ kg hr $mL^{-1}$, or greater than or equal to 2000 ± 25% × $10^{-6}$ kg hr $mL^{-1}$. Optionally, a $dbmnAUC_{0-24h}$ is greater than or equal to 500 ± 25% × $10^{-6}$ kg hr $mL^{-1}$, greater than or equal to 600 ± 25% × $10^{-6}$ kg hr $mL^{-1}$, greater than or equal to 700 ± 25% × $10^{-6}$ kg hr $mL^{-1}$, greater than or equal to 800 ± 25% × $10^{-6}$ kg hr $mL^{-1}$, or greater than or equal to 900 ± 25% × $10^{-6}$ kg hr $mL^{-1}$. Optionally, $dbmnAUC_{0-24h}$ is greater than or equal to 1000 ± 25% × $10^{-6}$ kg $mL^{-1}$, greater than or equal to 1100 ± 25% × $10^{-6}$ hr $mL^{-1}$, greater than or equal to 1200 ± 25% × $10^{-6}$ kg hr $mL^{-1}$, greater than or equal to 1300 ± 25% × $10^{-6}$ kg hr $mL^{-1}$, greater than or equal to 1400 ± 25% × $10^{-6}$ kg hr $mL^{-1}$, or greater than or equal to 1500 ± 25% × $10^{-6}$ kg hr $mL^{-1}$. Optionally, a $dbmnAUC_{0-24h}$ is greater than or equal to 1600 ± 25% × $10^{-6}$ kg hr $mL^{-1}$, greater than or equal to 1700 ± 25% × $10^{-6}$ kg hr $mL^{-1}$, greater than or equal to 1800 ± 25% × $10^{-6}$ kg hr $mL^{-1}$, greater than or equal to 1900 ± 25% × $10^{-6}$ kg hr $mL^{-1}$, greater than or equal to 2000 ± 25% × $10^{-6}$ kg hr $mL^{-1}$, or greater than or equal to 2100 ± 25% × $10^{-6}$ kg hr $mL^{-1}$. Optionally, a $dbmnAUC_{0-24h}$ is greater than or equal to 2200 ± 25% × $10^{-6}$ kg hr $mL^{-1}$, greater than or equal to 2400 ± 25% × $10^{-6}$ kg hr $mL^{-1}$, greater than or equal to 2500 ± 25% × $10^{-6}$ kg hr $mL^{-1}$, greater than or equal to 2600 ± 25% × $10^{-6}$ kg hr $mL^{-1}$, greater than or equal to 2700 ± 25% × $10^{-6}$ kg hr $mL^{-1}$, or greater than or equal to 2800 ± 25% × $10^{-6}$ kg hr $mL^{-1}$. Optionally, a $dnAUC_{0-\infty h}$ is greater than or equal to 240 ± 25% × $10^{-6}$ kg hr $mL^{-1}$, or greater than or equal to 250 ± 25% × $10^{-6}$ kg hr $mL^{-1}$, greater than or equal to 300 ± 25% × $10^{-6}$ kg hr $mL^{-1}$, greater than or equal to 35 ± 25% × $10^{-6}$ kg hr $mL^{-1}$, greater than or equal to 400 ± 25% × $10^{-6}$ kg hr $mL^{-1}$, greater than or equal to 500 ± 25% × $10^{-6}$ kg hr $mL^{-1}$, greater than or equal to 600 ± 25% × $10^{-6}$ kg hr $mL^{-1}$, greater than or equal to 700 ± 25% × $10^{-6}$ kg hr $mL^{-1}$, greater than or equal to 800 ± 25% × $10^{-6}$ kg hr $mL^{-1}$, or greater than or equal to 900 ± 25% × $10^{-6}$ kg hr $mL^{-1}$. Optionally, $dbmnAUC_{0-\infty h}$ is greater than or equal to 1000 ± 25% × $10^{-6}$ kg hr $mL^{-1}$, greater than or equal to 1100 ± 25% × $10^{-6}$ kg hr $mL^{-1}$, greater than or equal to 1200 ± 25% × $10^{-6}$ kg hr $mL^{-1}$, greater than or equal to 1300 ± 25% × $10^{-6}$ kg hr $mL^{-1}$, greater than or equal to 1400 ± 25% × $10^{-6}$ kg hr $mL^{-1}$, or greater than or equal to 1500 ± 25% × $10^{-6}$ kg hr $mL^{-1}$. Optionally, a $dbmnAUC_{0-\infty h}$ is greater than or equal to 1600 ± 25% × $10^{-6}$ kg hr $mL^{-1}$, greater than or equal to 1700 ± 25% × $10^{-6}$ kg hr $mL^{-1}$, greater than or equal to 1800 ± 25% × $10^{-6}$ kg hr $mL^{-1}$, greater than or equal to 1900 ± 25% × $10^{-6}$ kg hr $mL^{-1}$, greater than or equal to 2000 ± 25% × $10^{-6}$ kg hr $mL^{-1}$, or greater than or equal to 2100 ± 25% × $10^{-6}$ kg hr $mL^{-1}$. Optionally, a $dbmnAUC_{0-\infty h}$ is greater than or equal to 2200 ± 25% × $10^{-6}$ kg hr $mL^{-1}$, greater than or equal to 2400 ± 25% × $10^{-6}$ kg hr $mL^{-1}$, greater than or equal to 250 ± 25% × $10^{-6}$ kg hr $mL^{-1}$, greater than or equal to 2600 ± 25% × $10^{-6}$ kg hr $mL^{-1}$, greater than or equal to 2700 ± 25% × $10^{-6}$ hr $mL^{-1}$, or greater than or equal to 2800 ± 25% × $10^{-6}$ kg hr $mL^{-1}$. Optionally, a $dbmnAUC_{0-\infty h}$ is greater than or equal to 5000 ± 25% × $10^{-6}$ kg hr $mL^{-1}$, greater than or equal to 10000 ± 25% × $10^{-6}$ kg hr $mL^{-1}$, greater than or equal to 15000 ± 25% × $10^{-6}$ kg hr $mL^{-1}$, greater than or equal to 20000 ± 25% × $10^{-6}$ kg hr $mL^{-1}$, greater than or equal to 25000 ± 25% × $10^{-6}$ kg hr $mL^{-1}$, or greater than or equal to 30000 ± 25% × $10^{-6}$ kg hr $mL^{-1}$. Optionally, a $dbmnAUC_{0-\infty h}$ is greater than or equal to 35000 ± 25% × $10^{-6}$ kg hr $mL^{-1}$, greater than or equal to 40000 ± 25% × $10^{-6}$ kg hr $mL^{-1}$, greater than or equal to 45000 ± 25% × $10^{-6}$ kg hr $mL^{-1}$, greater than or equal to 50000 ± 25% × $10^{-6}$ kg hr $mL^{-1}$, greater than or equal to 55000 ± 25% × $10^{-6}$ hr $mL^{-1}$, or greater than or equal to 60000 ± 25% × $10^{-6}$ kg hr $mL^{-1}$.

A composition disclosed herein can be one that produces a bioequivalent effect to the compositions described herein. Bioequivalence may be determined by AUC, $C_{max}$, $t_{max}$, mean absorption time, metabolite plasma concentration, mean residence time, rate constants, rate profiles, and $C_{max}$ normalized to AUC. An exemplary test for bioequivalence is a confidence interval for $C_{max}$ and/or AUC that is approximately 80%, 85%, 90%, 91%, 92%, 93%, 94%, 95%, 96%, 97%, 98%, 99%, 100%, 101%, 102%, 103%, 104%, 105%, 106%, 107%, 108%, 109%, 110%, 115%, 120%, or 125% of a given compound.

A method disclosed herein is one that can optionally produce a bioequivalent effect to the compositions described herein. Bioequivalence may be determined by AUC, $C_{max}$, $t_{max}$, mean absorption time, metabolite plasma concentration, mean residence time, rate constants, rate profiles, and $C_{max}$ normalized to AUC. An exemplary test for bioequivalence is a confidence interval for $C_{max}$ and/or AUC that is approximately 80%, 85%, 90%, 91%, 92%, 93%, 94%, 95%, 96%, 97%, 98%, 99%, 100%, 101%, 102%, 103%, 104%, 105%, 106%, 107%, 108%, 109%, 110%, 115%, 120%, or 125% of a given compound.

Methods and compositions disclosed herein can optionally allow an administered compound including biologically active metabolites of the compound to be removed from the plasma more quickly than if the compound was administered orally. This is beneficial because the clearance of a compound can aid in the reduction of side effects. For example, if a subject takes a sublingual composition comprising cyclobenzaprine before going to sleep, the cyclobenzaprine may be rapidly absorbed but be substantially metabolized and excreted by the time the subject wakes up, minimizing fatigue, somnolence and grogginess felt upon waking. A plasma level of a compound can optionally decrease by at least 50% of the $C_{max}$ by 4 hours after administration, 5 hours after administration, 6 hours after administration, 7 hours after administration, 8 hours after administration, 9 hours after administration, 10 hours after administration, 11 hours after administration, 12 hours after administration, 13 hours after administration, 14 hours after administration, or 15 hours after administration. For example, a plasma level of a compound decreases by at least 50% of the $C_{max}$ by 4 hours after tmax, by at least 55% of the $C_{max}$ by 4 hours after tmax, by at least 60% of the $C_{max}$ by 4 hours after tmax, by at least 65% of the $C_{max}$ by 4 hours after tmax, by at least 70% of the $C_{max}$ by 4 hours after tmax, by at least 75% of the $C_{max}$ by 4 hours after tmax, by at least 80% of the $C_{max}$ by 4 hours after tmax, by at least 85% of the $C_{max}$ by 4 hours after tmax, by at least 90% of the $C_{max}$ by 4 hours after tmax, by at least 91% of the $C_{max}$ by 4 hours after tmax, by at least 92% of the $C_{max}$ by 4 hours after tmax, by at least 93% of the $C_{max}$ by 4 hours after tmax, by at least 94% of the $C_{max}$ by 4 hours after tmax, by at least 95% of the $C_{max}$ by 4 hours after tmax, by at least 96% of the $C_{max}$ by 4 hours after tmax, by at least 97% of the $C_{max}$ by 4 hours after tmax, by at least 98% of the $C_{max}$ by 4 hours after tmax, or by at least 99% of the $C_{max}$ by 4 hours after $t_{max}$. A plasma level of a compound can optionally decrease by at least 50% of the $C_{max}$ by 8 hours after administration, by at least 55% of the $C_{max}$ by 8 hours after administration, by at least 60% of the $C_{max}$ by 8 hours after administration, by at least 65% of the $C_{max}$ by 8 hours after administration, by at least 70% of the $C_{max}$ by 8 hours after administration, by at least 75% of the $C_{max}$ by 8 hours after administration, by at least 80% of the $C_{max}$ by 8 hours after administration, by at least 85% of the $C_{max}$ by 8 hours after administration, by at least 90% of the $C_{max}$ by 8 hours after administration, by at least 91% of the $C_{max}$ by 8 hours after administration, by at least 92% of the $C_{max}$ by 8 hours after administration, by at least 93% of the $C_{max}$ by 8 hours after administration, by at least 94% of the $C_{max}$ by 8 hours after administration, by at least 95% of the $C_{max}$ by 8 hours after administration, by at least 96% of the $C_{max}$ by 8 hours after administration, by at least 97% of the $C_{max}$ by 8 hours after administration, by at least 98% of the $C_{max}$ by 8 hours after administration, or by at least 99% of the $C_{max}$ by 8 hours after administration. A plasma level of a compound can optionally decrease by at least 50% of the $C_{max}$ by 4 hours after administration, by at least 55% of the $C_{max}$ by 4 hours after administration, by at least 60% of the $C_{max}$ by 4 hours after administration, by at least 65% of the $C_{max}$ by 4 hours after administration, by at least 70% of the $C_{max}$ by 4 hours after administration, by at least 75% of the $C_{max}$ by 4 hours after administration, by at least 80% of the $C_{max}$ by 4 hours after administration, by at least 85% of the $C_{max}$ by 4 hours after administration, by at least 90% of the $C_{max}$ by 4 hours after administration, by at least 91% of the $C_{max}$ by 4 hours after administration, by at least 92% of the $C_{max}$ by 4 hours after administration, by at least 93% of the $C_{max}$ by 4 hours after administration, by at least 94% of the $C_{max}$ by 4 hours after administration, by at least 95% of the $C_{max}$ by 4 hours after administration, by at least 96% of the $C_{max}$ by 4 hours after administration, by at least 97% of the $C_{max}$ by 4 hours after administration, by at least 98% of the $C_{max}$ by 4 hours after administration, or by at least 99% of the $C_{max}$ by 4 hours after administration.

A composition or method disclosed herein can optionally afford an increased $C_{max}$ and a decreased $t_{max}$, in combination with increased clearance of cyclobenzaprine. For example, a composition or method may afford a $C_{max}$ from about 20 to about 200 ng/mL from about 0.05 to about 2.5 hours after administration, while also affording a minimum plasma concentration from about 1 to about 5 ng/mL from about 22 to about 26 hours after administration, wherein the composition is administered for four days or more of daily administration. A composition can optionally be administered within two hours of sleep. A method can optionally be for reducing the symptoms of fibromyalgia in a human patient.

The methods and compositions disclosed herein can optionally allow a compound to be removed from the plasma more quickly than if the compound was administered orally. This is beneficial because the clearance of a compound can aid in the reduction of the accumulation of cyclobenzaprine from the body when administered by nightly dosing and in a chronic dosing schedule. The minimal concentration or $C_{min}$ may be determined by measuring the plasma concentration of the compound being administered can be measured either at fixed time points or at a variable time point, e.g., at time points after the time point corresponding to $C_{max}$, for example 23 hours after $C_{max}$. The $C_{min}$ can be measured after a single dose or after repeated, multiple, or chronic dosing, for example in daily dosing. For example, $C_{min}$ can be measured 3 hours after administration, 4 hours after administration, 5 hours after administration, 6 hours after administration, 7 hours after administration, 8 hours after administration, 9 hours after administration, 10 hours after administration, 11 hours after administration, or 12 hours after administration. $C_{min}$ can optionally be measured 10 hours after administration, 11 hours after administration, 12 hours after administration, 13 hours after administration, 14 hours after administration, 15 hours after administration, 16 hours after administration, 17 hours after administration, 18 hours after administration, 19 hours after administration, 20 hours after administration, 21 hours after administration, 22 hours after administration, 23 hours after administration, 24 hours after administration, or 36 hours after administration. A composition can optionally afford a $C_{min}$ of a compound less than or equal to 10 pg/mL, less than or equal to 11 pg/mL, less than or equal to 12 pg/mL, less than or equal to 13 pg/mL, less than or equal to 14 pg/mL, less than or equal to 15 pg/mL, less than or equal to 16 pg/mL, less than or equal to 17 pg/mL, less than or equal to 18 pg/mL, less than or equal to 19 pg/mL, less than or equal to 20 pg/mL, less than or equal to 21 pg/mL, less than or equal to 22 pg/mL, less than or equal to 23 pg/mL, less than or equal to 24 pg/mL, less than or equal to 25 pg/mL, less than or equal to 26 pg/mL, less than or equal to 27 pg/mL, less than or equal to 28 pg/mL, less than or equal to 29 pg/mL, less than or equal to 30 pg/mL, less than or equal to 31 pg/mL, less than or equal to 32 pg/mL, less than or equal to 33 pg/mL, less than or equal to 34 pg/mL, less than or equal to 35 pg/mL, less than or equal to 36 pg/mL, less than or equal to 37 pg/mL, less than or equal to 38 pg/mL, less than or equal to 39 pg/mL, less than or equal to 40 pg/mL, less than or equal to 50 pg/mL, less than or equal to 60 pg/mL, less than or equal to 70 pg/mL, less than or equal to 80 pg/mL, less than or equal to 90 pg/mL, less than or equal to 100 pg/mL, less than or equal to 120 pg/mL, less than or equal to 140 pg/mL, less than or equal to 160 pg/mL, less than or equal to 180 pg/mL, or less than or equal to 200 pg/mL. A composition can optionally afford a $C_{min}$ of a compound less than or equal to 100 pg/mL, less than or equal to 110 pg/mL, less than or equal to 120 pg/mL, less than or equal to 130 pg/mL, less than or equal to 140 pg/mL, less than or equal to 150 pg/mL, less than or equal to 160 pg/mL, less than or equal to 170 pg/mL, less than or equal to 180 pg/mL, less than or equal to 190 pg/mL, less than or equal to 200 pg/mL, less than or equal to 210 pg/mL, less than or equal to 220 pg/mL, less than or equal to 230 pg/mL, less than or equal to 240 pg/mL, less than or equal to 250 pg/mL, less than or equal to 260 pg/mL, less than or equal to 270 pg/mL, less than or equal to 280 pg/mL, less than or equal to 290 pg/mL, less than or equal to 300 pg/mL, less than or equal to 310 pg/mL, less than or equal to 320 pg/mL, less than or equal to 330 pg/mL, less than or equal to 340 pg/mL, less than or equal to 350 pg/mL, less than or equal to 360 pg/mL, less than or equal to 370 pg/mL, less than or equal to 380 pg/mL, less than or equal to 390 pg/mL, less than or equal to 400 pg/mL, less than or equal to 500 pg/mL, less than or equal to 600 pg/mL, less than or equal to 700 pg/mL, less than or equal to 800 pg/mL, less than or equal to 900 pg/mL, less than or equal to 1.0 ng/mL, less than or equal to 1.20 ng/mL, less than or equal to 1.40 ng/mL, less than or equal to 1.60 ng/mL, less than or equal to 1.80 ng/mL, or less than or equal to 2.00 ng/mL. A composition can optionally afford a $C_{min}$ of a compound less than or equal to 3.0 ng/mL, less than or equal to 4.0 ng/mL, less than or equal to 5.0 ng/mL, less than or equal to 6.0 ng/mL, less than or equal to 7.0 ng/mL, less than or equal to 8.0 ng/mL, or less than or equal to 10.0 ng/mL The minimal concentration at 24 hours or $C_{min(24)}$ may be determined by measuring the plasma concentration of the compound being administered approximately 24 hours after the last dose or immediately prior to the next dose. $C_{min(24)}$ is significant as a plasma value or by calculating the ratio of $C_{min(24)}$ and the dose administered, which is the dose-normalized minimum plasma concentration or $dnC_{min(24)}{}^*$. The $dnC_{min(24)}{}^*$ is calculated by determining the ratio of plasma level to dose administered. For example, in a study (shown in Figure 1) in which 5.0 mg of cyclobenzaprine in an immediate release tablet was administered PO, the mean $C_{min(24)}$ was 1.384 ng/mL at 24 hours and the $dnC_{min(24)}{}^*$ was ((1.384 ng/mL)/(5.0 mg)) = 0.27680 ng/(mg mL), or 0.27680 $\times$ $10^{-6}$ $ml^{-1}$. In another example, in a study in which 2.4 mg of cyclobenzaprine in an sublingual tablet was administered, the mean $C_{min(24)}$ was 706.55 ng/mL at 24 hours and the $dnC_{min(24)}{}^*$ was ((706.55 ng/mL)/(2.4 mg)) = 294.40 ng/(mg mL), or 0.29440 $\times$ $10^{-6}$ $mL^{-1}$. A $dnC_{min(24)}{}^*$ can optionally be less than or equal to 1.0 $\pm$ 25% $\times$ $10^{-6}$ $mL^{-1}$, less than or equal to 0.9 $\pm$ 25% $\times$ $10^{-6}$ $mL^{-1}$, less than or equal to 0.8 $\pm$ 25% $\times$ $10^{-6}$ $mL^{-1}$, less than or equal to 0.7 $\pm$ 25% $\times$ $10^{-6}$ $mL^{-1}$, less than or equal to 0.6 $\pm$ 25% $\times$ $10^{-6}$ $mL^{-1}$, less than or equal to 0.5 $\pm$ 25% $\times$ $10^{-6}$ $mL^{-1}$, less than or equal to 0.4 $\pm$ 25% $\times$ $10^{-6}$ $mL^{-1}$, or less than or equal to 0.3 $\pm$ 25% $\times$ $10^{-6}$ $mL^{-1}$. A $dnC_{min(24)}{}^*$ can optionally be less than or equal to 240 $\pm$ 25% $\times$ $10^{-9}$ $mL^{-1}$, less than or equal to 220 $\pm$ 25% $\times$ $10^{-9}$ $mL^{-1}$, less than or equal to 200 $\pm$ 25% $\times$ $10^{-9}$ $mL^{-1}$, less than or equal to 180 $\pm$ 25% $\times$ $10^{-9}$ $mL^{-1}$, less than or equal to 160 $\pm$ 25% $\times$ $10^{-9}$ $mL^{-1}$, less than or equal to 140 $\pm$ 25% $\times$ $10^{-9}$ $mL^{-1}$, less than or equal to 120 $\pm$ 25% $\times$ $10^{-6}$ $mL^{-1}$, less than or equal to 100 $\pm$ 25% $\times$ $10^{-6}$ $mL^{-1}$, less than or equal to 80 $\pm$ 25% $\times$ $10^{-9}$ $mL^{-1}$, less than or equal to 60 $\pm$ 25% $\times$ $10^{-9}$ $mL^{-1}$, less than or equal to 40 $\pm$ 25% $\times$ $10^{-9}$ $mL^{-1}$, less than or equal to 20 $\pm$ 25% $\times$ $10^{-9}$ $mL^{-1}$, or less than or equal to 10 $\pm$ 25% $\times$ $10^{-9}$ $mL^{-1}$. A $dnC_{min(24)}{}^*$ can optionally be less than or equal to 9 $\pm$ 25% $\times$ $10^{-9}$ $mL^{-1}$, less than or equal to 9 $\pm$ 25% $\times$ $10^{-6}$ $mL^{-1}$, less than or equal to 7 $\pm$ 25% $\times$ $10^{-9}$ $mL^{-1}$, less than or equal to 6 $\pm$ 25% $\times$ $10^{-9}$ $mL^{-1}$, less than or equal to 5 $\pm$ 25% $\times$ $10^{-9}$ $mL^{-1}$, less than or equal to 4 $\pm$ 25% $\times$ $10^{-9}$ $mL^{-1}$, less than or equal to 3 $\pm$ 25% $\times$ $10^{-9}$ $mL^{-1}$, less than or equal to 2 $\pm$ 25% $\times$ $10^{-9}$ $mL^{-1}$, or less than or equal to 1 $\pm$ 25% $\times$ $10^{-9}$ $mL^{-1}$.

The dose- and body mass- normalized $C_{min}$, or the $dbmnC_{min}{}^*$ is calculated by determining the ratio of the product of the plasma level and body mass to dose administered. The $dbmnC_{min}{}^*$ at 24 hours, or $dbmnC_{min(24)}{}^*$, may be determined by measuring the plasma concentration of the compound being administered approximately 24 hours after the last dose or immediately prior to the next dose in a daily dosing schedule, such as a bedtime dosing schedule. The $dbmnC_{min}{}^*$ may be determined by measuring the plasma concentration of the compound being administered can be measured either at fixed time points, for example 24 hours after administration ($C_{min(24)}{}^*$), or at a variable time point, e.g., at time points after the time point corresponding to $C_{max}$, for example 23 hours after $C_{max}$. For example, in a study (shown in Figure 1) in which 5.0 mg of cyclobenzaprine in an immediate release tablet was administered PO, the mean $C_{min(24)}{}^*$ was 1.384 ng/mL at 24 hours and, assuming a 70 kg human, the approximate $dbmnC_{min(24)}{}^*$ was 70 kg $\times$ ((1.384 ng/mL)/(5.0 mg)) = 19.4 $\times$ $10^{-6}$ kg $mL^{-1}$. For example, in a study in which 2.4 mg of cyclobenzaprine in an sublingual tablet was administered, the mean $C_{min(24)}$ was 706.55 ng/mL at 24 hours and the $dnC_{min(24)}{}^*$ was ((706.55 ng/mL)/(2.4 mg)) = 294.40 ng/(mg mL), or 294.40 $\times$ $10^{-9}$ $mL^{-1}$ and, assuming a 70 kg human, the approximate $dbmnC_{min(24)}{}^*$ was 70 kg $\times$ ((706.55 ng/mL)/(2.4 mg)) = 20.608 $\times$ $10^{-6}$ kg $mL^{-1}$. A $dbmnC_{min(24)}{}^*$ can optionally be less than or equal to 1.0 $\pm$ 25% $\times$ $10^{-6}$ kg $mL^{-1}$, less than or equal to 0.9 $\pm$ 25% $\times$ $10^{-6}$ kg $mL^{-1}$, less than or equal to 0.8 $\pm$ 25% $\times$ $10^{-6}$ kg $mL^{-1}$, less than or equal to 0.7 $\pm$ 25% $\times$ $10^{-6}$ kg $mL^{-1}$, less than or equal to 0.6 $\pm$ 25% $\times$ $10^{-6}$ kg $mL^{-1}$, less than or equal to 0.5 $\pm$ 25% $\times$ $10^{-6}$ kg $mL^{-1}$, less than or equal to 0.4 $\pm$ 25% $\times$ $10^{-6}$ kg $mL^{-1}$, or less than or equal to 0.3 $\pm$ 25% $\times$ $10^{-6}$ kg $mL^{-1}$. A $dbmnC_{min(24)}{}^*$ can optionally be less than or equal to 240 $\pm$ 25% $\times$ $10^{-9}$ $mL^{-1}$, less than or equal to 220 $\pm$ 25% $\times$ $10^{-9}$ kg $mL^{-1}$, less than or equal to 200 $\pm$ 25% $\times$ $10^{-9}$ kg $mL^{-1}$, less than or equal to 180 $\pm$

25% $\times$ $10^{-9}$ kg $mL^{-1}$, less than or equal to 160 $\pm$ 25% $\times$ $10^{-9}$ kg $mL^{-1}$, less than or equal to 140 $\pm$ 25% $\times$ $10^{-9}$ kg $mL^{-1}$, less than or equal to 120 $\pm$ 25% $\times$ $10^{-6}$ kg $mL^{-1}$, less than or equal to 100 $\pm$ 25% $\times$ $10^{-6}$ kg $mL^{-1}$, less than or equal to 80 $\pm$ 25% $\times$ $10^{-9}$ kg $mL^{-1}$, less than or equal to 60 $\pm$ 25% $\times$ $10^{-9}$ kg $mL^{-1}$, less than or equal to 40 $\pm$ 25% $\times$ $10^{-9}$ kg $mL^{-1}$, less than or equal to 20 $\pm$ 25% $\times$ $10^{-9}$ kg $mL^{-1}$, or less than or equal to 10 $\pm$ 25% $\times$ $10^{-9}$ kg $mL^{-1}$. A $dbmnC_{min(24)}$* can optionally be less than or equal to 9 $\pm$ 25% $\times$ $10^{-9}$ kg $mL^{-1}$, less than or equal to 9 $\pm$ 25% $\times$ $10^{-6}$ $mL^{-1}$, less than or equal to 7 $\pm$ 25% $\times$ $10^{-9}$ kg $mL^{-1}$, less than or equal to 6 $\pm$ 25% $\times$ $10^{-9}$ kg $mL^{-1}$, less than or equal to 5 $\pm$ 25% $\times$ $10^{-9}$ kg $mL^{-1}$, less than or equal to 4 $\pm$ 25% $\times$ $10^{-9}$ kg $mL^{-1}$, less than or equal to 3 $\pm$ 25% $\times$ $10^{-9}$ kg $mL^{-1}$, less than or equal to 2 $\pm$ 25% $\times$ $10^{-9}$ kg $mL^{-1}$, or less than or equal to 1 $\pm$ 25% $\times$ $10^{-9}$ kg $mL^{-1}$.

The methods and compositions disclosed herein can optionally allow a compound to be absorbed into plasma without gut or hepatic metabolism which reduces the extent of demethylation by p450. This is beneficial because demethylation by p450 transforms cyclobenzaprine into norcyclobenzaprine which has a long half life. Reducing the concentration of the secondary amine metabolite norcyclobenzaprine is beneficial because the tertiary amine cyclobenzaprine is cleared from the plasma and from the body more rapidly and the clearance of a compound can aid in the reduction of the accumulation of compounds (drug plus metabolites) that act on from the body when administered by nightly dosing and in a chronic dosing schedule. The ratio of the plasma concentration of a metabolite to the dose of the agent administered is the dose-normalized concentration of the metabolite or $dnC_{met*}$. The ratio of the plasma concentration of norcyclobenzaprine to the dose of cyclobenzaprine administered is the dose-normalized concentration of norcyclobenzaprine or $dnC_{met}$(norcycl)*. The $dnC_{met}$* may be measured at various times after administration of the compound either after a single dose or after multiple doses. The $dnC_{met}$* can be measured either at fixed time points or at a variable time point, e.g., the time point corresponding to $C_{max}$. For example, $dnC_{met}$* can be measured 5 minutes after administration, 10 minutes after administration, 15 minutes after administration, 30 minutes after administration, 45 minutes after administration, 1 hour after administration, 2 hours after administration, 3 hours after administration, 4 hours after administration, 5 hours after administration, 6 hours after administration, 7 hours after administration, 10 hours after administration, 11 hours after administration, or 12 hours after administration. $dnC_{met}$* can optionally be measured 10 hours after administration, 11 hours after administration, 12 hours after administration, 13 hours after administration, 14 hours after administration, 15 hours after administration, 16 hours after administration, 17 hours after administration, 18 hours after administration, 19 hours after administration, 20 hours after administration, 21 hours after administration, 22 hours after administration, 23 hours after administration, 24 hours after administration, or 36 hours after administration. The $dnC_{met}$* at 24 hours or $dnC_{met\ (24)}$* may be determined by measuring the plasma concentration of the compound being administered approximately 24 hours after the last dose or immediately prior to the next dose in a daily dosing schedule, such as a bedtime dosing schedule. For example, in a study in which 5.0 mg of cyclobenzaprine in an immediate release tablet was administered and the mean plasma concentration of norcyclobenzaprine was 1.227 ng/mL at 24 hours, the $dnC_{met\ (24)}$(norcycl) * is ((1.227 ng/mL)/(5.0 mg)) = 0.245 ng/(mL mg) or 0.245 $\times$ $10^{-6}$ $mL^{-1}$. The $dnC_{met\ (24)}$* for multiple dosing of cyclobenzaprine is expected to be higher. A $dnC_{met(24)}$* can optionally be less than or equal to 1.0 $\pm$ 25% $\times$ $10^{-6}$ $mL^{-1}$, less than or equal to 0.9 $\pm$ 25% $\times$ $10^{-6}$ $mL^{-1}$, less than or equal to 0.8 $\pm$ 25% $\times$ $10^{-6}$ $mL^{-1}$, less than or equal to 0.7 $\pm$ 25% $\times$ $10^{-6}$ $mL^{-1}$, less than or equal to 0.6 $\pm$ 25% $\times$ $10^{-6}$ $mL^{-1}$, less than or equal to 0.5 $\pm$ 25% $\times$ $10^{-6}$ $mL^{-1}$, less than or equal to 0.4 $\pm$ 25% $\times$ $10^{-6}$ $mL^{-1}$, or less than or equal to 0.3 $\pm$ 25% x $10^{-6}$ $mL^{-1}$. A $dnC_{met(24)}$* can optionally be less than or equal to 240 $\pm$ 25% $\times$ $10^{-9}$ $mL^{-1}$, less than or equal to 220 $\pm$ 25% $\times$ $10^{-9}$ $mL^{-1}$, less than or equal to 200 $\pm$ 25% $\times$ $10^{-9}$ $mL^{-1}$, less than or equal to 180 $\pm$ 25% x $10^{-9}$ $mL^{-1}$, less than or equal to 160 $\pm$ 25% $\times$ $10^{-9}$ $mL^{-1}$, less than or equal to 140 $\pm$ 25% $\times$ $10^{-9}$ $mL^{-1}$, less than or equal to 120 $\pm$ 25% $\times$ $10^{-6}$ $mL^{-1}$, less than or equal to 100 $\pm$ 25% $\times$ $10^{-6}$ $mL^{-1}$, less than or equal to 80 $\pm$ 25% $\times$ $10^{-9}$ $mL^{-1}$, less than or equal to 60 $\pm$ 25% $\times$ $10^{-9}$ $mL^{-1}$, less than or equal to 40 $\pm$ 25% $\times$ $10^{-9}$ $mL^{-1}$, less than or equal to 20 $\pm$ 25% $\times$ $10^{-9}$ $mL^{-1}$, or less than or equal to 10 $\pm$ 25% $\times$ $10^{-9}$ $mL^{-1}$. A $dnC_{met(24)}$* can optionally be less than or equal to 9 $\pm$ 25% $\times$ $10^{-9}$ $mL^{-1}$, less than or equal to 9 $\pm$ 25% $\times$ $10^{-6}$ $mL^{-1}$, less than or equal to 7 $\pm$ 25% $\times$ $10^{-9}$ $mL^{-1}$, less than or equal to 6 $\pm$ 25% $\times$ $10^{-9}$ $mL^{-1}$, less than or equal to 5 $\pm$ 25% $\times$ $10^{-9}$ $mL^{-1}$, less than or equal to 4 $\pm$ 25% $\times$ $10^{-9}$ $mL^{-1}$, less than or equal to 3 $\pm$ 25% $\times$ $10^{-9}$ $mL^{-1}$, less than or equal to 2 $\pm$ 25% $\times$ $10^{-9}$ $mL^{-1}$, or less than or equal to 1 $\pm$ 25% $\times$ $10^{-9}$ $mL^{-1}$.

The ratio of the product of the body mass and plasma concentration of norcyclobenzaprine to the dose of cyclobenzaprine administered is the dose-and body mass- normalized concentration of norcyclobenzaprine or $dbmnC_{met}$(norcycl)*. The $dbmnC_{met}$* may be measured at various times after administration of the compound either after a single dose or after multiple doses. The $dbmnC_{met}$* can be measured either at fixed time points or at a variable time point, e.g., the time point corresponding to $C_{max}$. For example, $dbmnC_{met}$* can be measured 5 minutes after administration, 10 minutes after administration, 15 minutes after administration, 30 minutes after administration, 45 minutes after administration, 1 hour after administration, 2 hours after administration, 3 hours after administration, 4 hours after administration, 5 hours after administration, 6 hours after administration, 7 hours after administration, 10 hours after administration, 11 hours after administration, or 12 hours after administration. $dbmnC_{met}$* can optionally be measured 10 hours after administration, 11 hours after administration, 12 hours after administration, 13 hours after administration, 14 hours after administration, 15 hours after administration, 16 hours after administration, 17 hours after administration, 18 hours after administration, 19 hours after administration, 20 hours after administration, 21 hours after administration, 22 hours after administration, 23 hours after administration, 24 hours after administration, or 36 hours after administration. The $dbmnC_{met}$* at 24 hours or $dbmnC_{met(24)}$* may be determined by measuring the plasma concentration of the compound being administered approximately 24 hours after the last dose or immediately prior to the next dose in a daily dosing schedule, such as a bedtime dosing schedule. For example, in a study in which 5.0 mg of cyclobenzaprine in an immediate release tablet was administered and the mean plasma concentration of norcyclobenzaprine was 1.227 ng/mL at 24 hours, and the average body mass of the human is estimated to be 70 kg, the $dbmnC_{met\ (24)}$(norcycl) * is 70 kg × ((1.227 ng/mL)/(5.0 mg)) = 17.2 × $10^{-6}$ kg $mL^{-1}$. The $dbmnC_{met(24)}$* for multiple dosing of cyclobenzaprine is expected to be higher. A $dbmnC_{met(24)}$* can optionally be less than or equal to 1.0 ± 25% x $10^{-6}$ kg $mL^{-1}$, less than or equal to 0.9 ± 25% × $10^{-6}$ kg $mL^{-1}$, less than or equal to 0.8 ± 25% × $10^{-6}$ kg $mL^{-1}$, less than or equal to 0.7 ± 25% × $10^{-6}$ kg $mL^{-1}$, less than or equal to 0.6 ± 25% × $10^{-6}$ kg $mL^{-1}$, less than or equal to 0.5 ± 25% × $10^{-6}$ kg $mL^{-1}$, less than or equal to 0.4 ± 25% × $10^{-6}$ kg $mL^{-1}$, or less than or equal to 0.3 ± 25% × $10^{-6}$ kg $mL^{-1}$. A $dbmnC_{met(24)}$* can optionally be less than or equal to 240 ± 25% × $10^{-9}$ kg $mL^{-1}$, less than or equal to 220 ± 25% × $10^{-9}$ kg $mL^{-1}$, less than or equal to 200 ± 25% × $10^{-9}$ kg $mL^{-1}$, less than or equal to 180 ± 25% × $10^{-9}$ kg $mL^{-1}$, less than or equal to 160 ± 25% × $10^{-9}$ kg $mL^{-1}$, less than or equal to 140 ± 25% × $10^{-9}$ kg $mL^{-1}$, less than or equal to 120 ± 25% × $10^{-6}$ kg $mL^{-1}$, less than or equal to 100 ± 25% × $10^{-6}$ kg $mL^{-1}$, less than or equal to 80 ± 25% × $10^{-9}$ kg $mL^{-1}$, less than or equal to 60 ± 25% × $10^{-9}$ kg $mL^{-1}$, less than or equal to 40 ± 25% × $10^{-9}$ kg $mL^{-1}$, less than or equal to 20 ± 25% × $10^{-9}$ kg $mL^{-1}$, or less than or equal to 10 ± 25% × $10^{-9}$ kg $mL^{-1}$. A $dbmnC_{met(24)}$* can optionally be less than or equal to 9 ± 25% × $10^{-9}$ kg $mL^{-1}$, less than or equal to 9 ± 25% × $10^{-6}$ kg $mL^{-1}$, less than or equal to 7 ± 25% × $10^{-9}$ kg $mL^{-1}$, less than or equal to 6 ± 25% × $10^{-9}$ kg $mL^{-1}$, less than or equal to 5 ± 25% × $10^{-9}$ kg $mL^{-1}$, less than or equal to 4 ± 25% × $10^{-9}$ kg $mL^{-1}$, less than or equal to 3 ± 25% × $10^{-9}$ kg $mL^{-1}$, less than or equal to 2 ± 25% × $10^{-9}$ kg $mL^{-1}$, or less than or equal to 1 ± 25% × $10^{-9}$ kg $mL^{-1}$.

Excipients

A composition of the invention is useful as a medicament. A composition disclosed herein can be used in the manufacture of a medicament. It may be beneficial to include one or more excipients in the compositions disclosed herein. One of skill in the art would appreciate that the choice of any one excipient may influence the choice of any other excipient. For example, the choice of a particular excipient may preclude the use of one or more additional excipient because the combination of excipients would produce undesirable effects. One of skill in the art would be able to empirically determine which additional excipients, if any, to include in the formulations disclosed herein. For example, a compound disclosed herein can optionally be combined with at least one pharmaceutically acceptable carrier such as a solvent, bulking agents, binder, humectant, disintegrating agent, solution retarder, disintegrant, glidant, absorption accelerator, wetting agent, solubilizing agent, lubricant, sweetening agent, or flavorant agent. A "pharmaceutically acceptable carrier" refers to any diluent or excipient that is compatible with the other ingredients of the formulation, and which is not deleterious to the recipient. A pharmaceutically acceptable carrier can be selected on the basis of the desired route of administration, in accordance with standard pharmaceutical practices.

Bulking agents

It may be beneficial to include a bulking agent in the compositions disclosed herein. Bulking agents are commonly used in pharmaceutical compositions to provide added volume to the composition. Bulking agents are well known in the art. Accordingly, the bulking agents described herein are not intended to constitute an exhaustive list, but are provided merely as exemplary bulking agents that may be used in the compositions and methods disclosed herein.

Exemplary bulking agents may include carbohydrates, sugar alcohols, amino acids, and sugar acids. Bulking agents include, but are not limited to, mono-, di-, or poly-, carbohydrates, starches, aldoses, ketoses, amino sugars, glyceraldehyde, arabinose, lyxose, pentose, ribose, xylose, galactose, glucose, hexose, idose, mannose, talose, heptose, glucose, fructose, methyl a-D-glucopyranoside, maltose, lactone, sorbose, erythrose, threose, arabinose, allose, altrose, gulose, idose, talose, erythrulose, ribulose, xylulose, psicose, tagatose, glucosamine, galactosamine, arabinans, fructans, fucans, galactans, galacturonans, glucans, mannans, xylans, inulin, levan, fucoidan, carrageenan, galactocarolose, pectins, amylose, pullulan, glycogen, amylopectin, cellulose, microcrystalline cellulose, pustulan, chitin, agarose, keratin, chondroitin, dermatan, hyaluronic acid, xanthin gum, sucrose, trehalose, dextran, lactose, alditols, inositols, sorbitol, mannitol, glycine, aldonic acids, uronic acids, aldaric acids, gluconic acid, isoascorbic acid, ascorbic acid, glucaric acid, glucuronic acid, gluconic acid, glucaric acid, galacturonic acid, mannuronic acid, neuraminic acid, pectic acids, maize starch, and alginic acid.

Disintegrants

It may be beneficial to include a disintegrant in the compositions disclosed herein. Disintegrants aid in the breakup of solid compositions, facilitating delivery of an active pharmaceutical composition. Disintegrants are well known in the art. Some disintegrants have been referred to as superdisintegrants because they have fast properties, and may be used as disintegrants in the context of this disclosure. Accordingly, the disintegrants described herein are not intended to constitute an exhaustive list, but are provided merely as exemplary disintegrants that may be used in the compositions and methods disclosed herein. Exemplary disintegrants include crospovidone, microcrystalline cellulose, sodium carboxymethyl cellulose, methyl cellulose, sodium starch glycolate, calcium carboxymethyl croscarmellose sodium, polyvinylpyrrolidone, lower alkyl-substituted hydroxypropyl cellulose, Indion 414, starch, pre-gelatinized starch, calcium carbonate, gums, sodium alginate, and Pearlitol Flash®. Pearlitol Flash® (Roquette) is a mannitol-maize starch disintegrant that is specifically designed for orally dispersible tablets (ODT). Certain disintegrants have an efferverscent quality.

Glidants

It may be beneficial to include a glidant in the compositions disclosed herein. Glidants aid in the ability of a powder to flow freely. Glidants are well known in the art. Accordingly, the glidants described herein are not intended to constitute an exhaustive list, but are provided merely as exemplary glidants that may be used in the compositions and methods disclosed herein. Exemplary glidants include colloidal silica (silicon dioxide), magnesium stearate, starch, talc, glycerol behenate, DL-leucine, sodium lauryl sulfate, calcium stearate, and sodium stearate.

Lubricants

It may be beneficial to include a lubricant in the compositions disclosed herein. Lubricants help keep the components of a composition from clumping. Lubricants are well known in the art. Accordingly, the lubricants described herein are not intended to constitute an exhaustive list, but are provided merely as exemplary lubricants that may be used in the compositions and methods disclosed herein. Exemplary lubricants include calcium stearate, magnesium stearate, stearic acid, sodium stearyl fumarate, vegetable based fatty acids, talc, mineral oil, light mineral oil, hydrogenated vegetable oil (e.g., peanut oil, cottonseed oil, sunflower oil, sesame oil, olive oil, corn oil, safflower oil, canola oil, coconut oil and soybean oil), silica, zinc stearate, ethyl oleate, ethyl laurate.

Sweeteners

It may be beneficial to include a sweetener in the compositions disclosed herein. Sweeteners help improve the palatability of the composition by conferring a sweet taste to the composition. Sweeteners are well known in the art. Accordingly, the sweeteners described herein are not intended to constitute an exhaustive list, but are provided merely as exemplary sweeteners that may be used in the compositions and methods disclosed herein. Exemplary sweeteners include, without limitation, compounds selected from the saccharide family such as the mono-, di-, tri-, poly-, and oligosaccharides; sugars such as sucrose, glucose (corn syrup), dextrose, invert sugar, fructose, maltodextrin and polydextrose; saccharin and salts thereof such as sodium and calcium salts; cyclamic acid and salts thereof; dipeptide sweeteners; chlorinated sugar derivatives such as sucralose and dihydrochalcone; sugar alcohols such as sorbitol, sorbitol syrup, mannitol, xylitol, hexa-resorcinol, and the like, and combinations thereof. Hydrogenated starch hydrolysate, and the potassium, calcium, and sodium salts of 3,6-dihydro-6-methyl-1-1,2,3-oxathiazin-4-one-2,2-dioxide many also be used.

Flavorants

It may be beneficial to include a flavorant in the compositions disclosed herein. Flavorants help improve the palatability of the composition by conferring a more desirable taste to the composition. Flavorants are well known in the art. Accordingly, the flavorants described herein are not intended to constitute an exhaustive list, but are provided merely as exemplary flavorants that may be used in the compositions and methods disclosed herein. Exemplary flavorants include, without limitation, natural and/or synthetic (i.e., artificial) compounds such as peppermint, spearmint, wintergreen, menthol, cherry, strawberry, watermelon, grape, banana, peach, pineapple, apricot, pear, raspberry, lemon, grapefruit, orange, plum, apple, lime, fruit punch, passion fruit, pomegranate, chocolate (e.g., white, milk, dark), vanilla, caramel, coffee, hazelnut, cinnamon, combinations thereof, and the like.

Coloring Agents

Coloring agents can be used to color code the composition, for example, to indicate the type and dosage of the therapeutic agent therein. Coloring Agents are well known in the art. Accordingly, the coloring agents described herein are not intended to constitute an exhaustive list, but are provided merely as exemplary coloring agents that may be used in the compositions and methods disclosed herein. Exemplary coloring agents include, without limitation, natural and/or artificial compounds such as FD & C coloring agents, natural juice concentrates, pigments such as titanium oxide, silicon dioxide, and zinc oxide, combinations thereof, and the like.

## Combination therapy

As described above, the compositions and methods disclosed herein may be used to treat PTSD, depression, fibromyalgia, traumatic brain injury, sleep disorder, non-restorative sleep, chronic pain, and anxiety disorder. Any of the methods of treatment described also may be combined with a psychotherapeutic intervention to improve the outcome of the treatment. Exemplary psychotherapeutic interventions directed at either modifying traumatic memories or reducing emotional responses to traumatic memories, including psychological debriefing, cognitive behavior therapy and eye movement desensitization and reprocessing, systematic desensitization, relaxation training, biofeedback, cognitive processing therapy, stress inoculation training, assertiveness training, exposure therapy, combined stress inoculation training and exposure therapy, combined exposure therapy, and relaxation training and cognitive therapy. In each case, the goal of the intervention involves either modifying traumatic memories or reducing emotional responses to traumatic memories. The intended result is generally a improvement in the symptoms of PTSD or the reduction of occurrences of symptoms, as evidenced in terms of physiological responding, anxiety, depression, and feelings of alienation.

A composition disclosed herein can optionally be combined with a drug which may further alleviate the symptoms of PTSD, depression, fibromyalgia, traumatic brain injury, sleep disorder, non-restorative sleep, chronic pain, or anxiety disorder. The drugs include an alpha-1-adrenergic receptor antagonist, a beta-adrenergic antagonist, an anticonvulsant, a selective serotonin reuptake inhibitor, a serotonin-norepinephrine reuptake inhibitor, and an analgesic. Exemplary anticonvulsants include carbamazepine, gabapentin, lamotrigine, oxcarbazepine, pregabalin, tiagabine, topiramate, and valproate. An exemplary alpha-1-adrenergic receptor antagonist is prazosin. Exemplary selective serotonin reuptake inhibitors or serotonin-norepinephrine reuptake inhibitors include, bupropion, citalopram, desvenlafaxine, duloxetine, escitalopram, fluoxetine, escitalopram, fluvoxamine, milnacipran, paroxetine, sertraline, trazodone, and venlafaxine. Exemplary analgesics include pregabalin, gabapentin, acetaminophen, tramadol, and non-steroidal anti-inflammatory drugs (e.g., ibuprofen and naproxen sodium). Additional drugs that can be used in combination with the compositions disclosed herein include sodium oxybate, zolpidem, pramipexole, modafinil, temazepam, zaleplon, and armodafinil.

It is to be understood that the embodiments of the present invention which have been described are merely illustrative of some of the applications of the principles of the present invention..

The following examples are set forth as being representative of the present disclosure. These examples are not to be construed as limiting the scope of the invention.

## Examples

### Reference Example 1

To study cyclobenzaprine metabolism, cyclobenzaprine HCl immediate release (Watson, bioequivalent to Flexeril 5 mg) was administered to 10 healthy human subjects in 5 mg tablets for oral administration and cyclobenzaprine and norcyclobenzaprine plasma concentrations were measured at 0.5 hours, 1 hour, 1.5 hours, 2 hours, 2.5 hours, 3 hours, 3 hours 20 minutes, 3 hours 40 minutes, 4 hours, 4 hours 20 minutes, 4 hours 40 minutes, 5 hours, 5.5 hours, 6 hours, 8 hours, 12 hours, 16 hours, 24 hours, 36 hours, 48 hours, 72 hours, 96 hours, and 168 hours (1 week) (Table 2, Figures 1a and 1b). A high performance liquid chromatographic method for the determination of cyclobenzaprine and norcyclobenzaprine in human EDTA $K_3$ plasma also was developed and validated. The conditions used were as follows:
Mobile Phase A (MPA) and Autosampler Rinsing Solution No. 1: Milli-Q Type Water/Methanol (40/60), Ammonium Formate 5 mM, Formic Acid 0.1% (0.1:100)
Mobile Phase B (MPB) and Autosampler Rinsing Solution No. 2: Methanol (100%)
Buffer Solution: Trizma®Base 500 mM, pH 11.0
Dissolving Solution: Milli-Q Type Water/Methanol (50/50)
Solvent delivery module: Hewlett Packard Series 1100, Agilent (Montréal, Canada)
Chromatographic mode: Reversed phase
Isocratic/gradient mode: Gradient
Time table program:

| Time (min.) | MPA (%) | MPB (%) |
|---|---|---|
| 0.00 | 100 | 0 |
| 0.50 | 100 | 0 |

(continued)

| Time (min.) | MPA (%) | MPB (%) |
|---|---|---|
| 0.51 | 0 | 100 |
| 1.50 | 0 | 100 |
| 1.51 | 100 | 0 |
| 3.50 | 100 | 0 |

Mobile phase A flow rate: 1.000 mL/min
Back-pressure: 130 bars (approximately)

CTC PAL (HTC-xt)* (stator wash station)

Injection macro:

Sample pickup

Needle dip in wash 1

Sample injection

Valve clean with solvent 2

Post clean with solvent 2

Valve clean with solvent 1

Post clean with solvent 1
(stator wash)

Autosampler method:

Air gap volume (mL): 3 Valve clean time solvent 2 (s): 2

Front volume (mL): 0 Post clean time solvent 2 (s): 2

Rear volume (mL): 0 Valve clean time solvent 1 (s): 3

Filling speed (μL/s): 5 Post clean time solvent 1 (s): 3

Pullup delay (ms): 3000 Stator wash: 1

Inject to LC VLV1 Delay stator wash (s): 120

Injection speed (mL/s): 5 Stator wash time solvent 2 (s): 5

Pre inject delay (ms): 500 Stator wash time solvent 1 (s): 5

Post inject delay (ms): 500 Method syringe (μL): 100

Needle gap valve clean (mm): 3

Note: Solvent 1 corresponds to autosampler rinsing solution No. 1 and solvent 2 corresponds to autosampler rinsing solution No. 2. Therefore, the valve, post and stator washes are performed with methanol first and mobile phase A last.

Autosampler loop: 100 μL, stainless steel

Injection volume: 20 μL

Injection temperature: Room temperature

Pre-column filter: Supelco Filter 0.5 μm

Column: Supplier / Manufacturer ACE

Brand / Model ACE 3 C18

Length × width (mm) 30 × 4.6

Particule size (μm) 3

Column temperature: Room temperature

Retention times (Retention times may vary between runs. Retention times obtained without dual injection (ex: cohesive)):

Cyclobenzaprine: 1.05 minutes

Norcyclobenzaprine: 1.16 minutes

Internal standard A: 1.05 minutes

Internal standard B: 1.15 minutes

Autosampler run time: 3.50 minutes (TIME SAVER ON)

Acquisition time: 3.50 minutes

Detector Parameters

Source: TurboIonSpray

Split ratio: not applicable

Ionization mode: Positive

API 5000 (may be modified to optimize chromatography conditions, sensitivity, or reproducibility

Auxiliary gas pressure (GS2): 70 psi
Nebulizer gas pressure (GS1): 50 psi
Curtain gas pressure: 45 psi
CAD gas: 4
Interface heater (Ihe): ON
TurboIonSpray temperature: 450°C
Ion Spray Voltage (ISV): 1800
State file parameters: DP = 70; EP = 10;
(cyclobenzaprine) CE = 55; CXP = 12
State file parameters: DP = 65; EP = 10;
(norcyclobenzaprine) CE = 48; CXP = 12
State file parameters: DP = 70; EP = 10;
(internal standard A) CE = 55; CXP = 12

State file parameters: DP = 65; EP = 10;
(internal standard B) CE = 48; CXP = 12

Mass acquisition parameters:

Analytes: MRM Dwell time = 150 msec; Pause time = 5 msec
IS: MRM Dwell time = 90 msec; Pause time = 5 msec
Cyclobenzaprine 276.4 ® 215.2 amu
Norcyclobenzaprine 262.4 ® 215.2 amu
Internal standard A 279.2 ® 215.1 amu
Internal standard B 265.4 ® 215.2 amu

Integration Parameters (may be changed to optimize peak integration)

| | Cyclo | Norcyclo | Int. Std. A | Int. Std. B |
|---|---|---|---|---|
| Noise Threshold: | 2 | 2 | 1 | 1 |
| Area Threshold: | 4 | 4 | 2 | 2 |
| RT Window (sec): | 30 | 30 | 30 | 30 |
| Bunching factor: | 1 | 2 | 2 | 2 |
| Num. Smooths: | 1 | 1 | 1 | 1 |
| Separation Width: | 0 | 0 | 0 | 0 |
| Separation Height: | 0 | 0 | 0 | 0 |

Calibration Parameters

Peak Attribute: Area

Calibration equation: y = mx + b

Calibration regression: Linear

Weighting factor: 1/C2

Determination factor: r2

Cyclobenzaprine and norcyclobenzaprine were extracted from a 0.200 mL aliquot of human EDTA $K_3$ plasma using an automated liquid-liquid extraction procedure, and then injected into a liquid chromatograph equipped with a tandem mass spectrometry detector. Quantitation was based on peak area ratio of the analytes versus their stable labeled internal standards. A weighted (1/C2), linear regression was performed to determine the concentration of the analytes. All regressions and figures presented in this validation report were generated by MDS Sciex Analyst version 1.4.2 and Thermo Electron Corporation Watson LIMS software, version 7.0.0.01b. The results of method validation were acceptable, which demonstrate that the method is suitable for the determination of cyclobenzaprine and norcyclobenzaprine in human EDTA $K_3$ plasma over the range of 50 to 10000 pg/mL for cyclobenzaprine and 5 to 1000 pg/mL for norcyclobenzaprine. Cyclobenzaprine and norcyclobenzaprine were measured in plasma. Equilibrium receptor binding assays were performed on cell lines expressing recombinant human receptors expressed to determine intrinsic potency of cyclobenzaprine and norcyclobenzaprine on human serotonin 5-HT1a, 5-HT2a, 5-HT2b, 5-HT2c, 5-HT5a, and 5-HT6 receptors, adrenergic $\alpha$-1A, adrenergic $\alpha$-2 (A,B,C), histamine H1, and the muscarinic M1 and M2 receptors. Select receptors were analyzed in ligand-induced intracellular calcium mobilization.

The amount of norcyclobenzaprine in plasma was unexpectedly high and this discovery related to the improved methods that we employed relative to the methods in the literature which did not detect norcyclobenzaprine. In addition, the half-life of norcyclobenzaprine was unexpectedly long. The $t_{max}$ of cyclobenzaprine was calculated to be approximately 3.5 hours ($AUC_{0-\infty h}$ = 103.1 $\pm$ 35.8 ng hr $mL^{-1}$), while the $t_{max}$ of norcyclobenzaprine was calculated to be approximately 24.0 hours ($AUC_{0-\infty h}$ = 169.5 ± 94.3 ng hr $mL^{-1}$). Furthermore, the ratio of cyclobenzaprine to norcyclobenzaprine decreased more rapidly than anticipated, reaching a ratio of 1.1 : 1 within 24 hours and falling to 1 : 2 by 72 hours (Table 3). This is supported by the surprisingly long half life of norcyclobenzaprine, which was calculated to be approximately 73 hours (72.75 ± 27.71 h), as compared to approximately 31 hours (30.95 ± 7.18 h) for cyclobenzaprine. These data indicate that norcyclobenzaprine remains in a subject's system long after most of the cyclobenzaprine has been eliminated. In vitro, cyclobenzaprine and norcyclobenzaprine exhibited high affinity binding ($K_i$) to receptors: 5-HT2a (5.2 and 13 nM) and 5-HT2c (5.2 and 43 nM), adrenergic α-1A (5.6 and 34 nM), α-2B ($K_i$ = 21 and 150 nM) and α-2C ($K_i$ = 21 and 48 nM,); H1 (1.3 nM and 5.6 nM); M1 (7.9 nM and 30 nM). Functionally, norcyclobenzaprine is an antagonist at 5-HT2a ($IC_{50}$ = 92 nM) by Ca+ mobilization. Cyclobenzaprine is a functional antagonist on 5-HT2B ($IC_{50}$ = 100 nM), which is consistent with a lack of association with any heart valve pathology. Cyclobenzaprine and norcyclobenzaprine are functional antagonists on 5-HT2c ($IC_{50}$ = 0.44 μM and 1.22 μM) and on α-2A ($IC_{50}$ = 4.3 μM and 6.4 μM). In contrast, both CBP and nCBP were shown to be functional agonists on 5-HT1a ($EC_{50}$ = 5.3 μM and 3.2 μM).

Table 2: Cyclobenzaprine and norcyclobenzaprine plasma concentrations

| | | | | | | | |
|---|---|---|---|---|---|---|---|
| Hours | 0 | 0.5 | 1 | 1.5 | 2 | 2.5 | 3 |
| Plasma cyclobenzaprine concentration (pg/mL) | Below limit of detection | 102 | 896 | 1999 | 3061 | 3206 | 3416 |
| Plasma norcyclobenzaprine concentration (pg/mL) | Below limit of detection | 5 | 63 | 193 | 391 | 505 | 610 |
| | | | | | | | |
| Hours | 3.33 | 3.67 | 4 | 4.33 | 4.67 | 5 | 5.5 |
| Plasma cyclobenzaprine concentration (pg/mL) | 3634 | 3538 | 3574 | 3680 | 3480 | 3469 | 3245 |
| Plasma norcyclo benzaprine concentration (pg/mL) | 703 | 730 | 753 | 681 | 722 | 773 | 839 |
| | | | | | | | |
| Hours | 6 | 8 | 12 | 16 | 24 | 36 | 48 |
| Plasma cyclobenzaprine concentration (pg/mL) | 3142 | 2578 | 1962 | 1486 | 1384 | 831 | 707 |
| Plasma norcyclo benzaprine concentration (pg/mL) | 928 | 967 | 1114 | 1118 | 1227 | 1166 | 1075 |
| | | | | | | | |
| Hours | 72 | 96 | 168 | | | | |
| Plasma cyclobenzaprine concentration (pg/mL) | 394 | 227 | 26 | | | | |
| Plasma norcyclo benzaprine concentration (pg/mL) | 820 | 659 | 278 | | | | |

Table 3: Cyclobenzaprine : norcyclobenzaprine ratio

| | | | | | | | |
|---|---|---|---|---|---|---|---|
| Hours | 0 | 0.5 | 1 | 1.5 | 2 | 2.5 | 3 |
| Ratio | N/A | 22.5 | 14.3 | 10.4 | 7.8 | 6.4 | 5.6 |
| | | | | | | | |
| Hours | 3.33 | 3.67 | 4 | 4.33 | 4.67 | 5 | 5.5 |
| Ratio | 5.2 | 4.8 | 4.7 | 5.4 | 4.8 | 4.5 | 3.9 |
| | | | | | | | |
| Hours | 6 | 8 | 12 | 16 | 24 | 36 | 48 |

(continued)

| Hours | 0 | 0.5 | 1 | 1.5 | 2 | 2.5 | 3 |
|---|---|---|---|---|---|---|---|
| Ratio | 3.4 | 2.7 | 1.8 | 1.3 | 1.1 | 0.7 | 0.7 |
| | | | | | | | |
| Hours | 72 | 96 | 168 | | | | |
| Ratio | 0.5 | 0.3 | 0.1 | | | | |

**Reference Example 2**

To ensure the accuracy of data collected from *in vivo* studies, it was important to first develop *in vitro* analytical methods for assaying an active pharmaceutical ingredient that may be used in the compositions and methods of the invention. Accordingly, an LC-MS/MS analytical method for assaying cyclobenzaprine in beagle dogs was developed as described below.

Reagents were prepared as follows:

Methanol/UHQ water 20/80 v/v with 0.1% formic acid: 20 mL of methanol (VWR) was mixed with 80 mL of UHQ water (ADME) and 0.1 mL of formic acid (Merck).

Carbonate buffer ($Na_2CO_3$ 0.1 M/$NaHCO_3$ 0.1 M 50/50 v/v (pH 9.8): 2.65g of $Na_2CO_3$ was diluted in 250 mL of UHQ water to make 0.1 M $Na_2CO_3$. 2.1 g of $NaHCO_3$ was diluted in 250 mL of UHQ water to make 0.1 M $NaHCO_3$. The final solution was prepared by mixing 250 mL of 0.1 M $Na_2CO_3$ and 250 mL of 0.1 M $NaHCO_3$.

Analyst® 1.5.1 (Applied Biosystems) software was used to capture and integrate the chromatographic peak area. Watson® 7.2.0.03 (Thermo Electro Corporation) software was used for concentration calculations, data analysis, to store concentration data and statistical calculation. Excel® (2003) (Microsoft) was used for the statistical calculation for carry over.

Labeled cyclobenzaprine ([$^{13}C$, $^{2}H_{3]}$ cyclobenzaprine HCl) (Alsachim) was used as an internal standard (IS) for the cyclobenzaprine HCl (Alsachim) reference sample. Cyclobenzaprine and IS standard solutions were prepared according to Tables 4 and 5 after dissolution of a definite amount in a dark flask in appropriate volume of solvent taking into account the respective corrective factors. The solutions were vortex mixed and sonicated until complete dissolution if necessary.

Table 4: Standard solutions

| | Cyclobenzaprine | Internal Standard |
|---|---|---|
| Stock solution (mg/mL) | 1 | 1 |
| Solvent | Methanol | Methanol |
| Container | Glass | Glass |

These solutions were aliquoted following their preparation, stored at -20°C ± 5°C, and each aliquot was discarded after use.

Table 5: Diluted cyclobenzaprine solutions

| Spiking solution (from Table 4) concentration (μg/mL) | Used volume (mL) | Solvent | Final volume (mL) | Final concentration (μg/mL) |
|---|---|---|---|---|
| 1000 | 0.05 | Methanol | 5 | 10 |
| 10 | 0.25 | Methanol | 5 | 0.5 |
| 10 | 0.2 | Methanol | 5 | 0.4 |
| 10 | 0.15 | Methanol | 5 | 0.3 |
| 10 | 0.125 | Methanol | 5 | 0.25 |

(continued)

| Spiking solution (from Table 4) concentration (μg/mL) | Used volume (mL) | Solvent | Final volume (mL) | Final concentration (μg/mL) |
|---|---|---|---|---|
| 10 | 0.05 | Methanol | 5 | 0.1 |
| 0.5 | 0.5 | Methanol | 5 | 0.05 |
| 0.5 | 0.25 | Methanol | 5 | 0.025 |
| 0.5 | 0.1 | Methanol | 5 | 0.01 |
| 0.1 | 0.15 | Methanol | 5 | 0.003 |
| 0.1 | 0.05 | Methanol | 5 | 0.001 |

Table 6: Diluted internal standard solutions

| Spiking solution concentration (μg/mL) | Used volume (mL) | Solvent | Final volume (mL) | Final concentration (μg/mL) |
|---|---|---|---|---|
| 1000 | 0.025 | Methanol | 2.5 | 10 |
| 10 | 0.125 | Methanol | 5 | 0.25 |

In a polypropylene microcentrifuge tube, 20 μl of the appropriate diluted solution of cyclobenzaprine was added to 200 μl of Heparin lithium Beagle dog plasma (also referred to herein as blank dog plasma) previously centrifuged at 3500 rpm between 0 and 9°C for approximately 5 min as follows:

Table 7: Preparation of calibration standards

| Spiking solution conc. (μg/mL) | 0.001 | 0.003 | 0.01 | 0.025 | 0.05 | 0.1 | 0.25 | 0.3 | 0.4 | 0.5 |
|---|---|---|---|---|---|---|---|---|---|---|
| Volume added (μl) | 20 | 20 | 20 | 20 | 20 | 20 | 20 | 20 | 20 | 20 |
| Volume of blank dog plasma (μl) | 200 | 200 | 200 | 200 | 200 | 200 | 200 | 200 | 200 | 200 |
| Conc. of cyclobenzaprine in plasma (ng/mL) | 0.1 | 0.3 | 1 | 2.5 | 5 | 10 | 25 | 30 | 40 | 50 |

Table 8: Preparation of blank and double blank

| Sample | S0 | S0SI |
|---|---|---|
| Vol. of methanol added (μl) | 20 | 20 |
| Vol. of blank dog plasma (μl) | 200 | 200 |

Table 9: Preparation of quality controls

| Spiking solution used (μg/mL) | 0.001 | 0.003 | 0.25 | 0.4 |
|---|---|---|---|---|
| Vol. of solution added (μl) | 20 | 20 | 20 | 20 |
| Vol. of blank dog plasma (μl) | 200 | 200 | 200 | 200 |
| Conc. of cyclobenzaprine in plasma (ng/mL) | 0.1 | 0.3 | 25 | 40 |

Calibration standards and quality control samples were prepared daily.

The extraction procedure was performed as follows: 20 μl of [$^{13}C$, $^{2}H_{3]}$ cyclobenzaprine at 0.25 μg/mL or 20 μl of methanol for S0 was added to 200 μl of blank dog plasma. To this solution, 200 μl of carbonate buffer was added and vortex mixed for 10 seconds. Subsequently, 1000 μl of hexane was added to this solution and mixed at 180 rpm for 10 minutes before centrifugation at 3500 rpm, between 0 and 9°C for 5 minutes. The organic phase was transferred in a polypropylene tube and dried under a nitrogen stream at 40°C. 200 μl of methanol/water (20/80, v/v) with 0.1 % formic acid was added to the dry residue, which was then sonicated for about 5 minutes and vortex mixed for 10 seconds. The mixture was then transferred into a polypropylene plate, centrifuged at 3500 rpm between 0 and 9°C for 5 minutes, and stored between 0 and 9°C before injection into the LC-MS/MS system. Chromatography was then performed using the parameters shown below.

Table 10: Chromatographic parameters

| |
|---|
| HPLC Column |
| Precolumn |
| Column temperature (°C) |
| Type of column oven |
| Onyx monolithic Phenomenex C 18 100 × 3.0 mm |
| |
| 40 |
| CTO-20AC, Shimadzu |

Table 11: Automatic sampler parameters

| |
|---|
| Type of automatic sampler |
| Injection volume (μl) |
| Automatic sampler temperature (°C) |
| Rinsing Volume (μl) |
| Rinsing solution |
| Rinse Mode |
| SIL-20AC, Shimadzu |
| 10 |
| 4 |
| 300 |
| Acetonitrile/Water (70/30, v/v) |
| After aspiration |

Table 12: Pump parameters

| |
|---|
| Type of pump |
| Flow rate (mL/min) |
| Mobile phase |
| Detection mode |
| Type of detector |
| LC-20AD, Shimadzu |
| 1 |
| Gradient:<br>Time (min) channel A channel B<br>0 30 70<br>1 90 10 |

(continued)

| Type of pump | | |
|---|---|---|
| 1.8 | 90 | 10 |
| 1.9 | 30 | 70 |
| 4 | 30 | 70 |
| Channel A: Methanol + 0.1% formic acid | | |
| Channel B: Water + 0.1% formic acid | | |
| LC-MS/MS | | |
| API4000 | | |

Table 13: Other parameters

| Run time (min) |
|---|
| Retention time<br>Cyclobenzaprine<br>[$^{13}C$,$^{2}H_3$] cyclobenzaprine |
| 4 |
| About 1.6 min<br>About 1.6 min |

Table 14: Comparison of cyclobenzaprine and [$^{13}C$, $^{2}H_3$] cyclobenzaprine in HPLC parameters

| Analyte | Parent (m/z) | Daughter (m/z) | Dwell (msec) | DP (V) | CE(eV) | CXP (V) | EP (V) |
|---|---|---|---|---|---|---|---|
| Cyclobenzaprine | 276.2 | 216.3 | 100 | 70 | 33 | 14 | 10 |
| [$^{13}C$, $^{2}H_3$] cyclobenzaprine | 280.3 | 216.3 | 100 | 70 | 33 | 14 | 10 |

Table 15: Acquisition parameters

| Ionisation mode: ESI+ |
|---|

Table 16: Tandem mass spectrometer tune conditions

| CAD (psi) (collision gas) |
|---|
| CUR (psi) (curtain gas) |
| GS1 (psi) (ion source gas 1) |
| GS2 (psi) (ion source gas 2) |
| IS (V) (ion spray voltage) |
| TEM (°C) (temperature) |
| 4 |
| 25 |
| 50 |
| 60 |
| 5500 |
| 550 |

The data collected during HPLC methods development is described in the following tables. In particular, the data show that there was no carry over of cyclobenzaprine in consecutive runs when comparing cyclobenzaprine peak areas and blank samples (Table 17). The data also show that, when comparing [$^{13}C$, $^{2}H_{3]}$ cyclobenzaprine HCl area and internal standard area in blank samples, carry over is evident only in the first run. However, at 0.1% interference, the extent of carry over is negligible (Table 18).

Table 17: Comparison of cyclobenzaprine areas in lower limit of quantification (LLOQ) sample and blank samples injected after upper limit of quantification (ULOQ) sample

| Run Identification | Area of CYCLOBENZAPRINE peak in S0 | Area of CYCLOBENZAPRINE peak in LLOQ calibration standard (LLOQ) | % interference |
|---|---|---|---|
| 1 | No peak | 1268 | 0.0 |
| 2 | No peak | 1268 | 0.0 |
| 3 | No peak | 1268 | 0.0 |

Table 18: Comparison of [$^{13}C$, $^{2}H_{3]}$ Cyclobenzaprine HCl area in S0SI sample and internal standard area in blank samples injected after ULOQ sample

| Run Identification | Area of $^{13}C$, $^{2}H_{3}$ CYCLOBENZAPRINE peak in S0 | Area of $^{13}C$, $^{2}H_{3}$ CYCLOBENZAPRINE peak in S0SI | % interference |
|---|---|---|---|
| 1 | 255 | 303814 | 0.1 |
| 2 | No peak | 303814 | 0.0 |
| 3 | No peak | 303814 | 0.0 |

Table 19: Back calculated dog plasma cyclobenzaprine concentrations (ng/mL) and regression parameters

| **Cone. (ng/mL)** | **0.1000** | **0.3000** | **1.000** | **2.500** | **5.000** | **10.00** | **25.00** | **30.00** | **40.00** | **50.00** | **Slope** | **Intercept** | **$R^2$** |
|---|---|---|---|---|---|---|---|---|---|---|---|---|---|
| Batch 1 | 0.09639 | 0.3322 | 1.009 | 2.495 | 4.89 | 10.19 | 25.38 | 28.78 | 39.74 | 47.85 | 0.03996 | 0.0005901 | 0.9978 |
| Batch 2 | 0.0940 | 0.3643 | 0.9103 | 2.302 | 5.066 | 10.16 | 26.4 | 29.81 | 40.61 | 45.98 | 0.03914 | 0.001496 | 0.9908 |
| n | 2 | 2 | 2 | 2 | 2 | 2 | 2 | 2 | 2 | 2 | 2 | 2 | 2 |
| Overall Mean | 0.09521 | 0.3483 | 0.9597 | 2.399 | 4.978 | 10.18 | 25.89 | 29.3 | 40.18 | 46.92 | 0.03955 | 0.001043 | 0.9943 |
| % Deviation | -4.79 | 16.1 | -4.03 | -4.04 | -0.44 | 1.8 | 3.56 | -2.33 | 0.45 | -6.16 | | | |

Table 20: Intra-run (repeatability), precision, and deviation of a cyclobenzaprine assay in dog plasma

| Measurement | Curve No. | D 0.1000 ng/mL | E 0.3000 ng/mL | F 25.00 ng/mL | G 40.00 ng/mL |
|---|---|---|---|---|---|
| Individual Runs | 1 | 0.1079 | 0.3143 | 25.03 | 39.07 |
| | | 0.1172 | 0.3253 | 25.57 | 40.04 |
| | | 0.1131 | 0.2969 | 25.51 | 40.26 |
| | | 0.1181 | 0.3312 | 25.95 | 40.11 |
| | | 0.1161 | 0.3419 | 25.05 | 39.61 |
| | | 0.1298 | 0.3295 | 25.58 | 41.21 |
| Intrarun Mean | | 0.117 | 0.3232 | 25.45 | 40.05 |
| Intrarun SD | | 0.007265 | 0.01568 | 0.3525 | 0.7138 |
| Intrarun %CV | | 6.21 | 4.85 | 1.39 | 1.78 |
| Intrarun % Deviation | | 17 | 7.73 | 1.8 | 0.13 |
| N | | 6 | 6 | 6 | 6 |
| Individual Runs | 2 | 0.1013 | 0.31 | 25.9 | 43.98 |
| | | 0.1069 | 0.3208 | 26.7 | 42.82 |
| | | 0.07961 | 0.2957 | 26.34 | 43.4 |
| | | 0.1076 | 0.3177 | 26.01 | 42.63 |
| | | 0.0942 | 0.3312 | 26.49 | 43.95 |
| | | 0.09532 | 0.3486 | 27.41 | 43.44 |
| Intrarun Mean | | 0.09749 | 0.3207 | 26.48 | 43.37 |
| Intrarun SD | | 0.0104 | 0.0181 | 0.5459 | 0.5594 |
| Intrarun %CV | | 10.67 | 5.64 | 2.06 | 1.29 |
| Intrarun % Deviation | | -2.51 | 6.9 | 5.92 | 8.43 |
| N | | 6 | 6 | 6 | 6 |

Table 21: Inter-run (reproducibility), precision, and deviation of a cyclobenzaprine assay in dog plasma

| Curve Number | D 0.1000 ng/mL | E 0.3000 ng/mL | F 25.00 ng/mL | G 40.00 ng/mL |
|---|---|---|---|---|
| 1 | 0.1079 | 0.3143 | 25.03 | 39.07 |
| | 0.1172 | 0.3253 | 25.57 | 40.04 |
| | 0.1131 | 0.2969 | 25.51 | 40.26 |
| | 0.1181 | 0.3312 | 25.95 | 40.11 |
| | 0.1161 | 0.3419 | 25.05 | 39.61 |
| | 0.1298 | 0.3295 | 25.58 | 41.21 |
| 2 | 0.1013 | 0.31 | 25.9 | 43.98 |
| | 0.1069 | 0.3208 | 26.7 | 42.82 |
| | 0.07961 | 0.2957 | 26.34 | 43.4 |
| | 0.1076 | 0.3177 | 26.01 | 42.63 |
| | 0.0942 | 0.3312 | 26.49 | 43.95 |
| | 0.09532 | 0.3486 | 27.41 | 43.44 |
| Mean Concentration Found (ng/mL) | 0.1073 | 0.3219 | 25.96 | 41.71 |
| Inter-run SD | 0.01332 | 0.01619 | 0.6924 | 1.838 |
| Inter-run %CV | 12.41 | 5.03 | 2.67 | 4.41 |
| Inter-run %Deviation | 7.3 | 7.3 | 3.84 | 4.28 |
| N | 12 | 12 | 12 | 12 |

**Reference Example 3**

The LC-MS/MS analytical method described above was validated using acceptance criteria of ± 25% (except for the lower limit of quantification (LLOQ), which was set at an acceptance criterion of ± 30%) with respect to the concentration response relationship and intra- and inter-run precision of deviation. Cyclobenzaprine HCl and the internal standard (IS) [$^{13}C$, $^{2}H_{3]}$ cyclobenzaprine HCl were used, as described above.

For validation of the LC-MS/MS method, the target LLOQ was set to 0.1 ng/mL. The upper limit of quantification (ULOQ) therefore corresponds to a maximum of 500-fold times the LLOQ, or 50 ng/mL. The method required a lithium heparinized plasma sample volume of 200 μl, and the extraction was a liquid liquid extraction with hexane. The extracts were injected using a HPLC system and an AP14000® (Applied Biosystems) for the MS/MS detection, with an "Onyx monolithic Phenomenex C18 100×3.0 mm" column. No data were excluded from the calculations. The difference between the mean concentration observed and the nominal concentration were used to estimate the accuracy of the method. The coefficient of variation was used to estimate the precision of the method.

Blank dog plasma was spiked with cyclobenzaprine HCl at four concentrations: LLOQ, 3 x LLOQ, 0.5 x ULOQ, and 0.8 x ULOQ. The concentration-response relationship was determined from the calibration standards at concentrations ranging from the LLOQ up to the ULOQ on 2 different runs. At least eight calibration points different from zero, prepared the same day of the analysis, were used for each calibration curve. One blank dog plasma samples not spiked (S0) and two spiked only with the internal standard (S0SI) were analyzed with each calibration curve. The weighting factor was determined during the qualification according to the results of calibration curve regression fits. The simplest model that adequately described the concentration-response relationship was used.

Target acceptance criteria included deviation for 75% of the calibration standards that ranged between ± 30.00% of the nominal value for the LLOQ concentration level and between ± 25.00% of the nominal values for the other concentration levels. Deviation % was measured as ((Cmeas - Cn)/Cn) x 100, wherein Cmeas is the measured or back-calculated concentration and Cn is the nominal concentration. The lowest and highest levels were included in the calibration curve, and calibration standards were excluded (except LLOQ and ULOQ) from the final calibration curve only if the back calculated deviation was not comprised within ± 25.00% of the nominal values. Using this method, for each S0 and S0SI sample, interferences at the cyclobenzaprine retention time in the multiple reaction monitoring (MRM) traces specific for cyclobenzaprine should be lower than 30.00% of the cyclobenzaprine peak area in the LLOQ calibration standard, and for each S0 sample, interferences at internal standard retention time in the MRM trace specific for the internal standard should be lower than 2.00% of the internal standard peak area in S0SI. If these criteria are not fulfilled, a documented investigation should be performed on 3 x LLOQ fresh QC samples in order to evaluate a possible impact on cyclobenzaprine measured concentrations to draw a conclusion on the analytical run qualification. A summary of the criteria to include an analytical batch or run is given in Table 22, below.

Table 22: Acceptance criteria

| Series description | QC samples acceptance criteria | Calibration curve acceptance criteria |
|---|---|---|
| Series with 24 fresh QC samples (4 concentration levels in sextuplet) | At least 67% (16 out of 24) of QC samples should have a deviation between ± 25.00% (or ± 30.00% for LLOQ concentration level) with at least 2 QC per level inside the acceptance criteria | At least 75% of calibration standards (including LLOQ and ULOQ) should be taken into account for calculation of calibration curve regression parameters<br>Possible interference peak area at cyclobenzaprine retention time in S0SI and S0 samples should be < 30.00% of cyclobenzaprine peak area in LLOQ calibration standard<br>Possible interference peak area at internal standard retention time in S0 samples should be < 2.00% of internal standard peak area in S0SI sample |

The intra-run and inter-run precision and deviation of the assay method were tested in dog plasma for each concentration level on two different runs. For each concentration, QC samples were extracted in sextuplet within the same run. Precision % was measured as (standard deviation/Cmean) x 100. All QC results were used for precision and mean deviation calculations except if any were rejected for a defined analytical problem. In order to meet the acceptance criteria, precision was calculated for each concentration and should be < 25.00% except for LLOQ where < 30.00% is acceptable (calculated with n=6 for each repeatability test and n=12 for the reproducibility test). The mean deviation also was calculated and should be within ± 30.00% at LLOQ and within ± 25.00% for the other concentrations accepted (calculated with n=6 for each repeatability test and n=12 for the reproducibility test).

One S0 sample was injected three times immediately following the last higher calibration standard (ULOQ) of at least one calibration curve. The MRM chromatograms were examined for the presence of peaks at the retention times of cyclobenzaprine and the IS, and the area of all peaks were measured. In order to meet the acceptance criteria, the peak area obtained at the retention time of the cyclobenzaprine in the MRM trace specific for cyclobenzaprine in each S0 should be less than30.00% of the peak area obtained for cyclobenzaprine in the first calibration standard (LLOQ). The peak area obtained at the retention time of the internal standard in the MRM trace specific for the internal standard in each S0 should be less than 2.00% of the peak area obtained for internal standard in the S0SI.

Cyclobenzaprine concentrations were calculated using Watson® 7.2.0.03 directly from the peak area obtained after automatic integration of chromatograms by Analyst® 1.5.1. Concentrations of the QC samples were calculated by interpolation with the weighted calibration curves prepared in the same conditions and assessed daily after automatic integration. The results of the concentrations, once accepted, were expressed in "ng/mL". Statistics (mean, SD, precision and deviation) were calculated with Watson® 7.2.0.03, except for carry over, which was calculated with Excel®.

**Example 4**

In order to study the effects of the compositions and methods of the invention, a protocol was developed to estimate the levels of unchanged cyclobenzaprine plasma concentrations after a single oral, sublingual or intravenous administration of cyclobenzaprine hydrochloride to a female beagle dog. The protocol was designed as outlined in Table 23.

The *in vivo* protocol uses the analytical method described above to test the cyclobenzaprine HCl, with a calibration range from 0.1 to 50 ng/mL. The method requires a lithium heparinized plasma sample volume of 200 μl. The extraction is a liquid liquid extraction with hexane, and the extracts are injected using an HPLC system and an AP14000® (Applied Biosystems) for the MS/MS detection, with a "Onyx monolithic Phenomenex C18 100x3.0 mm" column.

Table 23: *In vivo* study design

| | |
|---|---|
| Animals | 5 non naive female beagle dogs |
| Compound | Cyclobenzaprine hydrochloride |
| Administration routes | Oral by nasogastric tube (NG) (control)<br>Sublingual (SL)<br>Intravenous (IV) (control) |
| Administered dose | 0.14 mg/kg (corresponding to a 10 mg dose in a 70 kg human) |
| Formulation concentration | NG: 0.028 mg/mL<br>Sublingual: 2.5 mg/mL<br>IV: 0.14 mg/mL |
| Vehicle | Aqueous solution of monopotassium phosphate ($KH_2PO_4$) at pH 7.4 |
| Administration volume | NG: 5 mL/kg<br>Sublingual: 0.056 mL/kg<br>IV: 1 mL/kg |

To check the quality of the method during the biological sample assays, fresh QC samples are prepared at 3 x LLOQ, 0.5 x ULOQ and 0.8 x ULOQ concentration levels, in duplicate. Each assay series consists of one sample of blank lithium heparinized beagle dog plasma not spiked (S0), two samples of blank lithium heparinized beagle dog plasma spiked only with the internal standard (S0SI), eight calibration standards, a minimum of six quality control (QC) samples covering three different concentrations of cyclobenzaprine in duplicate, distributed throughout and at the end of the series, and the biological samples being assayed.

The criteria described hereafter are used to validate calibration curves: deviation for 75% of calibration standards should be ± 25.00% of the nominal value and ± 30.00 % for the LLOQ; the lowest and the highest levels have to be included in the calibration curve; and the calibration standard should be excluded from the final calibration line if the back-calculated concentration was not ± 25.00% of the nominal value. The criteria described hereafter are used to perform an additional investigation if necessary: in each S0SI, possible interferences at retention time of cyclobenzaprine should be lower than 30.00% of the cyclobenzaprine peak area in the LLOQ calibration standard. If these criteria are not met, an investigation should be performed on pre-dose and 3 x LLOQ QC samples in order to evaluate a possible impact on measured cyclobenzaprine concentrations and, therefore, on the analytical run validation.

A series is considered validated if at least 67% of QC samples have a deviation range of ± 25.00% of the nominal concentrations. Additionally, any rejected QC samples should not correspond to the last analyzed QC samples of the series. Therefore, only concentrations measured between validated QC samples are considered validated. If dilutions are necessary, diluted QCs will be added in order to validate the dilution procedure. Diluted concentrations will be validated if at least 1 QC out of 2 has a deviation range of ± 25.00% of the nominal concentration.

Concentrations of the samples are calculated using Watson® 7.2.0.03 directly from chromatograms after automatic integration by Analyst® 1.5.1 and expressed as ng/mL. Mean plasma concentrations will be calculated (when calculable, i.e. $n \geq 2$) using individual concentrations and will be expressed with the corresponding standard deviation value and variation coefficients (when calculable, i.e. $n \geq 3$) (with CV(%) = (SD/mean) x 100). The individual plasma concentrations are tabulated for each dog and scheduled sampling time. Concentrations below the LLOQ are designated as "BLQ." All BLQ concentrations are substituted by zero for calculation of the descriptive statistics of the concentrations.

Pharmacokinetic analysis is carried out using KINETICA® (Version 4.3 (Thermo Electron Corporation)). An independent model (non compartmental analysis) is used. All BLQ values in the absorption phase are substituted by zero, except for BLQ values between evaluable concentrations, which are treated as missing values, before the calculation of the pharmacokinetic variables. The terminal BLQ values are ignored. The following pharmacokinetic parameters are calculated:

$C_{max}$ (ng/mL): observed maximal plasma concentration (for oral and sublingual administration)

$T_{max}$ (h): time since administration at which maximum plasma concentration is found (for oral and sublingual administration).

AUCt (ng/(mL x h)): area under the plasma concentration time curve from administration to the last observed concentration at time t measured by trapezoidal rules.

$AUC_{inf}$ (ng/mL x h): area under the plasma concentration time curve from administration up to infinity with extrapolation of the terminal phase. This can also be represented as $AUC_{0-\infty}$.

$\%AUC_{extra}$: percentage of extrapolated AUC, calculated as ($AUC_{inf}$-$AUC_t$/$AUC_{inf}$) x 100

$T_{1/2}$* (h): half-life of elimination, calculated as ln2/$K_{el}$

$K_{el}$* (1/h): estimated by the linear regression of the logarithm of the terminal concentration as a function of time using Kinetica® software.

Cl and Cl/F* (L/h): apparent plasma clearance, calculated as dose/$AUC_{inf}$

Vd and Vd/F* (L): apparent volume of distribution, calculated as Cl/$K_{el}$

$$\text{Absolute bioavailability F\% (\%)} = ((\text{AUC by extravascular administration})/\text{AUC by IV administration}) \times 100$$

*These parameters are calculated only if $\%AUC_{extra}$ is lower than 20% and if the elimination phase contains three time points.

**Example 5**

To test the effects of the methods and compositions of the invention described herein, a protocol was designed for the administration of cyclobenzaprine HCl in beagle dogs. The protocol is described below.

5 non-naive female dogs are used, each dog receiving the test substance by oral administration via nasogastric tube to the stomach (NG) administration (control), sublingual administration (SL), and by intravenous (IV) administration (control). There is a "wash-out" period of at least two weeks between each type of administration. Blood samples are drawn as follows:

Session 1: oral administration (control)

A single NG dose of 0.14 mg/kg (under a volume of 5 ml/kg and a solution concentration of 0.028 mg/mL) is administered. Blood samples are taken pre-dosing, and then at 30 min, 1, 2, 3, 4, 5, 6, 8, 10, 12 and 24 hours after administration (for a total of 12 blood samples per animal).

Session 2: sublingual administration

After a wash-out period of at least 2 weeks, dogs are sedated using propofol (6.5 mg/kg IV). They will be then given a single sublingual dose of 0.14 mg/kg (under a volume of 0.056 mL/kg and a solution concentration of 2.5 mg/mL). Blood samples are taken pre-dosing, and then at 10 min, 20 min, 30 min, 1, 2, 3, 4, 6, 8, 10 and 24 hours after administration (for a total of 12 blood samples per animal). After administration, animals do not have any access to water for 30 minutes.

Session 3: intravenous administration (control)

After a wash-out period of at least 2 weeks, dogs are given a single IV dose of 0.14 mg/kg (under a volume of 1 mL/kg, bolus of approximately 30 sec, and a solution concentration of 0.14 mg/mL). Blood samples are taken pre-dosing, and then at 10 min, 20 min, 30 min, 1, 2, 3, 4, 6, 8, 10 and 24 hours after administration (for a total of 12 blood samples per animal).

Blood sampling was designed to minimize animal suffering and to ensure the quality of the biological samples, and was adapted from basic procedures commonly used in studies performed in dogs. Serial blood samples (1 tube of approximately 5 mL) are collected from a jugular vein using vacuum tubes containing lithium heparin. After sealing each tube, the blood samples are manually agitated and stored on ice until centrifugation (within 30 minutes of sampling). The samples are centrifuged at 1500 g, at 4°C, for 10 minutes. The entire resultant plasma obtained from each tube is immediately transferred to suitably labeled polypropylene tubes (3 aliquots of plasma of at least 500 μl each), which are stored upright at approximately -80°C and protected from light until bioanalysis.

The dogs are fasted overnight before each administration, and food is given to the dogs 4 hours after each treatment. The cyclobenzaprine HCl is dosed at 0.14 mg/kg for each of the three routes of administration (PO, sublingual, and IV). In each of the three routes, a potassium phosphate buffer (pH 7.4) is used as the vehicle.

Female beagle dogs, weighing 12 - 18 kg, obtained from HARLAN or CEDS are used in these trials. The dogs are housed in groups in a kennel with free access to food and water, under natural lighting and in a controlled ambient temperature of 18 ± 3°C. During the pharmacokinetic phase, dogs are housed singly in a floor area of approximately 1 or 2 $m^2$. During this phase, the animal house is maintained under artificial lighting (12 hours) between 7:00 and 19:00 in a controlled ambient temperature of 18 ± 3°C.

**Example 6**

The solubility of cyclobenzaprine HCl was tested both in purified water (control) (Table 24) and in an aqueous solution containing monobasic potassium phosphate ($KH_2PO_4$) with NaOH to adjust the solution to pH 7.4. The ($KH_2PO_4$) solution was prepared according to the current USP (USP 34) (Table 25). Briefly, 50 mL of 0.2 M monobasic potassium phosphate ($KH_2PO_4$) was mixed with 39.1 mL of 0.2 M NaOH, and water was added to bring the solution to a final volume of 200 mL. During the test, the change in the pH of the solution also was measured for each addition. The test was performed on a 100 mL sample, adding 5 g of cyclobenzaprine HCl each time to the initial aliquot of 10 g. Each pH value was measured only after complete dissolution of the added quantity of cyclobenzaprine HCl. The measured values are reported in the tables below. The solubility data for cyclobenzaprine HCl reported in the literature (30g for 100 g of water) was consistent with use of the aqueous $KH_2PO_4$ solution (pH 7.4) as a solvent.

Table 24: Dissolution in purified water (volume = 100 mL) (control)

| Grams of cyclobenzaprine HCl added | pH value | Molarity |
|---|---|---|
| 0 | 5.80 | 0 |
| 10 | 3.31 | 0.32 |
| 15 | 3.24 | 0.48 |
| 20 | 3.16 | 0.64 |
| 25 | 3.11 | 0.80 |

(continued)

| Grams of cyclobenzaprine HCl added | pH value | Molarity |
|---|---|---|
| 30 | 3.07 | 0.96 |

Table 25: Dissolution in Phosphate Buffer Solution pH 7.4 (volume = 100 mL)

| Grams of cyclobenzaprine HCl added | pH value | Molarity |
|---|---|---|
| 0 | 7.37 | 0 |
| 10 | 6.43 | 0.32 |
| 15 | 6.29 | 0.48 |
| 20 | 6.21 | 0.64 |
| 25 | 6.15 | 0.80 |
| 30 | 6.10 | 0.96 |

According to the protocol relative to the pre-clinical study described above, the administered solution for a sublingual route had to be at a concentration of 2.5 mg/mL (solvent: aqueous $KH_2PO_4$ solution, pH 7.4, as described above). The volume of the cyclobenzaprine HCl solution administered was 0.056 mL/kg. Looking to the available literature, and in anticipation of gathering data after the setup of the preliminary formulation, we hypothesized an average weight of 10 kg for each dog. The quantity of cyclobenzaprine HCl solution administered under the tongue was therefore 0.56 mL, equivalent to 1.4 mg of cyclobenzaprine HCl.

In order to simulate the ambient pH under the animal tongue, artificial saliva was prepared taking into account the standard procedure in the available literature (see, for example, UNI EN 12868:2002), with some modifications. Briefly, 4.2 g of sodium bicarbonate, 500 mg of sodium chloride, 200 g of potassium carbonate and 30 mg of sodium nitrite were dissolved into 900 mL of purified water under agitation with a magnetic stirrer. The final pH of the solution was between about pH 8 and pH 9, which was acidified to approximately pH 5.5 using lactic acid.

In order to consider different possible volumes of saliva under the tongues of the animals, the 0.56 mL of cyclobenzaprine HCl solution (solvent: $H_2O$; basifying agent: aqueous $KH_2PO_4$ solution, pH 7.4, as described above) corresponding to the dose administered to the dogs, was added to 1 mL, 3 mL and 5 mL of saliva, and the pH values were measured as described in Table 26. The same pH measurements were performed adding the formulation without the basifying agent to the saliva instead of the cyclobenzaprine HCl alone (Table 24).

Table 26: pH measurements of a cyclobenzaprine HCl solution in artificial saliva

| Solution corresponding to the dose (0.56 mL of aqueous solution containing $KH_2PO_4$ with 2.5 mg/mL of cyclobenzaprine HCl) | 1 mL artificial saliva | 3 mL artificial saliva | 5 mL artificial saliva |
|---|---|---|---|
| | pH=7.19 | pH=7.04 | pH=6.73 |

Table 27: pH measurements of a cyclobenzaprine HCl tablet in artificial saliva (control: lacking a basifying agent)

| 1 tablet (corresponding to 2.4 mg of cyclobenzaprine HCl) | 1 mL artificial saliva | 3 mL artificial saliva | 5 mL artificial saliva |
|---|---|---|---|
| | pH=5.40 | pH=5.47 | pH=5.51 |

In order to increase the pH under the tongue after the administration of the tablet to a pH value as similar as possible to the aqueous $KH_2PO_4$ solution, pH 7.4, as described above, $K_2HPO_4$ was added to the tablet formulation. The quantity had been determined by pH trials on solutions obtained by dissolving the tablets of the formulation lacking the $K_2HPO_4$ basifying agent in the artificial saliva. By adding 1.05 mg of $K_2HPO_4$ to the control formulation (lacking the $K_2HPO_4$ basifying agent), the results depicted in Table 28 were obtained.

Table 28: pH measurements of a cyclobenzaprine HCl tablet in artificial saliva (basifying-agent-containing)

| 1 tablet (corresponding to 2.4 mg of cyclobenzaprine HCl) | 1 mL artificial saliva | 3 mL artificial saliva | 5 mL artificial saliva |
|---|---|---|---|
| | pH=7.11 | pH=6.83 | pH=6.56 |

**Example 7**

An exemplary composition formulated for sublingual administration is a sublingual tablet designed to quickly disintegrate under the tongue. To develop this type of composition, the galenic properties of cyclobenzaprine HCl were studied. A solubility test confirmed that slightly basic media (e.g., aqueous $KH_2PO_4$ solution, pH 7.4, described above) is a possible solvent for cyclobenzaprine HCl.

The specifications for sublingual dosage forms are not defined by pharmacopoeias, so a preliminary pool of excipients was selected in order to obtain a tablet with a disintegration time in compliance with USP specifications referred to an oral dispersible forms (disintegration in less than 30 seconds). This specification is one target for formulations, but is not a mandatory specification. On the basis of this characteristic, a disintegrant (Crospovidone) and a highly palatable disintegrant (Pearlitol Flash) had been selected. On the basis of the characteristics of the aqueous $KH_2PO_4$ solution, pH 7.4, described above, a stoichiometric ratio of $K_2HPO_4$ was introduced in one of the preliminary formulations as basifying agent. In addition, a formulation lacking a basifying agent was produced. For each formulation approach, batches of cyclobenzaprine HCl from two different suppliers were tested with the aim of selecting the best performing batch for further testing. The final mixing was obtained in both cases by dry mixing, adding a lubricant only in the last mixing step. Due to the low concentration of the active ingredient, a progressive dilution method was applied.

The tabletting phase was performed on a GP1 tabletting machine equipped with a 4mm punch. The punch selection took into account the method of administration, as it affects both the diameter and the shape of the tablet in order to comply with the sublingual route and transmucosal absorption. Final mixing and the corresponding tablets were tested for all the standard parameters as reported here below. Two formulations containing the $K_2HPO_4$ as a basifying agent were manufactured as described in Table 35. These formulations differed only in that they use cyclobenzaprine HCl from different suppliers. The analytical results of each formulation is summarized in Table 36.

Table 35: Basifying-agent-containing cyclobenzaprine HCl formulations

| Component | Commercial Name | Function | Supplier | Prototype A (mg/tab) | Prototype B (mg/tab) |
|---|---|---|---|---|---|
| Cyclobenzaprine HCl | Cyclobenzaprine HCl | Active Ingredient | Dipharma | 2.40 | - |
| Cyclobenzaprine HCl | Cyclobenzaprine HCl | Active Ingredient | Sifavitor | - | 2.40 |
| Mannitol and Maize starch | Pearlitol Flash | Disintegrant | Roquette | 31.55 | 31.55 |
| Crospovidone | Kollidon CL | Disintegrant | BASF | 2.00 | 2.00 |
| Colloidal Silica | Aerosil 200 | Glidant | Evonik | 0.50 | 0.50 |
| Sodium Stearyl Fumarate | Lubrisanaq | Lubricant | Pharma Trans Sanaq | 0.50 | 0.50 |
| Dipotassium hydrogen phosphate | Dipotassium hydrogen phosphate | Basifying agent | VWR | 1.05 | 1.05 |
| Total Weight | | | | 38.00 | 38.00 |

Table 36: Analytical results from basifying-agent-containing cyclobenzaprine HCl formulations

| Analysis | Specifications | Prototype A | Prototype B |
|---|---|---|---|
| Powder | | | |

(continued)

| Analysis | Specifications | Prototype A | Prototype B |
|---|---|---|---|
| Distribution | TBD | Assay= 96.2%<br>SD= 3.5<br>RSD= 3.7% | Assay= 99.9%<br>SD= 6.5<br>RSD= 6.5% |
| Bulk Density | / | 0.53 g/mL | 0.50 g/mL |
| Tapped Density (300 strokes) | / | 0.60 g/mL | 0.59 g/mL |
| Carr's Index | / | 12.28% | 15.00% |
| Hausner Ratio | / | 1.14 | 1.18 |
| Tablets | | | |
| Uniformity of Content | AV <15 | 13.1 | 13.7 |
| Assay | 95-105 % | 99.4% | 103.8% |
| Purity :<br>Impurity 1<br>Impurity 2<br>Impurity 3<br>Impurity 4<br>Total unknown<br>Each other impurity | TBD | Absent<br>Absent<br>Absent<br>0.097<br>Absent<br>0.097 | Absent<br>Absent<br>Absent<br>0.129<br>Absent<br>0.129 |
| Disintegration test | TBD | 54" | 40" |
| Hardness | To be Defined | 32.40N | 20.20N |
| Friability Test | < 1.0 % | 0.3% | 0.3% |
| KF | To be Defined | 1.50% | 1.36% |
| * Preliminary analytical conditions were applied to these preliminary trials; TBD: to be determined | | | |

Formulations lacking the $K_2HPO_4$ basifying agents (control) were manufactured as described below in Table 37. The analytical results from these formulations are described in Table 38.

Table 37: Cyclobenzaprine HCl formulations lacking a basifying agent (control)

| Component | Commercial Name | Function | Supplier | Prototype C (mg/tab) | Prototype D (mg/tab) |
|---|---|---|---|---|---|
| Cyclobenzaprine HCl | Cyclobenzaprine HCl | Active Ingredient | Dipharma | 2.40 | - |
| Cyclobenzaprine HCl | Cyclobenzaprine HCl | Active Ingredient | Sifavitor | - | 2.40 |
| Mannitol and Maize starch | Pearlitol Flash | Disintegrant | Roquette | 32.60 | 32.60 |
| Crospovidone | Kollidon CL | Disintegrant | BASF | 2.00 | 2.00 |
| Colloidal Silica | Aerosil 200 | Glidant | Evonik | 0.50 | 0.50 |
| Sodium Stearyl Fumarate | Lubrisanaq | Lubricant | Pharma Trans Sanaq | 0.50 | 0.50 |
| Total weight | | | | 38.00 | 38.00 |

Table 38: Analytical results from basifying-agent-containing cyclobenzaprine HCl formulations

| Analyses | Specifications | Prototype C | Prototype D |
|---|---|---|---|
| Powder | | | |
| Distribution | TBD | Assay= 102.8% SD= 2.3 RSD= 2.2% | Assay= 102.2% SD= 2.6 RSD= 2.5% |
| Bulk Density | / | 0.52 g/mL | 0.51 g/mL |
| Tapped Density (300 strokes) | / | 0.59 g/mL | 0.58 g/mL |
| Carr's Index | / | 12.07% | 11.86% |
| Hausner Ratio | / | 1.14 | 1.14 |
| Tablets | | | |
| Uniformity of Content | AV <15 | 4.7 | 7.5 |
| Assay | 95-105 % | 102.2% | 98.8% |
| Purity :<br>Impurity 1<br>Impurity 2<br>Impurity 3<br>Impurity 4<br>Each other impurity<br>Total impurities | TBD | <br>Absent<br>Absent<br>Absent<br>0.085<br>Absent<br>0.085 | <br>Absent<br>Absent<br>Absent<br>0.115<br>Absent<br>0.115 |
| Disintegration test | TBD | 1' 5" | 42" |
| Hardness | To be Defined | 31.4N | 20.80N |
| Friability Test | < 1.0 % | 0.2% | 0.3% |
| KF | To be Defined | 1.41% | 1.31% |
| *Preliminary analytical conditions were applied to these preliminary trials; TBD: to be determined | | | |

On the basis of the distribution, assays and uniformity of content results, both formulations were identified as suitable.

**Example 8**

In order to evaluate the stability of the formulations described above, prototype A, B, C, D were maintained under the 50°C stress condition for 30 days. The results are described below in Tables 39 and 40, respectively. Cyclobenzaprine HCl, without excipients, also was tested from each manufacturer over the course of 15 days, as shown in Table 41. In sum, the formulations were stable, especially considering the high-stress storage conditions. Differences between the formulation containing the $K_2HPO_4$ basifying agents and the formulation lacking the $K_2HPO_4$ basifying agents (control) were minimal.

Table 39: Basifying-agent-containing cyclobenzaprine HCl formulations at 50°C, for 30 days

| Purity Profile of Prototypes (%) | **Basifying-age proto nt-containing type A** | | **Basifying-age proto ent-containing type B** | |
|---|---|---|---|---|
| | T0* | T30days | T0* | T30days |
| Impurity 1 (RT = about 56') | Absent | Absent | Absent | Absent |
| Impurity 2 (RT = about 6') | Absent | 0.006% | Absent | 0.008% |
| Impurity 4 (RT = about 15') | Absent | Absent | Absent | Absent |
| Each Unknown Impurity | | | | |

(continued)

| Purity Profile of Prototypes (%) | **Basifying-age proto nt-containing type A** | | **Basifying-age proto ent-containing type B** | |
|---|---|---|---|---|
| -Unknown 3 (RT= about 7.5') | Absent | 0.014% | Absent | 0.010% |
| -Unknown 5 RT=about 11.1') | 0.003% | 0.018% | 0.003% | 0.019% |
| -Unknown 1 (RT = about 16') | 0.034% | 0.002% | 0.028% | 0.011% |
| -Unknown 2 (RT= about 17.5') | Absent | 0.113% | Absent | 0.107% |
| -Unknown 4 (RT = about 26') | Absent | Absent | Absent | Absent |
| Total Impurities | 0.037% | 0.155% | 0.031% | 0.147% |
| *Data obtained on samples stored at room temperature | | | | |

Table 40: Cyclobenzaprine HCl formulations without basifying agent at 50°C, for 30 days

| Purity Profile of Prototypes (%) | **Prototype C** | | **Prototype D** | |
|---|---|---|---|---|
| | T0* | T30days | T0* | T30days |
| Impurity 1 (RT = about 56') | Absent | Absent | Absent | Absent |
| Impurity 2 (RT = about 6') | 0.004% | 0.006% | 0.005% | 0.008% |
| Impurity 4 (RT = about 15') | Absent | Absent | Absent | Absent |
| Each Unknown Impurity<br>-Unknown 3 (RT= about 7.5')<br>-Unknown 5 (RT=about 11.1')<br>-Unknown 1 (RT = about 16')<br>-Unknown 2 (RT= about 17.5')<br>-Unknown 4 (RT = about 26') | 0.003%<br>0.002%<br>Absent<br>0.037%<br>0.011% | 0.011%<br>0.017%<br>Absent 0.040%<br>Absent% | Absent<br>0.004%<br>0.018%<br>0.019%<br>0.004% | 0.009%<br>0.018%<br>0.011%<br>0.163%<br>Absent% |
| Total Impurities | 0.053% | 0.068% | 0.045% | 0.068% |
| *Data obtained on samples stored at room temperature | | | | |

Table 41: Cyclobenzaprine HCl active ingredient from two manufacturers

| Purity Profile of APIs (%) | **Cyclobenzaprine HCl (Sifavitor)** | | **Cyclobenzaprine HCl (Dipharma)** | |
|---|---|---|---|---|
| | T0* | T15days | T0* | T15days |
| Impurity 1 (RT = about 56') | Absent | Absent | Absent | Absent |
| Impurity 2 (RT = about 6') | Absent | Absent | Absent | Absent |
| Impurity 4 (RT = about 15') | Absent | Absent | Absent | Absent |
| Each Unknown Impurity<br>-Unknown 3 (RT= about 7.5')<br>-Unknown 1 (RT = about 16')<br>-Unknown 2 (RT = about 17.5') | Absent<br>0.020%<br>Absent | Absent<br>Absent<br>0.019% | 0.028%<br>0.005%<br>Absent | 0.008%<br>Absent<br>0.003% |
| Total Impurities | 0.020% | 0.019% | 0.005% | 0.003% |
| *Data obtained on samples stored at room temperature | | | | |

As described above, the micronized cyclobenzaprine HCl supplied by Dipharma and the un-micronized cyclobenzaprine HCl supplied by Sifavitor are equivalent in terms of their galenic activities and properties. In order to verify this conclusion, a further trial was designed using un-micronized cyclobenzaprine HCl supplied by Dipharma in both the formulations (with and without the $K_2HPO_4$ basifying agent). This formulation was created to compare the same characteristics of the un-micronized cyclobenzaprine HCl supplied by two different manufacturers (Table 42). The formulation then was analyzed and compared with the previous formulations as shown in Table 43.

Table 42: Un-micronized cyclobenzaprine HCl formulation (basifying-agent-containing)

| Component | Commercial Name | Function | Supplier | Cyclobenzaprine HCl (not micronized) (mg/tab) |
|---|---|---|---|---|
| Cyclobenzaprine HCl | Cyclobenzaprine HCl | Active Ingredient | Dipharma | 2.40 |
| Mannitol and Maize starch | Pearlitol Flash | Disintegrant | Roquette | 31.55 |
| Crospovidone | Kollidon CL | Disintegrant | BASF | 2.00 |
| Colloidal Silica | Aerosil 200 | Glidant | Evonik | 0.50 |
| Sodium Stearyl Fumarate | Lubrisanaq | Lubricant | Pharma Trans Sanaq | 0.50 |
| $K_2HPO_4$ | $K_2HPO_4$ | Basifying agent | VWR | 1.05 |
| Total weight | | | | 38.00 |

Table 43: Analysis of un-micronized cyclobenzaprine HCl formulation (containing $K_2HPO_4$ basifying agent)

| Analyses | Specifications | Cyclobenzaprine HCl formulation (Dipharma, not micronized) | Cyclo benzaprine HCl formulation (Dipharma, micronized) | Cyclobenzaprine HCl formulation (Sifavitor, not micronized) |
|---|---|---|---|---|
| Distribution | TBD | Assay= 97.5%<br>SD= 4.5<br>RSD= 4.6% | Assay= 96.2%<br>SD= 3.5<br>RSD= 3.7% | Assay= 99.9%<br>SD= 6.5<br>RSD= 6.5% |
| Tablets | | | | |
| Assay | 95-105 % | 101.5% | 99.4% | 103.8% |
| Disintegration test | < 30" | 17" | 54" | 40" |
| Hardness | TBD | 27.20N | 32.40N | 20.20N |

The control cyclobenzaprine HCl formulations (lacking a basifying agent) described above were duplicated, substituting un-micronized cyclobenzaprine HCl for micronized cyclobenzaprine HCl (Table 44). The formulation then was analyzed and compared with the previous formulations lacking the $K_2HPO_4$ basifying agent as shown in Table 45.

Table 44: Un-micronized cyclobenzaprine HCl formulation (lacking the basifying agent)

| Component | Commercial Name | Function | Supplier | Cyclobenzaprine HCl (not micronized) (mg/tab) |
|---|---|---|---|---|
| Cyclobenzaprine HCl | Cyclobenzaprine HCl | Active Ingredient | Dipharma | 2.40 |
| Mannitol and Maize starch | Pearlitol Flash | Disintegrant | Roquette | 32.60 |
| Crospovidone | Kollidon CL | Disintegrant | BASF | 2.00 |
| Colloidal Silica | Aerosil 200 | Glidant | Evonik | 0.50 |

(continued)

| Component | Commercial Name | Function | Supplier | Cyclobenzaprine HCl (not micronized) (mg/tab) |
|---|---|---|---|---|
| Sodium Stearyl Fumarate | Lubrisanaq | Lubricant | Pharma Trans Sanaq | 0.50 |
| Total weight | | | | 38.00 |

Table 45: Analysis of un-micronized cyclobenzaprine HCl formulation (control: lacking a basifying agent)

| Analyses | Specifications | Cyclobenzaprine HCl formulation (Dipharma, not micronized) | Cyclobenzaprine HCl formulation (Dipharma, micronized) | Cyclobenzaprine HCl formulation (Sifavitor, not micronized) |
|---|---|---|---|---|
| Powder | | | | |
| Distribution | TBD | Assay= 101.3% SD= 2.69 RSD= 2.65% | Assay= 102.8% SD= 2.3 RSD= 2.2% | Assay= 102.2% SD= 2.6 RSD= 2.5% |
| Tablets | | | | |
| Assay | 95-105 % | 106.9% | 102.2% | 98.8% |
| Disintegration test | < 30" | 20" | 65" | 42" |
| Hardness | TBD | 28.40N | 31.40N | 20.80N |

In summary, the Dipharma batch of un-micronized cyclobenzaprine HCl demonstrated, for the tested formulations, good distribution and performance (e.g., it had a particularly low disintegration time in tablet form). The Dipharma batch of micronized cyclobenzaprine HCl demonstrated, for the tested formulations, good distribution and performance, with the exception of some electrostatic phenomena typical of a micronized powder. The Sifavitor batch of not-micronized cyclobenzaprine HCl demonstrated, for the tested formulations, good distribution and performance.

All the analytical results reported in Example 8 and referred to the formulations reported above had been performed according to the following analytical conditions:

Assay

| | |
|---|---|
| **ANALYTICAL EQUIPMENT:** | HPLC JASCO equipped with Auto-Sampler AS-1555, Pump PU-1580 and detector UV-2075 Plus |
| **ANALYTICAL COLUMN:** | ALLTIMA $C_{18}$ 5μm 150 x 4.6 mm or equivalent |
| **MOBILE PHASE:** | 49.8% Water<br>25% Methanol<br>25% Acetonitrile<br>0.2% Methanesulphonic acid<br>Mobile phase corrected to pH 3.60 ± 0.10 with diethylamine |
| **FLOW RATE:** | 1.5 ml/min |
| **WAVELENGHT:** | 240 nm |
| **INJECTED VOLUME:** | 10 μl |
| **TEMPERATURE:** | 25°C |
| **ACQUISITION LENGTH:** | 8 minutes |
| **SOLVENT:** | 50% Methanol |

50% Phosphate buffer: (dissolve 6.80 g/l Potassium dihydrogen phosphate and 1.57 g/l Sodium hydroxide in water; correct to pH 7.40 ± 0.10 with Sodium hydroxide IN if necessary)

**CYCLOBENZAPRINE RETENTION TIME:** About 5.0 minutes

**PRODUCT STANDARD PREPARATION:** Weigh about 20 mg of cyclobenzaprine HCl reference (or working) standard in a 50 mL volumetric flask and add 40 ml of solvent; sonicate for 5 minutes and dilute to volume with solvent. Transfer 2.5 mL of this solution in a 10 ml volumetric flask and dilute to volume with solvent (Concentration of cyclobenzaprine HCl = about 100 μg/mL).

**SAMPLE PREPARATION (powder):** Weigh about 160 mg of cyclobenzaprine HCl powder in a 100 mL volumetric flask, add 70 mL of solvent, agitate with magnetic stirrer for 10 minutes and sonicate for 5 minutes; dilute to volume with solvent. Filter with Syringe filter with Hydrophilic 0.45μm PVDF membrane before injection. The concentration of cyclobenzaprine HCl is 100 μg /mL].

**SAMPLE PREPARATION (tablets):** Weigh 10 tablets of Cyclobenzaprine HCl in a 100 mL volumetric flask, add 80 mL of solvent, agitate with magnetic stirrer for 10 minutes and sonicate for 5 minutes; dilute to volume with solvent. Transfer 4 mL in a 10 mL volumetric flask and dilute to volume with solvent. Filter with syringe filter with Hydrophilic 0.45μm PVDF membrane before injection. [The concentration of cyclobenzaprine HCl is 100 μg /mL].

Purity

| | |
|---|---|
| **ANALYTICAL EQUIPMENT:** | HPLC JASCO equipped with Auto-Sampler AS-1555, Pump PU-1580, and detector UV-2075 Plus |
| **ANALYTICAL COLUMN:** | ALLTIMA $C_{18}$ 5μm 150 x 4.6 mm or equivalent |
| **MOBILE PHASE:** | 59.8% Water<br>20% Methanol<br>20% Acetonitrile<br>0.2% Methanesulphonic acid<br>Mobile phase corrected to pH 3.60 ± 0.10 with diethylamine |
| **FLOW RATE:** | 2.0 ml/min |
| **WAVELENGTH:** | 240 nm |
| **INJECTED VOLUME:** | 20 μl |
| **TEMPERATURE:** | 25°C |
| **ACQUISITION LENGTH:** | 60 minutes |
| **SOLVENT:** | Methanol |
| **RETENTION TIME:** | About 12.0 minutes |
| **RELATED SUBSTANCE RETENTION TIME** : | Dibenzosuberenone (Impu 1) about 56.0 minutes |

Carbinole (Impu 2): about 6.0 minutes
Amitriptyline (Impu 4): about 15.0 minutes

**RELATED SUBSTANCES STANDARD PREPARATION (for Known and Unknown impurities):** Weigh about 10 mg of cyclobenzaprine HCl reference (or working) standard and about 10 mg of cyclobenzaprine HCl Impurity 1, 2, 4 reference (or working) standard in a 100 mL volumetric flask, add 80 ml of Methanol and sonicate for 10 minutes; dilute to volume with Methanol.

Transfer 1.0 mL of this solution in a 100 ml volumetric flask and dilute to volume with solvent. The concentration of impurity is 1 μg /mL, corresponding to 0.1%.

**SAMPLE PREPARATION:** Weigh exactly 4 cyclobenzaprine HCl tablets in a 10 mL volumetric flask, add 5 mL of solvent and sonicate for 10 minutes; dilute to volume with solvent. Filter with syringe filter with hydrophilic 0.45μm PVDF membrane before injection. The concentration of Cyclobenzaprine HCl is about 1000 μg /mL.

Starting from the all the data collected in the formulation setups, further batches of formulations both with and without basifying agent (control) were manufactured. As the final step of the galenic development, the big laboratory batches were manufactured according to the formulations reported in Table 46 and 47:

Table 46: Basifying-agent-containing cyclobenzaprine HCl formulations

| Component | Commercial Name | Function | Supplier | (mg/tab) |
|---|---|---|---|---|
| Cyclobenzaprine HCl* | Cyclobenzaprine HCl | Active Ingredient | Dipharma | 2.40 |
| Mannitol and Maize starch | Pearlitol Flash | Disintegrant | Roquette | 31.55 |
| Crospovidone | Kollidon CL | Disintegrant agent | BASF | 2.00 |

(continued)

| Component | Commercial Name | Function | Supplier | (mg/tab) |
|---|---|---|---|---|
| Colloidal Silica | Aerosil 200 | Glidant agent | Evonik | 0.50 |
| Sodium Stearyl Fumarate | Lubrisanaq | Lubricant | Pharma Trans Sanaq | 0.50 |
| Potassium Phosphate Dibasic | Potassium Phosphate Dibasic | Basifying Agent | VWR | 1.05 |
| | | | | *Total Weight 38.00* |

Table 47: Cyclobenzaprine HCl formulations lacking a basifying agent (control)

| Component | Commercial Name | Function | Supplier | (mg/tab) |
|---|---|---|---|---|
| Cyclobenzaprine HCl* | Cyclobenzaprine HCl | Active Ingredient | Dipharma | 2.40 |
| Mannitol and Maize starch | Pearlitol Flash | Disintegrant | Roquette | 32.60 |
| Crospovidone | Kollidon CL | Disintegrant agent | BASF | 2.00 |
| Colloidal Silica | Aerosil 200 | Glidant agent | Evonik | 0.50 |
| Sodium Stearyl Fumarate | Lubrisanaq | Lubri cant | Pharma Trans Sanaq | 0.50 |
| | | | | *Total Weight 38.00* |

An additional formulation was manufactured starting from the basifying-agent-containing cyclobenzaprine HCl formulation. This coated version was obtained using a a coating mixture also containing the basifying agent as described in Table 48.

Table 48: Basifying-agent-containing cyclobenzaprine HCl formulations (coated formulation)

| Component | Commercial Name | Function | Supplier | (mg/tab) |
|---|---|---|---|---|
| Cyclobenzaprine HCl* | Cyclobenzaprine HCl | Active Ingredient | Dipharma | 2.40 |
| Mannitol and Maize starch | Pearlitol Flash | Diluter | Roquette | 31.55 |
| Crospovidone | Kollidon CL | Disintegrant agent | BASF | 2.00 |
| Colloidal Silica | Aerosil 200 | Glidant agent | Evonik | 0.50 |
| Sodium Stearyl Fumarate | Lubrisanaq | Lubricant | Pharma Trans Sanaq | 0.50 |
| Potassium Phosphate Dibasic | Potassium Phosphate Dibasic | Basifying Agent | VWR | 1.05 |
| Coating Mixture (water, Opadry Clear, Potassium phosphate dibasic) | Water Opadry Clear Potassium phosphate dibasic | Coating Layer | APR Colorcon VWR | 0.30 |
| | | | | *Total Weight 38.30* |

Time zero data from a stability study on the large laboratory batches in different packaging materials is are reported in the Table 49.

Table 49: Stability study, time zero data

| *Parameter* | *Batch with basifying agent (core)* | *Batch lacking basifying agent (core) (control)* | *Batch with basifying agent (coated)* |
|---|---|---|---|
| Appearance | White to off-white, biconvex round tablet | White to off-white, biconvex round tablet | White to off-white, biconvex round tablet |
| Cyclobenzaprin e HCl Assay (%) | 93.1 | 99.7 | 107.1 |
| ImpurityA (%) | Absent | Absent | Absent |
| ImpurityB (%) | <LOQ | <LOQ | <LOQ |
| ImpurityC (%) | Absent | Absent | Absent |
| Each Unk impurity (%) -Unk1 (RRT=0.83) | 0.01 | <LOQ | <LOQ 0.02 |
| Total impurities (%) | 0.01 | <LOQ | |
| pH in water | 7.23 | 5.47 | 7.25 |
| Hardness (N) | 28.80 | 37.40 | 43.20 |
| Disintegration Time (sec) | 26 (16;23;40;21;27;3 1) | 22 (18;40;16;23;20;1 6) | 64 (76;76;57;59;60;5 7) |
| KF(%) | 2.02 | 2.06 | 2.59 |

To study stressed stability in the batch lacking the basifying agent, the samples are stored at 40°C and 50°C and are analyzed after 1, 2, 3, and 4 weeks. Data from the 1 week timepoint are reported in Table 50.

Table 50: Stability study, 1 week, no basifying agent

| Parameter | Tentative Specifications | 1week, 50°C | 1week, 40°C |
|---|---|---|---|
| ImpurityA (%) | 0.2 | <LOQ | Absent |
| ImpurityB (%) | 0.2 | <LOQ | <LOQ |
| ImpurityC (%) | 0.2 | Absent | Absent |
| Each Unk impurity (%) - Unk 1 (RRt =0.83) | 0.5 | 0.02 | <LOQ |
| Total impurities (Area) | 2.0 | 0.02 | <LOQ |

All of the analysis perfomed starting from the large laboratory batches were performed according to the following optimized and validated conditions:

Purity

Analytical equipment: HPLC JASCO equipped with Auto-Sampler AS-2055, PU-2080, and detector Diode Array MD 2010 Plus

Analytical column: SYMMETRY C18 5μm 250 x 4.6 mm or equivalent

Mobile phase: 25% methanol, 25% acetonitrile, 50% Butylamine buffer/$CH_3COOH$ (In 950ml of water add 10ml of 99.5% Butylamine, correct to pH 6.20 ± 0.10 with glacial acetic acid and dilute to volume of 1L with water)

Flow rate: 1.5 mL/min

Wavelength: 239 nm

Injected volume: 10 μl

Temperature: 28°C

Acquisition length: 35 minutes

Solvent: Mobile phase

Retention time: Impurity A (Carbinole) about 6', Impurity B (Amitriptyline) about 14', Impurity C (Dibenzosuberenone) about 29', Cyclobenzaprine HCl about 11'

For the related substances standard preparation (for Known and Unknown impurities): weigh about 10 mg of cyclobenzaprine HCl reference (or working) standard and about 10 mg of cyclobenzaprine HCl Impurity A, B and C reference (or working) standard in a 100 mL volumetric flask, add 5 mL of Acetonitrile and then 75 mL of solvent; sonicate for 5 minutes and dilute to volume with solvent.
Transfer 1.0 mL of this solution in a 50 ml volumetric flask and dilute to volume with solvent for a final concentration of impurity of 2 μg /mL.
LOQ level: Transfer 1.0 mL of the last solution in a 10 ml volumetric flask and dilute to volume with solvent for a concentration of impurity of 0.2 μg /mL.
For the sample preparation: place 10 cyclobenzaprine HCl tablets exactly weighed in a 25 mL volumetric flask (or 20 cyclobenzaprine HCl tablets in a 50 mL volumetric flask), add 20 mL (or 40 mL) of solvent and sonicate for 5 minutes; dilute to volume with solvent. Filter with a syringe filter with hydrophilic 0.45μm PVDF membrane before injection. The concentration of Cyclobenzaprine HCl is about 1000 μg /mL.
Assay

| | | |
|---|---|---|
| **ANALYTICAL EQUIPMENT:** | HPLC JASCO equipped with Auto-Sampler AS-1555, Pump PU-1580, and detector UV-2075 Plus | |
| **ANALYTICAL COLUMN:** | ALLTIMA $C_{18}$ 5μm 150 x 4.6 mm or equivalent | |
| **MOBILE PHASE:** | | 49.8% Water |
| | | 25% Methanol |
| | | 25% Acetonitrile |
| | | 0.2% Methanesulphonic acid |
| | | Mobile phase corrected to pH 3.60 ± 0.10 with diethylamine |
| **FLOW RATE:** | 1.5 ml/min | |
| **WAVELENGHT:** | 240 nm | |

| | |
|---|---|
| **INJECTED VOLUME:** | 10 μl |
| **TEMPERATURE:** | 25°C |
| **ACQUISITION LENGTH:** | 8 minutes |
| **SOLVENT:** | 50% Methanol |
| | 50% Phosphate buffer (dissolve 6.80 g/l Potassium dihydrogen phosphate and 1.57 g/l Sodium hydroxide in water; correct to pH 7.40 ± 0.10 with Sodium hydroxide 1N if necessary) |

**CYCLOBENZAPRINE RETENTION TIME:** about 5.0 minutes
**PRODUCT STANDARD PREPARATION:** Weigh about 20 mg of cyclobenzaprine HCl reference (or working) standard in a 50 mL volumetric flask and add 40 ml of solvent; sonicate for 5 minutes and dilute to volume with solvent. Transfer 2.5 mL of this solution in a 10 ml volumetric flask and dilute to volume with solvent, for a concentration of cyclobenzaprine HCl equalling about 100 μg/mL.
**SAMPLE PREPARATION (powder):** Weigh about 160 mg of cyclobenzaprine HCl powder in a 100 mL volumetric flask, add 70 mL of solvent, agitate with magnetic stirrer for 10 minutes and sonicate for 5 minutes; dilute to volume with solvent. Filter with a syringe filter with hydrophilic 0.45μm PVDF membrane before injection, for a cyclobenzaprine HCl concentration of 100 μg /mL.

**SAMPLE PREPARATION (tablets):** Weigh 10 tablets of cyclobenzaprine HCl in a 100 mL volumetric flask, add 80 mL of solvent, agitate with magnetic stirrer for 10 minutes and sonicate for 5 minutes; dilute to volume with solvent. Transfer 4 mL in a 10 mL volumetric flask and dilute to volume with solvent. Filter with a syringe filter with hydrophilic 0.45μm PVDF membrane before injection, for a cyclobenzaprine HCl concentration of 100 μg /mL.

**Example 9**

As referred to in Example 4, a study was designed in order to assess the effects of the compositions and methods of the invention. The study utilized beagle dogs, which were administered cyclobenzaprine HCl orally, sublingually, or intravenously(control). The pharmacokinetic parameters were then calculated. A summary of the study design is as follows:

Test substance: cyclobenzaprine hydrochloride

Administration routes: oral (PO) (control), sublingual, and intravenous (IV;control)

Species: beagle dog

Gender: female

Matrix: plasma

Vehicle: aqueous $KH_2PO_4$ solution adjusted with NaOH to pH 7.4

Dose: 0.14 mg/kg (approximately corresponding to 10 mg dose in 70 kg human)

Formulation concentrations: PO: 0.028 mg/mL; sublingual: 2.5 mg/mL; IV: 0.14 mg/mL

Administration volumes: PO: 5 mL/kg; sublingual: 0.056 mL/kg; IV: 1 mL/kg

Blood was collected in a fasted condition for all three routes of administration. For PO administration, blood was collected at 0 h (pre-dose) and at 0.5 h, 1 h, 2 h, 3 h, 4 h, 5 h, 6 h, 8 h, 10 h, 12 h and 24 h post-administration. For sublingual and IV administration, blood was collected after the predose and at 10 min, 20 min, 0.5 h, 1 h, 2 h, 3 h, 4 h, 6 h, 8 h, 10 h, and 24 h post-administration. Analysis of the cyclobenzaprine was performed substantially as described above. Briefly, the method involved liquid-liquid extraction with hexane. The extracts were injected using an HPLC system and an API4000® (Applied Biosystems) for the MS/MS detection, with an "Onyx monolithic Phenomenex C18 100x3.0 mm" column. The method was linear from 0.1 to 50 ng/mL for cyclobenzaprine in dog plasma. Quality control samples were prepared at 0.3, 25 and 40 ng/mL for cyclobenzaprine in dog plasma. Results obtained from the analysis of quality control samples were within the limits of acceptance defined in the protocol, therefore validating the concentrations measured in plasma samples.

Intravenous (IV) administration (as a control) of cyclobenzaprine HCl in a solution containing a basifying agent resulted in surprisingly dynamic range in the plasma concentration-time profile and a surprisingly low bioavailability of cyclobenzaprine administered by nasogastric tube. In previous Beagle experiments, 2 mg/kg of cyclobenzaprine was administered to Beagles (6.7-8.2 kg) with a radioactive label (Hucker, 1978 Drug Metabolism and Disposition 6(6):659). Doses were expressed as free base, and so should be adjusted by MW Free Base 275 g/mole/MW HCl salt 312 g/mole. Thus, 1.76 mg/kg of cyclobenzaprine HCl was administered. Cyclobenzaprine was administered by in saline solution for intravenous (IV) dosing or in gelatin capsules for oral (PO) dosing. In the Hucker study, the plasma levels of cyclobenzaprine were measured by recovery of radioactive equivalents of a $^{14}C$ labeled cyclobenzaprine, so the plasma levels were "equivalent" to certain values (in μg/ml) of cyclobenzaprine. By this method, Beagles who received 1.76 mg/kg cyclobenzaprine HCl by mouth had plasma levels of 0.72 μg/ml [equivalent] (at 0.5 h), 1.14 μg/ml [equivalent] (1 hr), 1.46 μg/ml [equivalent] (2 hr), 0.92 μg/ml [equivalent] (4 hr), 0.58 μg/ml [equivalent] (6 hr), 0.10 μg/ml [equivalent] (24 hr) and dogs who received 2 mg/kg cyclobenzaprine by intravenous injection in saline had plasma levels of 0.43 μg/ml [equivalent] (0.5 h), 0.53 μg/ml [equivalent] (1 hr), 0.60 μg/ml [equivalent] (2 hr), 0.55 μg/ml [equivalent] (4 hr), 0.45 μg/ml [equivalent] (6 hr), 0.12 μg/ml [equivalent] (24 hr). In our Beagle studies, the dose was 0.14 mg/kg, which is approximately 1/12.6 the dose of the Hucker studies. Solely for the purposes of comparing the pharmacokinetic profiles of Hucker and our studies, assuming dose proportionality, the data from Hucker were adjusted to be equivalent to dose of 0.14 mg/kg cyclobenzaprine HCl and are shown in Table 51.

Table 51: Comparison of pharmacokinetics

| Hucker Dose Adjusted for 0.14 mg/kg | | | Current study | | |
|---|---|---|---|---|---|
| t | PO | IV | NG | IV | SL |
| 0.167 | | | | 21.07 | 122.3 |
| 0.33 | | | | 17.33 | 81.03 |
| 0.5 | 57.1 | 34.1 | 0.32 | 14.3 | 52.3 |
| 1 | 90.5 | 42.1 | 0.41 | 9.0 | 40.8 |
| 2 | 115.9 | 47.6 | 0.33 | 6.5 | 15.0 |
| 4 | 73.0 | 43.7 | 0.21 | 3.2 | 5.8 |
| 6 | 46.0 | 35.7 | 0.15 | 1.7 | 3.4 |
| 24 | 7.9 | 9.5 | - | 0.1 | 0.1 |
| all plasma levels in ng/ml | | | | | |

Comparison revealed that adjusted for dose and dose-proportionality to our study, Hucker's PO bioavailability from a gelatin capsule was much higher (peak at 115.9 ng/ml at 2 hours) than the bioavailability of cyclobenzaprine oral solution via a nasogastric tube (NG) (peak at 0.41 ng/ml at 1 h). The profile of IV administered cyclobenzaprine in Hucker was relatively flate from 0.5 h to 6 h, adjusted for dose and dose-proportionality to our study, varying from 34.1 ng/ml at 0.5 h to 47.6 ng/ml (at 2 hours) to 35.7 ng/ml whereas our profile was dyanamic in that period of time and decreased from 52.3 to 3.4 ng/ml. Hucker did not measure plasma levels at 0.167 h or 0.33 h which in our study were the highest plasma levels and and shows an even more dynamic range. Hucker did not study sublingual administration.

Sublingual administration of cyclobenzaprine HCl resulted in surprisingly improved pharmacokinetic properties and bioavailability as compared to PO administration (Table 52, Figures 2 and 3; individual PO, sublingual (SL), and IV data: Tables 53-56). In particular, the $C_{max}$ for cyclobenzaprine was significantly higher when administered sublingually, from approximately 0.48 ng/mL (PO) to approximately 137 ng/mL (SL). The $T_{max}$ also diminished from one hour to 10 minutes, and the bioavailability increased from approximately 3.8% to 292%. As is standard practice, bioavailability for IV administration was considered to be 100%. We believe that a possible explanation for the sublingual bioavailability may be that the IV measurement was taken after the true IV $C_{max}$ value should have been recorded. Regardless, in comparing the bioavailability of PO and sublingual administration, there is a nearly 77-fold increase.

Table 52: Mean ± SD plasma pharmacokinetic parameters

| Route | Cmax (ng/mL) | Tmax* (h) | $AUC_t$ (ng/mL*h) | $AUC_{inf}$ (ng/mL*h) | %AUCextra | Kel (1/h) | $t_{1/2}$ (h) | Cl** (L/h/kg) | Vd** (L/kg) | Bioavailability F% |
|---|---|---|---|---|---|---|---|---|---|---|
| **PO** | 0.4782 ± 0.2616 | 1.00 | 1.591 ± 0.8142 | 3.626 ± 3.339 | 36.68 ± 28.68 | 0.4782 | 1.449 | 72.34 | 151.3 | 3.778 ±2.294 |
| **Sublingual** | 136.6 ± 69.94 | 0.17 | 126.9 ± 37.05 | 129.1 ± 36.45 | 1.84 ± 1.81 | 0.2964 ± 0.1442 | 2.852 ± 1.437 | 1.158 ± 0.3288 | 5.260 ± 3.987 | 292.4 ± 84.97 |
| **IV** | NA | NA | 43.51 ± 3.766 | 44.94 ± 4.149 | 3.13 ± 1.77 | 0.1677 ± 0.07679 | 4.723 ±1.685 | 3.138 ± 0.3068 | 21.27 ± 7.173 | - |

* Median ** Cl and Vd are Cl/F and Vd/F for PO and sublingual routes NA: Not Applicable

Table 53: Cyclobenzaprine plasma concentrations (ng/mL) measured after oral administration of cyclobenzaprine HCl

| Time Hours | Dog 1 | Dog 2 | Dog 3 | Dog 4 | Dog 5 | Low | High | Mean | S.D. | %CV | n |
|---|---|---|---|---|---|---|---|---|---|---|---|
| 0 | BLQ | BLQ | BLQ | BLQ | BLQ | BLQ | BLQ | BLQ | NC | NC | 5 |
| 0.5 | 0.2324 | 0.2011 | 0.2843 | 0.3300 | 0.5700 | 0.2011 | 0.5700 | 0.3236 | 0.1463 | 45.21 | 5 |
| 1 | 0.3215 | 0.2671 | 0.2033 | 0.3860 | 0.8486 | 0.2033 | 0.8486 | 0.4053 | 0.2568 | 63.36 | 5 |
| 2 | 0.242 | 0.2345 | 0.1859 | 0.6610 | 0.3261 | 0.1859 | 0.6610 | 0.3299 | 0.1918 | 58.14 | 5 |
| 3 | 0.2465 | 0.2756 | 0.206 | 0.2414 | 0.5494 | 0.2060 | 0.5494 | 0.3038 | 0.1395 | 45.92 | 5 |
| 4 | 0.1589 | 0.2057 | 0.1264 | 0.2193 | 0.3384 | 0.1264 | 0.3384 | 0.2097 | 0.08092 | 38.59 | 5 |
| 5 | 0.1570 | 0.1623 | 0.1035 | 0.1386 | 0.2119 | 0.1035 | 0.2119 | 0.1547 | 0.03942 | 25.48 | 5 |
| 6 | 0.1530 | 0.1326 | BLQ | BLQ | 0.1671 | BLQ | 0.1671 | 0.09054 | 0.08356 | 92.29 | 5 |
| 8 | BLQ | BLQ | BLQ | BLQ | 0.1255 | BLQ | 0.1255 | BLQ | NC | NC | 5 |
| 10 | BLQ | BLQ | BLQ | BLQ | 0.1121 | BLQ | 0.1121 | BLQ | NC | NC | 5 |
| 12 | BLQ | BLQ | BLQ | BLQ | BLQ | BLQ | BLQ | BLQ | NC | NC | 5 |
| 24 | BLQ | BLQ | BLQ | BLQ | BLQ | BLQ | BLQ | BLQ | NC | NC | 5 |

Table 54: Cyclobenzaprine plasma concentrations (ng/mL) measured after sublingual administration of cyclobenzaprine HCl

| Time Hours | Dog 1 | Dog 2 | Dog 3 | Dog 4 | Dog 5 | Low | High | Mean | S.D. | %CV | n |
|---|---|---|---|---|---|---|---|---|---|---|---|
| 0 | BLQ | BLQ | BLQ | BLQ | BLQ | BLQ | BLQ | BLQ | NC | NC | 5 |
| 0.16667 | 38.94 | 86.95 | 164.9 | 244.6 | 76.21 | 38.94 | 244.6 | 122.3 | 82.30 | 67.28 | 5 |
| 0.33333 | 38.39 | 41.03 | 64.52 | 147.2 | *114.0* | 38.39 | 147.2 | 81.03 | 47.84 | 59.05 | 5 |
| 0.5 | 72.36 | 32.33 | 28.82 | *66.00* | 61.78 | 28.82 | 72.36 | 52.26 | 20.19 | 38.63 | 5 |
| 1 | 22.62 | 23.49 | 34.74 | *60.88* | 62.42 | 22.62 | 62.42 | 40.83 | 19.61 | 48.02 | 5 |
| 2 | 11.50 | 8.706 | 14.25 | 17.76 | 22.55 | 8.706 | 22.55 | 14.95 | 5.409 | 36.18 | 5 |
| 3 | 10.91 | 6.663 | 9.565 | 5.866 | 9.348 | 5.866 | 10.91 | 8.470 | 2.120 | 25.03 | 5 |
| 4 | 5.323 | 3.946 | 6.611 | 7.016 | 6.027 | 3.946 | 7.016 | 5.785 | 1.210 | 20.92 | 5 |
| 6 | 3.882 | 2.361 | 3.682 | 3.687 | 3.369 | 2.361 | 3.882 | 3.396 | 0.6072 | 17.88 | 5 |
| 8 | 2.573 | 1.430 | 1.085 | 0.7974 | 1.287 | 0.7974 | 2.573 | 1.434 | 0.6793 | 47.37 | 5 |
| 10 | 1.364 | 0.8100 | 0.5219 | 0.5297 | 0.7659 | 0.5219 | 1.364 | 0.7983 | 0.3427 | 42.93 | 5 |
| 24 | BLQ | 0.1430 | 0.2610 | BLQ | BLQ | BLQ | 0.2610 | 0.0808 | 0.1182 | 146.29 | 5 |
| BLQ: Below the Limit of Quantification (0.1 ng/mL)<br>*italic:* out of range (50 ng/mL): value given for indication | | | | | | | | | | | |

Table 55: Cyclobenzaprine plasma concentrations (ng/mL) measured after IV administration of cyclobenzaprine HCl

| Time Hours | Dog 1 | Dog 2 | Dog 3 | Dog 4 | Dog 5 | Low | High | Mean | S.D. | %CV | n |
|---|---|---|---|---|---|---|---|---|---|---|---|
| 0 | BLQ | BLQ | BLQ | BLQ | BLQ | BLQ | BLQ | BLQ | NC | NC | 5 |
| 0.16667 | 15.41 | 18.56 | 17.38 | 25.48 | 28.52 | 15.41 | 28.52 | 21.07 | 5.633 | 26.73 | 5 |
| 0.33333 | 13.44 | 13.74 | 13.70 | 22.02 | 23.74 | 13.44 | 23.74 | 17.33 | 5.106 | 29.46 | 5 |
| 0.5 | 10.73 | 12.23 | 14.55 | 17.49 | 16.61 | 10.73 | 17.49 | 14.32 | 2.855 | 19.94 | 5 |
| 1 | 11.08 | 8.868 | 7.459 | 10.31 | 7.071 | 7.071 | 11.08 | 8.958 | 1.743 | 19.46 | 5 |
| 2 | 8.553 | 4.797 | 6.823 | 5.831 | 6.528 | 4.797 | 8.553 | 6.506 | 1.385 | 21.29 | 5 |
| 3 | 4.427 | 4.248 | 5.060 | 4.047 | 3.282 | 3.282 | 5.060 | 4.213 | 0.6440 | 15.29 | 5 |
| 4 | 5.139 | 1.881 | 3.879 | 2.989 | 1.866 | 1.866 | 5.139 | 3.151 | 1.394 | 44.24 | 5 |
| 6 | 1.682 | 1.963 | 2.193 | 1.663 | 0.9617 | 0.9617 | 2.193 | 1.693 | 0.4633 | 27.37 | 5 |
| 8 | 1.413 | 1.000 | 1.036 | 1.089 | 0.7013 | 0.7013 | 1.413 | 1.048 | 0.2537 | 24.21 | 5 |
| 10 | 0.8358 | 0.8134 | 0.6166 | 0.6831 | 0.2325 | 0.2325 | 0.8358 | 0.6363 | 0.2433 | 38.24 | 5 |

(continued)

| Time Hours | Dog 1 | Dog 2 | Dog 3 | Dog 4 | Dog 5 | Low | High | Mean | S.D. | %CV | n |
|---|---|---|---|---|---|---|---|---|---|---|---|
| 24 | BLQ | 0.1328 | 0.1787 | 0.1164 | 0.1216 | BLQ | 0.1787 | 0.1099 | 0.06617 | 60.21 | 5 |
| BLQ: Below the Limit of Quantification (0.1 ng/mL) | | | | | | | | | | | |

Table 56: Cyclobenzaprine pharmacokinetic parameters after administration of cyclobenzaprine HCl

| Route | Animal | Cmax (ng/mL) | Tmax (h) | $AUC_t$ (ng/mL*h) | $AUC_{inf}$ (ng/mL*h) | %AUCextra | Kel (1/h) | $t_{1/2}$ (h) | Cl* (L/h/kg) | Vd* (L/kg) | Bioavailability F% |
|---|---|---|---|---|---|---|---|---|---|---|---|
| PO | **dog 1** | 0.3215 | 1.00 | 1.238 | 9.326 | 86.72 | NC | NC | NC | NC | 2.79 |
| | **dog 2** | 0.2756 | 3.00 | 1.245 | 1.849 | 32.66 | NC | NC | NC | NC | 3.01 |
| | **dog 3** | 0.2843 | 0.50 | 0.8647 | *1.165* | 25.81 | NC | NC | NC | NC | 1.88 |
| | **dog 4** | 0.6610 | 2.00 | 1.646 | 1.935 | 14.97 | 0.4782 | 1.449 | 72.34 | 151.3 | 3.46 |
| | **dog 5** | 0.8486 | 1.00 | 2.961 | 3.856 | 23.22 | NC | NC | NC | NC | 7.75 |
| | **N** | 5 | 5 | 5 | 5 | 5.00 | 1 | 1 | 1 | 1 | 5 |
| | **Mean** | 0.4782 | 1.50 | 1.591 | 3.626 | 36.68 | 0.4782 | 1.449 | 72.34 | 151.3 | 3.78 |
| | **Stdev** | 0.2616 | 1.00 | 0.8142 | 3.339 | 28.68 | NC | NC | NC | NC | 2.29 |
| | **%CV** | 54.71 | 66.67 | 51.17 | 92.09 | 78.21 | NC | NC | NC | NC | 60.74 |
| | **SEM** | 0.1170 | 0.45 | 0.3641 | 1.493 | 12.83 | NC | NC | NC | NC | 1.03 |
| | **Harmean** | 0.3862 | 1.03 | 1.347 | 2.191 | 26.21 | NC | NC | NC | NC | 3.05 |
| | **GeoMean** | 0.4266 | 1.25 | 1.454 | 2.724 | 30.27 | NC | NC | NC | NC | 3.35 |
| | **Median** | 0.3215 | 1.00 | 1.245 | 1.935 | 25.81 | NC | NC | NC | NC | 3.01 |
| | **Min** | 0.2756 | 0.50 | 0.8647 | 1.165 | 14.97 | NC | NC | NC | NC | 1.88 |
| | **Max** | 0.8486 | 3.00 | 2.961 | 9.326 | 86.72 | NC | NC | NC | NC | 7.75 |
| Sublingual | **dog 1** | 72.36 | 0.50 | 98.64 | 103.9 | 5.02 | 0.2615 | 2.651 | 1.348 | 5.155 | 222.11 |
| | **dog 2** | 86.95 | 0.17 | 86.08 | 87.12 | 1.20 | 0.1365 | 5.077 | 1.607 | 11.77 | 208.22 |
| | **dog 3** | 164.9 | 0.17 | 123.2 | 124.4 | 0.99 | 0.2126 | 3.260 | 1.125 | 5.294 | 268.00 |
| | **dog 4** | 244.6 | 0.17 | 176.6 | 177.6 | 0.59 | 0.5084 | 1.364 | 0.7881 | 1.550 | 370.85 |
| | **doq 5** | 114.0 | 0.33 | 150.1 | 152.2 | 1.39 | 0.3630 | 1.909 | 0.9196 | 2.533 | 392.93 |
| | **N** | 5 | 5 | 5 | 5 | 5.00 | 5 | 5 | 5 | 5 | 5 |
| | **Mean** | 136.6 | 0.27 | 126.9 | 129.1 | 1.84 | 0.2964 | 2.852 | 1.158 | 5.260 | 292.42 |
| | **Stdev** | 69.94 | 0.15 | 37.05 | 36.45 | 1.81 | 0.1442 | 1.437 | 0.3288 | 3.987 | 84.97 |
| | **%CV** | 51.22 | 55.90 | 29.19 | 28.24 | 98.30 | 48.65 | 50.39 | 28.40 | 75.79 | 29.06 |
| | **SEM** | 31.28 | 0.07 | 16.57 | 16.30 | 0.81 | 0.06448 | 0.6427 | 0.1470 | 1.783 | 38.00 |
| | **Harmean** | 113.0 | 0.22 | 118.5 | 120.9 | 1.12 | 0.2430 | 2.339 | 1.085 | 3.316 | 273.56 |
| | **GeoMean** | 123.7 | 0.24 | 122.6 | 124.9 | 1.37 | 0.2687 | 2.580 | 1.121 | 4.170 | 282.72 |
| | **Median** | 114.0 | 0.17 | 123.2 | 124.4 | 1.20 | 0.2615 | 2.651 | 1.125 | 5.155 | 268.00 |
| | **Min** | 72.36 | 0.17 | 86.08 | 87.12 | 0.59 | 0.1365 | 1.364 | 0.7881 | 1.550 | 208.22 |
| | **Max** | 244.6 | 0.50 | 176.6 | 177.6 | 5.02 | 0.5084 | 5.077 | 1.607 | 11.77 | 392.93 |

(continued)

| Route | Animal | Cmax (ng/mL) | Tmax (h) | $AUC_t$ (ng/mL*h) | $AUC_{inf}$ (ng/mL*h) | %AUCextra | Kel (1/h) | $t_{1/2}$ (h) | Cl* (L/h/kg) | Vd* (L/kg) | Bioavailability F% |
|---|---|---|---|---|---|---|---|---|---|---|---|
| IV | **dog 1** | NA | NA | 44.41 | 47.23 | 5.98 | 0.2960 | 2.342 | 2.964 | 10.01 | - |
| | **dog 2** | NA | NA | 41.34 | 42.38 | 2.46 | 0.1274 | 5.441 | 3.303 | 25.93 | - |
| | **dog 3** | NA | NA | 45.97 | 47.72 | 3.67 | 0.1020 | 6.798 | 2.934 | 28.77 | - |
| | **dog 4** | NA | NA | 47.62 | 48.49 | 1.78 | 0.1348 | 5.141 | 2.887 | 21.41 | - |
| | **dog 5** | NA | NA | 38.20 | 38.88 | 1.76 | 0.1781 | 3.891 | 3.601 | 20.21 | - |
| | **N** | 0 | 0 | 5 | 5 | 5.00 | 5 | 5 | 5 | 5 | - |
| | **Mean** | NA | NA | 43.51 | 44.94 | 3.13 | 0.1677 | 4.723 | 3.138 | 21.27 | - |
| | **Stdev** | NA | NA | 3.766 | 4.149 | 1.77 | 0.07679 | 1.685 | 0.3068 | 7.173 | - |
| | **%CV** | NA | NA | 8.655 | 9.232 | 56.64 | 45.80 | 35.68 | 9.776 | 33.73 | - |
| | **SEM** | NA | NA | 1.684 | 1.855 | 0.79 | 0.03434 | 0.7536 | 0.1372 | 3.208 | - |
| | **Harmean** | NA | NA | 43.24 | 44.62 | 2.53 | 0.1468 | 4.134 | 3.115 | 18.56 | - |
| | **GeoMean** | NA | NA | 43.37 | 44.78 | 2.79 | 0.1560 | 4.444 | 3.126 | 20.04 | - |
| | **Median** | NA | NA | 44.41 | 47.23 | 2.46 | 0.1348 | 5.141 | 2.964 | 21.41 | - |
| | **Min** | NA | NA | 38.20 | 38.88 | 1.76 | 0.1020 | 2.342 | 2.887 | 10.01 | - |
| | **Max** | NA | NA | 47.62 | 48.49 | 5.98 | 0.2960 | 6.798 | 3.601 | 28.77 | - |

* Cl and Vd are Cl/F and Vd/F for PO and sublingual routes
*italic:* %AUCextra >20%: value given for indication
NC: Not Calculated
NA: Not Applicable

To further investigate the cause of the sublingual bioavailability measured at greater than 100%, several additional hypotheses were proposed. These included an analytical interaction between Propofol (used as an anaesthesic in the sublingual route) and cyclobenzaprine, binding of cyclobenzaprine to the device used for administrations, and *in vivo* enzymatic competition in the liver between Propofol and cyclobenzaprine.

To address the hypothesis that there was *in vivo* enzymatic competition in the liver between Propofol and cyclobenzaprine, four dogs were treated either sublingually or by IV, with or without Propofol as follows: one dog was treated by IV without Propofol pre-anesthesia; one dog was treated by IV with Propofol pre-anesthesia; one dog was treated sublingually without Propofol pre-anesthesia; and one dog was treated sublingually with Propofol pre-anesthesia. Each dog was sampled at pre-dose before and after Propofol (when possible) and at 5, 10 and 20 minutes post-dose. Figures 4 and 5 depict the mean cyclobenzaprine concentration time profiles after IV and sublingual administration, respectively, of cyclobenzaprine HCl as compared to the principal study described above. Table 57 shows the plasma pharmacokinetic parameters for the dogs. The bioavailability calculated in the same condition is about 200%. Concentrations obtained in dogs pre-treated with Propofol or not are similar. Therefore, enzymatic liver competition does not seem to be responsible for the high bioavailability. Dog 4 (treated by sublingual route without Propofol) had concentrations slightly lower than Dog 3 (with Propofol), but the animal swallowed and slobbered, which may explain the difference.

Table 57: Plasma pharmacokinetic parameters for dogs treated with or without Propofol

| **Route** | **Animal** | **Cmax (ng/mL)** | **Tmax (h)** | **AUC, (ng/mL*h)** | **Bioavailability F%** | **Bioavailability F% IV without propofol vs sublingual with propofol** |
|---|---|---|---|---|---|---|
| **IV** | **Dog 1 with propofol** | 26.84 | 0.0833 | 7.666 | - | - |
| | **Dog 2 without propofol** | 40.10 | 0.0833 | 11.56 | - | - |
| **Sublingual** | **Dog 3 with propofol** | 92.23 | 0.1667 | 22.78 | 297.1 | 197.0 |
| | **Dog 4 without propofol** | 75.57 | 0.1667 | 17.45 | 150.9 | |
| Dog 4 swallowed and slobbered | | | | | | |

To address the hypothesis that cyclobenzaprine bound to the device used for administrations, a binding test was performed with the formulations containing or not containing cyclobenzaprine. The results are depicted in Table 58, which shows that binding of cyclobenzaprine to the administration device was about 5% for the sublingual material and 10% for the intravenous material. Therefore, the binding hypothesis does not explain the bioavailability.

Table 58: Binding test results

| Formulation | Mean | SD | %CV | % from initial formulation |
|---|---|---|---|---|
| Formulation IV | 31.62 | 0.67 | 2.11 | - |
| Mock-dose IV | 28.53 | 0.36 | 1.26 | 90.22 |
| Formulation sublingual | 17.68 | 0.42 | 2.35 | - |
| Mock-dose sublingual | 16.96 | 0.30 | 1.74 | 95.93 |

To determine if propofol may be interfering with the analysis of cyclobenzaprine in either a pure solution or in plasma, a study was performed to combine propofol and cyclobenzaprine *in vitro* and determine if the measurement of cyclobenzaprine was affected. The results of the test are shown in Table 59. Pre-doses before and after propofol are below the level of quanitification (i.e., below 0.1 ng/mL), with no interfering chromatographic peak and no analytical contribution of Propofol observed. Therefore, there does not appear to be an interaction between Propofol and cyclobenzaprine.

Table 59: Analytical contribution of Propofol to cyclobenzaprine

| | Measured concentration (ng/mL) | % from Cyclobenzaprine at 0.5xULOQ |
|---|---|---|
| Pure solution of Cyclobenzaprine at 0.5xULOQ | 24.14 | - |
| Pure solution of Cyclobenzaprine at 0.5xULOQ ± propofol at 10 000 ng/mL | 26.23 | 108.6 |
| Plasma sample spiked with Cylobenzaprine at 0.5xULOQ | 25.18 | - |
| Plasma sample spiked with Cylobenzaprine at 0.5xULOQ + propofol at 10 000 ng/mL | 24.18 | 96.0 |

Based on the above tests, it seems that our initial hypothesis that the bioavailability phenomenon could be explained by the delay between administration and blood sampling (which would be slightly different for intravenous and sublingual routes) may be the most likely. This delay could result in metabolism occurring for a longer period of time after intravenous administration than by the sublingual route. Regardless, we conclude that the bioavailability of cyclobenzaprine administered sublingually in Beagle dogs is very high, and likely close to 100%.

**Example 10**

To study the levels of cyclobenzaprine plasma concentrations after a single sublingual administration of cyclobenzaprine HCl in female beagle dogs, two sublingual tablet formulations were compared: a control tablet without a basifying agent (control) and a tablet with a basifying agent ($K_2HPO_4$). Pharmacokinetic parameters were then calculated and compared.

Each dog received a sublingual cyclobenzaprine tablet, first in a formulation with basifying agent, and then in a formulation without basifying agent, with a washout period of two weeks between each administration. For sublingual dosing, dogs were given propofol and were put in a lying position (ventral decubitis). The mouth was gently opened and the tablet was placed under the tongue. The tongue was then immediately replaced in its initial position and the mouth was closed. The dog was left in the same position until waking. Dogs treated with cyclobenzaprine in the formulation with basifying agent ($K_2HPO_4$) were sedated using 6.5 mg/kg of propofol intravenously. They were then given a single sublingual dose of 1 tablet equivalent to 2.4 mg of cyclobenzaprine HCl (i.e., one tablet per dog). After the washout period of two weeks, dogs were sedated in the same way and given a single sublingual dose of the formulation without basifying agent. Blood samples after both types of treatment were taken pre-dose and at 5, 10, 20, 30, and 45 minutes, and at 1, 2, 3, 4, 6, 8, and 10 hours after dose administration. Blood was taken from the jugular vein. In all cases, after administration, animals did not have access to water for 30 minutes.

Cyclobenzaprine analysis was performed according to the analytical method described above. The method involved liquid-liquid extraction with hexane. The extracts were injected using an HPLC system and an AP14000® (Applied Biosystems) for MS/MS detection, with an "Onyx monolithic Phenomenex C18 100x3.0 mm" column. The method was linear from 0.1 to 50 ng/mL for cyclobenzaprine in dog plasma. Quality control samples were prepared at 0.3, 25, and 40 ng/mL for cyclobenzaprine in dog plasma. Results obtained from the analysis of quality control samples were within the limits of acceptance defined in the protocol, therefore verifying the concentrations measured in plasma samples.

Sublingual administration was performed by placing the control tablet or tablet with $K_2HPO_4$under the tongue when female beagles were slightly anesthetized with propofol. The results obtained showed that cyclobenzaprine in the tablet with $K_2HPO_4$ seemed to be about 25% more bioavailable than cyclobenzaprine when administered in the tablet without $K_2HPO_4$ (Figures 6 and 7; Tables 60-64). In addition, the AUC values calculated from 0 to 0.75 hours in beagles treated with cyclobenzaprine with $K_2HPO_4$ (AUC = 135.6 ng h/mL) versus control cyclobenzaprine (AUC = 82.4 ng h/mL) are quite different. Thus, the pharmacokinetic parameters associated with cyclobenzaprine in sublingual formulations appear to be improved with the addition of a basifying agent such as $K_2HPO_4$.

Table 60: Mean ± SD plasma PK parameters

| Formulation | Cmax (ng/mL) | Tmax* (h) | AUCt (ng/mL*h) | AUCinf (ng/mL*h) | %AUCextra | Kel (1/h) | $t_{1/2}$ (h) | Cl/F (L/h) | Vd/F (L) |
|---|---|---|---|---|---|---|---|---|---|
| **Buffered tablet** | 256.3 ± 108.5 | 0.33 | 176.6 ± 49.91 | 179.0 ± 50.23 | 1.390 ± 0.5573 | 0.3665 ± 0.05934 | 1.926 ± 0.2747 | 14.13 ± 3.297 | 40.06 ± 12.75 |
| **Unbuffered tablet** | 192.4 ± 76.69 | 0.33 | 151.6 ± 63.95 | 155.4 ± 64.57 | 2.489 ± 1.342 | 0.3001 ± 0.07501 | 2.425 ± 0.5932 | 17.18 ± 5.349 | 59.39 ± 20.12 |

* Median

Table 61: Individual cyclobenzaprine plasma concentrations, with $K_2HPO_4$

| Time Hours | Dog 1 | Dog 2 | Dog 3 | Dog 4 | Dog 5 | Low | High | Mean | S.D. | %CV | n |
|---|---|---|---|---|---|---|---|---|---|---|---|
| 0 | BLQ | BLQ | BLQ | BLQ | BLQ | BLQ | BLQ | BLQ | NA | NA | 5 |
| 0.08333 | 5.801 | 226.6 | 1.212 | *BLQ* | 1.689 | 0.000 | 226.6 | 47.06 | 100.4 | 213.34 | 5 |
| 0.16667 | 381.2 | 366.6 | 81.84 | 4.318 | 65.44 | 4.318 | 381.2 | 179.9 | 179.5 | 99.78 | 5 |
| 0.33333 | 101.7 | 242.6 | 165.3 | 120.3 | 112.2 | 101.7 | 242.6 | 148.4 | 57.97 | 39.06 | 5 |
| 0.5 | 45.39 | 171.9 | 117.6 | 165.7 | 202.8 | 45.39 | 202.8 | 140.7 | 61.38 | 43.62 | 5 |
| 0.75 | 56.10 | 81.05 | 49.61 | 68.50 | 99.84 | 49.61 | 99.84 | 71.02 | 20.12 | 28.33 | 5 |
| 1 | 25.39 | 63.98 | 29.21 | 99.64 | 34.43 | 25.39 | 99.64 | 50.53 | 31.38 | 62.10 | 5 |
| 2 | 12.61 | 26.63 | 14.55 | 16.46 | 21.07 | 12.61 | 26.63 | 18.26 | 5.632 | 30.84 | 5 |
| 3 | 8.422 | 12.27 | 8.636 | 9.107 | 9.878 | 8.422 | 12.27 | 9.663 | 1.561 | 16.15 | 5 |
| 4 | 3.454 | 6.701 | 8.924 | 5.240 | 7.067 | 3.454 | 8.924 | 6.277 | 2.053 | 32.71 | 5 |
| 6 | 1.880 | 3.258 | 2.515 | 2.188 | 4.122 | 1.880 | 4.122 | 2.793 | 0.9027 | 32.32 | 5 |
| 8 | 1.059 | 1.190 | 2.151 | 1.275 | 1.939 | 1.059 | 2.151 | 1.523 | 0.4887 | 32.09 | 5 |
| 10 | 0.4612 | 1.350 | 0.9891 | 0.5514 | 1.117 | 0.4612 | 1.350 | 0.8937 | 0.3780 | 42.30 | 5 |

BLQ: Below the Limit of Quantification (0.1 ng/mL)
*BLQ: Below the Limit of Quantification (1 ng/mL) (diluted plasma sample according to the protocol)*
NA: Not Applicable

Table 62: Individual cyclobenzaprine plasma concentrations, without $K_2HPO_4$

| Time Hours | Dog 1 | Dog 2 | Dog 3 | Dog 4 | Dog 5 | Low | High | Mean | S.D. | %CV | n |
|---|---|---|---|---|---|---|---|---|---|---|---|
| 0 | BLQ | BLQ | BLQ | BLQ | BLQ | BLQ | BLQ | BLQ | NA | NA | 5 |
| 0.08333 | 1.448 | 4.871 | *BLQ* | 72.10 | 6.170 | 0.000 | 72.10 | 16.92 | 30.95 | 182.92 | 5 |
| 0.16667 | 13.27 | 6.661 | 199.7 | 4.470 | 31.79 | 4.470 | 199.7 | 51.18 | 83.72 | 163.58 | 5 |
| 0.33333 | 137.6 | 98.65 | 178.0 | 214.6 | 147.1 | 98.65 | 214.6 | 155.2 | 43.65 | 28.13 | 5 |
| 0.5 | 46.23 | 71.56 | 57.51 | 322.8 | 76.76 | 46.23 | 322.8 | 115.0 | 116.8 | 101.57 | 5 |
| 0.75 | 41.83 | 154.8 | 51.25 | 38.07 | 44.52 | 38.07 | 154.8 | 66.09 | 49.82 | 75.38 | 5 |
| 1 | 42.66 | 46.86 | 22.13 | 127.3 | 30.35 | 22.13 | 127.3 | 53.86 | 42.21 | 78.37 | 5 |
| 2 | 14.85 | 19.42 | 10.73 | 30.58 | 11.29 | 10.73 | 30.58 | 17.37 | 8.156 | 46.95 | 5 |
| 3 | 8.077 | 11.78 | 6.866 | 11.82 | 6.506 | 6.506 | 11.82 | 9.010 | 2.613 | 29.00 | 5 |
| 4 | 5.598 | 6.742 | 3.844 | 6.271 | 4.108 | 3.844 | 6.742 | 5.313 | 1.289 | 24.26 | 5 |
| 6 | 2.186 | 3.386 | 2.565 | 2.809 | 2.222 | 2.186 | 3.386 | 2.634 | 0.4928 | 18.71 | 5 |
| 8 | 1.290 | 2.205 | 1.365 | 1.559 | 1.286 | 1.286 | 2.205 | 1.541 | 0.3874 | 25.14 | 5 |
| 10 | 0.4694 | 1.459 | 1.180 | 1.209 | 0.7364 | 0.4694 | 1.459 | 1.011 | 0.3991 | 39.48 | 5 |

BLQ: Below the Limit of Quantification (0.1 ng/mL)
*BLQ: Below the Limit of Quantification (1 ng/mL) (diluted plasma sample according to the protocol)*
NA: Not Applicable

Table 63: Pharmacokinetic parameters, individual beagle dogs

| Formulation | Animal | Cmax (ng/mL) | Tmax (h) | AUCt (ng/mL*h) | AUCinf (ng/mL*h) | %AUCextra | Kel (1/h) | t1/2 (h) | Cl/F (L/h) | Vd/F (L) |
|---|---|---|---|---|---|---|---|---|---|---|
| With $K_2HPO_4$ | Dog 1 | 381.2 | 0.167 | 136.5 | 137.9 | 1.012 | 0.3307 | 2.096 | 17.41 | 52.64 |
| | Dog 2 | 366.6 | 0.167 | 260.1 | 263.0 | 1.101 | 0.4663 | 1.486 | 9.124 | 19.57 |
| | Dog 3 | 165.3 | 0.33 | 140.0 | 142.8 | 1.942 | 0.3568 | 1.943 | 16.81 | 47.12 |
| | Dog 4 | 165.7 | 0.50 | 175.7 | 177.2 | 0.8532 | 0.3647 | 1.900 | 13.55 | 37.14 |
| | Dog 5 | 202.8 | 0.50 | 170.8 | 174.3 | 2.040 | 0.3141 | 2.207 | 13.77 | 43.84 |
| | N | 5 | 5 | 5 | 5 | 5 | 5 | 5 | 5 | 5 |
| | Mean | 256.3 | 0.3328 | 176.6 | 179.0 | 1.390 | 0.3665 | 1.926 | 14.13 | 40.06 |
| | Stdev | 108.5 | 0.1665 | 49.91 | 50.23 | 0.5573 | 0.05934 | 0.2747 | 3.297 | 12.75 |
| | %CV | 42.34 | 50.03 | 28.26 | 28.06 | 40.11 | 16.19 | 14.26 | 23.33 | 31.84 |
| | SEM | 48.54 | 0.07446 | 22.32 | 22.46 | 0.2492 | 0.02654 | 0.1229 | 1.474 | 5.704 |
| | Harmean | 223.5 | 0.2631 | 167.4 | 169.8 | 1.227 | 0.3598 | 1.891 | 13.41 | 35.44 |
| | GeoMean | 238.8 | 0.2967 | 171.7 | 174.1 | 1.304 | 0.3630 | 1.909 | 13.79 | 37.98 |
| | Median | 202.8 | 0.33 | 170.8 | 174.3 | 1.101 | 0.3568 | 1.943 | 13.77 | 43.84 |
| | Min | 165.3 | 0.167 | 136.5 | 137.9 | 0.8532 | 0.3141 | 1.486 | 9.124 | 19.57 |
| | Max | 381.2 | 0.50 | 260.1 | 263.0 | 2.040 | 0.4663 | 2.207 | 17.41 | 52.64 |
| Without $K_2HPO_4$ | Dog 1 | 137.6 | 0.33 | 110.2 | 111.4 | 1.020 | 0.4132 | 1.678 | 21.55 | 52.15 |
| | Dog 2 | 154.8 | 0.75 | 154.6 | 161.6 | 4.291 | 0.2105 | 3.293 | 14.86 | 70.58 |
| | Dog 3 | 199.7 | 0.167 | 125.4 | 129.7 | 3.287 | 0.2768 | 2.504 | 18.51 | 66.86 |
| | Dog 4 | 322.8 | 0.50 | 260.9 | 264.7 | 1.410 | 0.3240 | 2.139 | 9.068 | 27.99 |
| | Dog 5 | 147.1 | 0.33 | 106.9 | 109.5 | 2.435 | 0.2761 | 2.511 | 21.91 | 79.36 |
| | N | 5 | 5 | 5 | 5 | 5 | 5 | 5 | 5 | 5 |
| | Mean | 192.4 | 0.4154 | 151.6 | 155.4 | 2.489 | 0.3001 | 2.425 | 17.18 | 59.39 |
| | Stdev | 76.69 | 0.2210 | 63.95 | 64.57 | 1.342 | 0.07501 | 0.5932 | 5.349 | 20.12 |
| | %CV | 39.86 | 53.21 | 42.18 | 41.56 | 53.94 | 24.99 | 24.46 | 31.14 | 33.87 |
| | SEM | 34.30 | 0.09884 | 28.60 | 28.88 | 0.6004 | 0.03355 | 0.2653 | 2.392 | 8.996 |
| | Harmean | 174.6 | 0.3251 | 136.2 | 139.7 | 1.896 | 0.2858 | 2.310 | 15.45 | 51.74 |
| | GeoMean | 182.4 | 0.3688 | 142.9 | 146.6 | 2.181 | 0.2928 | 2.367 | 16.37 | 55.92 |
| | Median | 154.8 | 0.33 | 125.4 | 129.7 | 2.435 | 0.2768 | 2.504 | 18.51 | 66.86 |
| | Min | 137.6 | 0.167 | 106.9 | 109.5 | 1.020 | 0.2105 | 1.678 | 9.068 | 27.99 |
| | Max | 322.8 | 0.75 | 260.9 | 264.7 | 4.291 | 0.4132 | 3.293 | 21.91 | 79.36 |

Table 64: Relative bioavailability, individual beagle dogs

| Animal | **F** (%) = **AUCinf** (**with** $K_2HPO_4$)/**AUCinf** (**without** $K_2HPO_4$) * **100** |
|---|---|
| **Dog 1** | 123.79 |
| **Dog 2** | 162.75 |
| **Dog 3** | 110.10 |
| **Dog 4** | 66.94 |
| **Dog 5** | 159.18 |
| **N** | 5 |
| **Mean** | 124.55 |
| **Stdev** | 39.33 |
| **%CV** | 31.57 |
| **SEM** | 17.59 |
| **Harmean** | 112.29 |
| **GeoMean** | 118.77 |
| **Median** | 123.79 |
| **Min** | 66.94 |
| **Max** | 162.75 |

**Example 11**

To study whether sublingual cyclobenzaprine is efficiently absorbed in humans, a solution for sublingual administration containing 2.4 mg of cyclobenzaprine HCl (2.4 mg/mL) formulation in an aqueous solution containing $K_2HPO_4$ at pH 7.0 - 7.4 may be used in lieu of sublingual tablets that would introduce a disintegration factor. Sublingual administration of cyclobenzaprine in the context of the invention may take place through, *inter alia,* sublingual tablets or a liquid solution. As described above, with a sublingual formulation, cyclobenzaprine is expected to be more bioavailable and to provide more predictable absorption of cyclobenzaprine than oral tablets, such as those currently available. As a result, patients may be less likely to receive too little drug to receive therapeutic effect, and yet may also be less likely to be overdosed, reducing the potential for side-effects, i.e., next-day drowsiness and/or noncompliance due to intolerance. Sublingual administration also is expected to bypass the first-pass hepatic metabolism that results in, among other metabolites, demethylation of cyclobenzaprine to norcyclobenzaprine.

The extent to which the pH of sublingual oral solutions affects the rate or efficiency of sublingual absorption is not completely understood. The results described in Example 9 found rapid absorption at pH 7.4. To more completely characterize the extent to which absorption is affected by the pH of oral solution formulations, the cyclobenzaprine HCl formulation 2.4 mg sublingual solution was tested at a pH of 7.4 and a pH of 3.5 (as a control). To establish a baseline for absolute bioavailability, cyclobenzaprine 2.4 mg also was administered as an IV solution (0.6 mg/mL) at pH 7.4.

The single-center, randomized, open-label, single-dose, comparative, parallel-design pharmacokinetic study described below was appropriately designed to compare the rate and extent of absorption of cyclobenzaprine HCl formulation 2.4 mg sublingual solution (2.4 mg/mL) at pH 7.4 versus cyclobenzaprine HCl formulation 2.4 mg sublingual solution (2.4 mg/mL) at pH 3.5 (control), oral cyclobenzaprine 5 mg immediate-release tablets, and intravenous cyclobenzaprine at a dose of 2.4 mg (0.6 mg/mL) in an aqueous solution containing $K_2HPO_4$ at pH 7.4 (control). The safety and tolerability of the cyclobenzaprine HCl formulation 2.4 mg sublingual solution at pH 3.5 and 7.4 also was assessed and compared to the safety and tolerability of cyclobenzaprine 5 mg tablets and cyclobenzaprine 2.4 mg intravenous solution.

Potential subjects were screened by medical and psychiatric history and laboratory and physical examinations 2 to 30 days prior to dose administration. Baseline evaluations were conducted and subjects are randomly assigned to a formulation/treatment 1 day prior to drug administration (Day -1). The next morning, after all pre-dose assessments were completed and eligible subjects agreed to continue, subjects were randomly assigned to study medication and received the assigned dose of test or reference drug. Subjects were required to have fasted for at least 10 hours prior to dosing and for 4 hours thereafter.

Subjects were confined from at least 10 hours before dosing until after the 72-hour discharge procedures. Subjects were required to remain seated or semi-reclined and to avoid lying down or sleeping, unless medically necessary or procedurally required, for up to 4 hours after administration of the assigned study drug. Due to the long confinement period, supervised outings were permitted during the period of confinement. No outing was allowed on the day of dosing (Day 1).

Tests for thyroid-stimulating hormone (TSH), human immunodeficiency virus types 1 and 2 (HIV1 and HIV2), hepatitis B (HBsAg), and hepatitis C (HCAb) are conducted at screening only, and urine drug screens, alcohol breath tests, and urine cotinine tests were conducted at screening and admission. Physical examinations, laboratory tests, vital signs, 12-lead electrocardiograms (ECGs), weight/body mass index (BMI), and pregnancy tests were performed at specified intervals. Monitoring for adverse events (AEs) and concomitant medications were conducted continuously. Blood and urine samples were collected at specified intervals for the measurement of levels of cyclobenzaprine and norcyclobenzaprine in plasma and urine. A post-study follow-up telephone call was scheduled 10 $\pm$ 3 days after administration of the assigned study medication. For subjects who discontinued prematurely, every effort was made to complete the discharge assessments and the follow-up telephone call.

The objective of comparing the rate and extent of absorption of sublingual cyclobenzaprine HCl solution 2.4 mg (2.4 mg/mL) in an aqueous solution containing $K_2HPO_4$ at pH 3.5 versus sublingual cyclobenzaprine HCl solution 2.4 mg (2.4 mg/mL) in aqueous solution containing $K_2HPO_4$ at pH 7.4 was met through analysis of multiple plasma and urine samples collected from the subjects and by comparing those associated with the two formulations. The objective of comparing the rate and extent of absorption of sublingual cyclobenzaprine HCl solution 2.4 mg (2.4 mg/mL) at pH 3.5 and pH 7.4 (test formulations) versus oral cyclobenzaprine 5 mg tablets, and intravenous cyclobenzaprine 2.4 mg (0.6 mg/mL) in an aqueous solution containing $K_2HPO_4$ (reference formulations) was met through analysis of multiple plasma and urine samples collected from the subjects and comparing those associated with these formulations or treatments. The objective of assessing the safety and tolerability of cyclobenzaprine HCl 2.4 mg (2.4 mg/mL) sublingual solution formulations at pH 3.5 and 7.4 was addressed by monitoring AEs, clinical laboratory values, vital signs, 12-lead ECGs, weight/BMI, concomitant medications, and overall well-being prior to, during, and at the close of the 4-day in-house dosing period.

Before undergoing any study-related screening procedures, each potential subject provided signed informed consent. The investigator determined the potential subject's suitability for the study by interviewing the subject and by performing per-protocol screening assessments. Subjects were administered a single-dose treatment according to a block randomization scheme. Six subjects were randomly assigned to each of the four groups, for a total enrollment of 24 subj ects.

The four treatments were as follows:

Treatment A (control): 1 dose of 2.4 mg cyclobenzaprine HCl sublingual solution (2.4 mg/mL) in aqueous solution containing $K_2HPO_4$ at pH 3.5, administered as 1 mL held under the tongue for 90 seconds without swallowing

Treatment B: 1 dose of 2.4 mg cyclobenzaprine HCl sublingual solution (2.4 mg/mL) in aqueous solution containing $K_2HPO_4$ at pH 7.4, administered as 1 mL held under the tongue for 90 seconds without swallowing

Treatment C (control): 1 dose of 5 mg cyclobenzaprine immediate-release tablets, swallowed with 240 mL of room-temperature water

Treatment D (control): 1 dose of 2.4 mg cyclobenzaprine USP in aqueous solution containing $K_2HPO_4$ at pH 7.4 (0.6 mg/mL), administered intravenously as a 4 mL bolus injection over 30 seconds

Each subject participated for up to approximately 43 days, including an up to 30-day screening period, a 4-day in-house dosing period, and a follow-up telephone call 10 $\pm$ 3 days after study drug administration.

The low-dose cyclobenzaprine HCl sublingual solution 2.4 mg (2.4 mg/mL) was administered sublingually via Becton Dickinson 1 mL needle-less syringe. This sublingual solution consisted of cyclobenzaprine USP dissolved in aqueous solution containing $K_2HPO_4$ at a concentration of 2.4 mg/mL. The solution was manufactured as two formulations that were identical except that one is provided at pH 7.4 and the other is provided at pH 3.5. The two cyclobenzaprine HCl 2.4 mg sublingual formulations were filled in single-use 3 mL vials (1.5 mL per 3 mL vial), labeled, packaged, and provided for use in the study.

Cyclobenzaprine intravenous solution containing 0.6 mg/mL of cyclobenzaprine USP was used as a reference comparator in this trial. This solution was identical to the cyclobenzaprine HCl 2.4 mg sublingual solution except that it was formulated to a concentration of 0.6 mg/mL, with a pH adjusted to 7.4. This cyclobenzaprine 2.4 mg intravenous solution was filled in sterile 10 mL single-use vials (5 mL per 10 mL vial), labeled, packaged, and provided for use in the study.

Blood and urine samples for pharmacokinetic analysis were collected and vital signs recorded pre-dose and at specified intervals after dosing. Laboratory tests and 12-lead ECGs were conducted on Day -1 and prior to discharge on the morning of Day 4. A serum β-HCG pregnancy test was conducted for all female subjects on Day -1. A urine β-HCG pregnancy test was conducted for all female subjects prior to discharge on the morning of Day 4. Monitoring for adverse events and concomitant medications was conducted continuously.

For pharmacokinetic analysis, a total of 25 blood samples (6 mL per sample) are taken: within 30 minutes pre-dose and 5, 10, 20, 30, and 45 minutes and 1, 2, 2.5, 3, 3.33, 3.67, 4, 4.33, 4.67, 5, 5.5, 6, 8, 12, 16, 24, 36, 48, and 72 hours post-dose. Actual sampling times were used for statistical analyses. Unless otherwise specified or for subject safety, when blood draws and other procedures coincide, blood draws had precedence. A dead-volume intravenous catheter was used for blood collection to avoid multiple skin punctures, when appropriate. Otherwise, blood samples were collected by direct venipuncture. Charts of daily and hourly assessments are shown in Figures 8 and 9, respectively.

Also for pharmacokinetic analysis, a single urine sample was collected within 30 minutes pre-dose (one sample), after which urine was pooled for the duration of the dosing period, from 0 to 24, 24 to 48, and 48 to 72 hours post-dose. If a subject could not void his or her bladder within 30 minutes before dosing, a sample from earlier that morning may have beeb used as the pre-dose sample. Urine voided by subjects within 10 minutes of the end of the interval were included in the earlier sample. Subjects were asked to void their bladders within 5 minutes before the end of each collection interval, so that each new interval would begin with an empty bladder. Any urine voided by subjects but not collected was documented. Pharmacokinetic parameters (plasma) included AUCo-t, $AUC_{0\text{-inf}}$, $C_{max}$, Residual area, $T_{max}$, $T_{½el}$, $K_{el}$, and F. F was calculated for the sublingual and oral formulations of cyclobenzaprine only. Pharmacokinetic parameters (urine) included Aeo-t, $R_{max}$, and $T_{max}$.

For blood samples, ANOVA was performed on $T_{max}$, $K_{el}$, and $T_{½\ el}$ and on AUCo-t, $AUC_{0\text{-inf}}$, and $C_{max}$ at the alpha level of 0.05. The ratio of means (treatments A/B, A/C, A/D, B/C, and B/D) and 90% geometric confidence interval (CI) for the ratio of means, based on least-squares means from the ANOVA of the In-transformed data, was calculated for AUCo-t, $AUC_{0\text{-inf}}$, and $C_{max}$. For all analytes, the ratio of means (treatments A/B, A/C, A/D, B/C, and B/D) and 90% geometric CI for the ratio of means, based on least-squares means from the ANOVA of the In-transformed data, were calculated for Aeo-t and $R_{max}$.

Discharge from the study unit occurred following completion of the scheduled discharge assessments 72 ± 1.5 hours following study drug administration (morning of Day 4). Barring safety concerns, the subjects were discharged from the study unit after these examinations (i.e., 4 days after dosing), at the discretion of the investigator.

A follow-up call was made to each subject by a study staff member 3-9 days subsequent to discharge from the study unit (that is, 10 ± 3 days after dosing). The subject was asked to report any adverse events he or she may have experienced since discharge from the study unit. The subject was considered to have completed the study after the follow-up call.

This experiment was designed to be a stringent test of transmucosal absorption after sublingual administration of the oral cyclobenzaprine solution, so subjects were instructed to hold the solution under their tongues for 90 seconds, spit out the contents of their mouths, rinse their mouths with 60 mL of water, and then drink 240 mL of water. Unlike the anesthetized beagles, some of the alert humans were expected to swallow part of the sublingual solutions as part of an involuntary reflex. Table 65 shows the results of the study.

Table 65: Mean plasma cyclobenzaprine pharmacokinetics

| Treatment | N | $AUC_{0\text{-t}}$ (pg•hr/mL) | $AUC_{0\text{-inf}}$ (pg•hr/mL) | Residual Area (%) | Cmax (pg/mL) | Tmax (hr) | $T_{½}$el (hr) | $K_{el}$ (1/hr) |
|---|---|---|---|---|---|---|---|---|
| TNX-102 2.4 mg SL solution at pH 7.1 | 4[a] | 16,065 | 20,304 | 20.5 | 742.7 | 5.208 | 35.1 | 0.020275 |
| TNX-102 2.4 mg SL solution at pH 3.5 | 4[b] | 2,083 | 3,142 | 33.5 | 180.6 | 5.208 | 13.5 | 0.0833 |
| Cyclobenzapr ine 2.4 mg IV solution | 6 | 65,751 | 78,151 | 15.9 | 10,652.2 | 0.041 | 31.5 | 0.0224 |
| Cyclobenzapr ine 5 mg oral tablets [c] | 6 | 57,840 | 69,696 | 14.8 | 3,019.6 | 5.470 | 28.4 | 0.0256 |

[a] The means for 2.4 mg sublingual solution at pH 7.1 exclude Subject 7 (outlier) and Subject 10, who appeared to have swallowed the sublingual solution medication.
[b] The means for 2.4 mg sublingual solution at pH 3.5 exclude Subject 4, who appeared to have swallowed the sublingual solution medication.
[c]Each subject received a single cyclobenzaprine 5 mg oral tablet.

As expected, several subjects swallowed their sublingual doses. Subject 7, who was excluded from the analysis summarized in Table 65 because her absorption of a sublingual dose was so much more rapid than that of others given one of the sublingual treatments, seems likely to have been the most fully compliant with the intended dosing procedure and to best represent the potential of sublingual dosing. Analysis of the data show that Subject 7 in the cohort receiving 2.4 mg sublingual solution at pH 7.1 had a pharmacokinetic profile strikingly similar to the mean of the entire cohort receiving 2.4 mg IV solution during the 0-to 1-hour time frame, while the cohort receiving cyclobenzaprine 5 mg tablets showed almost no absorption during this time. The efficiency of absorption in Subject 7 was approximately half the absorption in the cohort receiving 2.4 mg IV solution. The concentration-time profiles of the cohort receiving 2.4 mg IV solution and of Subject 7 in the cohort receiving 2.4 mg sublingual solution at pH 7.1 had two distinct phases in the first hour, with the first phase showing a rapid increase and clearance from plasma before 5 min (0.83 h) and a second phase showing relatively flat plasma concentrations from 10 min (0.167 hour) to 60 min (1 hour). The plasma levels for oral dosing shown in Figure 10 were decreased by a factor of 2.4/5.0 to facilitate comparison of data for the 2.4 mg sublingual (SL) solution to data for the cyclobenzaprine 5 mg oral (PO) tablet group, assuming dose proportionality. During the first hour, the plasma samples were obtained at 0 min, 2 min (0.033 h), 3 min (0.058 h), 5 min (0.083 hr), 10 min (0.167 h), 20 min (0.33 h), 30 min (0.5 h), 45 min (0.75 h) and 60 min (1 h) (Figure 10).

Preliminary analysis of pharmacokinetic data over 24 hours showed that Subject 7, in the cohort receiving 2.4 mg SL solution at pH 7.1, continued to show a concentration-time profile over 24 hours similar to that of the entire cohort receiving 2.4 mg IV solution. Analysis of the pharmacokinetic data over 24 hours also showed that the plasma levels of the cohort receiving 2.4 mg IV solution fell rapidly between hours 1 and 5, while the plasma levels of the cohort receiving cyclobenzaprine 5 mg tablets increased to $T_{max}$ at approximately 5 hours. The plasma levels of the cohort receiving 2.4 mg IV solution showed a small increase after 5 hours consistent with some of the initial dose entering the bile and subsequently being taken up by the portal circulation (enterohepatic recirculation). The plasma levels for oral dosing shown were decreased by a factor of 2.4/5.0 to facilitate comparison of data for the 2.4 mg SL solution to data for the cyclobenzaprine 5 mg oral tablet group, assuming dose proportionality (Figure 11).

Because Subject 7 was an outlier, we also compared the mean pharmacokinetics of four of the other subjects from the cohort receiving 2.4 mg SL solution at pH 7.1 against subjects receiving 2.4 mg SL solution at pH 3.5 and subjects receiving cyclobenzaprine 5 mg oral tablets. The group receiving 2.4 mg SL solution at pH 7.1 showed a rapid rise in plasma cyclobenzaprine over the first hour (Figure 12). In contrast, very low levels of cyclobenzaprine were observed over this time period in either the cohort receiving 2.4 mg SL solution at pH 3.5 or the cyclobenzaprine 5 mg oral tablet cohort. The group mean for 2.4 mg SL solution at pH 7.1 excludes Subject 7 (outlier) and Subject 10 (who appeared to have swallowed the SL solution medication), and the group mean for 2.4 mg SL solution at pH 3.5 excludes Subject 4 (who appeared to have swallowed the SL solution medication). The plasma levels shown for cyclobenzaprine 5 mg oral tablets were decreased by a factor of 2.4/5.0 to facilitate comparison of data for the 2.4 mg SL solution groups to data for the cyclobenzaprine 5 mg oral tablet group, assuming dose proportionality. These findings indicated that the phosphate in the 2.4 mg SL solution at pH 7.1 was associated with increased transmucosal absorption of cyclobenzaprine after sublingual administration.

In order to determine whether sublingual administration of 2.4 mg SL solution (cyclobenzaprine HCl) had an effect on the formation of the long-lived metabolite, norcyclobenzaprine, plasma levels of this metabolite were determined. As shown in Figure 13, levels of norcyclobenzaprine from the IV cohort and from Subject 7 trended lower than the group mean observed for the cyclobenzaprine 5 mg oral tablet cohort, which is consistent with a reduction in first-pass metabolism by sublingual or IV administration.

The 2.4 mg SL solution at pH 7.1 had a benign safety profile. All treatment emergent adverse events (TEAEs) in this cohort were mild, and all but one (lipase increased) had resolved by the time of discharge from the study site (the lipase elevation was detected in a sample obtained at discharge, and the subject could not be reached for follow-up). TEAEs in all four cohorts were mild or moderate and generally compatible with the labeling for marketed cyclobenzaprine tablets.

An additional pharmacokinetic analysis of the data was performed by calculating partial AUCs from time 0 hr to each sampling time point up to 8 hours for each individual subject. The partial AUC data in group C was then dose-normalized to the same dose as in group B (5 mg to 2.4 mg), assuming linear pharmacokinetics. Geometric means were calculated for the partial AUCs in the two groups. Geometric means were preferred compared to arithmetic means due to the usually log-normal distribution of pharmacokinetic parameters such as AUC. Finally, the partial AUCs among the group were statistically compared. In line with the using geometric means, the partial AUCs were log-transformed prior to performing a t-test for unpaired samples. The results indicated that AUC(0-0.5hr), AUC(0-0.75hr), AUC(0-1hr), AUC(0-2hr) and AUC(0-2.5hr) were significantly higher after the administration of the phosphate-containing solution as compared to the tablet (Table 66). This means that at any time for the first 2.5hr after dosing, the phosphate-containing solution achieved a higher systemic exposure as compared to the tablet.

Table 66: Partial AUC for Groups B and C

| Group | $AUC_{0-0.5}$ hr | $AUC_{0-0.75}$ hr | $AUC_{0-1\ hr}$ | $AUC_{0-2}$ hr | $AUC_{0-2.5}$ hr |
|---|---|---|---|---|---|
| B | 73 | 152 | 246 | 828 | 1210 |
| C | 1.0 | 5.4 | 22 | 296 | 564 |
| p-value | 0.0068 | 0.0094 | 0.0037 | 0.0870 | 0.1419 |

**Example 12**

A single-dose, open-label, randomized, parallel-design study of the comparative pharmacokinetics and safety of sublingual cyclobenzaprine tablets was performed. The study compared sublingual cyclobenzaprine tablets (with phosphate) at 2.4 mg and 4.8 mg doses, sublingual cyclobenzaprine tablets (without phosphate) at 2.4 mg, and cyclobenzaprine 5 mg oral tablets. The study compares 1) the rate and extent of absorption of 2.4 mg sublingual cyclobenzaprine HCl tablets with and without phosphate; and 2) the rate and extent of absorption of 2.4 mg sublingual cyclobenzaprine HCl tablets (with phosphate) administered at doses of 2.4 mg and 4.8 mg vs. 2.4 mg sublingual cyclobenzaprine HCl tablets (without phosphate) at a 2.4 mg dose vs. cyclobenzaprine 5 mg oral tablets; and 3) assesses the safety and tolerability of 2.4 mg sublingual cyclobenzaprine HCl tablets (with phosphate) at doses of 2.4 mg and 4.8 mg vs. 2.4 mg sublingual cyclobenzaprine HCl tablets (without phosphate) at a 2.4 mg dose vs. cyclobenzaprine 5 mg oral tablets.

Patients were selected substantially as described in Example 11. Cyclobenzaprine was administered as follows:

Treatment A: A single dose of one TNX-102 2.4 mg SL tablet (with phosphate). Subjects were asked to keep the tablet under the tongue until dissolved and not to crush or chew it. Subjects were asked not to drink any water until at least 1 hour after dosing.

Treatment B (control): A single dose of one TNX-102-A 2.4 mg SL tablet (without phosphate). Subjects were asked to keep the tablet under the tongue until dissolved and not to crush or chew it. Subjects were asked not to drink any water until at least 1 hour after dosing.

Treatment C: A single dose of two TNX-102 2.4 mg SL tablets (with phosphate). Subjects will be asked to keep the tablets under the tongue until dissolved and not to crush or chew them. Subjects were asked not to drink any water until at least 1 hour after dosing.

Treatment D (control): A single dose of one cyclobenzaprine 5 mg oral immediate-release tablet (Watson Pharmaceuticals), to be swallowed with 240 mL of room-temperature water. Subjects were asked to swallow the administered tablet whole and not to crush or chew it.

The sublingual cyclobenzaprine HCl tablets were well tolerated, with no serious adverse affects, although some subjects experienced numbness of the tongue. Compared to 5 mg of oral cyclobenzaprine, 4.8 mg of sublingual cyclobenzaprine HCl significantly increased the rate of absorption in the first two hours after administration (Figures 14 and 15). Indeed, at some time points, the sublingual cyclobenzaprine produced approximately 20 fold higher mean dose-adjusted plasma levels of cyclobenzaprine as compared to oral administration. The sublingually administered cyclobenzaprine also resulted in a higher AUC than the orally administered cyclobenzaprine. Sublingual cyclobenzaprine HCl also resulted in approximate dose proportionality between the 2.4 mg and the 4.8 mg doses (Figure 16). Additionally, the sublingual tablets with phosphate showed a trend towards faster cyclobenzaprine absorption than the tablets without phosphate (Figure 17). Table 67 shows the statistical significance of partial AUC analysis using a one-way ANOVA test with Bonferroni one-way comparisons between the different treatment groups.

Partial AUC at different times were calculated to examine the effect of sublingual administration on absorption. Statistical comparison for In-transformed AUC data was analyzed by One-Way ANOVA with Bonferroni one-way comparisons (95% confidence level). Comparing the partial AUC for cyclobenzaprine HCl SL 2.4 mg (1 tablet) versus an AUC for cyclobenzaprine 5 mg IR tablets (dose-normalized to 2.4 mg) revealed a statistical significant increase in absorption for the SL 2.4 mg 1 tablet administration versus cyclobenzaprine 5 mg IR: $AUC_{0-20min}$, 37 ng hr $L^{-1}$ vs. 0 ng hr $L^{-1}$, $p < 0.05$; $AUC_{0-30min}$, 128 ng hr $L^{-1}$ vs. 1 ng hr $L^{-1}$, $p < 0.05$; $AUC_{0-45min}$, 333 ng hr $L^{-1}$ vs. 2 ng hr $L^{-1}$, $p < 0.05$; $AUC_{0-1h}$, 614 ng hr $L^{-1}$ vs. 5 ng hr $L^{-1}$, $p < 0.05$; $AUC_{0-2h}$, 2098 ng hr $L^{-1}$ vs. 386 ng hr $L^{-1}$, $p < 0.05$; $AUC_{0-2.5h}$, 2955 ng hr $L^{-1}$ vs. 791 ng hr $L^{-1}$, $p < 0.05$; $AUC_{0-3h}$, 3931 ng hr $L^{-1}$ vs. 1355 ng hr $L^{-1}$, $p < 0.05$.

Partial AUC at different times were analyzed to show the effect of cyclobenzaprine HCl SL administration on absorption. Comparing the mean partial AUC for 2 tablets of 2.4 mg cyclobenzaprine HCl versus a mean partial AUC for cyclobenzaprine 5 mg IR tablets (dose-normalized to 2.4 mg) revealed a statistically significant increase in absorption: $AUC_{0\text{-}20min}$, 23 ng hr $L^{-1}$ vs. 0 ng hr $L^{-1}$, $p < 0.05$; $AUC_{0\text{-}30min}$, 86 ng hr $L^{-1}$ vs. 1 ng hr $L^{-1}$, $p < 0.05$; $AUC_{0\text{-}45min}$, 223 ng hr $L^{-1}$ vs. 2 ng hr $L^{-1}$, $p < 0.05$; $AUC_{0\text{-}1h}$, 405 ng hr $L^{-1}$ vs. 5 ng hr $L^{-1}$, $p < 0.05$; $AUC_{0\text{-}2h}$, 1478 ng hr $L^{-1}$ vs. 386 ng hr $L^{-1}$, $p < 0.05$; $AUC_{0\text{-}2.5h}$, 2167 ng hr $L^{-1}$ vs. 791 ng hr $L^{-1}$, $p < 0.05$.

Table 67: One-Way ANOVA w/Bonferroni one-way comparisons (95% confidence level)

| Comparison/p-value | $AUC_{0-20min}$ | $AUC_{0-30min}$ | $AUC_{0-45min}$ | $AUC_{0-1hr}$ | $AUC_{0-}2hr$ | $AUC_{0-2.5hr}$ | $AUC_{0-}3hr$ | $AUC_{0-3.33hr}$ |
|---|---|---|---|---|---|---|---|---|
| A vs. D | <0.05 | <0.05 | <0.05 | <0.05 | <0.05 | <0.05 | <0.05 | - |
| B vs. D | <0.05 | <0.05 | <0.05 | <0.05 | <0.05 | <0.05 | - | - |
| C vs. D | <0.05 | <0.05 | <0.05 | <0.05 | <0.05 | <0.05 | - | - |

**Reference Example 13**

Although cyclobenzaprine has been shown to interact with both the serotonergic and noradrenergic receptor systems, the functional interactions of cyclobenzaprine with isolated receptors are not fully characterized and those of norcyclobenzaprine are unknown. Therefore, plasma norcyclobenzaprine was measured in healthy subjects after oral administration of cyclobenzaprine and the binding and functional activity of cyclobenzaprine and norcyclobenzaprine was studied on a set of CNS targets with potential relevance to cyclobenzaprine actions.

Cyclobenzaprine and norcyclobenzaprine were screened on a broad panel of receptors, channels, enzymes and transporters. Equilibrium receptor binding assays were performed on cell lines expressing select recombinant human serotonin, adrenergic, histamine, and muscarinic receptors. Select receptors also were analyzed for functional antagonism in ligand-induced intracellular calcium mobilization and β-arrestin signaling. Figures 18a-h depict the equilibrium binding of cyclobenzaprine (circles) and norcyclobenzaprine (triangles) to cells expressing various recombinant human receptors. In particular, Figure 18b depicts binding to the 5-$HT_{2A}$ receptor from a Ki about approximately $10^{-6.8}$ to $10^{-8.8}$, which may be consistent with a dynamic effect of bedtime dosing on the central nervous system despite baseline levels of cyclobenzaprine in the blood or central nervous system. Table 68 also shows the Hill plot (also called the Hill slope or the slope factor) ratios depicting the slopes of the curves for the receptors of Figures 18a-h. A wide dynamic range is indicated by a slope ratio of less than 1, while a narrow dynamic range is indicated by a slope ratio of greater than 1. A narrow dynamic range means that over a narrow range of concentrations of cyclobenzaprine, if the cyclobenzaprine concentration is increasing, then the population of receptors (e.g., the H-1 receptors) will go from unoccupied to occupied. Likewise, if the concentration of cyclobenzaprine is decreasing, then the population of receptors will go from occupied to unoccupied. A broad dynamic range means that over a wide range of concentrations of cyclobenzaprine, if the cyclobenzaprine concentration is increasing, then the population of receptors (e.g., 5HT2A receptors) will go from unoccupied to occupied. Likewise, if the concentration of cyclobenzaprine is decreasing, then the receptors will go from occupied to unoccupied. Because $K_i$ is the point of inflection (50:50 bound:unbound) in the curve, at a concentration of cyclobenzaprine of 10 times the $K_i$, a "narrow dynamic range" receptor would be expected to have Henderson-Hasselbach-like characteristics and the receptor would be expected to be 95:5 bound:unbound and in a range beyond the linear range where more ligand would bind a diminishingly small amount of receptor. However, for a "broad dynamic range" receptor, it would be expected that the receptor would be less than 95% bound and still in a linear range where more ligand would bind more receptor. These considerations are important because the $K_i$ values for the 5HT2A and H-1 receptors are close to the therapeutic levels of cyclobenzaprine, where 2.75 ng/ml of cyclobenzaprine (10 nM) is in the plasma. Therapeutically, bedtime doses of cyclobenzaprine should change the occupancy of the 5HT2A receptors and the H-1 receptors, but 5HT2A receptors should be affected to a greater degree because the 5HT2A receptor has a broad dynamic range. Figure 19 depicts similar binding studies for transporters expressed in the central nervous system. Table 69 shows the binding affinities and functional potency of cyclobenzaprine and norcyclobenzaprine on various central nervous system proteins.

Table 68: Hill plot ratios (or Hill slope)

| Receptor | Cyclobenzaprine | Norcyclobenzaprine |
|---|---|---|
| 5-HT1A | 0.87 | 4.07 |
| 5-HT2A | 0.86 | 0.50 |
| 5-HT2B | 1.24 | 1.10 |
| 5-HT2C | 1.13 | 1.40 |
| H1 | 1.61 | 1.04 |
| α-1A | 1.01 | 1.16 |
| M1 | 1.28 | 0.70 |
| D1 | 0.61 | 1.15 |

Table 69: Binding and functional potency of cyclobenzaprine and norcyclobenzaprine on targets expressed in the central nervous system

| | $K_i$ (nM) | | $IC_{50}$ (nM) (Antagonist) | | $EC_{50}$ (nM) (Agonist) | |
|---|---|---|---|---|---|---|
| | CBP | nCBP | CBP | nCBP | CBP | nCBP |
| 5-HT1A | 1100 | 76 | | 5300 | 3200 | |
| 5-$HT_{2A}$ | 5.2 | 13 | 230 | 140 | | |
| 5-$HT_{2A}$ | | | 210 (IP) | 450 (IP) | | |
| 5-$HT_{2A}$ | | | 99 (β-arrestin) | 181 (β -arrestin) | | |
| 5-$HT_{2B}$ | 15 | 12 | 100 | 580 | | |
| 5-$HT_{2B}$ | | | 760 (IP) | 1400 (IP) | | |
| 5-$HT_{2C}$ | 43 | 43 | 444 | 1220 | | |
| 5-$HT_{2C}$ | | | 770 (IP) | 2000 (IP) | | |
| 5-$HT_{5A}$ | 730 | 1600 | ND | ND | | |
| 5-$HT_6$ | 480 | 1400 | 2000 | 2800 | | |
| 5-$HT_7$ | 67 | 140 | ND | ND | | |
| $H_1$ | 1.3 | 5.9 | 5.2 | 16 | | |
| $H_1$ | | | 2.7 (β-arrestin) | 6.1 (β-arrestin) | | |
| $\alpha_{1A}$ | 5.6 | 34 | 4.9 | 16 | | |
| $\alpha_{1B}$ | 9.1 | 11 | 530 | 790 | | |
| $\alpha_{1B}$ | | | 144 (β -arrestin) | 173 (β -arrestin) | | |
| $\alpha_{2A}$ | 360 | 1800 | 4300 | 6400 | | |
| $\alpha_{2B}$ | 21 | 150 | 9800 | 41000 (cAMP) | | |
| $\alpha_{2C}$ | 25 | 48 | NA | NA (cAMP) | | |
| $M_1$ | 7.9 | 30 | 0.71 | 8.7 | | |
| $M_1$ | | | 81 (β -arrestin) | 266 (β -arrestin) | | |
| $M_2$ | 250 | 76 | 3.3 | 33 | | |
| $D_1$ | 12 | 57 | 65 | 300 | | |
| $D_{2S}$ | 120 | 410 | ND | ND | | |
| $D_3$ | 34 | 98 | ND | ND | | |
| $D_{4.4}$ | 180 | 250 | ND | ND | | |
| $D_5$ | 60 | 280 | ND | ND | | |
| Dopamine TP | >10,000 | >10,000 | ND | ND | | |
| Norepinephrine | TP 35 | 2.6 | ND | ND | | |
| Serotonin TP | | 29 | 91 | ND | ND | |
| Sigma 1 | | 120 | 790 | ND | ND | |
| Sigma 2 | | 480 | 2000 | ND | ND | |

IP: inositol triphosphate; cAMP: 3'-5'-adenosine monophosphate; TP: transporter; NA: No Activity; ND: Not Done

After performing the binding studies described above, functional studies of cyclobenzaprine and norcyclobenzaprine on central nervous system receptors were performed. These assays tested both G-protein-dependent signal transduction by intracellular $Ca^{2+}$ mobilization (Figures 20a-h, Table 70), cAMP production or turnover of inositol triphosphate, and G-protein-independent signal transduction by β-arrestin signaling (Figures 21a-d, Table 71). Cyclobenzaprine and norcyclobenzaprine exhibited high affinity binding ($K_i$) to receptors: 5-$HT_{2A}$ ($K_i$ = 5.2 and 13 nM, respectively), 5-$HT_{2B}$ (15 and 12nM), and 5-$HT_{2C}$ (43 and 43 nM), adrenergic $\alpha_{1A}$ (5.6 and 34 nM), $\alpha_{1B}$ (9.1 and 11 nM), $\alpha_{2B}$ (21 and 150 nM) and $\alpha_{2C}$ (25 and 48 nM,); $H_1$ (1.3 and 5.9 nM); and $M_1$ (7.9 and 30 nM). Cyclobenzaprine and norcyclobenzaprine were functional antagonists of 5-$HT_{2A}$ ($IC_{50}$ = 230 and 140 nM), 5-$HT_{2B}$ (100 and 580 nM), $H_1$ (5.2 and 16 nM), $\alpha_{1A}$(4.9 and 16 nM), $M_1$ (0.71 and 8.7) and $M_2$ (3.3 and 33 nM) via $Ca^{2+}$ mobilization. By contrast, both cyclobenzaprine and norcyclobenzaprine were functional agonists of 5-HT1A ($EC_{50}$= 5.3 and 3.2 μM). Cyclobenzaprine and norcyclobenzaprine also were functional antagonists of 5-$HT_{2A}$ ($IC_{50}$ = 99 and 181 nM), $H_1$ (2.7 and 6.1 nM), $\alpha_{1B}$ (144 and 173 nM), and $M_1$ (81 and 266 nM) via β-arrestin signaling.

Table 70: Four parameter Hill slope (or fit slope) ratios; $Ca^{2+}$ signaling

| Receptor | Cyclobenzaprine | Norcyclobenzaprine |
|---|---|---|
| 5-HT1A | -0.97 | -0.79 |
| 5-HT2A | 0.96 | 1.27 |
| 5-HT2B | 1.71 | 1.54 |
| 5-HT2C | 0.96 | 0.94 |
| H1 | 1.22 | 0.79 |
| α-1A | 1.83 | 2.27 |
| M1 | 2.94 | 0.62 |
| D1 | 0.62 | 0.98 |

Table 71: Four parameter Hill slope (or fit slope) ratios; β-arrestin signaling

| Receptor | Cyclobenzaprine | Norcyclobenzaprine |
|---|---|---|
| H1 | 1.69 | 1.09 |
| α-1B | 0.73 | 0.70 |
| M1 | 0.97 | 1.05 |
| 5-HT2A | 0.99 | 1.11 |

**Reference Example 14**

An amitriptyline formulation (Formulation (a)) not containing the basifying agent was prepared by blending 0.049 g of amitriptyline hydrochloride with 0.052 g of sodium starch glycolate, 0.399 g of spray-dried lactose, and 0.200 g of microcrystalline cellulose. This powder was then blended with 0.024 g of magnesium stearate, and the entire mixture was compressed into a tablet. When placed in 15 mL of water, the tablet disintegrated in less than 30 seconds. The pH of the resulting slurry was measured to be 4.92.

An amitriptyline formulation (Formulation (b)) containing the basifying agent was prepared by first blending 0.051 g of amitriptyline hydrochloride 0.105 g of sodium bicarbonate. After mixing, this powder was blended with 0.052 g of sodium starch glycolate, 0.356 g of spray-dried lactose, and 0.205 g of microcrystalline cellulose. Finally, the resulting powder was blended with 0.025 g of magnesium stearate, and the entire mixture was compressed into a tablet. When placed in 15 mL of water, the tablet disintegrated in less than 30 seconds. The pH of the resulting slurry was measured to be 7.49.

Additional studies of formulations containing the basifying agent were performed following the procedure described above. After compression, tablets of each formulation were noted to disintegrate in less than 30 seconds when placed in 15 mL of water. The compositions of these formulations, and the pH of the resulting slurries, are summarized in Table 72.

Table 72: Amitriptyline formulations and pHs

| Ingredient | Formulation (c) | Formulation (d) | Formulation (e) | Formulation (f) | July 3, 2012Formulation (g) |
|---|---|---|---|---|---|
| Amitriptyline hydrochloride | 0.051 g | 0.050 g | 0.050 g | 0.049 g | 0.048 g |
| Basifying agent | $KH_2PO_4$: 0.099 g | $Na_2CO_3$: 0.100 g | $CaCO_3$: 0.098 g | TRIS: 0.101 g | Na citrate: 0.100 g |
| sodium starch glycolate | 0.050 g | 0.051 g | 0.050 g | 0.052 g | 0.051 g |

(continued)

| Ingredient | Formulation (c) | Formulation (d) | Formulation (e) | Formulation (f) | July 3, 2012Formulation (g) |
|---|---|---|---|---|---|
| spray-dried lactose | 0.349 g | 0.351 g | 0.356 g | 0.351 g | 0.354 g |
| microcrystalline cellulose | 0.200 g | 0.199 g | 0.201 g | 0.201 g | 0.204 g |
| magnesium stearate | 0.025 g | 0.025 g | 0.026 g | 0.025 g | 0.024 g |
| pH of slurry after disintegration of tablet in 15 mL of water | 5.83 | 10.33 | 7.10 | 8.86 | 7.28 |

## Claims

1. A composition suitable for oral transmucosal absorption comprising a pharmaceutically acceptable cyclobenzaprine salt and a basifying agent.

2. The composition of claim 1, wherein the oral transmucosal absorption is sublingual absorption.

3. The composition of claim 2, wherein the composition is in a form selected from the group consisting of a sublingual tablet, a sublingual film, a sublingual powder, and a sublingual spray solution.

4. The composition of any one of claims 1 to 3, wherein the pharmaceutically acceptable cyclobenzaprine salt is cyclobenzaprine HCl.

5. The composition of any one of claims 1 to 4, wherein the basifying agent is selected from the group consisting of potassium dihydrogen phosphate, dipotassium hydrogen phosphate, tripotassium phosphate, sodium carbonate, sodium bicarbonate, calcium carbonate, calcium bicarbonate, TRIS buffer, sodium dihydrogen phosphate, disodium hydrogen phosphate, trisodium phosphate, potassium carbonate, potassium bicarbonate, potassium acetate, sodium acetate, dipotassium citrate, tripotassium citrate, disodium citrate and trisodium citrate.

6. The composition of claim 5, wherein the basifying agent is dipotassium hydrogen phosphate.

7. The composition of claim 5, wherein the basifying agent is potassium dihydrogen phosphate.

8. A composition according to any one of claims 1 to 7 for use in a method for treating a disease or condition in an individual, the method comprising administering said composition by transmucosal absorption, wherein the disease or condition is post-traumatic stress disorder (PTSD).

9. A composition according to any one of claims 1 to 7 for use in a method for treating a disease or condition in an individual, the method comprising administering said composition by transmucosal absorption, wherein the disease or condition is selected from the group consisting of fibromyalgia, depression, traumatic brain injury, sleep disorder, non-restorative sleep, chronic pain, muscle spasm, and anxiety disorder.

## Patentansprüche

1. Zusammensetzung, die zur oralen transmukoesen Absorption geeignet ist, umfassend ein pharmazeutisch annehmbares Cyclobenzaprinsalz und ein Alkalisierungsmittel.

2. Zusammensetzung nach Anspruch 1, wobei die orale transmukoese Absorption eine sublinguale Absorption ist.

3. Zusammensetzung nach Anspruch 2, wobei die Zusammensetzung in einer Form vorliegt, die ausgewählt ist aus der Gruppe bestehend aus einer sublingualen Tablette, einem sublingualen Film, einem sublingualen Pulver und einer sublingualen Sprühlösung.

4. Zusammensetzung nach einem der Ansprüche 1 bis 3, wobei das pharmazeutisch annehmbare Cyclobenzaprinsalz Cyclobenzaprin-HCl ist.

5. Zusammensetzung nach einem der Ansprüche 1 bis 4, wobei das Alkalisierungsmittel ausgewählt ist aus der Gruppe bestehend aus Kaliumdihydrogenphosphat, Dikaliumhydrogenphosphat, Trikaliumphosphat, Natriumcarbonat, Natriumbicarbonat, Calciumcarbonat, Calciumbicarbonat, TRIS-Puffer, Natriumdihydrogenphosphat, Dinatriumhydrogenphosphat, Trinatriumphosphat, Kaliumcarbonat, Kaliumbicarbonat, Kaliumacetat, Natriumacetat, Dikaliumcitrat, Trikaliumcitrat, Dinatriumcitrat und Trinatriumcitrat.

6. Zusammensetzung nach Anspruch 5, wobei das Alkalisierungsmittel Dikaliumhydrogenphosphat ist.

7. Zusammensetzung nach Anspruch 5, wobei das Alkalisierungsmittel Kaliumdihydrogenphosphat ist.

8. Zusammensetzung nach einem der Ansprüche 1 bis 7 zur Verwendung in einem Verfahren zum Behandeln einer Erkrankung oder Krankheit bei einem Individuum, wobei das Verfahren das Verabreichen der Zusammensetzung durch transmukoese Absorption umfasst, wobei die Erkrankung oder Krankheit eine posttraumatische Belastungsstörung (PTBS) ist.

9. Zusammensetzung nach einem der Ansprüche 1 bis 7 zur Verwendung in einem Verfahren zum Behandeln einer Erkrankung oder Krankheit bei einem Individuum, wobei das Verfahren das Verabreichen der Zusammensetzung durch transmukoese Absorption umfasst, wobei die Erkrankung oder Krankheit ausgewählt ist aus der Gruppe bestehend aus Fibromyalgie, Depression, Schädel-Hirn-Trauma, Schlafstörung, nicht erholsamem Schlaf, chronischen Schmerzen, Muskelkrämpfen und Angststörung.

**Revendications**

1. Composition convenant à l'absorption transmucosale orale comprenant un sel de cyclobenzaprine pharmaceutiquement acceptable et un agent alcalinisant.

2. Composition selon la revendication 1, dans laquelle l'absorption transmucosale orale est une absorption sublinguale.

3. Composition selon la revendication 2, dans laquelle la composition est sous une forme choisie dans le groupe constitué d'un comprimé sublingual, d'un film sublingual, d'une poudre sublinguale et d'une solution de pulvérisation sublinguale.

4. Composition selon l'une quelconque des revendications 1 à 3, dans laquelle le sel de cyclobenzaprine pharmaceutiquement acceptable est le HCl de cyclobenzaprine.

5. Composition selon l'une quelconque des revendications 1 à 4, dans laquelle l'agent alcalinisant est choisi dans le groupe constitué du dihydrogénophosphate de potassium, hydrogénophosphate dipotassique, phosphate tripotassique, carbonate de sodium, bicarbonate de sodium, carbonate de calcium, bicarbonate de calcium, tampon TRIS, dihydrogénophosphate de sodium, hydrogénophosphate disodique, phosphate trisodique, carbonate de potassium, bicarbonate de potassium, acétate de potassium, acétate de sodium, citrate dipotassique, citrate tripotassique, citrate disodique et citrate trisodique.

6. Composition selon la revendication 5, dans laquelle l'agent alcalinisant est l'hydrogénophosphate dipotassique.

7. Composition selon la revendication 5, dans laquelle l'agent alcalinisant est le dihydrogénophosphate de potassium

8. Composition selon l'une quelconque des revendications 1 à 7 destinée à être utilisée dans une méthode de traitement d'une maladie ou d'un état chez un individu, la méthode comprenant l'administration de ladite composition par absorption transmucosale, dans laquelle la maladie ou l'état est un trouble de stress post-traumatique (TSPT).

9. Composition selon l'une quelconque des revendications 1 à 7 destinée à être utilisée dans une méthode de traitement d'une maladie ou d'un état chez un individu, la méthode comprenant l'administration de ladite composition par absorption transmucosale, dans laquelle la maladie ou l'état est choisi dans le groupe constitué de la fibromyalgie, dépression, lésion cérébrale traumatique, trouble du sommeil, sommeil non réparateur, douleur chronique, spasme musculaire et trouble anxieux.

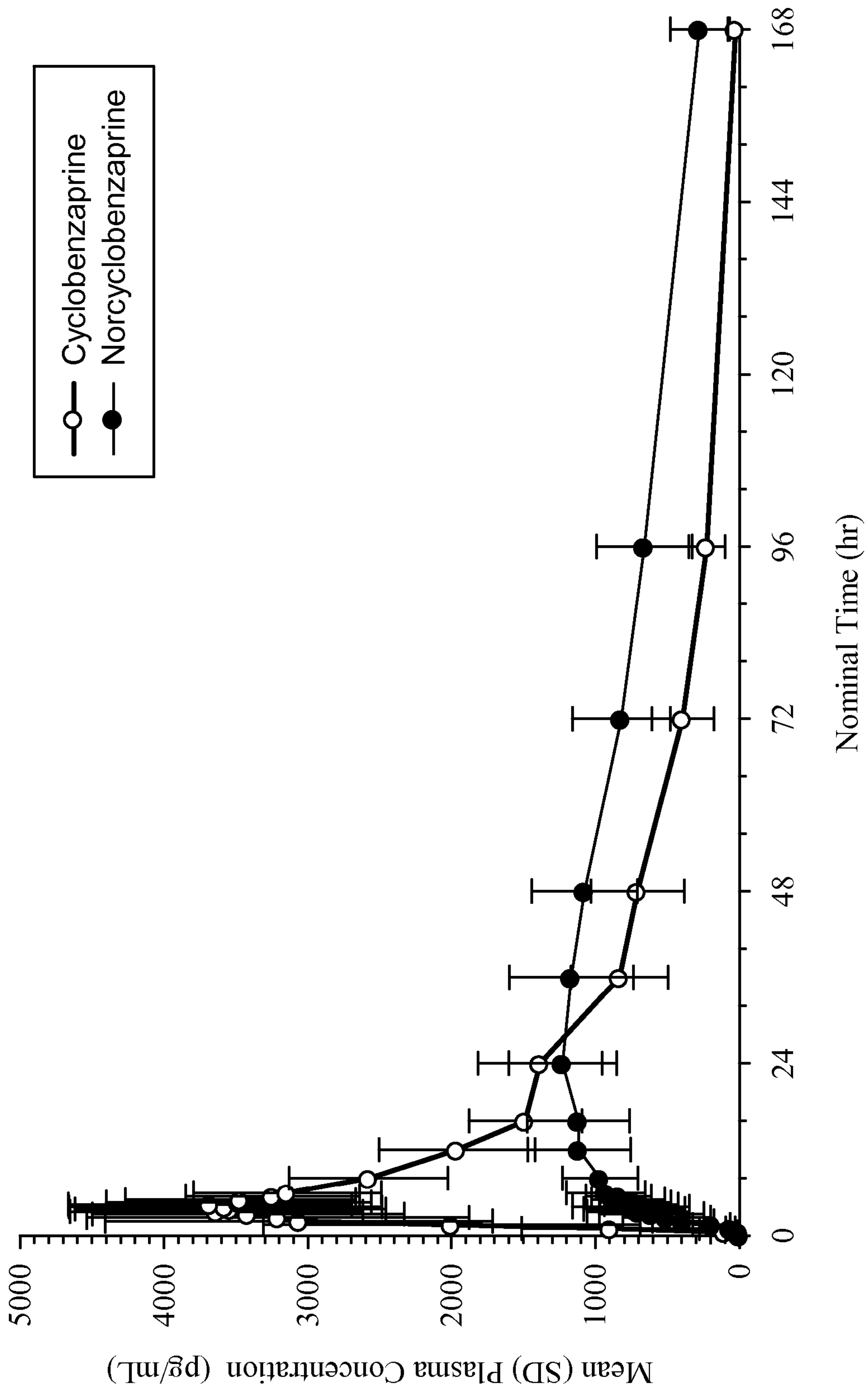
Cyclobenzaprine
Norcyclobenzaprine
Mean (SD) Plasma Concentration (pg/mL)
0
1000
2000
3000
4000
5000
Nominal Time (hr)
0
24
48
72
96
120
144
168

Figure 1a

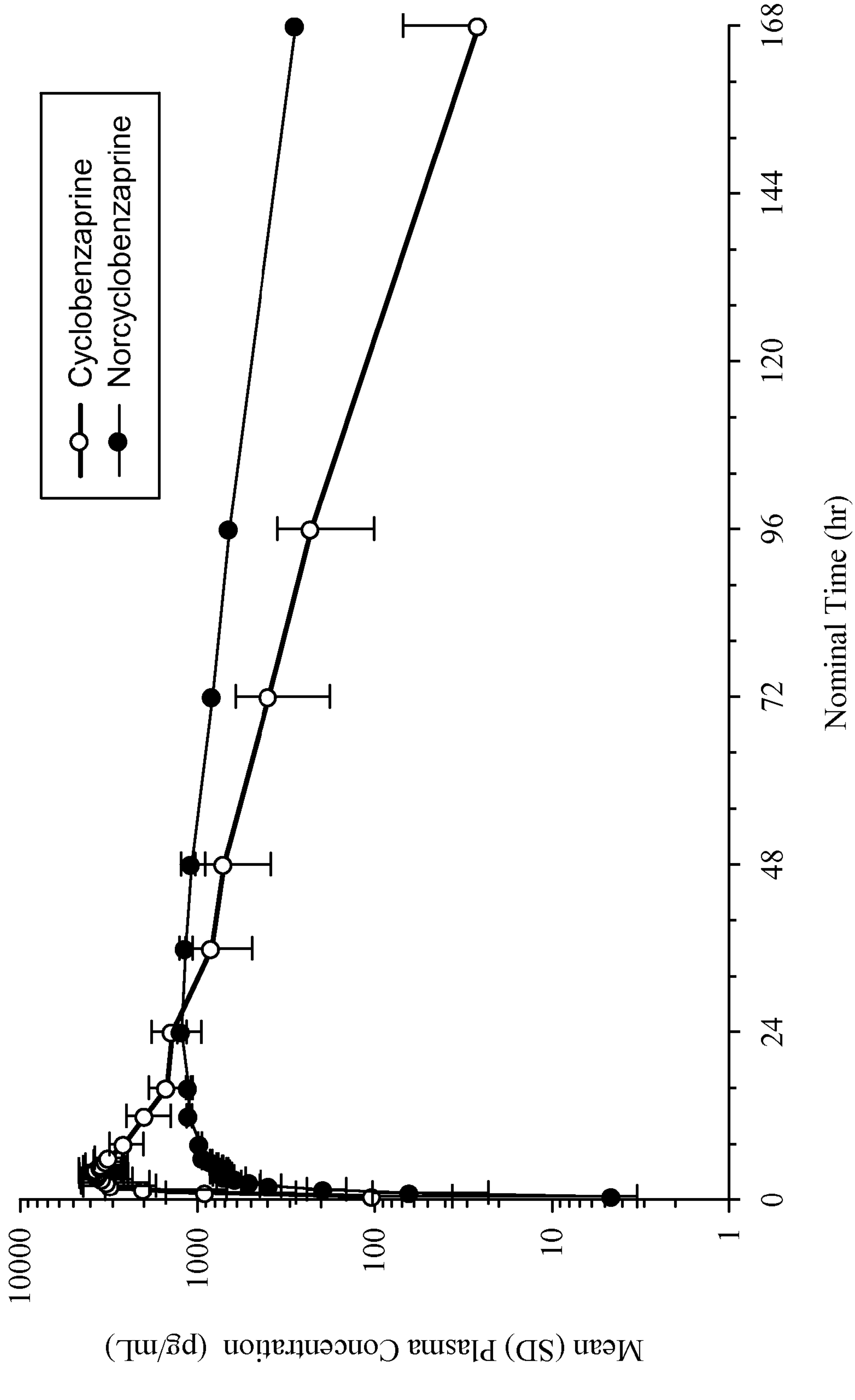

Cyclobenzaprine
Norcyclobenzaprine
Mean (SD) Plasma Concentration (pg/mL)
1
10
100
1000
10000
Nominal Time (hr)
0
24
48
72
96
120
144
168


Figure 1b

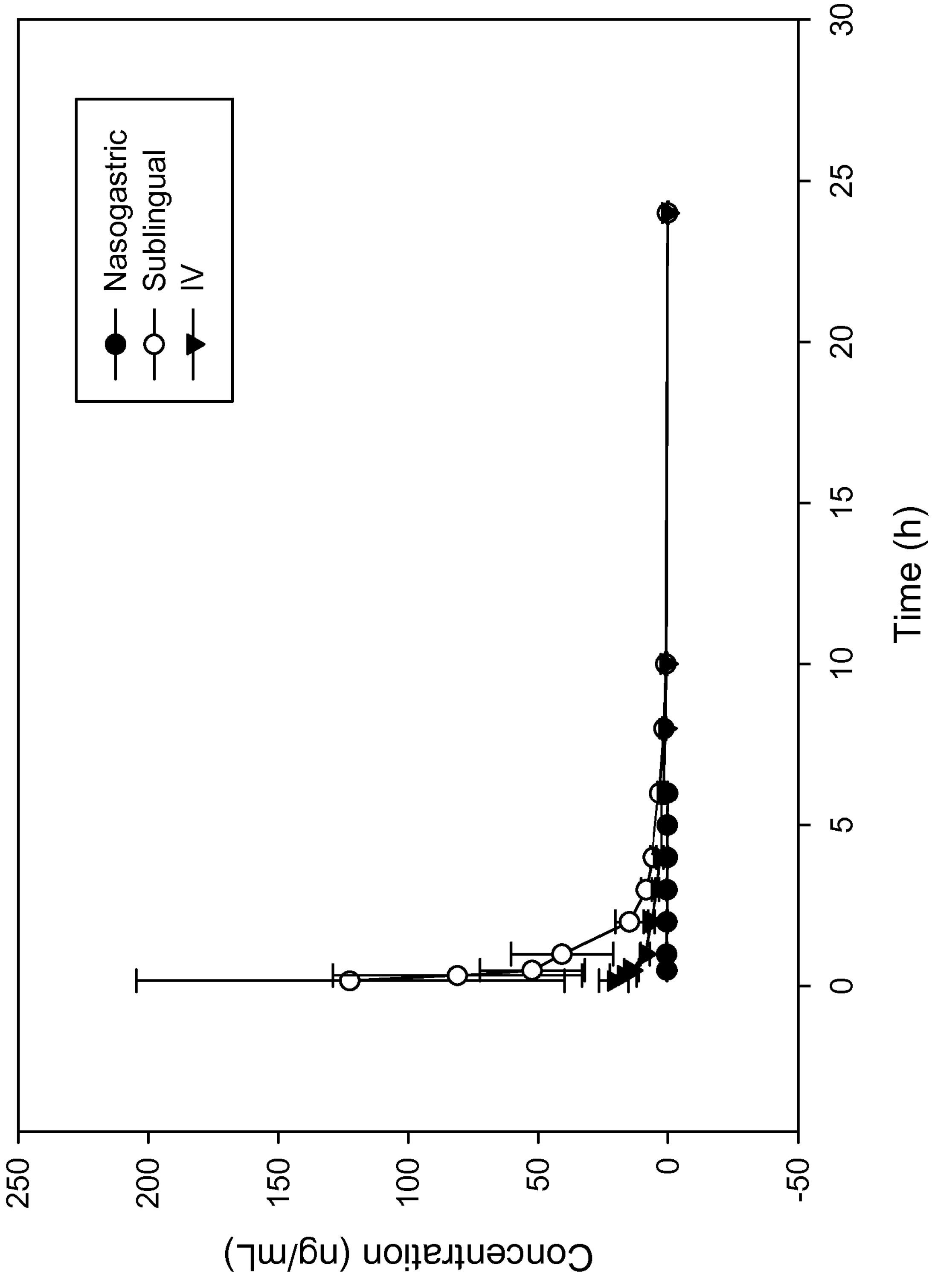
Nasogastric
Sublingual
IV
Concentration (ng/mL)
Time (h)
250
200
150
100
50
0
-50
0
5
10
15
20
25
30

Figure 2

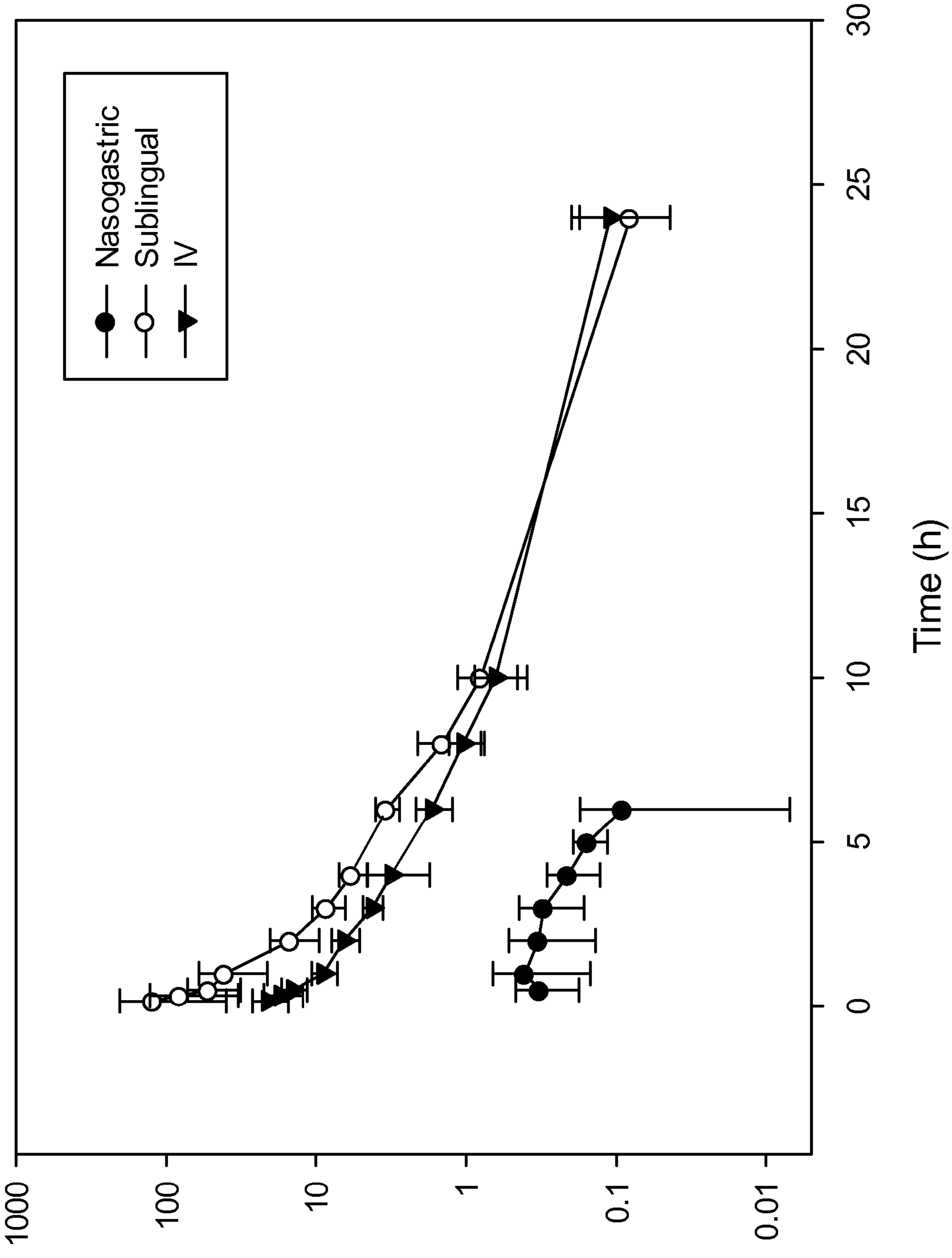
Nasogastric
Sublingual
IV
Log Concentration (ng/mL)
1000
100
10
1
0.1
0.01
Time (h)
0
5
10
15
20
25
30

Figure 3

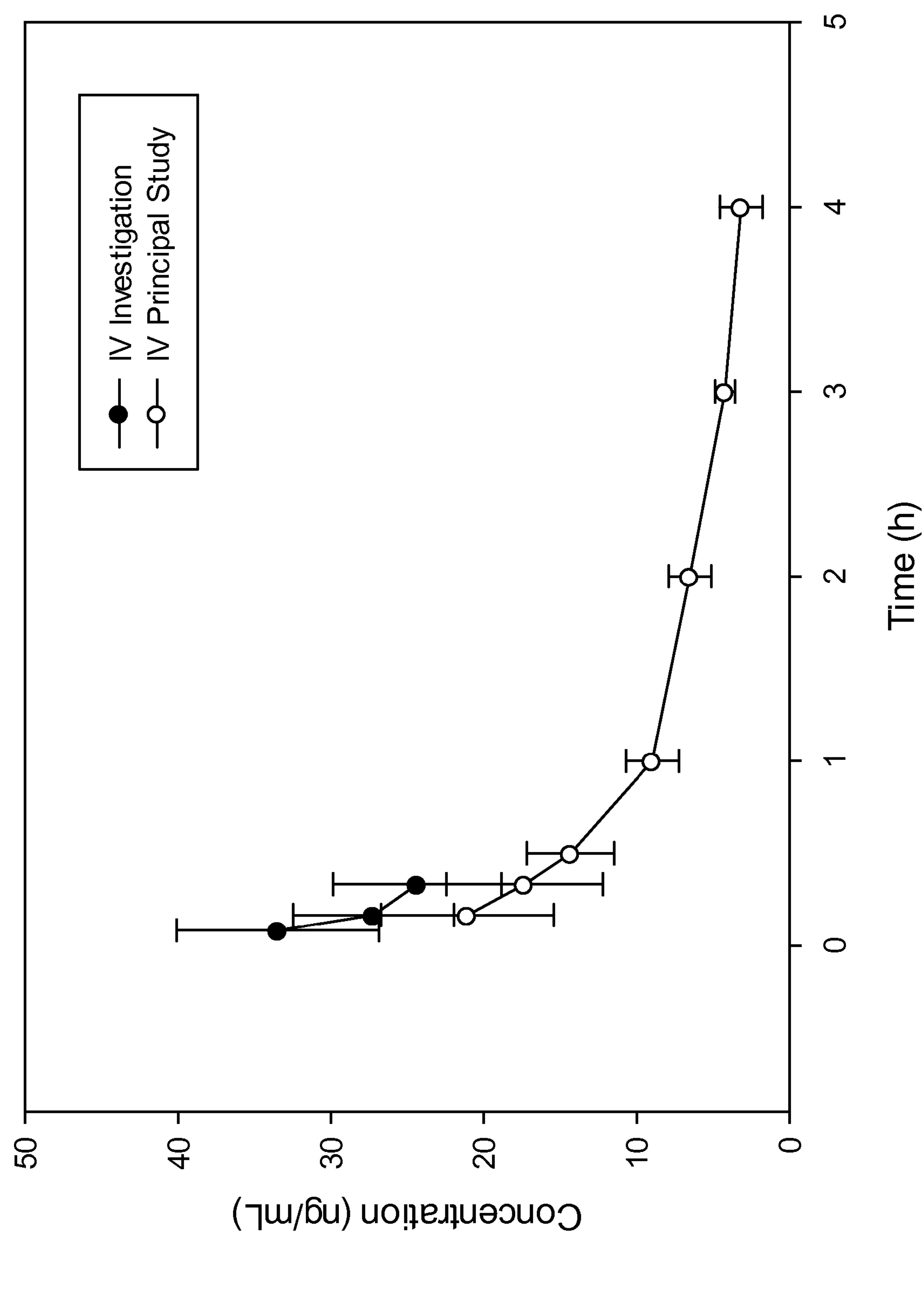
IV Investigation
IV Principal Study
Concentration (ng/mL)
0
10
20
30
40
50
Time (h)
0
1
2
3
4
5

Figure 4

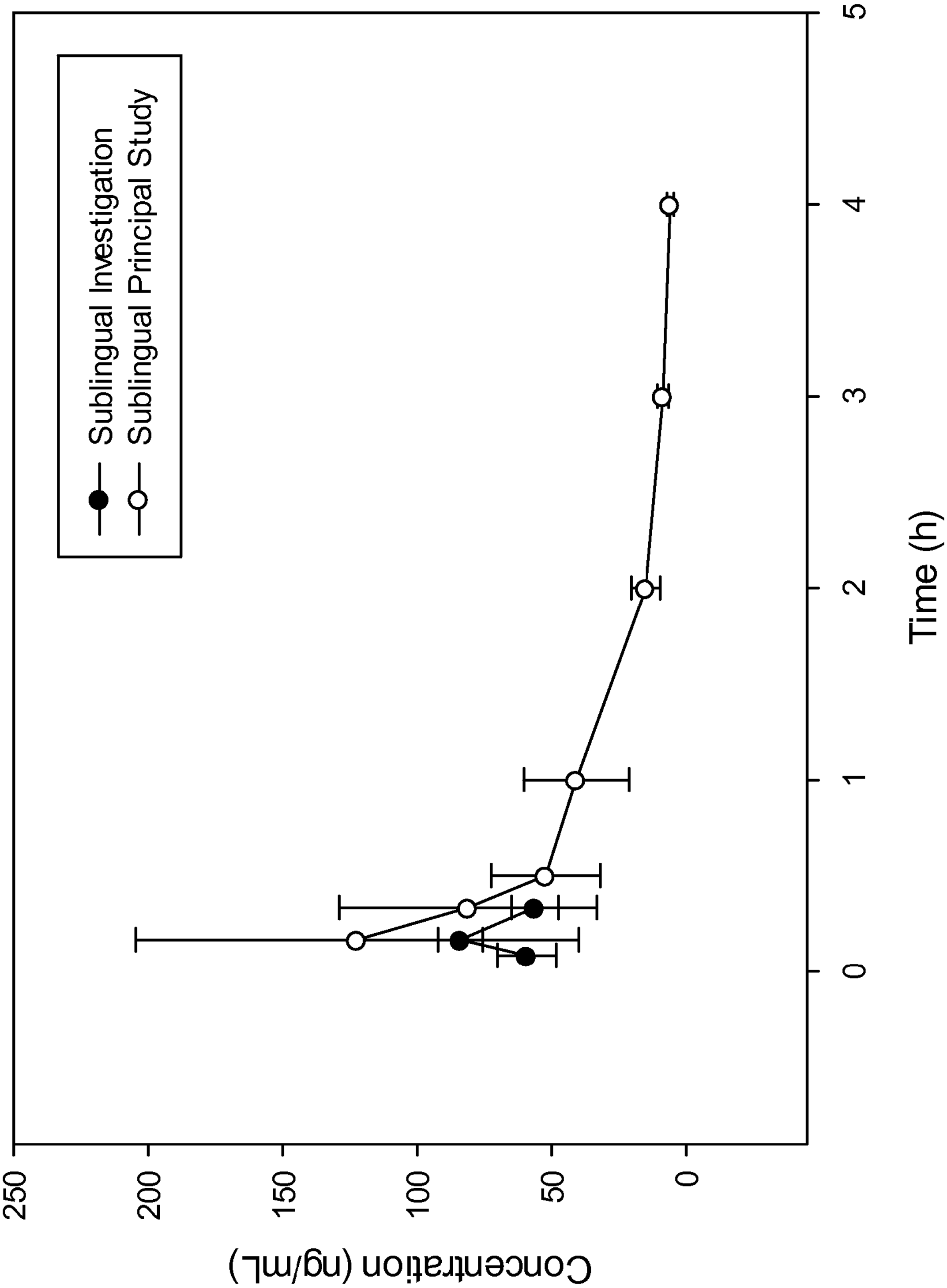
Sublingual Investigation
Sublingual Principal Study
Concentration (ng/mL)
Time (h)
0
50
100
150
200
250
0
1
2
3
4
5

Figure 5

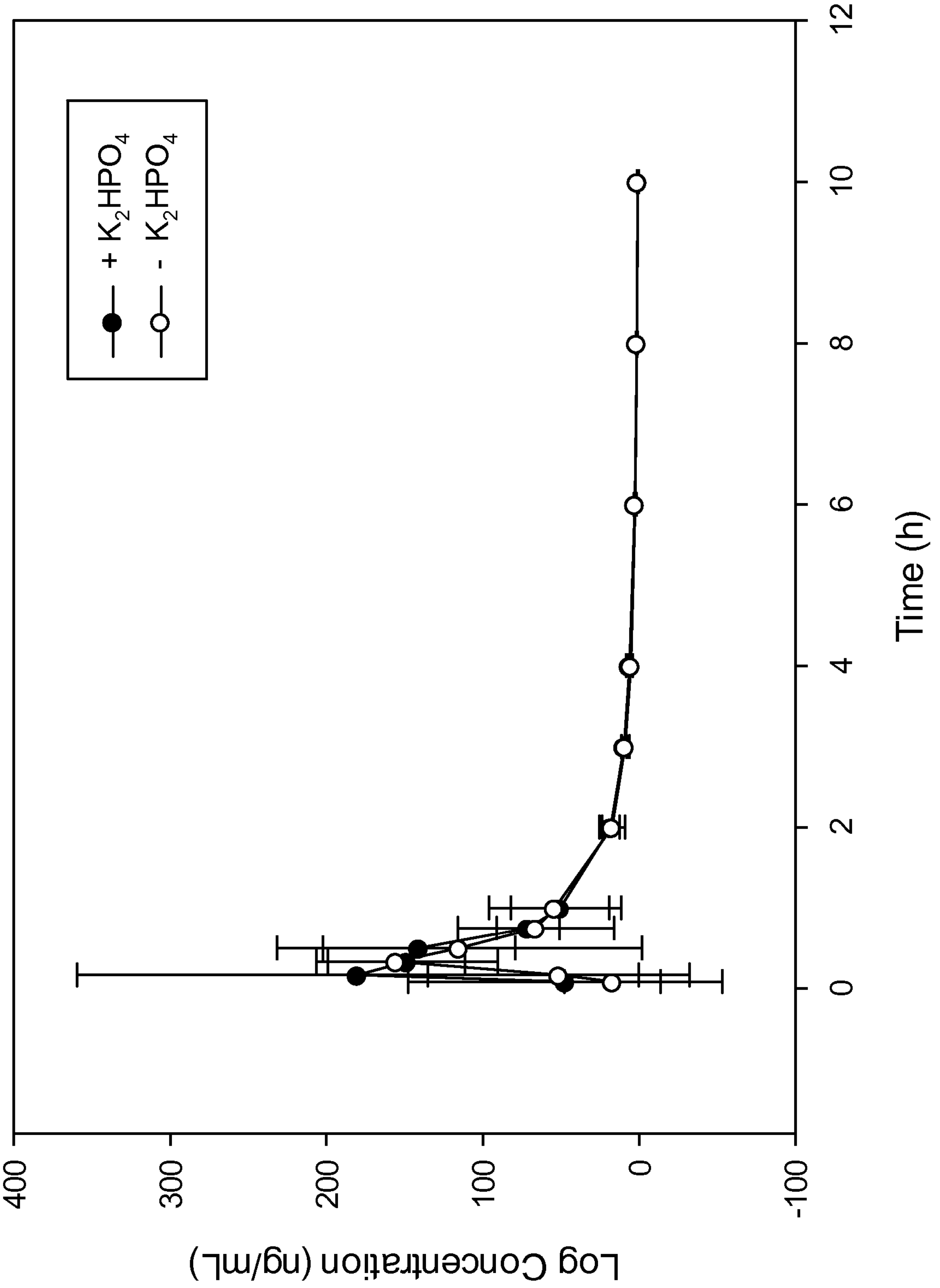
+ $K_2HPO_4$
- $K_2HPO_4$
Log Concentration (ng/mL)
Time (h)
400
300
200
100
0
-100
0
2
4
6
8
10
12

Figure 6

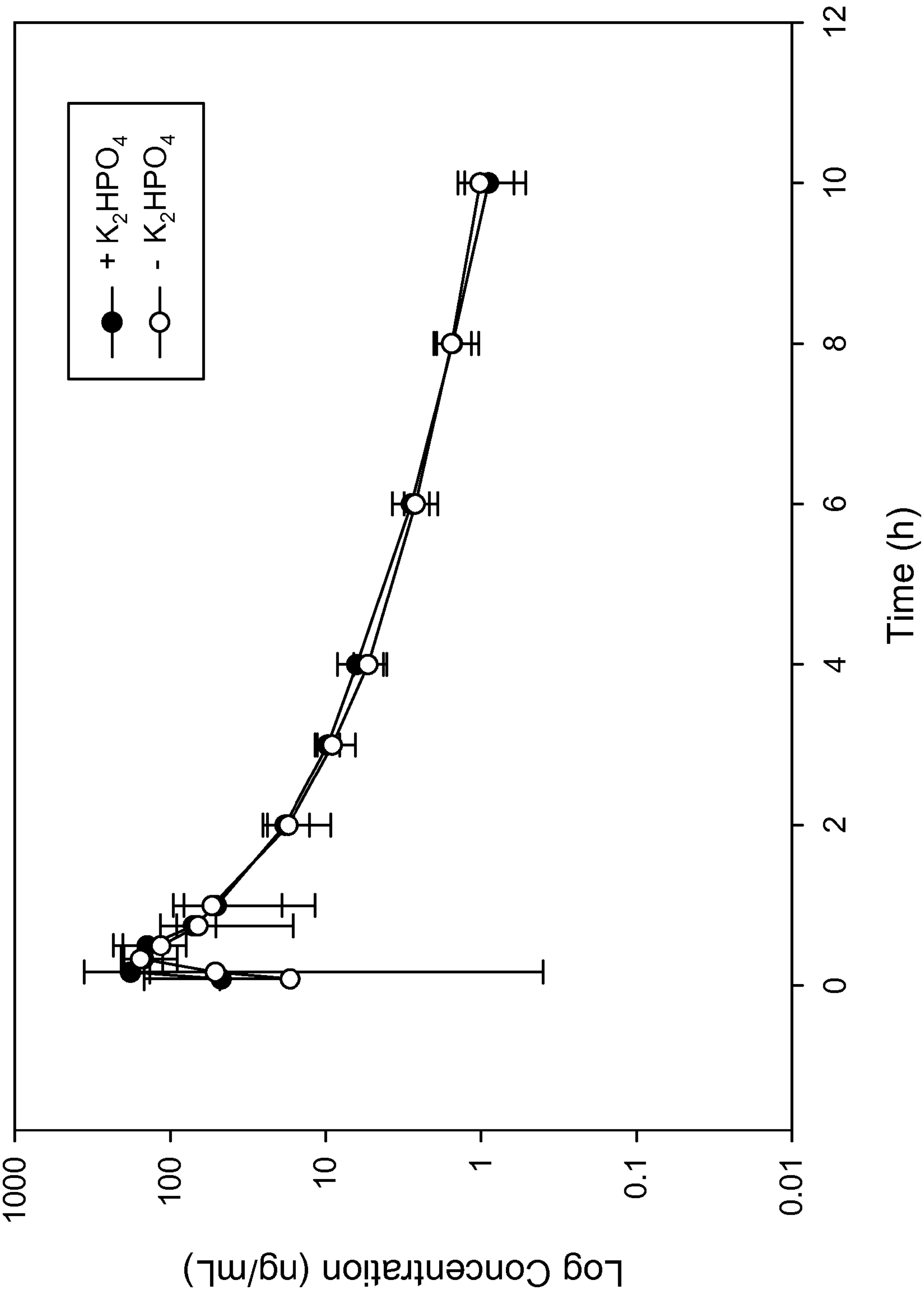
+ $K_2HPO_4$
- $K_2HPO_4$
Log Concentration (ng/mL)
1000
100
10
1
0.1
0.01
Time (h)
0
2
4
6
8
10
12

Figure 7

| Assessment | Screening, Days -30 to -2 | Baseline, Day -1 (On-Site) | Dosing Period (Confined to Study Site) | | | | Follow-up Call, 10 ± 3 Days After Dosing |
|---|---|---|---|---|---|---|---|
| | | | Day 1 | Day 2 | Day 3 | Day 4 | |
| Informed consent | x | — | — | — | — | — | — |
| Medical history | x | — | — | — | — | — | — |
| Urine β-HCG test (all women) | x | — | — | — | — | x | — |
| Serum β-HCG test (all women) | — | x | — | — | — | — | — |
| TSH, HIV, hepatitis B and C | x | — | — | — | — | — | — |
| Practice sublingual dosing test | — | x | — | — | — | — | — |
| Alcohol breath test | x | x | — | — | — | — | — |
| Urine drug screen | x | x | — | — | — | — | — |
| Urine cotinine | x | x | — | — | — | — | — |
| Height | x | — | — | — | — | — | — |
| Physical examination | x | x[a] | — | — | — | x[a] | — |
| Weight/body mass index | x | x | — | — | — | x | — |
| Prior/concomitant medications | x | x | x | x | x | x | x |
| Clinical laboratory tests | x | x | — | — | — | x | — |
| Vital signs (sitting) [b] | x | x | x | x | x | x | — |
| 12-Lead ECG (supine) | x | x | — | — | — | x | — |
| Adverse events | x | x | x | x | x | x | x |
| Administer drug | — | — | x | — | — | — | — |
| Plasma samples (PK) [c] | — | — | x | x | x | x | — |
| Urine samples (PK) [d] | — | — | x | x | x | x | — |

*Abbreviations:* ECG, electrocardiogram; HCG, human chorionic gonadotropin; HIV, human immunodeficiency virus; PK, pharmacokinetic; TSH, thyroid-stimulating hormone.

[a] Brief physical examination only.

[b] Vital signs (sitting blood pressure, respiratory rate, oral temperature, and heart rate) will be assessed at screening, upon admission to the study unit, within 1 hour pre-dose, and 1, 3, 6, 8, 12, 24, 48, and 72 hours post-dose. Vital signs will be recorded in advance of other assessments scheduled for a given timepoint, except at the Hour 72 timepoint, when vital signs will be recorded after other assessments have been completed. Otherwise, when another safety measurement coincides with a blood draw, the blood draw will be completed first.

[c] For pharmacokinetic analysis, a total of 25 blood samples will be taken: within 30 minutes pre-dose and 5, 10, 20, 30, and 45 minutes and 1, 2, 2.5, 3, 3.33, 3.67, 4, 4.33, 4.67, 5, 5.5, 6, 8, 12, 16, 24, 36, 48, and 72 hours post-dose.

[d] A single urine sample will be collected within 30 minutes pre-dose (one sample), and urine will be pooled from 0-24, 24-48, and 48-72 hours post-dose. If a subject cannot void his or her bladder within 30 minutes before dosing, a sample from earlier that morning may be used as the pre-dose sample.

Figure 8

| Assessment | Time Relative to Dosing, Days 1-4 (Dosing Period) | | | | | | | | | | | | | | | | | | | | | | | | |
|---|---|---|---|---|---|---|---|---|---|---|---|---|---|---|---|---|---|---|---|---|---|---|---|---|---|
| | Day 1 | | | | | | | | | | | | | | | | | | | | | Day 2 | | Day 3 | Day 4 |
| | Pre-dose[a] | 0 | 5 min | 10 min | 20 min | 30 min | 45 min | 1 h | 2 h | 2.5 h | 3 h | 3.33 h | 3.67 h | 4 h | 4.33 h | 4.67 h | 5 h | 5.5 h | 6 h | 8 h | 12 h | 16 h | 24 h | 36 h | 48 h | 72 h[b] |
| Dosing | | x | | | | | | | | | | | | | | | | | | | | | | | |
| Vital signs[c] | x | | | | | | | x | | | x | | | | | | | | x | x | x | | x | | x | x |
| Plasma PK | x | | x | x | x | x | x | x | x | x | x | x | x | x | x | x | x | x | x | x | x | x | x | x | x | x |
| Urine PK | x | Pooled, 0-24 hours | | | | | | | | | | | | | | | | | | | | | 24-48 h | | 48-72 h | |
| Clinlabs | | | | | | | | | | | | | | | | | | | | | | | | | | x |
| Urine β-HCG | | | | | | | | | | | | | | | | | | | | | | | | | | x |
| Brief physical examination | | | | | | | | | | | | | | | | | | | | | | | | | | x |
| Weight/BMI | | | | | | | | | | | | | | | | | | | | | | | | | | x |
| 12-Lead ECG | | | | | | | | | | | | | | | | | | | | | | | | | | x |
| AEs | Ongoing | | | | | | | | | | | | | | | | | | | | | | | | | |
| Conmeds | Ongoing | | | | | | | | | | | | | | | | | | | | | | | | | |

*Abbreviations:* Adverse events; AEs; BMI, body mass index; clinlabs, clinical laboratory tests; conmeds, concomitant medications; ECG, electrocardiogram; h, hour(s); HCG, human chorionic gonadotropin; min, minute(s); PK, pharmacokinetics.

[a] Predose evaluations are to be performed within 30 minutes prior to dosing, with the exception of vital signs, which may be recorded within 1 hour prior to dosing. If a subject cannot void his or her bladder within 30 minutes before dosing, a sample from earlier that morning may be used as the predose sample.

[b] Day 4 evaluations will be performed at 72 ± 1.5 hours after dosing. Vital signs will be performed last on Day 4 only.

[c] Vital signs will be recorded in advance of other assessments scheduled for a given timepoint, except at the Hour 72 timepoint, when vital signs will be recorded after other assessments have been completed. Otherwise, when another safety measurement coincides with a blood draw, the blood draw will be completed first.

Figure 9

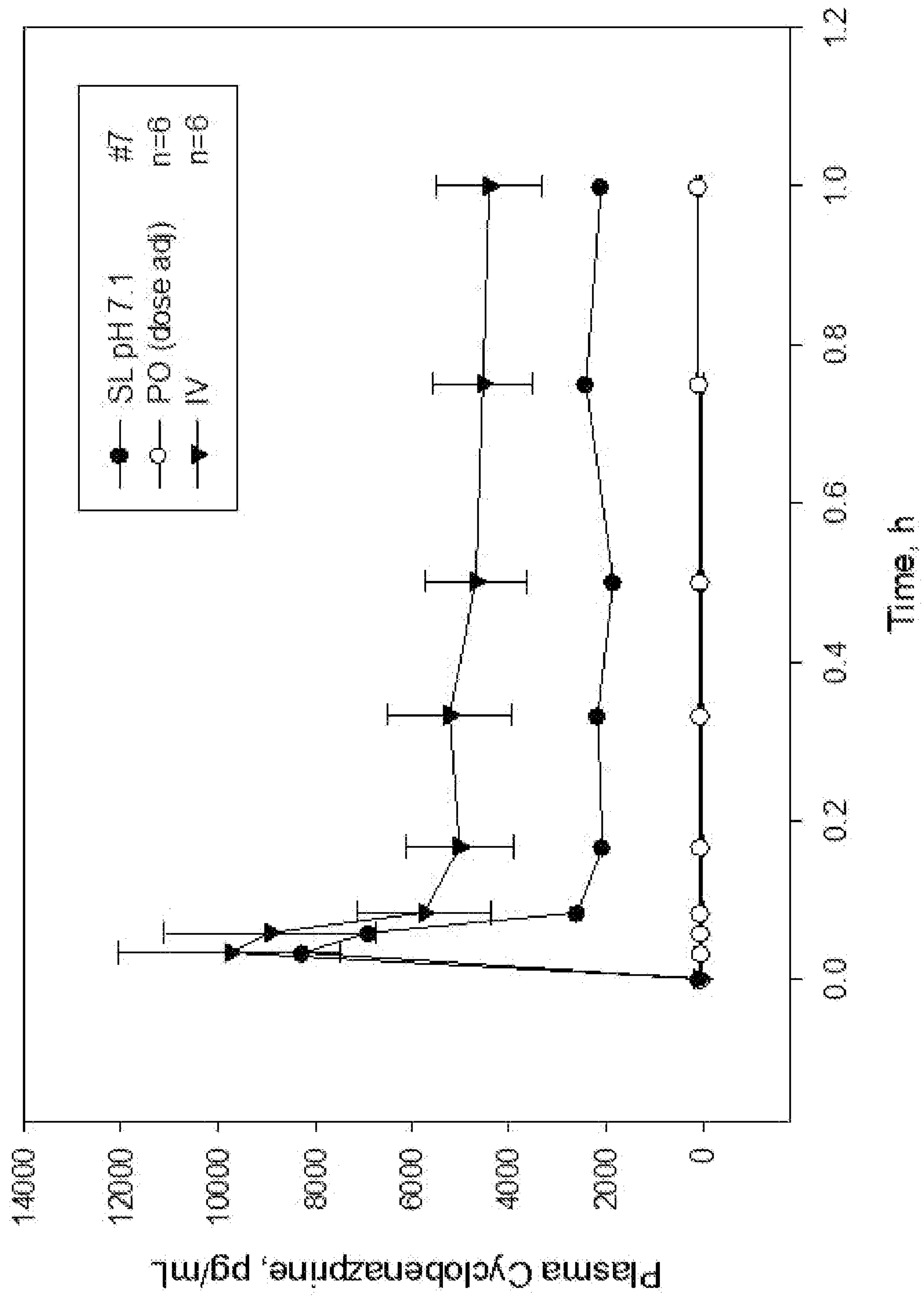
Plasma Cyclobenazprine, pg/mL
0
2000
4000
6000
8000
10000
12000
14000
Time, h
0.0
0.2
0.4
0.6
0.8
1.0
1.2
SL pH 7.1 #7
PO (dose adj) n=6
IV n=6

Figure 10

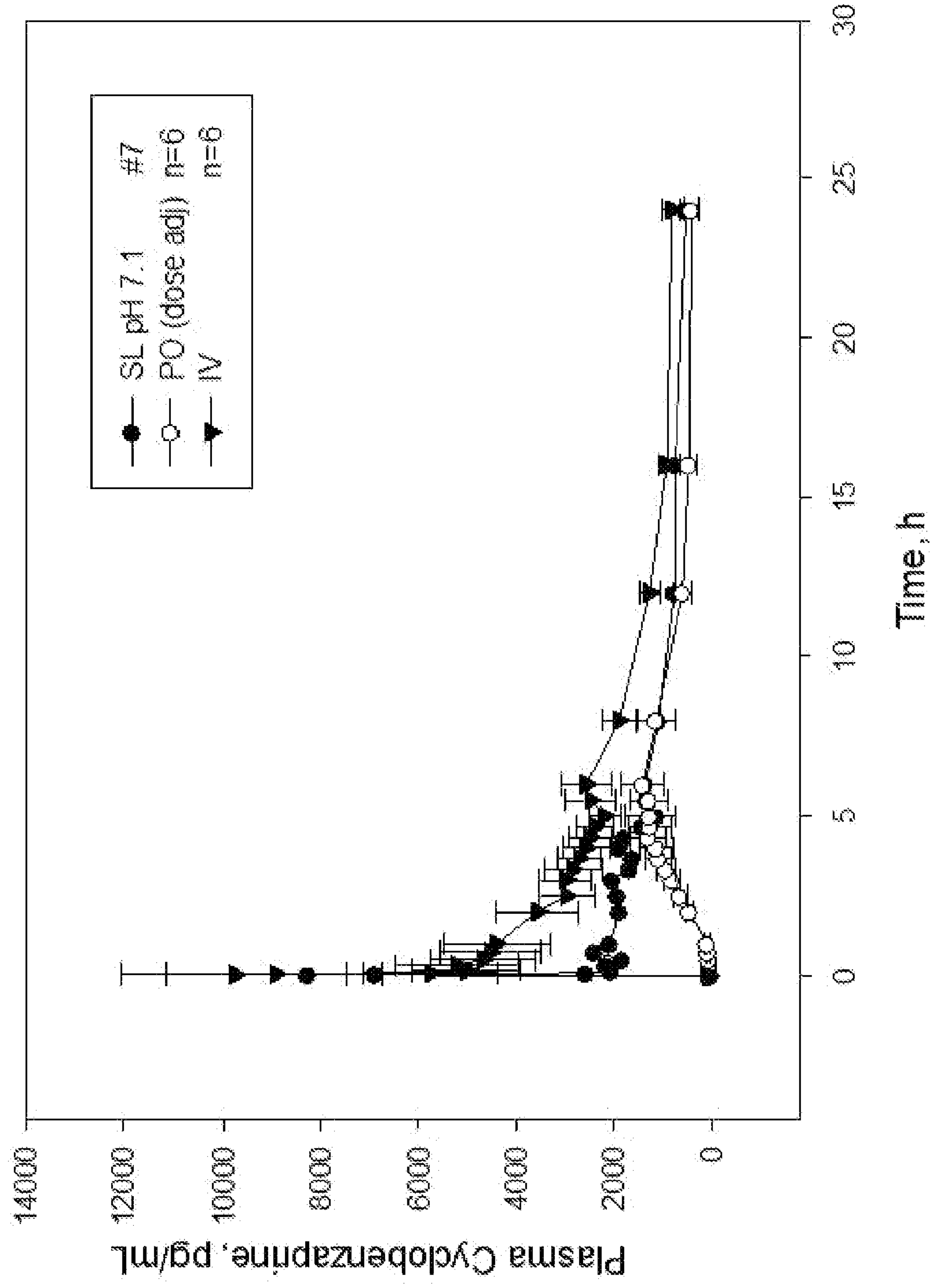
Plasma Cyclobenzaprine, pg/mL
14000
12000
10000
8000
6000
4000
2000
0
0
5
10
15
20
25
30
Time, h
SL pH 7.1 #7
PO (dose adj) n=6
IV n=6

Figure 11

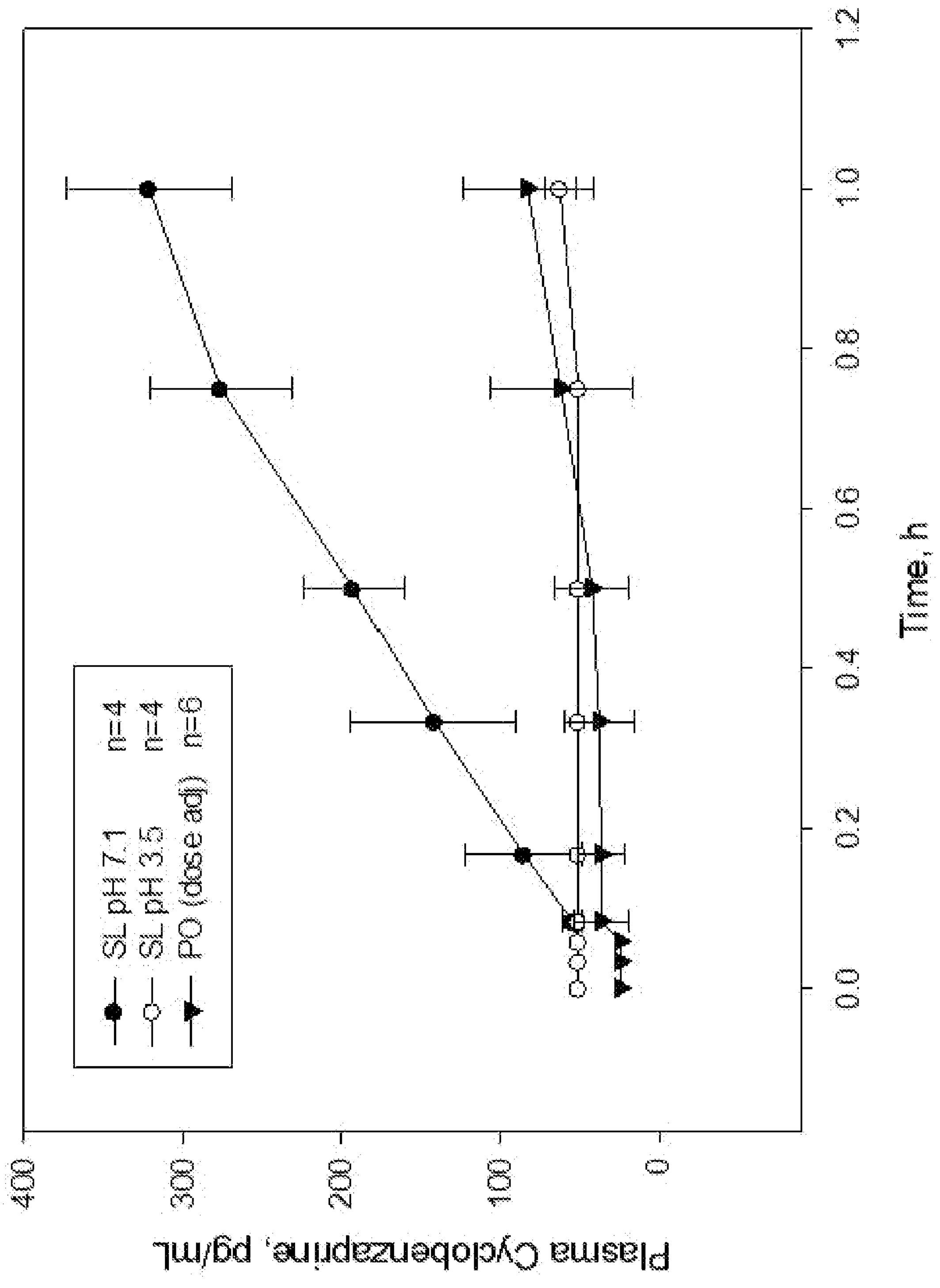
SL pH 7.1 n=4
SL pH 3.5 n=4
PO (dose adj) n=6
Plasma Cyclobenzaprine, pg/mL
0
100
200
300
400
Time, h
0.0
0.2
0.4
0.6
0.8
1.0
1.2

Figure 12

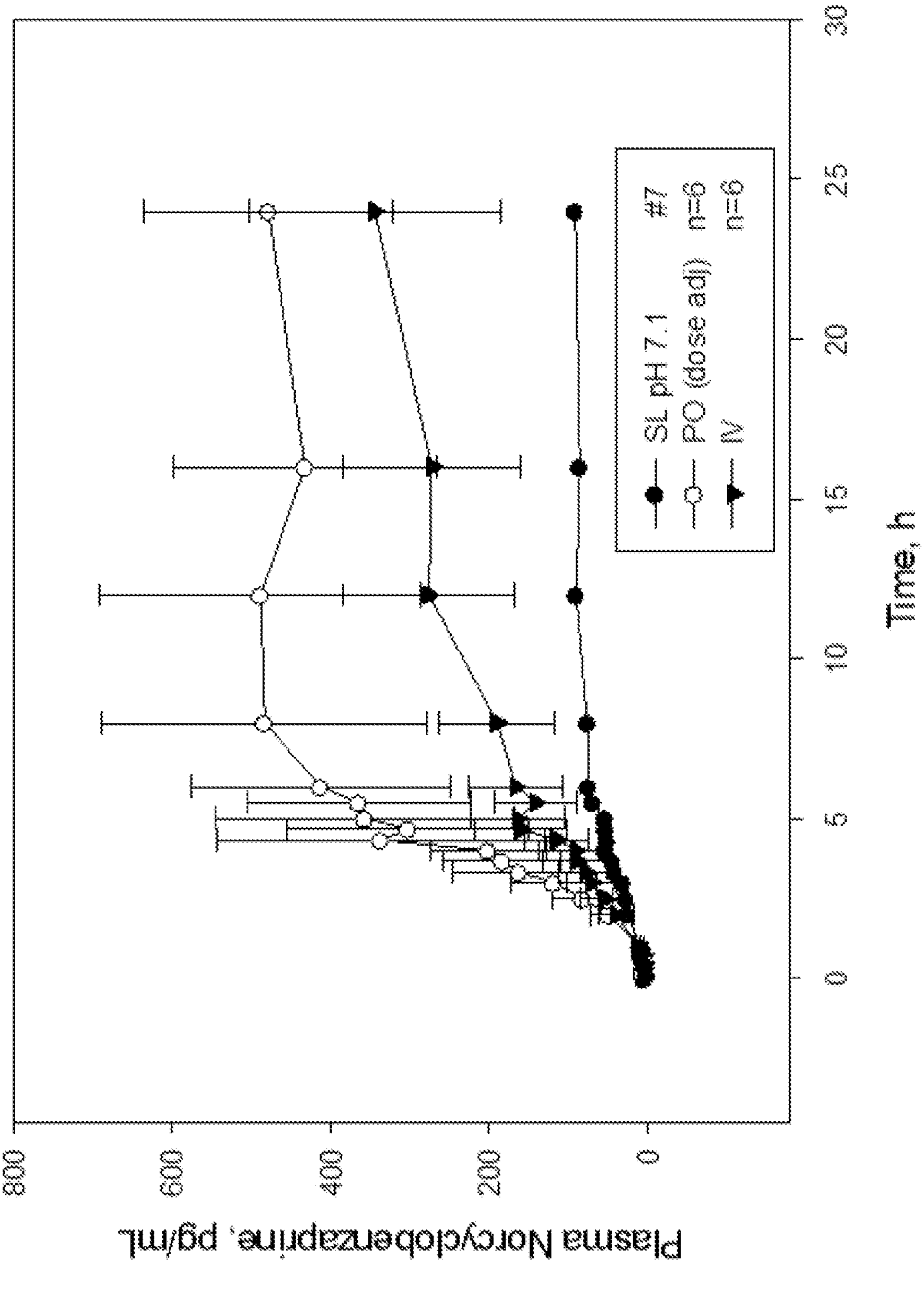
Plasma Norcyclobenzaprine, pg/mL
800
600
400
200
0
0
5
10
15
20
25
30
Time, h
SL pH 7.1 #7
PO (dose adj) n=6
IV n=6

Figure 13

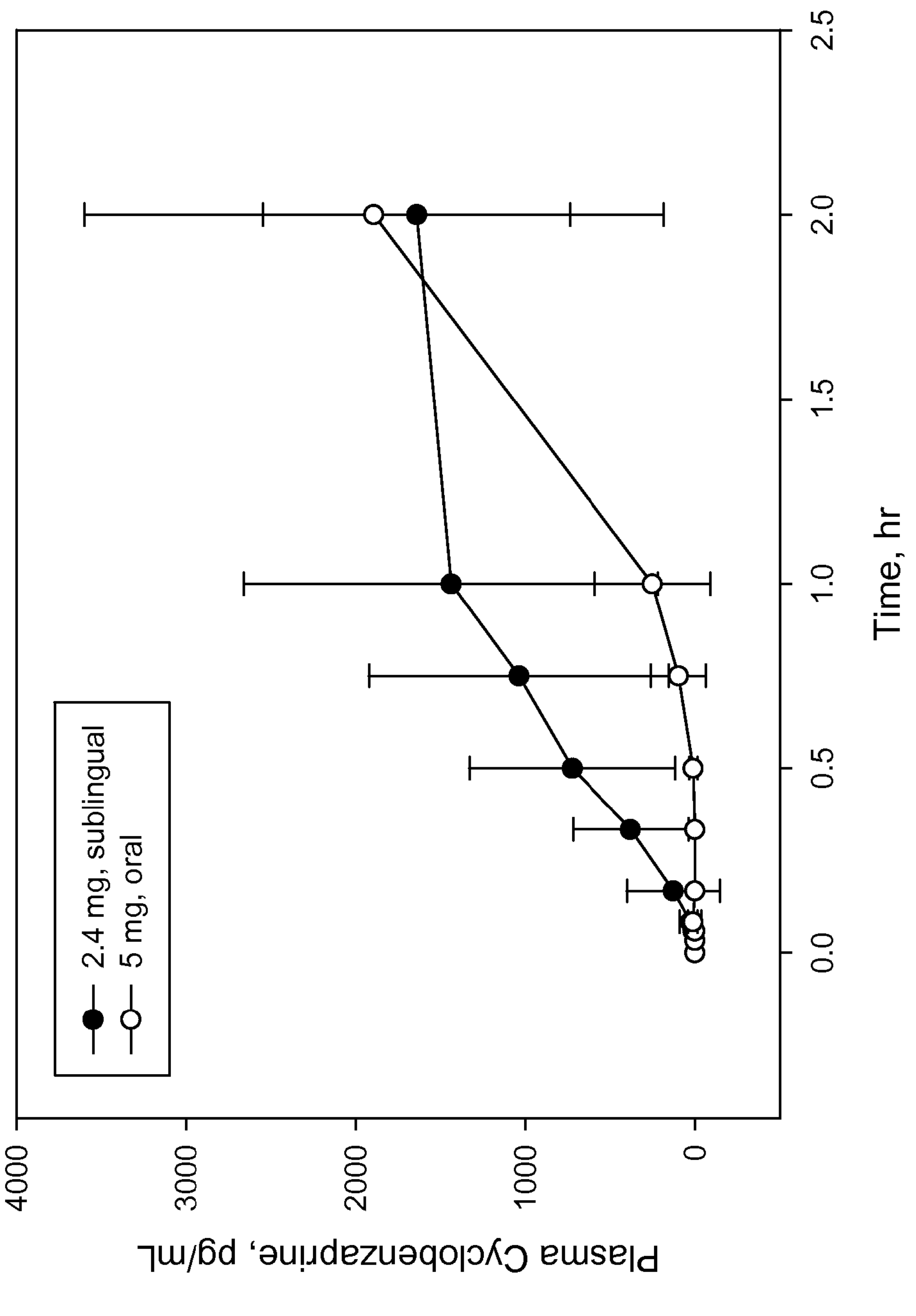
2.4 mg, sublingual
5 mg, oral
Plasma Cyclobenzaprine, pg/mL
4000
3000
2000
1000
0
Time, hr
0.0
0.5
1.0
1.5
2.0
2.5

Figure 14a

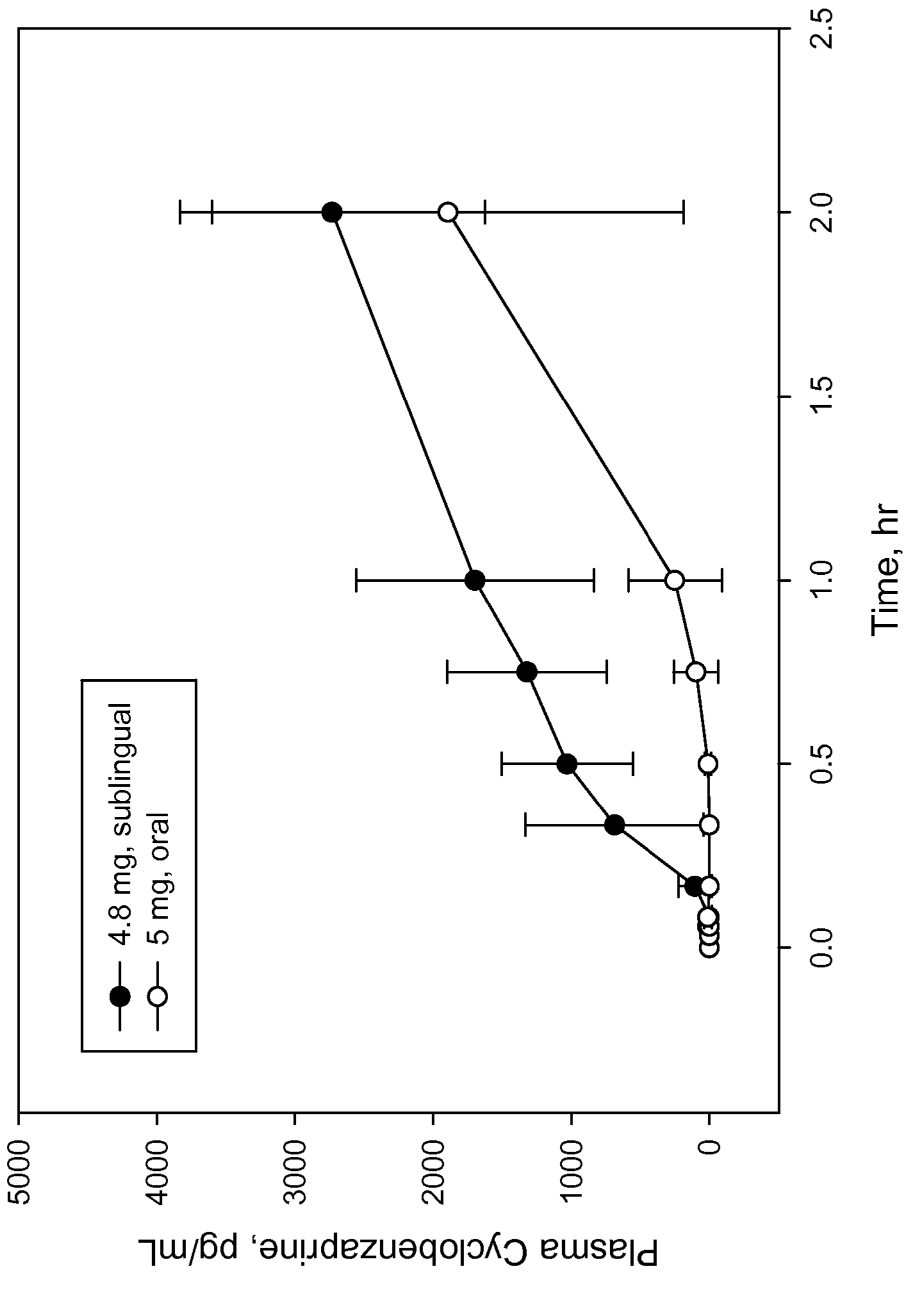
4.8 mg, sublingual
5 mg, oral
Plasma Cyclobenzaprine, pg/mL
5000
4000
3000
2000
1000
0
Time, hr
0.0
0.5
1.0
1.5
2.0
2.5

Figure 14b

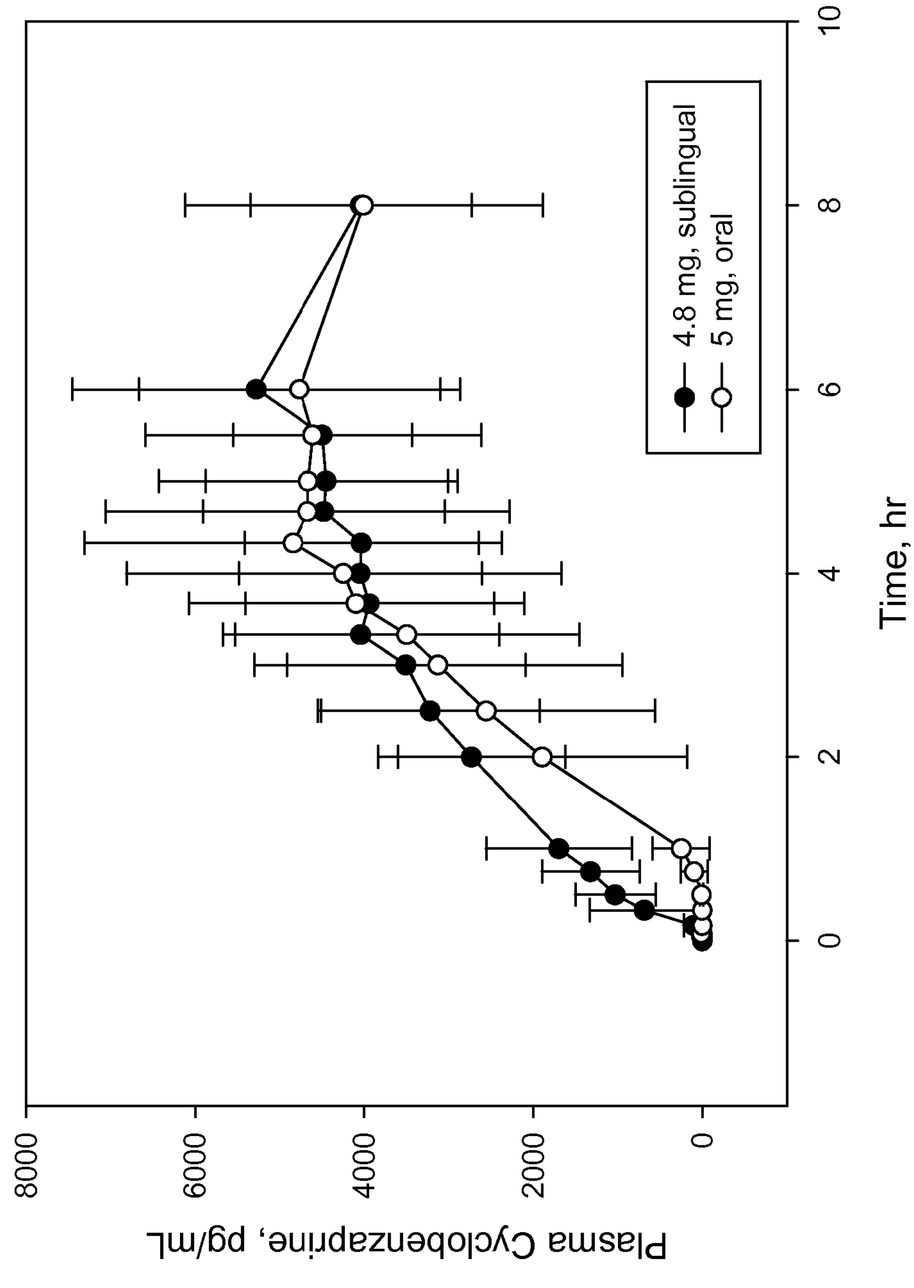
Plasma Cyclobenzaprine, pg/mL
8000
6000
4000
2000
0
Time, hr
0
2
4
6
8
10
4.8 mg, sublingual
5 mg, oral

Figure 15

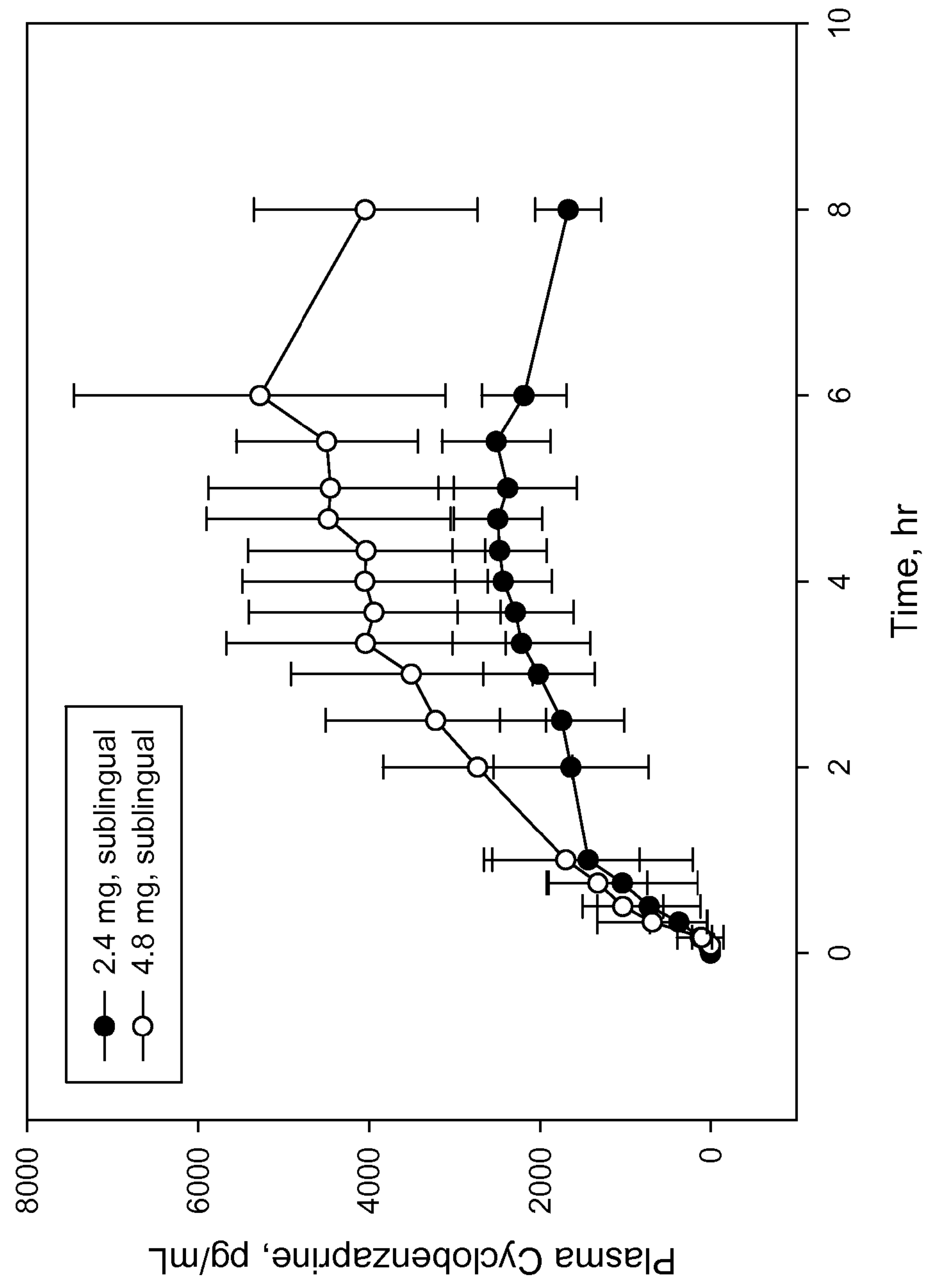
2.4 mg, sublingual
4.8 mg, sublingual
Plasma Cyclobenzaprine, pg/mL
8000
6000
4000
2000
0
Time, hr
0
2
4
6
8
10

Figure 16

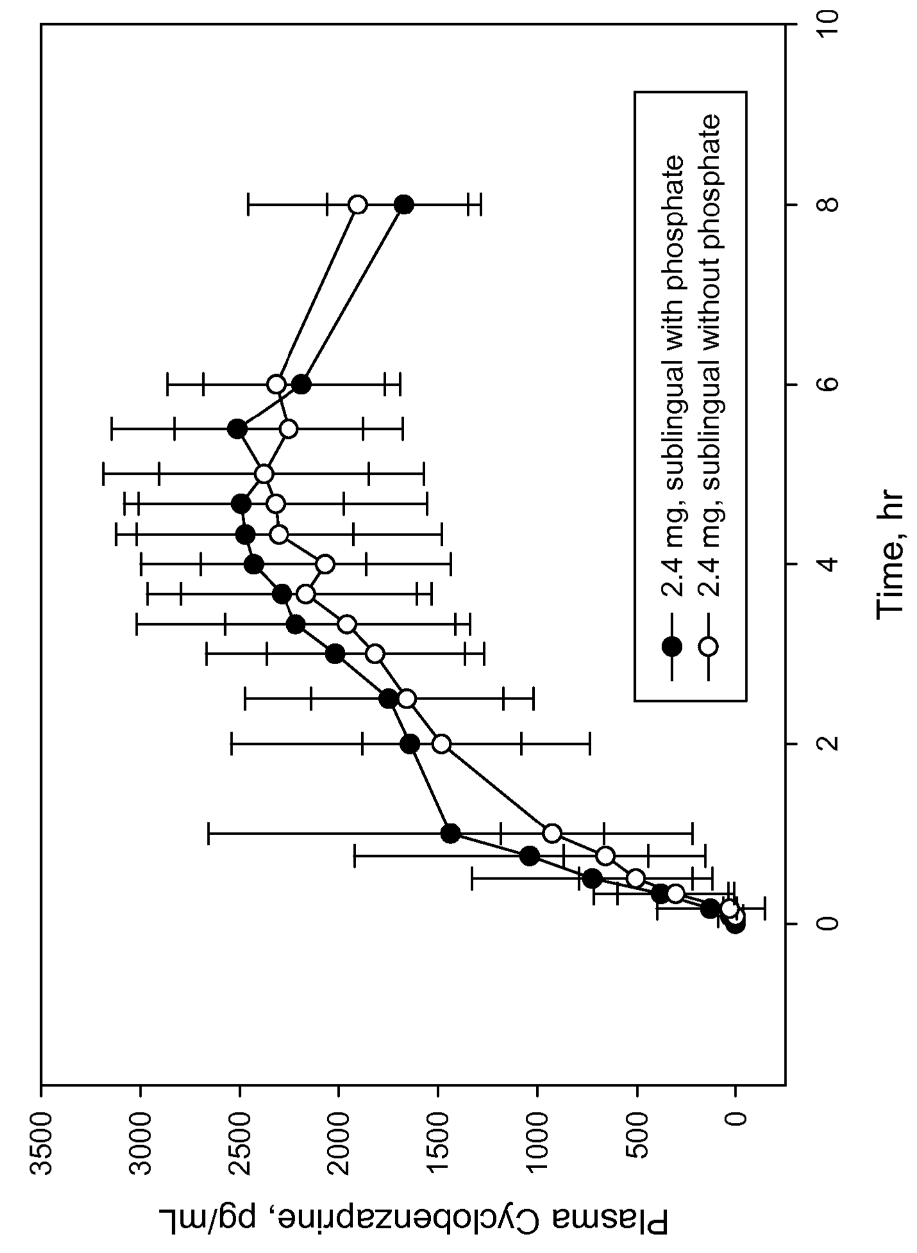
Plasma Cyclobenzaprine, pg/mL
Time, hr
2.4 mg, sublingual with phosphate
2.4 mg, sublingual without phosphate
0
500
1000
1500
2000
2500
3000
3500
0
2
4
6
8
10

Figure 17a

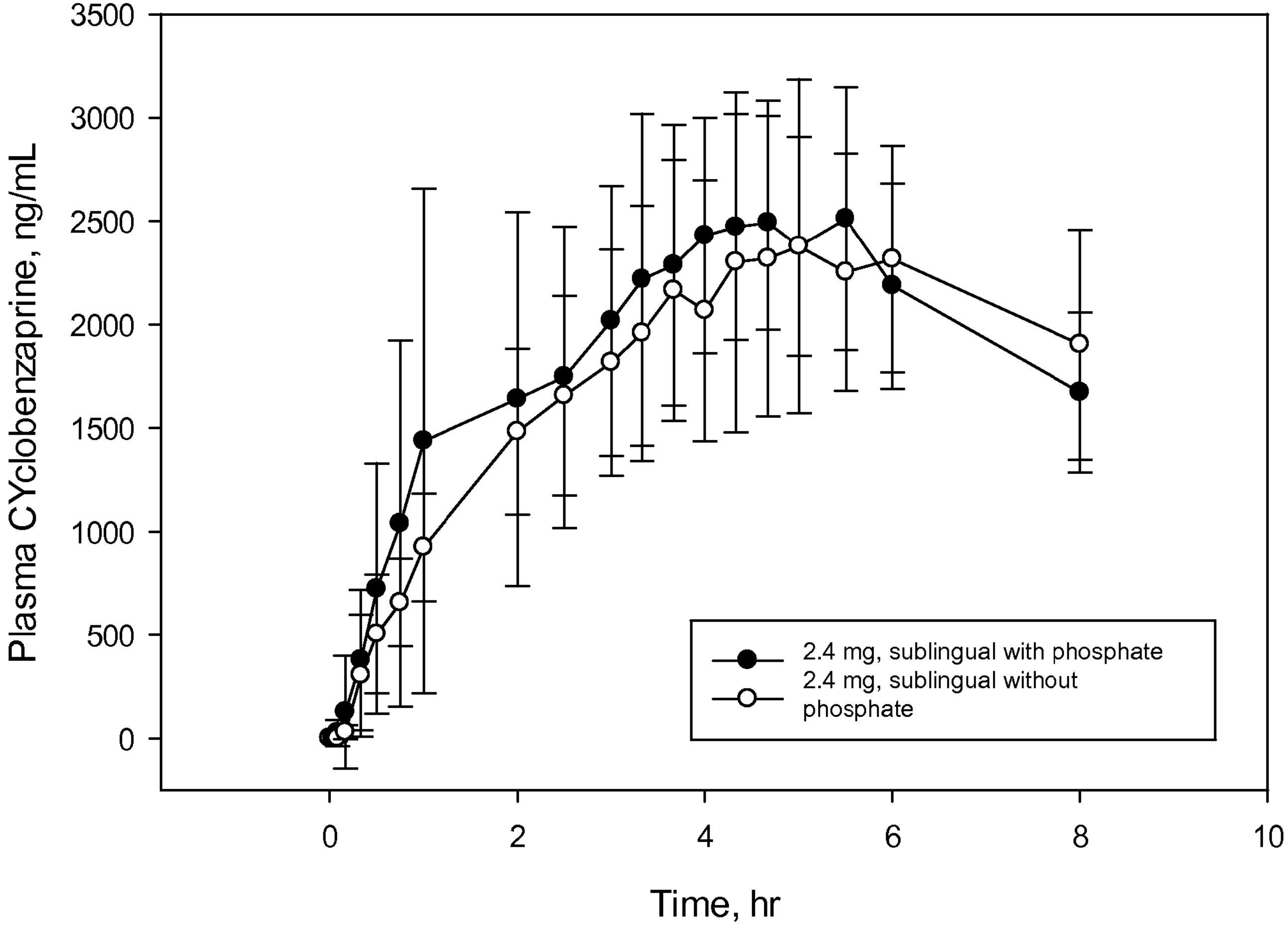

3500
3000
2500
2000
1500
1000
500
0
Plasma CYclobenzaprine, ng/mL
0
2
4
6
8
10
Time, hr
2.4 mg, sublingual with phosphate
2.4 mg, sublingual without phosphate


Figure 17b

# $5\text{-}HT_{1A}$

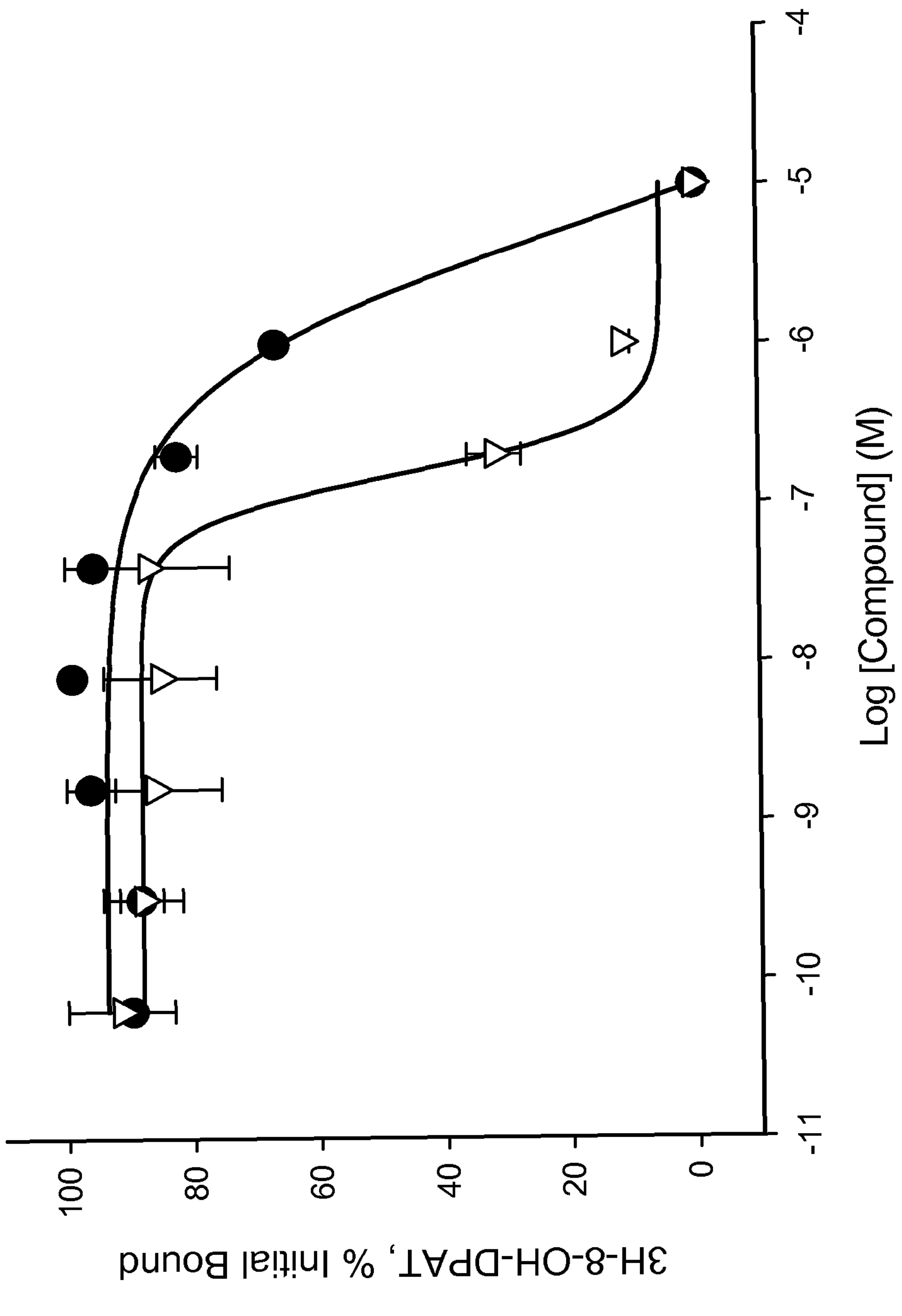


Figure 18a

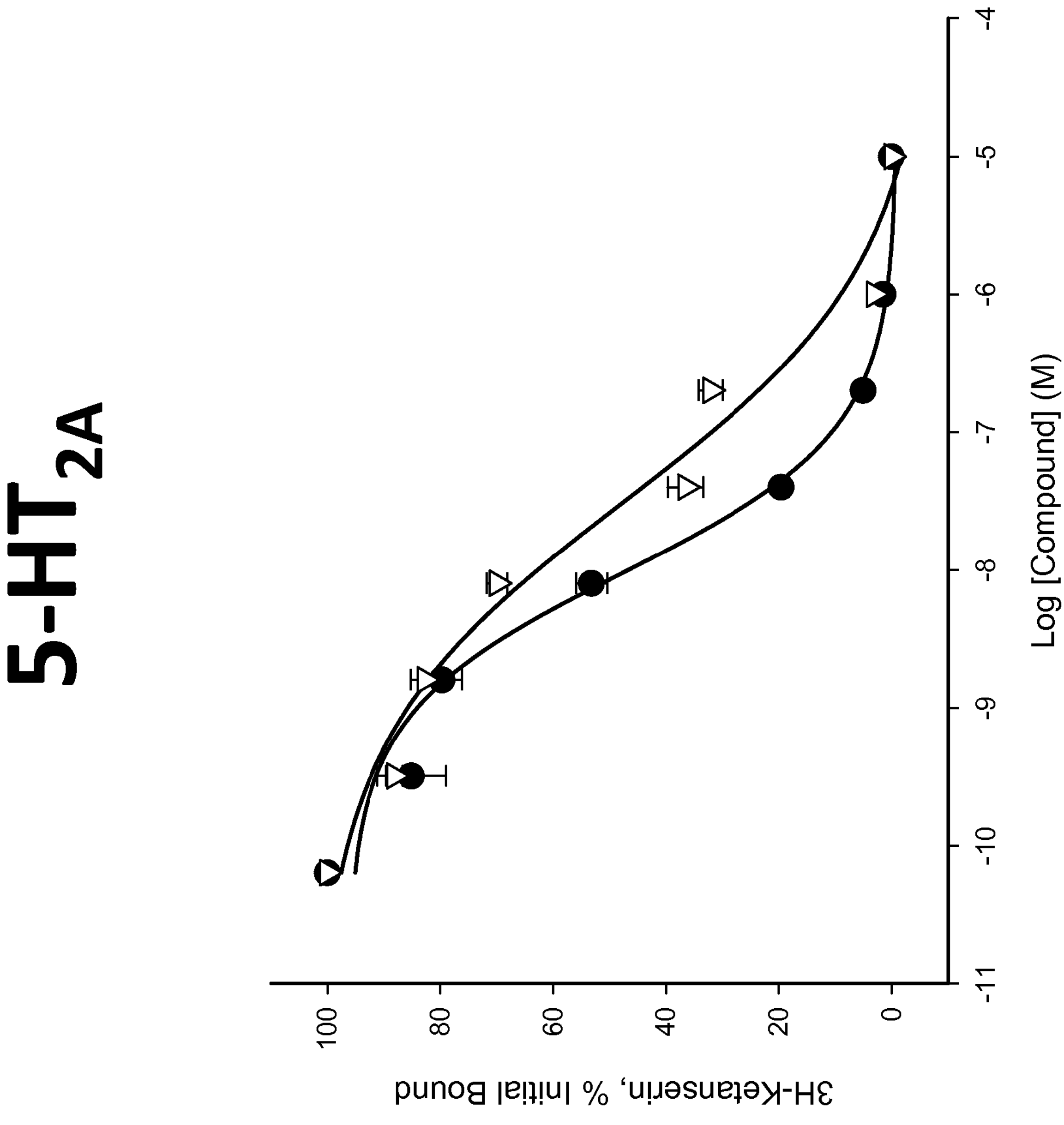

5-HT2A
3H-Ketanserin, % Initial Bound
0
20
40
60
80
100
-11
-10
-9
-8
-7
-6
-5
-4
Log [Compound] (M)


Figure 18b

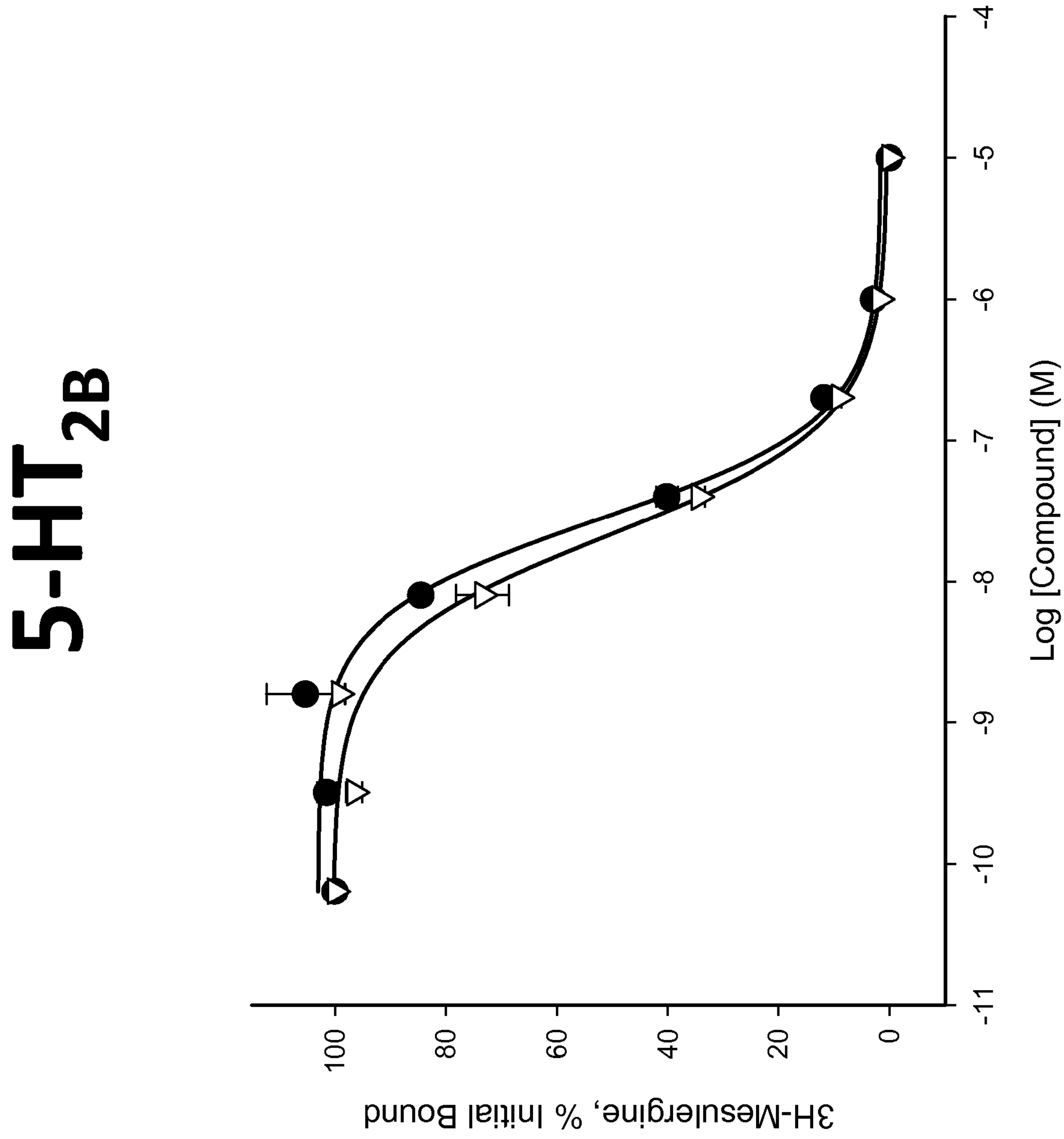
5-HT2B
3H-Mesulergine, % Initial Bound
100
80
60
40
20
0
-11
-10
-9
-8
-7
-6
-5
-4
Log [Compound] (M)

Figure 18c

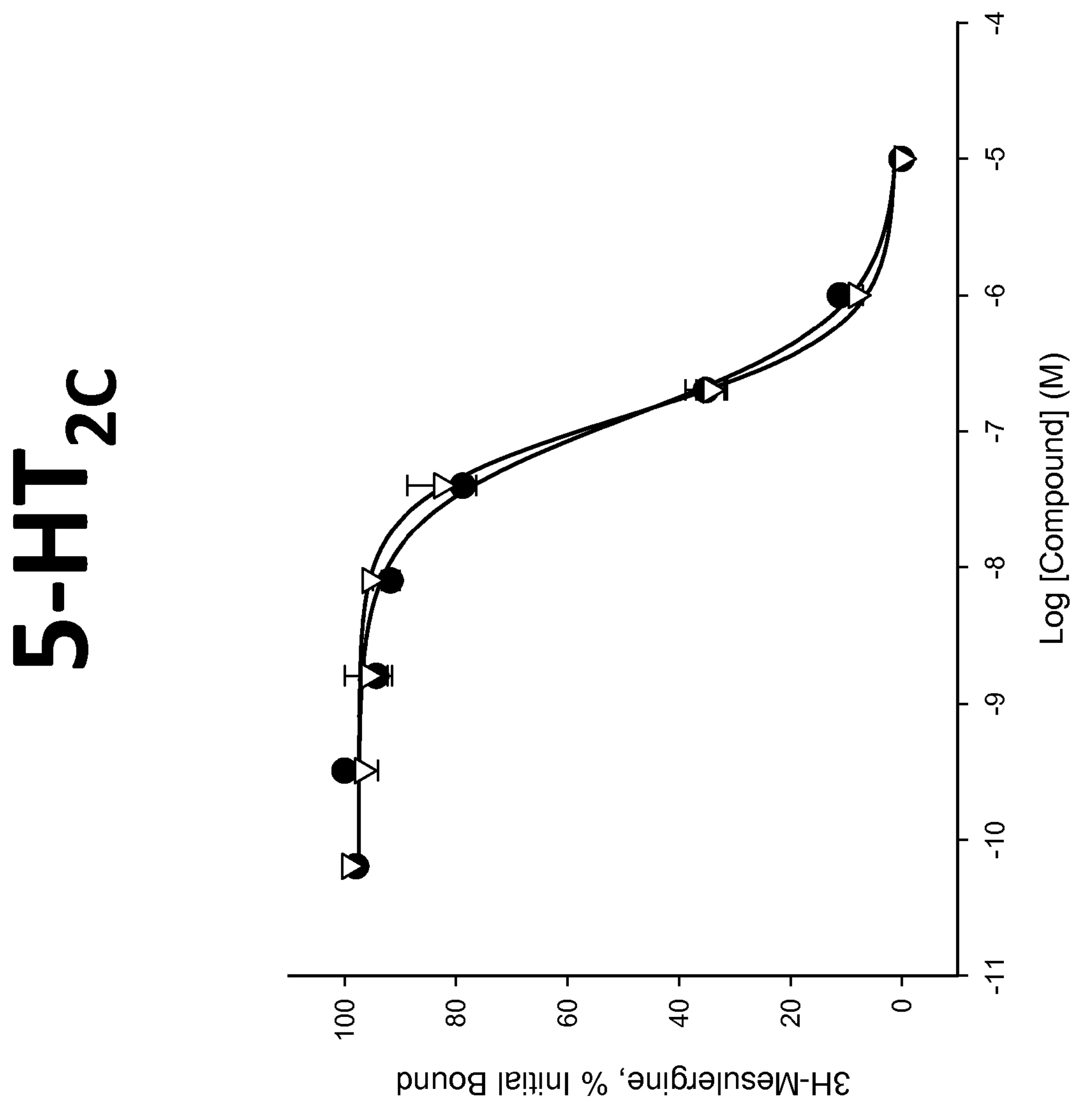
5-HT2C
3H-Mesulergine, % Initial Bound
100
80
60
40
20
0
-11
-10
-9
-8
-7
-6
-5
-4
Log [Compound] (M)

Figure 18d

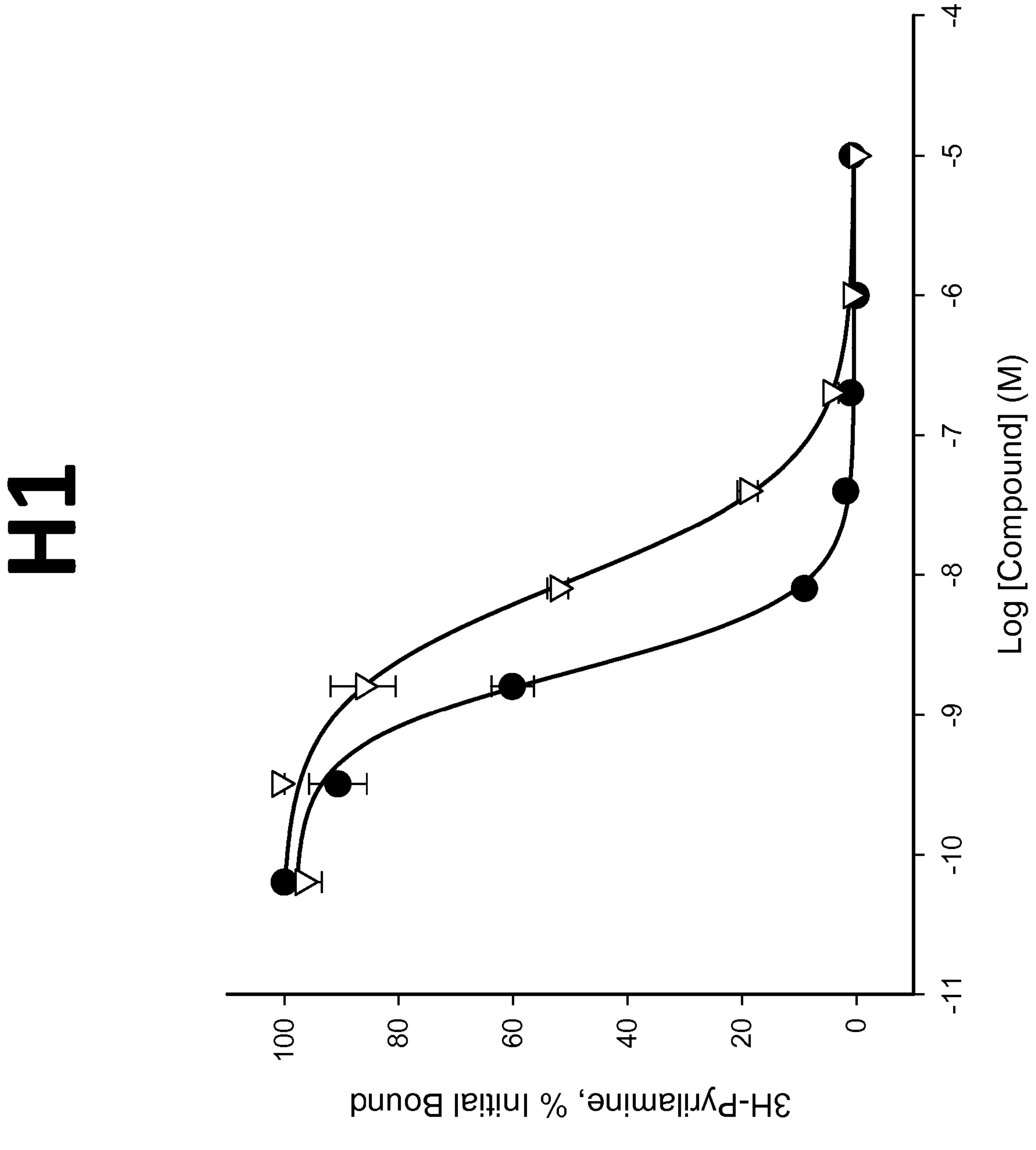
H1
3H-Pyrilamine, % Initial Bound
100
80
60
40
20
0
-11
-10
-9
-8
-7
-6
-5
-4
Log [Compound] (M)

Figure 18e

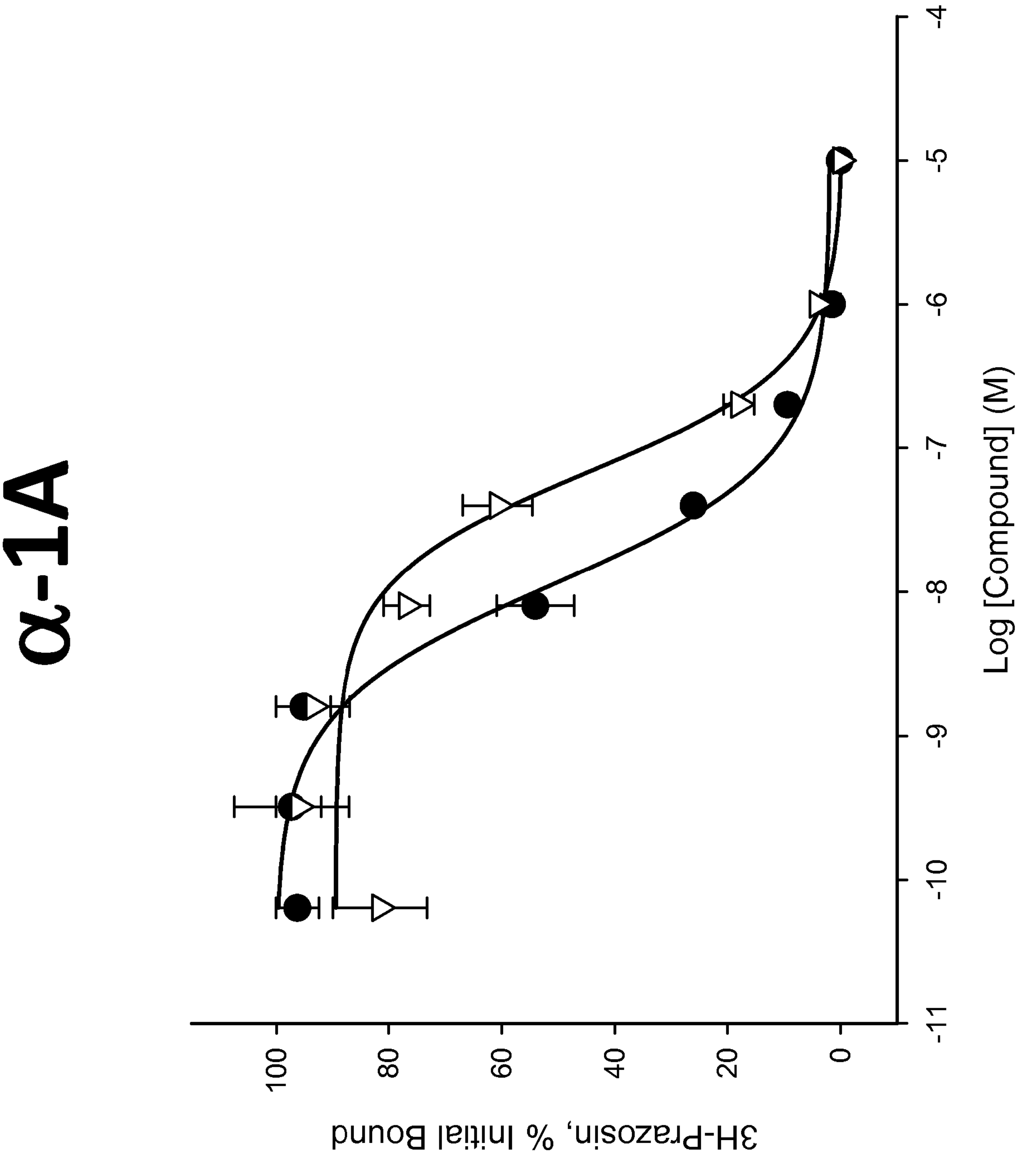
α-1A
3H-Prazosin, % Initial Bound
100
80
60
40
20
0
-11
-10
-9
-8
-7
-6
-5
-4
Log [Compound] (M)

Figure 18f

M1

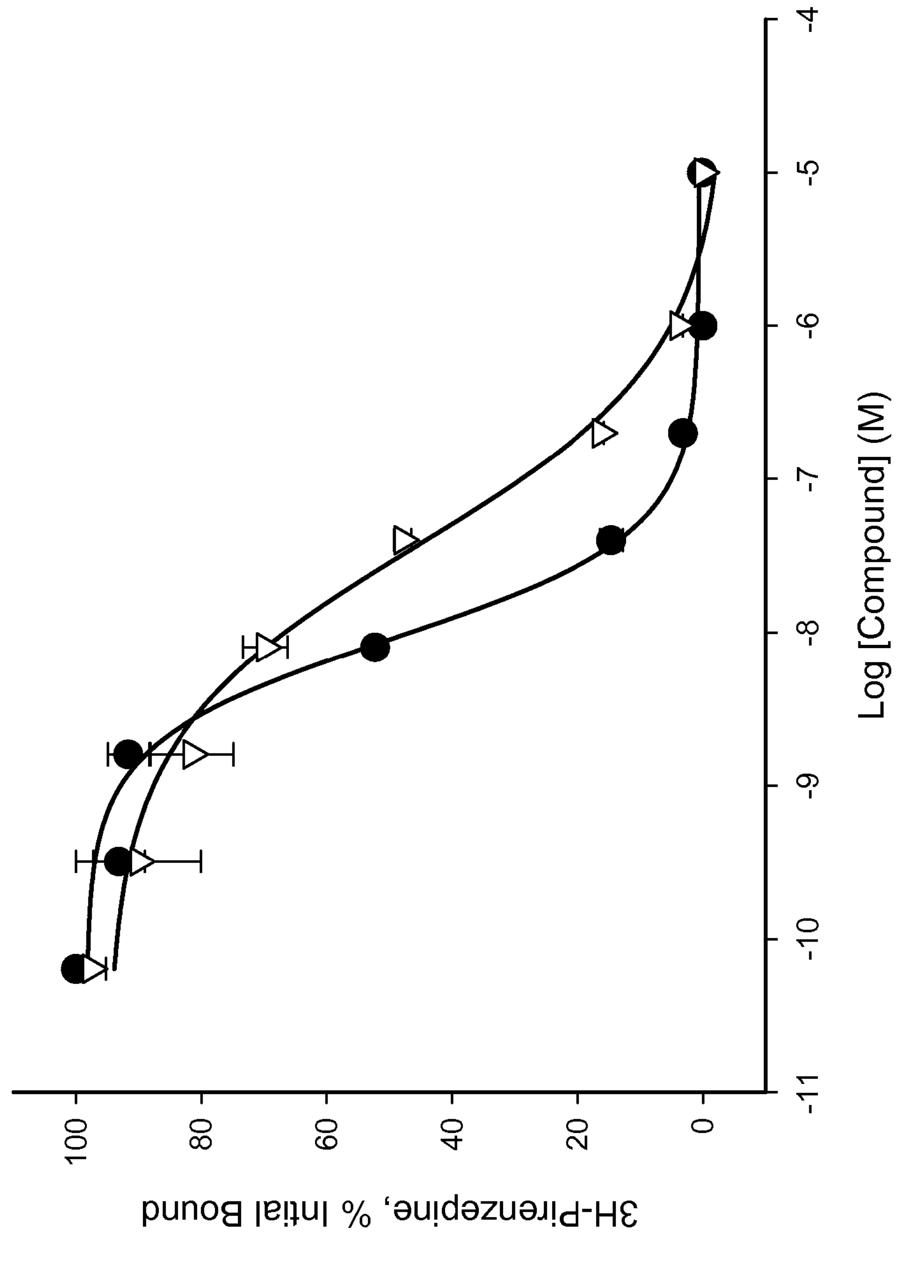


Figure 18g

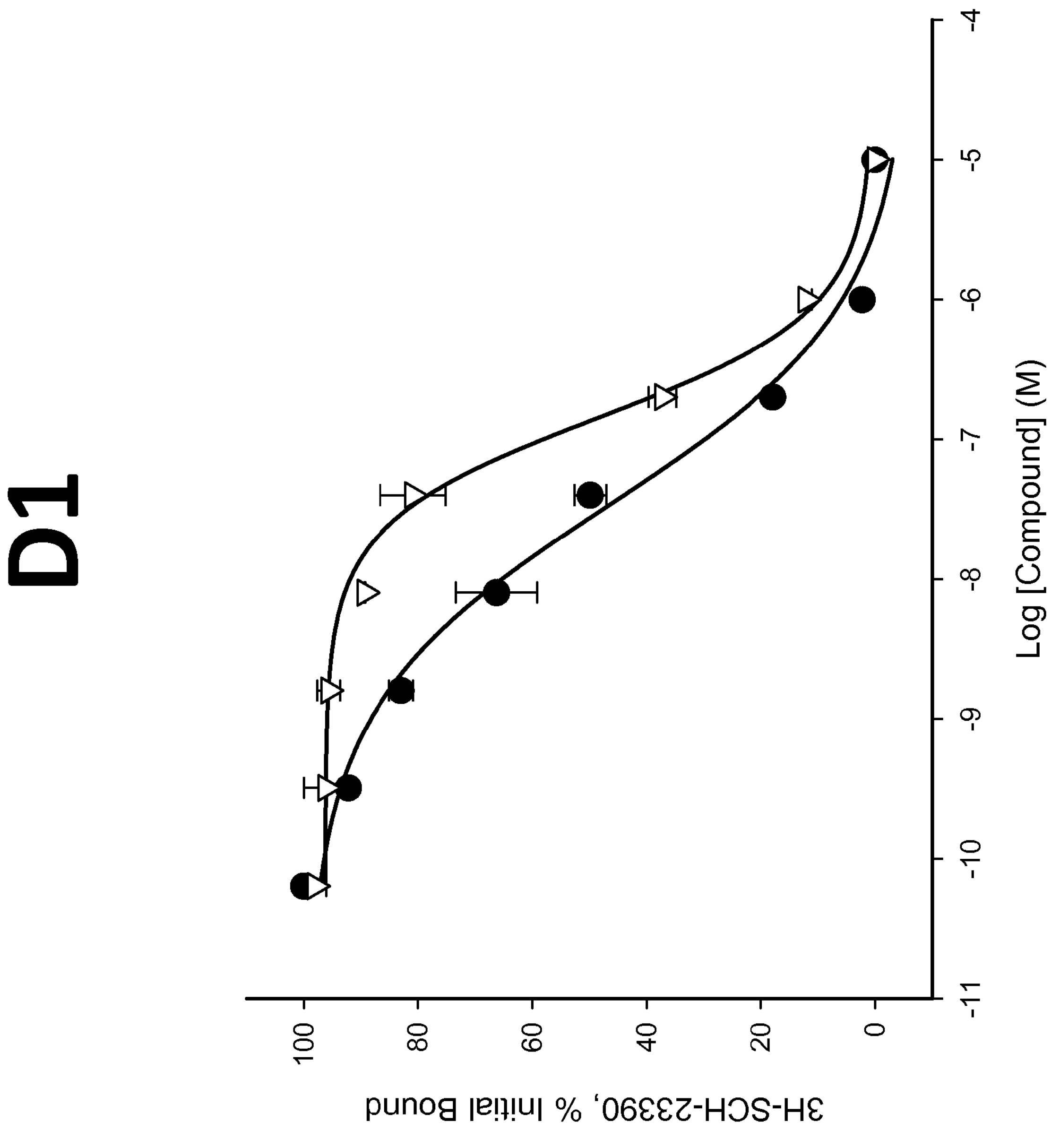
D1
3H-SCH-23390, % Initial Bound
100
80
60
40
20
0
-11
-10
-9
-8
-7
-6
-5
-4
Log [Compound] (M)

Figure 18h

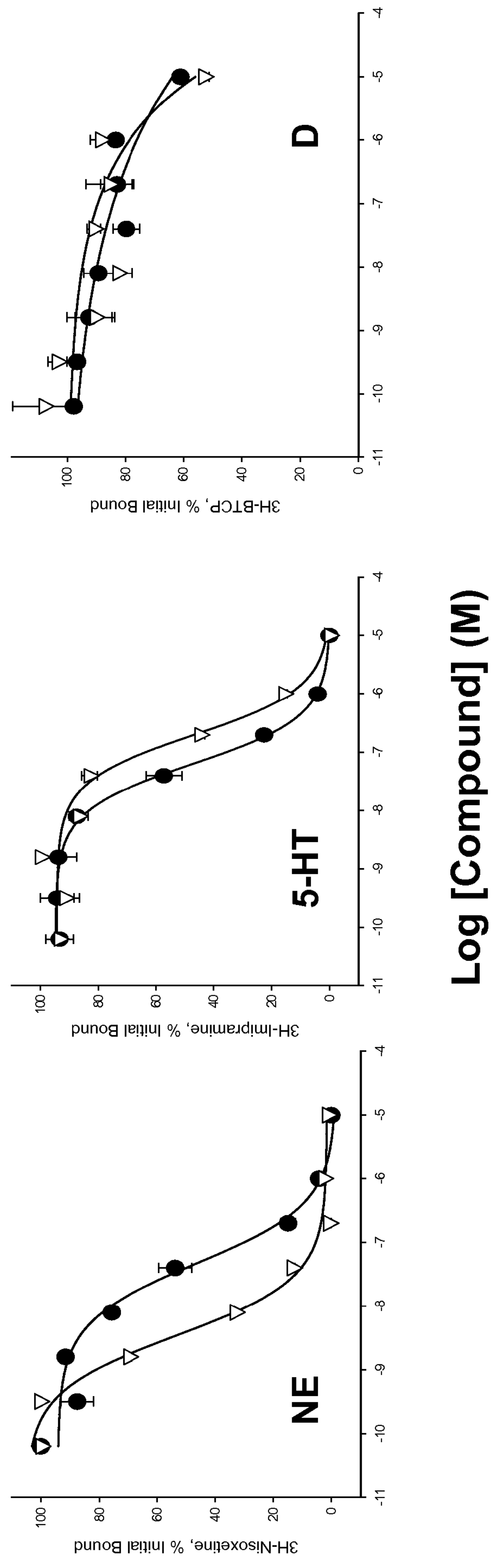

NE
5-HT
D
3H-Nisoxetine, % Initial Bound
3H-Imipramine, % Initial Bound
3H-BTCP, % Initial Bound
0
20
40
60
80
100
-11
-10
-9
-8
-7
-6
-5
-4
Log [Compound] (M)


Figure 19

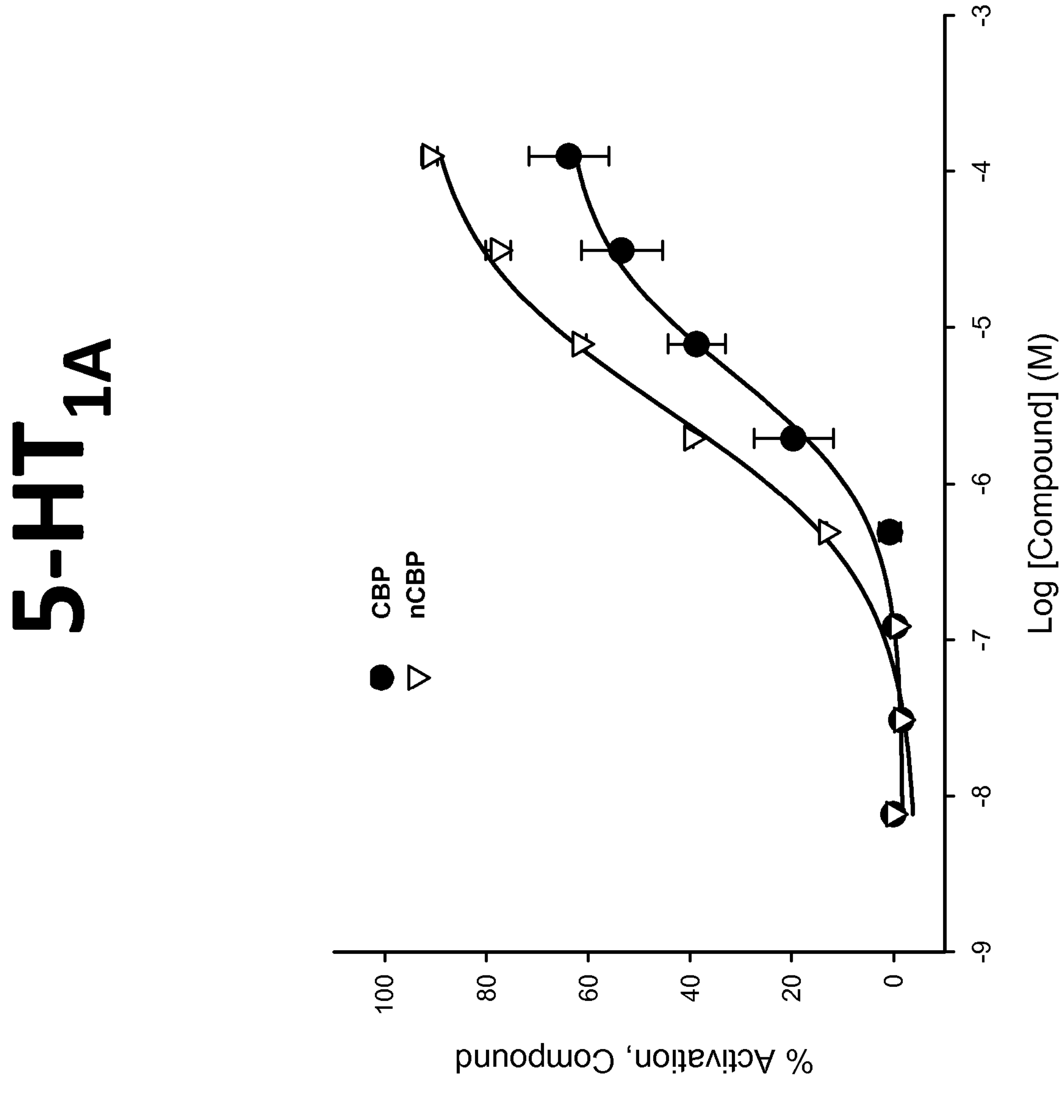
5-HT1A
CBP
nCBP
% Activation, Compound
100
80
60
40
20
0
-9
-8
-7
-6
-5
-4
-3
Log [Compound] (M)

Figure 20a

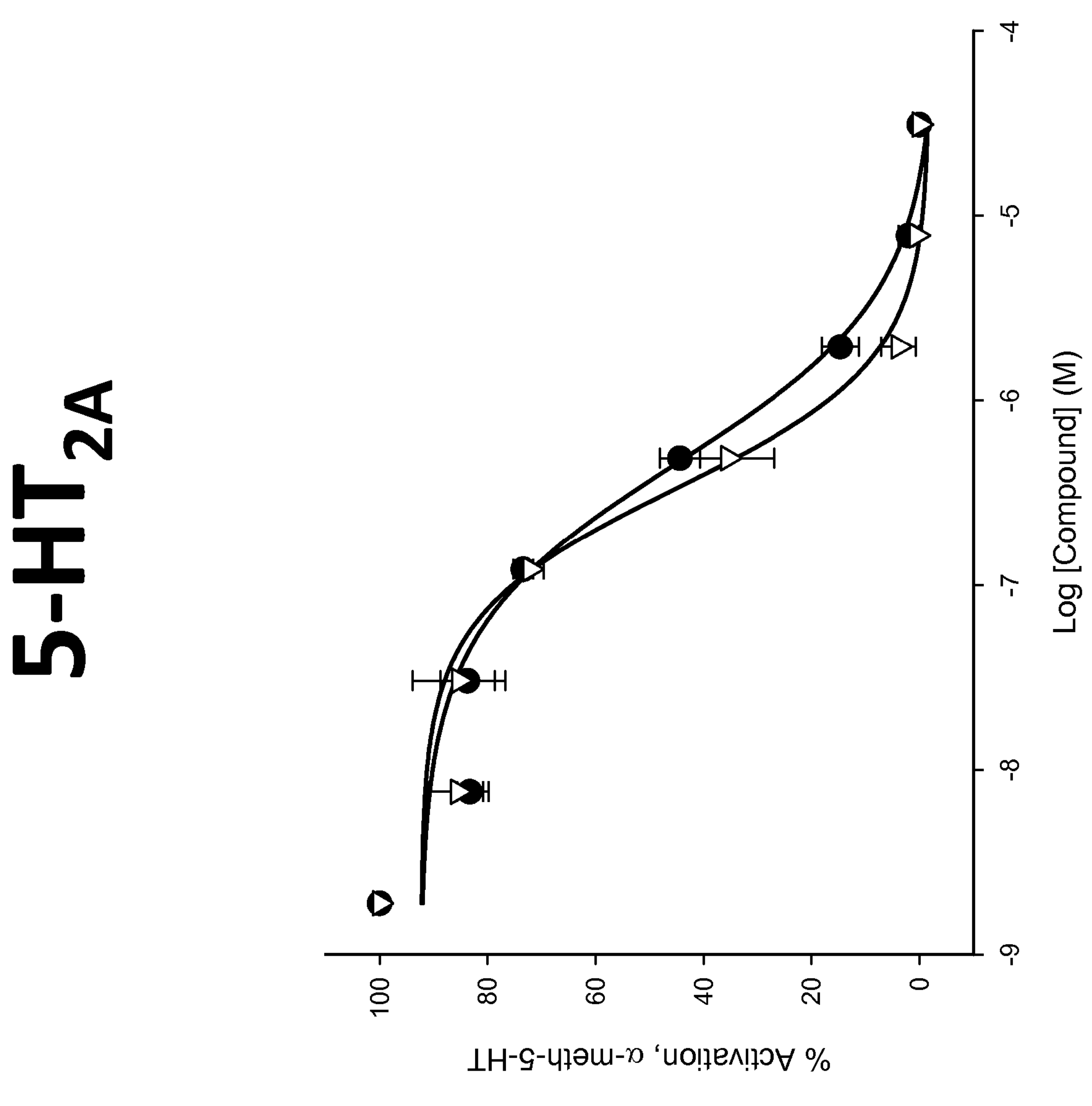
5-HT2A
% Activation, α-meth-5-HT
100
80
60
40
20
0
-9
-8
-7
-6
-5
-4
Log [Compound] (M)

Figure 20b

## 5-$HT_{2B}$

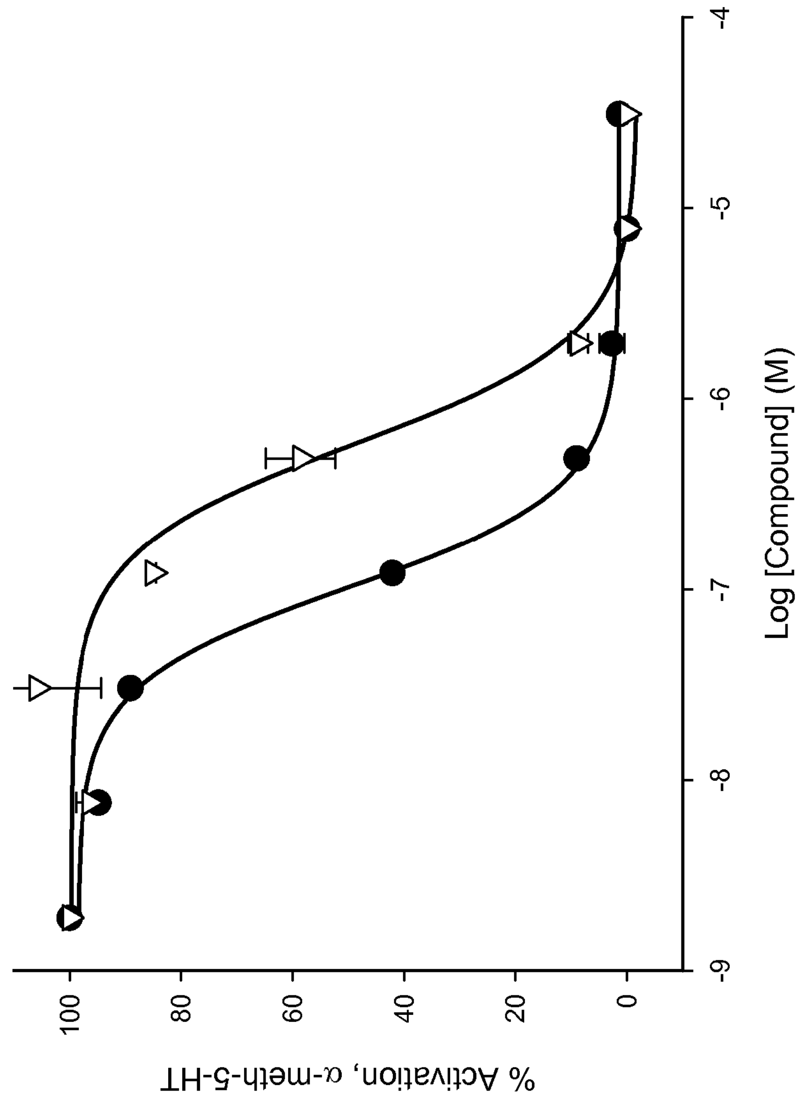


Figure 20c

# 5-HT$_{2C}$

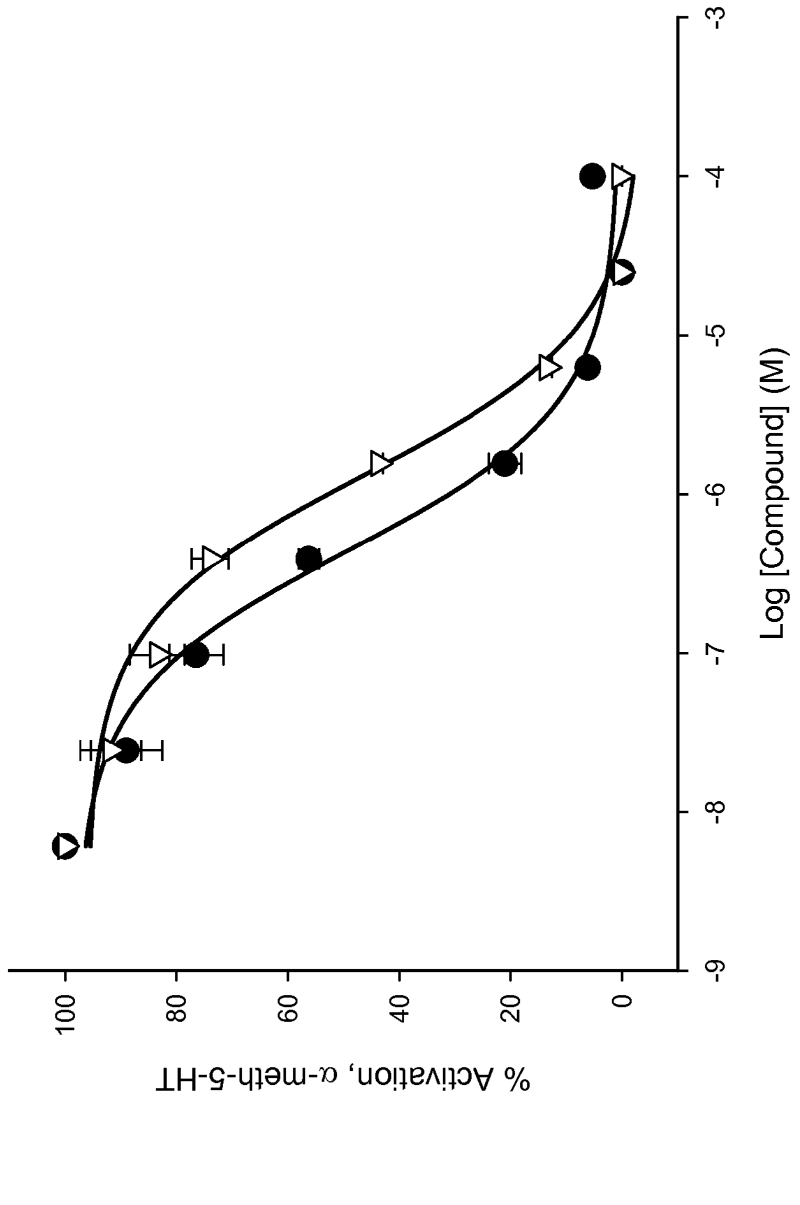


Figure 20d

# H1

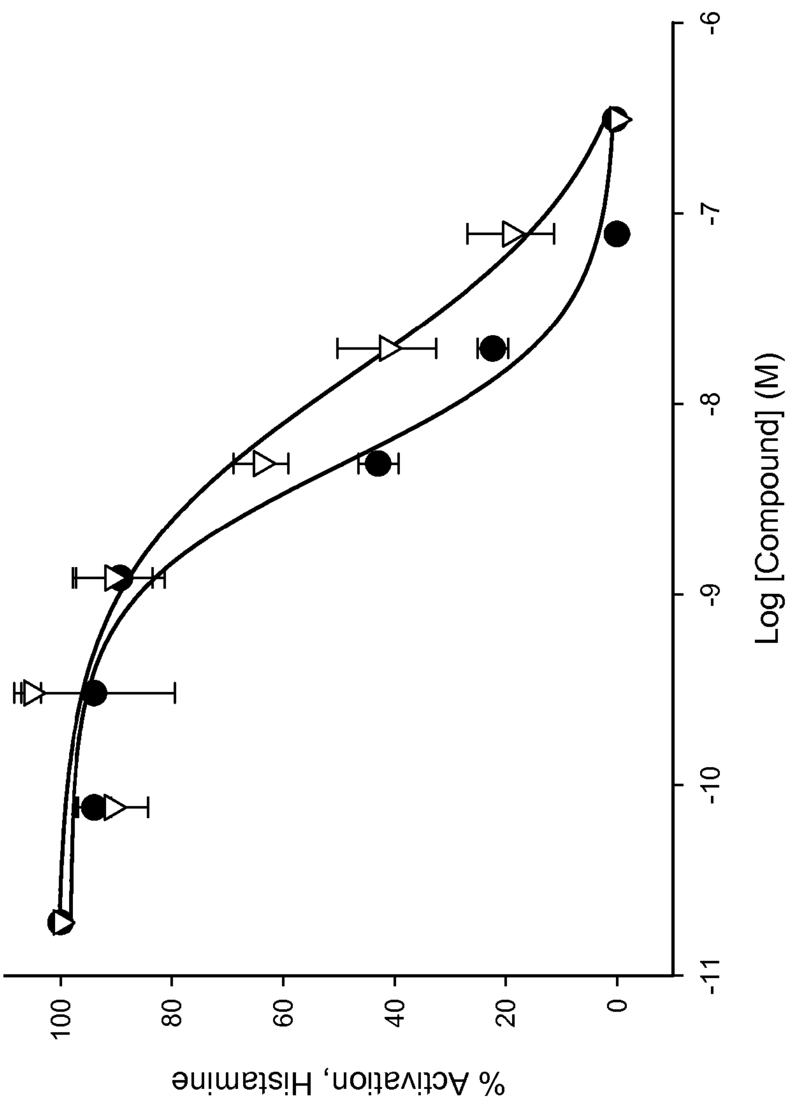


Figure 20e

α-1A

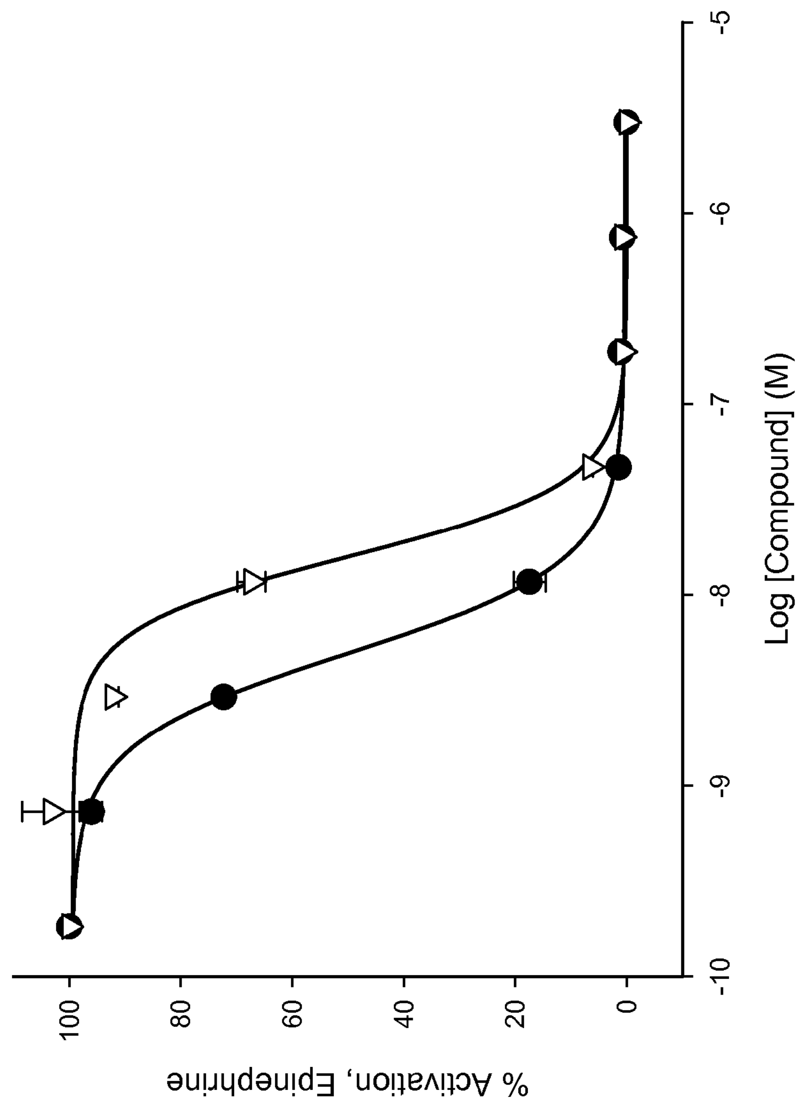


Figure 20f

# M1

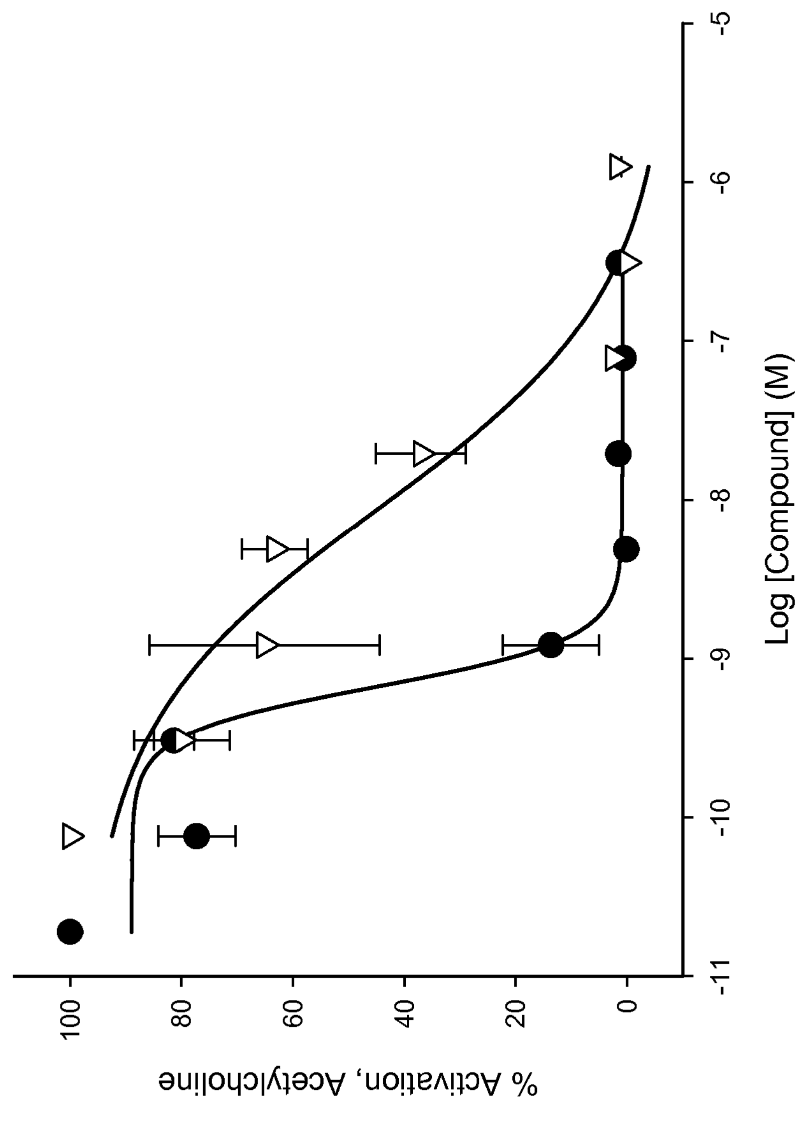


Figure 20g

D1

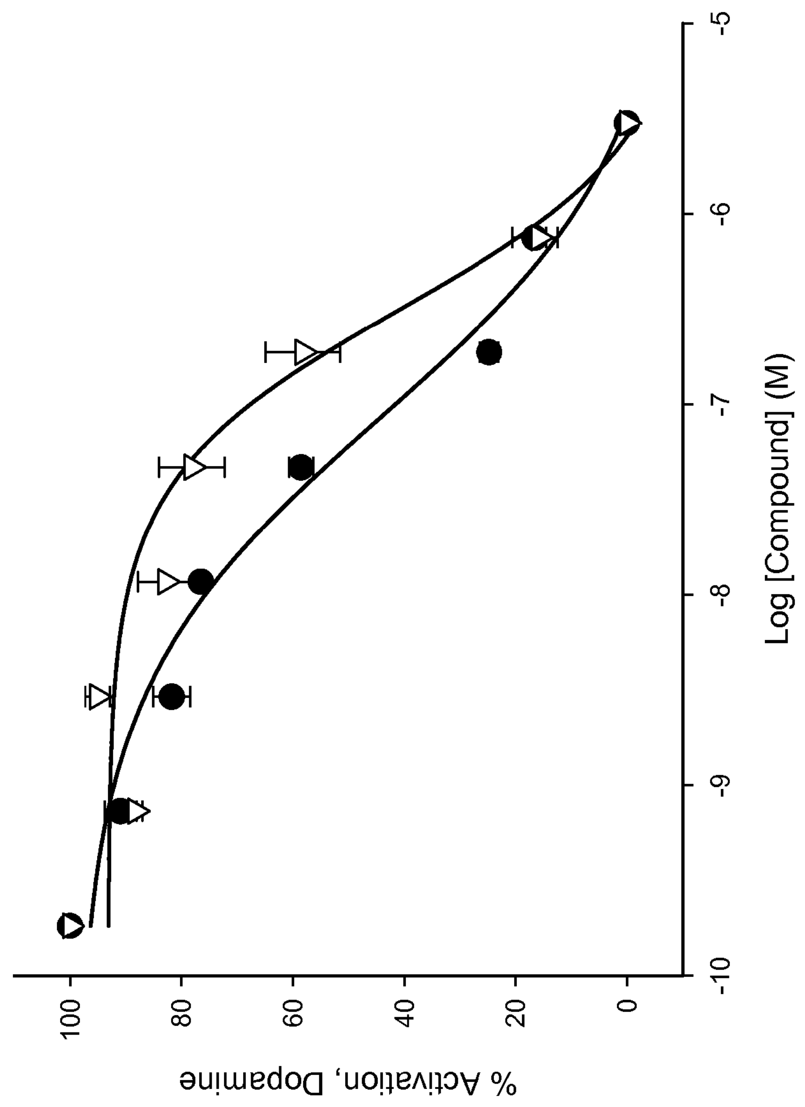


Figure 20h

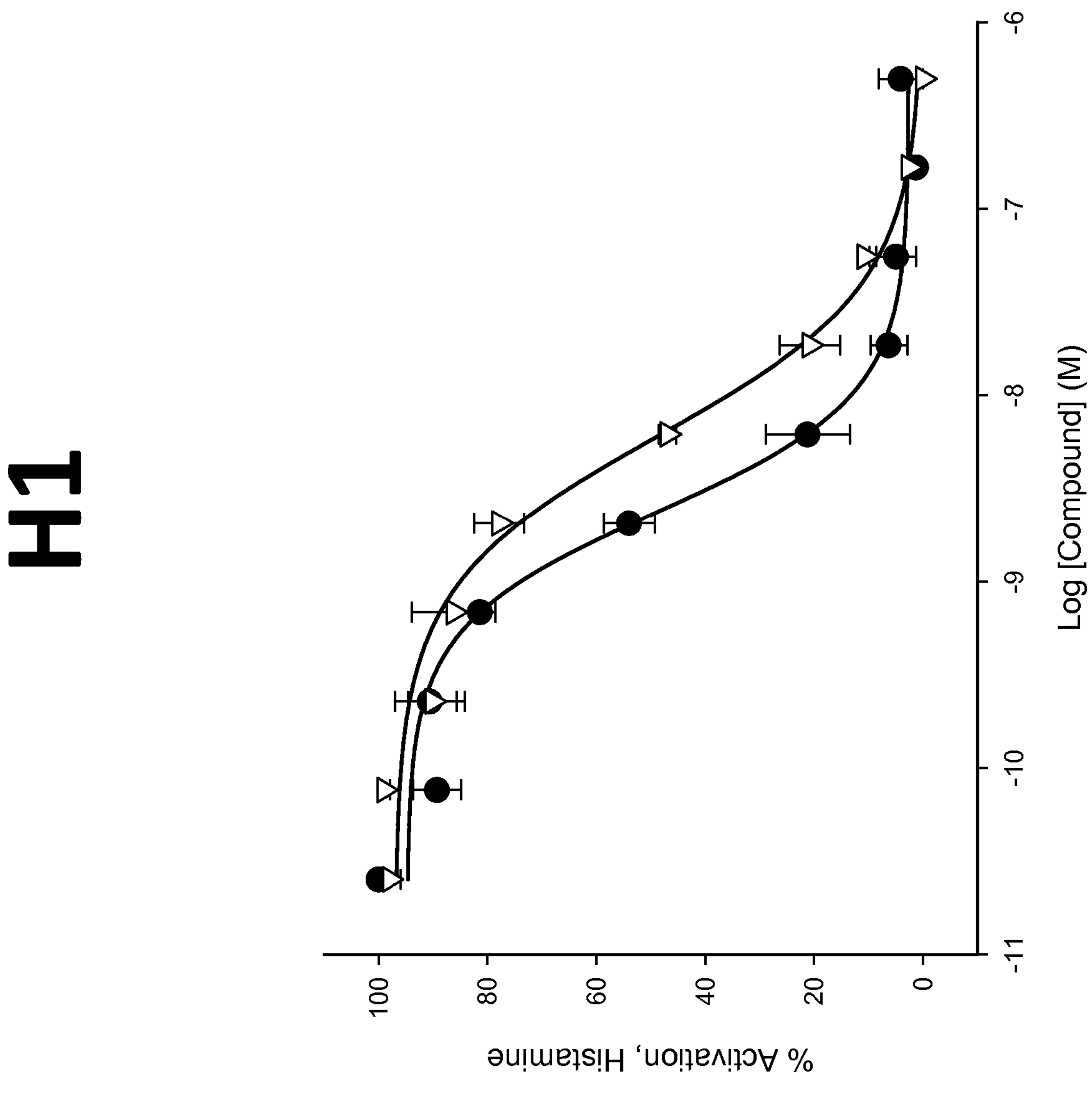
H1
% Activation, Histamine
100
80
60
40
20
0
-11
-10
-9
-8
-7
-6
Log [Compound] (M)

Figure 21a

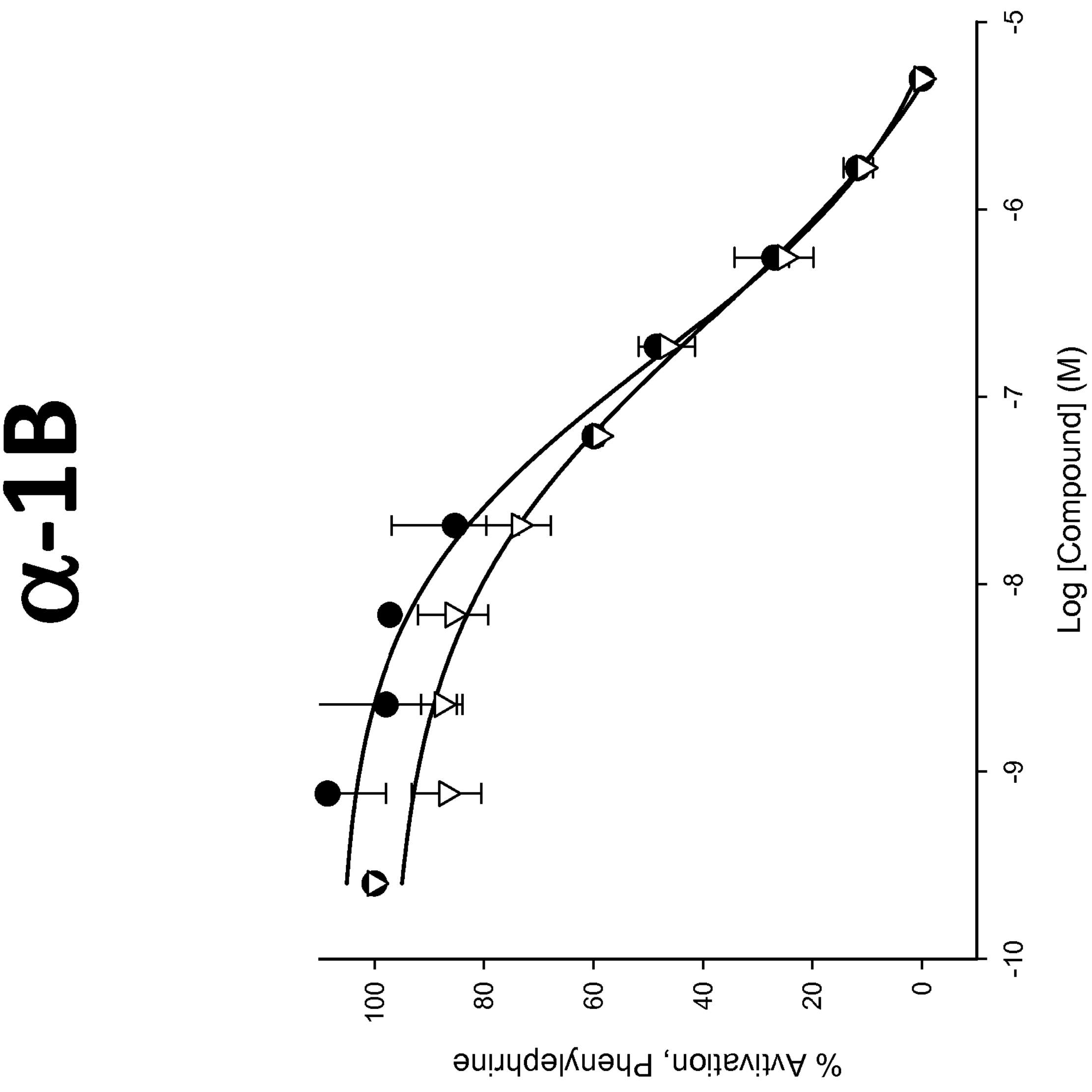
α-1B
% Avtivation, Phenylephrine
100
80
60
40
20
0
-10
-9
-8
-7
-6
-5
Log [Compound] (M)

Figure 21b

M1

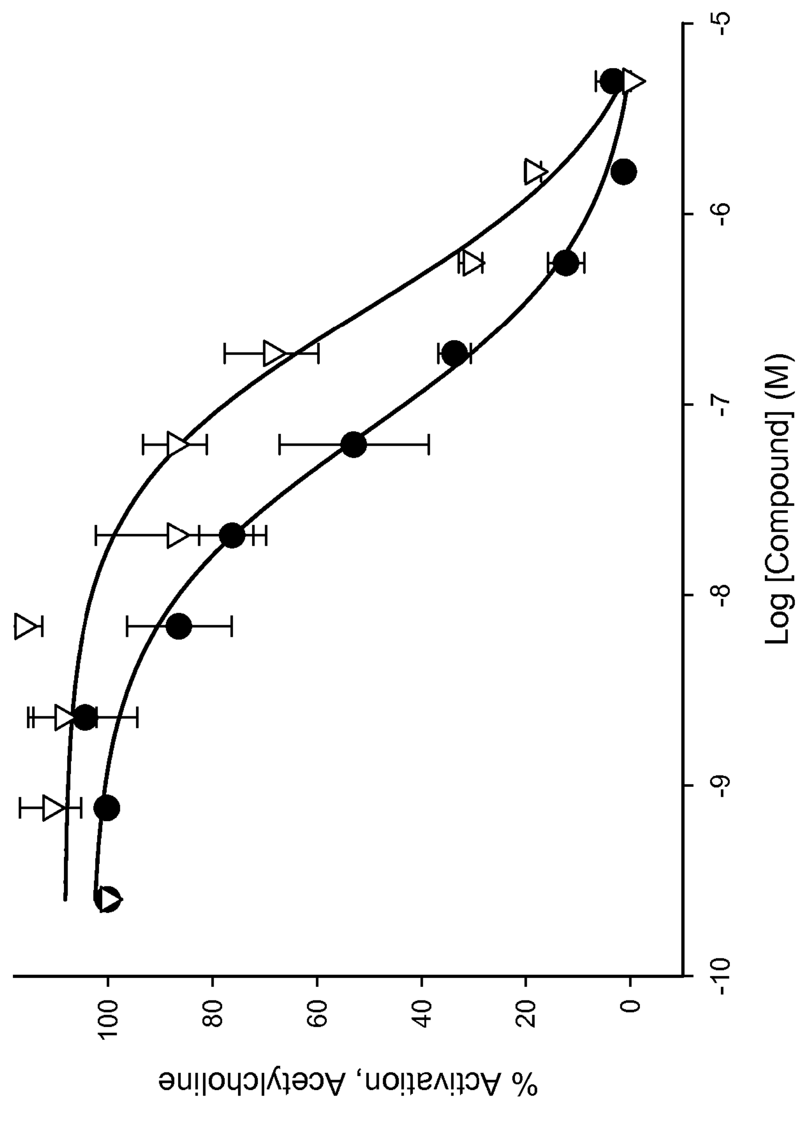


Figure 21c

# 5-$HT_{2A}$

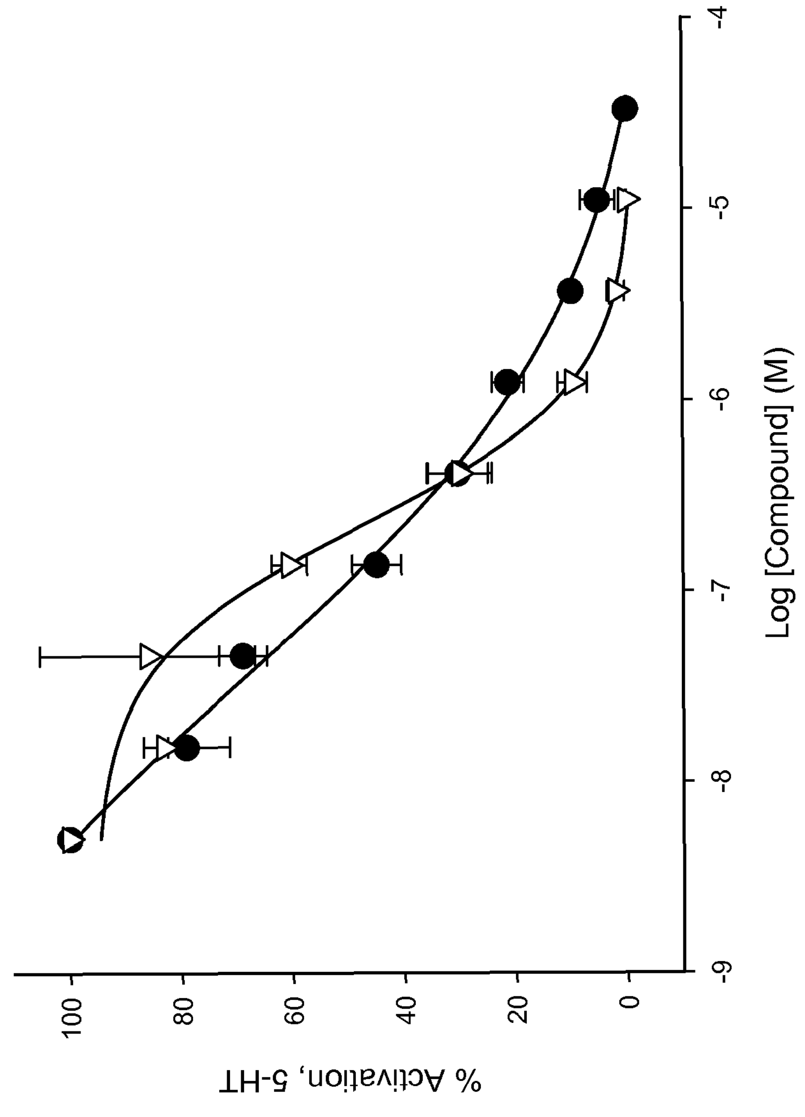


Figure 21d

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6395788 B
- US 6358944 B
- US 2003077227 A1

### Non-patent literature cited in the description


- *U.S. Food and Drug Administration,* 1977
- **KATZ, W. et al.** *Clinical Therapeutics,* 1988, vol. 10, 216-228
- The McGraw-Hill Dictionary of Chemical Terms. McGraw-Hill, 1985
- **MOLDOFSKY et al.** *J Rheumatol,* 2011, vol. 38 (12), 2653-2663
- **THOMAS.** *J Rheumatol,* 2011, vol. 38 (12), 2499-2500
- **WOLFE, F. et al.** *Arthritis and Rheumatism,* 1990, vol. 33, 160-172
- **WOLFE et al.** *J Rheumatol,* 2011, vol. 38 (6), 1113-22
- **MEASE P et al.** *J Rheumatol.,* 2009, vol. 36 (10), 2318-29
- *DSM-IV,* 551-607
- The International Classification of Sleep Disorders: (ICSD) Diagnostic and Coding Manual. American Sleep Disorders Association, 1990
- **M. L. COTTON ; G. R. B. DOWN.** *Anal. Profiles Drug Subs.,* 1988, vol. 17, 41-72
- **HUCKER et al.** *J Clin Pharmacol,* 1977, vol. 17, 719-727
- **HUCKER et al.** *Drug Metab Dispos,* 1978, vol. 6, 659-672
- **TILL.** *Annu Rev Pharmacol Toxicol,* 2000, vol. 40, 581-616
- **WINCHELL et al.** *J Clin Pharmacol,* 2002, vol. 42, 61-69
- **CARETTE, S.** *Arthritis Rheum.,* January 1994, vol. 37 (1), 32-40
- **BENNETT et al.** *Arthritis Rheum.,* 1988, vol. 31, 1535-1542
- **BHATT.** *Biomed Chromatogr,* 2010, vol. 24 (11), 1247-54
- **HAWES.** *Drug Metab Dispos,* 1998, vol. 26, 830-837
- **ZHOU et al.** *Drug Metab Dispos,* 2010, vol. 38, 863-870
- **BREYER-PFAFF et al.** *Drug Metab Dispos,* 1997, vol. 25, 340-345
- **NAKAJIMA et al.** *Drug Metab Dispos,* 2002, vol. 30, 636-642
- **WONG et al.** *J Anal Toxicol,* 1995, vol. 19, 218-224
- **LANGIN et al.** *Eur J Pharmacol,* 1989, vol. 167, 95-104
- **DEVEDJIAN et al.** *Eur J Pharmacol,* 1994, vol. 252, 43-49
- **SMIT et al.** *Brit. J. Pharmacol,* 1996, vol. 117, 1071-1080
- **DORJE et al.** *J Pharmacol Exp Ther,* 1991, vol. 256, 727-733
- **MULHERON et al.** *J Biol Chem,* 1994, vol. 269, 12954-12962
- **BONHAUS et al.** *Brit J Pharmacol,* 1995, vol. 115, 622-628
- **STAM et al.** *Eur J Pharmacol,* 1994, vol. 269, 339-348